# EUROPÄISCHE PATENTSCHRIFT

(11) **EP 1 246 808 B1**
(45) Veröffentlichungstag und Bekanntmachung des Hinweises auf die Patenterteilung: **17.08.2011**
(21) Anmeldenummer: 01915133.1
(22) Anmeldetag: 12.01.2001
(51) Int. Cl.: C07D 235/18, A61K 31/415, A61P 25/28, C07D 405/04, C07D 401/04, A61K 31/44, C07D 417/04, C07D 401/12, C07D 403/12

(54) **1,2-DIARYLBENZIMIDAZOLE ZUR BEHANDLUNG VON ERKRANKUNGEN DIE MIT EINER MICROGLIA-AKTIVIERUNG ASSOZIIERT SIND**
1,2-DIARYL BENZIMIDAZOLES FOR TREATING ILLNESSES ASSOCIATED WITH A MICROGLIA ACTIVATION
1,2-DIARALBENZIMIDAZOLES UTILISES POUR TRAITER DES AFFECTIONS ASSOCIEES A UNE ACTIVATION DE LA MICROGLIE

(30) Priorität: 14.01.2000 DE 10002898
(43) Veröffentlichungstag der Anmeldung: 09.10.2002
(73) Patentinhaber: Bayer Schering Pharma Aktiengesellschaft, 13353 Berlin (DE)
(72) Erfinder: KUHNKE, Joachim, 14469 Potsdam (DE); HALFBRODT, Wolfgang, 13505 Berlin (DE); MOENNING, Ursula, 15589 Woltersdorf (DE)
(86) Internationale Anmeldenummer: PCT/EP2001/000334
(87) Internationale Veröffentlichungsnummer: WO 2001/051473

(56) Entgegenhaltungen:
- EP-A- 0 104 727
- EP-A- 0 520 200
- EP-A- 0 528 164
- EP-A- 0 531 883
- WO-A-95/07263
- WO-A-97/12613
- WO-A-97/33873
- US-A- 5 552 426

## Beschreibung

Die Erfindung betrifft neue Benzimidazol-Derivate und die Verwendung von Benzimidazol-Derivaten zur Herstellung von Arzneimitteln zur Behandlung und Prophylaxe von Erkrankungen. die mit einer Mikroglia-Aktivierung assoziiert sind.

Nahezu alle degenerativen Erkrankungen des zentralen Nervensystems sind mit einer chronischen Entzündung verbunden. Ein zentraler Schritt des Entzündungsgeschehens ist die Aktivierung von mononukleären phagozytären Zellen, den Mikroglia. Dies erfolgt z.B. bei der Alzheimerschen Krankheit durch die senilen Plaques, bei der Creuzfeld-Jacob Krankheit durch ein Prion-Protein und beim ischämischen Schlaganfall durch abgestorbene Zellen. Die Mikroglia können über einen längeren Zeitraum in dem aktivierten Zustand bleiben, in dem sie verschiedene Entzündungsfaktoren, z.B. reaktive Sauerstoff/Stickstoff-Intermediate, Proteasen, Cytokine, Komplement-Faktoren und Neurotoxine produzieren und sekretieren. Diese wiederum bewirken neuronale Dysfunktion und Degeneration.

EP 0 528 164 beschreibt die Verwendung von Xanthin-Derivaten zur Herstellung eines Medikamentes zur Behandlung sekundärer Hirnschädigung und Funktionsstörungen nach Schädel-Hirn-Trauma. WO95/07263 behandelt 1,2-Di(hetero)arylbenzimidazol-Derivate sowie deren Herstellung und Verwendung als Prostacyclin (PGI2)-Mimetika.

Für eine mögliche Therapie der Neuroinflammation sind bisher nicht-steroidale Entzündungshemmer (COX II Inhibitoren) (McGeer, P.L., Roger, Neurology 42, 447-449 (1992), Rogers, J., Kirby, L.C., Hempleman, S.R., Berry, D.L McGeer, P.L., Kaszniak, A.W.,Zalinski, J.,Cofield, M., Mansukhani, L:, Wilson, P., Kogan, F. Neurology 43, 1609-1611 (1993), Andersen, K., Launer, L.J., Ott, A., Hoes, A.W., Breteler, M.M.B., Hofman, A. Neurology 45, 1441-1445 (1995), Breitner, J.C.S.. Gau, B.A. Welsh, KA. Plassman, B.L., McDonald, W.M., Helms, M.J., Anthony, J.C. Neurology 44, 227-232 (1994).The Canadian Study of Health and Aging, Neurology 44, 2073-2079 (1994)), Cytokin-Modulatoren (McGeer, P.L., McGeer, E.G. Brain Res. Rev 21:195-218 (1995*),* McGeer, E.G., McGeer, P.L., CNS Drugs 7, 214-228 (1997*),*Barone, F.C. and Feuerstein, G.Z, J. Cerebral Blood Flow and Metabolism 19, 819-834 (1999*)* und Komplement-Kaskaden-Inhibitoren (Chen., S., Frederickson, R.C.A., and Brunden, KR., Neurobiol. Aging (1996), McGeer, E.G.., McGeer, P.L, Drugs 55:739-746 (1998)) beschrieben worden. Diese Substanzen hemmen die Synthese oder die Wirkung einzelner Entzündungsfaktoren. Es wäre jedoch wünschenswert, Substanzen zu haben, die einen früheren Schritt im Entzündungsgeschehen hemmen und damit die Entstehung oder Wirkung vieler Entzündungfaktoren verhindern.

Das Problem wurde gelöst, durch Bereitstellung von Benzimidazol-Derivaten der allgemeinen Formel I, deren tautomere oder isomere Formen oder Salze 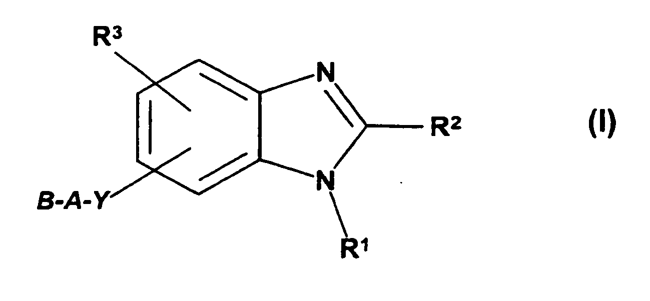 worin
- ***R¹***: eine mono- oder bicyclische C₆₋₁₂-Arylgruppe oder eine mono- oder bicyclische 5- 10 gliedrige Heteroarylgruppe mit 1-4 Heteroatomen ausgewählt aus der Gruppe bestehend aus N, S oder O, wobei die genannte Aryl-oder Heteroarylgruppe mit bis zu drei der folgenden Substituenten unabhängig voneinander substituiert sein kann: F, Cl, Br, I, C(NH)NH₂, C(NH)NHR⁴, C(NH)NR⁴R^{4'}, C(NR⁴)NH₂, C(NR⁴)NHR^{4'}, C(NR⁴)NR⁴R⁴, XOH, XOR⁴, XOCOR⁴, XOCONHR⁴, XOCOOR⁴, XCOR⁴, XC(NOH)R⁴, XC(NOR⁴)R^{4'}, XC(NO(COR⁴))R^{4'} XCN, XCOOH, XCOOR⁴, XCONH₂, XCONR⁴R^{4'}, XCONHR⁴, XCONHOH, XCONHOR⁴, XCOSR⁴ XSR⁴, XSOR⁴, XSO₂R⁴, SO₂NH₂, SO₂NHR⁴, SO₂NR⁴R^{4'}, NO₂, XNH₂, XNHR⁴, XNR⁴R^{4'}, XNHSO₂R⁴, XN(SO₂R⁴) SO₂R^{4'}, XNR⁴SO₂R^{4'}, XNHCOR⁴, XNHCOOR⁴, XNHCONHR⁴, Tetrahydro-2,5-dioxopyrrol-1-yl, 2,5- Dihydro-2,5-dioxopyrrol-1-yl, 2,7-Dihydro-2,7-dioxoisoindol-1-yl, R⁴, wobei zwei Substituenten an ***R¹,*** wenn sie zueinander orthoständig sind, so miteinander verknüpft sein können, daß sie gemeinsam Methandiylbisoxy, Ethan- 1,2-diylbisoxy, Propan-1,3-diyl, Butan-1,4-diyl bilden,
- ***R²***: eine mono- oder bicyclische C₆₋₁₀- Arylgruppe oder eine mono- oder bicyclische 5- 10 gliedrige Heteroarylgruppe mit 1-4 Heteroatomen ausgewählt aus der Gruppe bestehend aus N, S oder O, wobei die genannte Aryl-oder Heteroarylgruppe mit bis zu drei der folgenden Substituenten unabhängig voneinander substituiert sein kann: F, Cl, Br, I, XOH, XOR⁴, XOCOR⁴, XOCONHR⁴, XOCOOR⁴, XCOR⁴, XC(NOH)R⁴, XC(NOR⁴)R^{4'}. XC(NO(COR⁴))R^{4'}. XCOOH, XCOOR⁴, XCONH₂, XCONHR⁴, XCONR⁴R^{4'}, XCONHOH, XCONHOR⁴, XCOSR⁴, XSR⁴, XSOR⁴, XSO₂R⁴, SO₂NH₂, SO₂NHR⁴, SO₂NR⁴R^{4'}, NO₂, XNHR⁴, XNR⁴R^{4'}, XNHSO₂R⁴, XN(SO₂R⁴) SO₂R^{4'}, XNRSO₂R^{4'}, Tetrahydro- 2,5-dioxopyrrol-1-yl, 2,5-Dihydro-2,5-dioxopyrrol-1-yl, 2,7-Dihydro-2,7- dioxoisoindol-1-yl, R⁴, wobei zwei Substituenten an ***R²**,* wenn sie zueinander orthoständig sind, so miteinander verknüpft sein können, daß sie gemeinsam Methandiylbisoxy, Ethan- 1,2-diylbisoxy, Propan-1,3-diyl, Butan-1,4-diyl bilden,
- ***R³***: ein oder zwei Substituenten, die unabhängig voneinander: Wasserstoff, F, Cl, Br, I, XOH, XOR⁴, XOCOR⁴, XOCONHR⁴, XOCOOR⁴, XCOR⁴, XC(NOH)R⁴, XC(NOR⁴)R^{4'}. XC(NO(COR⁴))R^{4'} XCN, XCOOH, XCOOR⁴, XCONH₂, XCONHR⁴, XCONR⁴R^{4'}, XCONHOH, XCONHOR⁴, XCOSR⁴, XSR⁴, XSOR⁴, XSO₂R⁴, SO₂NH₂, SO₂NHR⁴, SO₂NR⁴R^{4'}, NO₂, XNH₂, XNHR⁴, XNR⁴R^{4'}, XNHSO₂R⁴, XNR⁴SO₂R^{4'}, XN(SO₂R⁴)(SO₂R^{4'}), XNHCOR⁴, XNHCOOR⁴, XNHCONHR⁴, Tetrahydro-2,5-dioxopyrrol-1-yl, 2,5- Dihydro-2,5-dioxopyrrol-1-yl, 2,7-Dihydro-2,7-dioxoisoindol-1-yl, oder R⁴ sein können, wobei zwei Substituenten ***R³**,* wenn sie zueinander orthoständig sind, so miteinander verknüpft sein können, daß sie gemeinsam Methandiylbisoxy, Ethan- 1,2-diylbisoxy, Propan-1,3-diyl, Butan-1,4-diyl bilden,
- ***R⁴***: und **R^{4'}** unabhängig voneinander C₁₋₄ Perfluoralkyl, C₁₋₆ Alkyl, C₂₋₆ Alkenyl, C₂₋₆ Alkinyl, C₃₋₇ Cycloalkyl, (C₁₋₃ Alkyl-C₃₋₇ Cycloalkyl), C₁₋₃ Alkyl-C₆₋₁₀-aryl, C₁₋₃ Alkyl-5- 10 gliedriges Heteroaryl mit 1-4 N-, S- oder O-Atomen, C₆₋₁₀-Aryl oder 5-10 gliedriges Heteroaryl mit 1-4 N-, S- oder O-Atomen, wobei die Aryl- und Heteroarylgruppen mit ein oder zwei Substituenten aus der Gruppe bestehend aus F, Cl, Br, CH₃, C₂H₅, NO₂, OCH₃, OC₂H₅, CF₃, C₂F₅ substituiert sein können oder auch eine annelierte Methandiylbisoxy, Ethan-1,2-diylbisoxygruppe tragen können, und weiterhin in einem 5-gliedrigen Cycloalkylring ein Ringglied ein N oder ein O sein kann und in einem 6- oder 7-gliedrigen Cycloalkylring ein oder zwei Ringglieder N und/oder O sein können, wobei Ringstickstoffe gegebenenfalls mit C₁₋₃ Alkyl oder C₁₋₃Alkanoyl substituiert sein können,
- ***R⁵***: und **R^{5'}** unabhängig voneinander C₁₋₆ Alkyl, C₂₋₆ Alkenyl, C₂₋₆ Alkinyl, wobei ein Kohlenstoffatom gegen O, S, SO, SO₂, NH, N C₁₋₃ Alkyl oder N C₁₋₃ Alkanoyl ausgetauscht sein kann, C₃₋₇ Cycloalkyl-C₀₋₃ Alkyl, wobei in einem 5-gliedrigen Cycloalkylring ein Ringglied ein N oder ein O sein kann und in einem 6- oder 7-gliedrigen Cycloalkylring ein oder zwei Ringglieder N und/oder O sein können, wobei Ringstickstoffe gegebenenfalls mit C₁₋₃ Alkyl oder C₁₋₃ Alkanoyl substituiert sein können C₆₋₁₀-Aryl oder 5-10 gliedriges Heteroaryl mit 1-4 Heteroatomen aus N, S und O, wobei die genannten Alkyl-, Alkenyl- und Alkinylketten mit einem der zuvor genannten Cycloalkyle, Aryle oder Heteroaryle substituiert sein können, wobei alle zuvor genannten Alkyl- und Cycloalkylreste mit bis zu zwei Substituenten aus CF₃, C₂F₅, OH, O C₁₋₃ Alkyl, NH2, NH C₁₋₃ Alkyl, NH C₁₋₃ Alkanoyl, N (C₁₋₃ Alkyl)₂, N(C₁₋₃ Alkyl)(C₁₋₃ Alkanoyl), COOH, CONH₂, COO C₁₋₃ Alkyl und alle zuvor genannten Aryl- und Heteroarylgruppen mit ein oder zwei Substituenten aus der Gruppe bestehend aus F, Cl, Br, CH₃, C₂H₅, NO₂, OCH₃, OC₂H₅, CF₃, C₂F₅ substituiert sein können oder auch eine annelierte Methandiylbisoxy, Ethan-1,2-diylbisoxygruppe tragen können, oder R⁵ und R^{5'} gemeinsam mit dem Stickstoffatom einen 5-7 gliedrigen Heterocyclus bilden, der ein weiteres Sauerstoff-, Stickstoff- oder Schwefelatom enthalten kann und substituiert sein kann mit C₁₋₄-Alkyl, C₁₋₄-Alkoxy-C₀₋₂-alkyl, C₁₋₄- Alkoxy-carbonyl, Aminocarbonyl oder Phenyl,
- ***A***: C₁₋₁₀ Alkandiyl, C₂₋₁₀ Alkendiyl, C₂₋₁₀ Alkindiyl, (C₀₋₅ Alkandiyl-C₃₋₇ Cycloalkandiyl- C₀₋₅ Alkandiyl), wobei in einem 5-gliedrigen Cycloalkylring ein Ringglied ein N oder ein O sein kann und in einem 6- oder 7-gliedrigen Cycloalkylring ein oder zwei Ringglieder N und/oder O sein können, wobei Ringstickstoffe gegebenenfalls mit C₁₋₃ Alkyl oder C₁₋₃ Alkanoyl substituiert sein können wobei in den oben genannten aliphatischen Ketten ein Kohlenstoffatom oder zwei Kohlenstoffatome gegen O, NH, N C₁₋₃ Alkyl, N C₁₋₃ Alkanoyl ausgetauscht sein können und wobei Alkyl- oder Cycloalkygruppen mit bis zu zwei Substituenten aus =O, OH, O C₁₋₃ Alkyl, NH2, NH C₁₋₃ Alkyl, NH C₁₋₃ Alkanoyl, N (C₁₋₃ Alkyl)₂, N(C₁₋₃ Alkyl)(C₁₋₃ Alkanoyl) substituiert sein können,
- ***B***: COOH, COOR⁵, CONH₂, CONHNH₂, CONHR⁵, CONR⁵R^{5'}, CONHOH, CONHOR⁵, SO₃H, SO₂NH₂, SO₂NHR⁵, SO₂NR⁵R^{5'}, PO₃H, PO(OH)(OR⁵), PO(OR⁵) (OR^{5'}), PO(OH)(NHR⁵), PO(NHR⁵) (NHR^{5'}), Tetrazolyl,
jeweils gebunden an ein Kohlenstoffatom der Gruppe ***A***,
oder die gesamte Gruppe ***Y-A-B*** N(SO₂R⁴)(SO₂R^{4'}) oder NHSO₂R⁴,
- ***X***: eine Bindung, CH₂, (CH₂)₂, CH(CH₃), (CH₂)₃, CH(CH₂CH₃), CH(CH₃)CH₂, CH₂CH(CH₃),
- ***Y***: O, NH, NR⁴,NCOR⁴, NSO₂R⁴,
bedeuten,
mit der Maßgabe,dass,
A) falls Y NH, NR⁴, NCOR⁴ oder NSO₂R⁴ bedeutet, a) und der Substituent ***R²*** einen stickstoffhaltigen gesättigten Heterocyclus enthält, dieser Heterocyclus nicht am Iminstickstoff mit H, Methyl, Ethyl, Propyl oder Isopropyl substituiert ist, oder b) in gegebenenfalls vorhandenen Gruppen XNHR⁴ oder XNR⁴R^{4'} des Substituenten ***R²*** R⁴ und/oder R^{4'} nicht C₁₋₄-Alkyl bedeuten,
   und dass
B) nicht gleichzeitig ***B*** COOH, SO₃H, PO₃H₂ oder Tetrazolyl und ***R¹*** und ***R²*** unabhängig voneinander C₅₋₆ Heteroaryl oder Phenyl bedeuten, wenn diese unabhängig voneinander unsubstituiert, einfach mit C₁₋₆ Alkyl, C₁₋₄ Perfluoralkyl, O C₁₋₆ Alkyl, O C₁₋₄ Perfluoralkyl, COOH, COO C₁₋₆ Alkyl, CO C₁₋₆ Alkyl, CONH₂, CONHR⁴, NO₂, NH₂, NHCOR⁴, NHSO₂R⁴ oder mit 1 oder 2 Halogenatomen aus der Gruppe F, Cl, Br, J substituiert sind, und
wobei die folgenden Verbindungen ausgeschlossen sind:
[(1,2-Diphenyl-1H-benzimidazol-6-yl)oxy]essigsäuremethylester,
5-[(1,2-Diphenyl-1H-benzimidazol-6-yl)oxy]pentansäuremethylester,
4-[(1,2-Diphenyl-1H-benzimidazol-6-yl)oxy]butansäureethylester,
5-[[1-(4-Nitrophenyl)-2-phenyl-1H-benzimidazol-6-yl]oxy]pentansäuremethylester,
6-[[1-(4-Nitrophenyl)-2-phenyl-1H-benzimidazol-6-yl]oxy]hexansäuremethylester.
5-[[1-(4-Aminophenyl)-2-phenyl-1H-benzimidazol-6-yl]oxy]pentansäuremethylester,
5-[[1-[4-[[(4-Chlorphenyl)sulfonyl]amino]phenyl]-2-phenyl-1H-benzimidazol-6-yl]oxy]pentansäuremethylester,
5-[[1-[4-[(Acetyl)amino]phenyl]-2-phenyl-1H-benzimidazol-6-yl]oxy]pentansäuremethylester,
5-[[1-(3-Nitrophenyl)-2-phenyl-1H-benzimidazol-6-yl]oxy]pentansäuremethylester,
6-[[1-(3-Nitrophenyl)-2-phenyl-1H-benzimidazol-6-yl]oxy]hexansäuremethylester,
5-[[1-(3-Aminophenyl)-2-phenyl-1H-benzimidazol-6-yl]oxy]pentansäuremethylester,
5-[[1-[3-[[(4-Chlorphenyl)sulfonyl]amino]phenyl]-2-phenyl-1H-benzimidazol-6-yl]oxy]pentansäuremethylester,
5-[[1-[3-[(Acetyl)amino]phenyl]-2-phenyl-1H-benzimidazol-6-yl]oxy]pentansäuremethylester.

Die physiologisch verträglichen Salze können mit anorganischen und organischen Säuren gebildet werden wie beispielsweise Oxalsäure, Milchsäure, Zironensäure, Fumarsäure, Essigsäure, Maleinsäure, Weinsäure, Phosphorsäure, HCl, HBr, Schwefelsäure, p-Toluolsulfonsäure, Methansulfonsäure.
Zur Salzbildung von Säuregruppen sind auch die anorganischen oder organischen Basen geeignet, die zur Bildung physiologisch verträglicher Salze bekannt sind wie beispielsweise Alkalihydroxide, Natrium- und Kaliumhydroxid, Erdalkalihydroxide wie Calciumhydroxid, Ammoniak, Amine wie Ethanolamin, Diethanolamin, Triethanilamin, N-Methylglucamin, Tris-(hydroxymethyl)-methylamin.
Unter einer "Arylgruppe" ist insbesondere eine gegebenenfalls substituierte Phenylgruppe oder Biphenyl, Naphthyl, Indan oder Fluorenyl zu verstehen.
Eine Heteroarylgruppe ist aus 5-10 Gerüstatomen aufgebaut und kann 1-4 Heteroatome enthalten. Heteroatome sind Sauerstoff (O), Stickstoff (N) und Schwefel (S). Beispiele für eine monocyclische Heteroarylgruppe sind Pyrrolyl, Thienyl, Furanyl, Imidazolyl, Thiazolyl, Isothiazolyl, Oxazolyl, Isoxazolyl, Pyrazolyl, Furazanyl, Pyridyl, Pyrimidinyl, Pyrazinyl und Pyridazinyl. Beispiele für eine bicyclisches Heteroarylgruppe sind Thienoimidazolyl, Indolyl, Isoindolyl, Benzothiophenyl, Benzofuranyl, Benzimidazolyl, Indazolyl, Imidazopyridinyl, Purinyl, Chinolyl, Isochinolyl, Phthalazinyl, Chinazolinyl, Chinaxolinyl, Cinnolinyl, Naphthyridinyl und Pteridinyl. Wenn die Arylgruppen oder Heteroarylgruppen Teil von R¹ sind, erfolgt die Bindung zum N des Benzimidazoles über ein Kohlenstoffatom.

Alkylgruppen können geradkettig oder verzweigt sein. Beispiele sind Methyl, Ethyl, Propyl, Isopropyl, n-Butyl, sek. Butyl, tert. Butyl, n-Pentyl, sek. Pentyl, tert. Pentyl, Neopentyl, n-Hexyl, sek. Hexyl, Heptyl, Octyl, Nonyl, Hexyl.

Perfluorierte Alkyle sind vorzugsweise CF₃ und C₂F₅ und Alkanoyle vorzugsweise Formyl, Acetyl, Propionyl.

Alkenylgruppen können geradkettig oder verzweigt sein. Beispielsweise seien die folgenden Reste genannt: Vinyl, 2-Propenyl, 1-Propenyl, 2-Butenyl, 1-Butenyl, 1-Methyl-1-propenyl, 2-Methyl-2-propenyl, 3-Methyl-2-propenyl.

Alkinylgruppen können geradkettig oder verzweigt sein. Beispiele hierfür sind: Ethinyl, 1-Propinyl, 2-Propinyl, 1-Butinyl, 2-Butinyl.

Unter Cycloalkylgruppen sind jeweils Cyclopropyl, Cyclobutyl, Cyclopentyl, Cyclohexyl oder Cycloheptyl zu verstehen.

Als gesättigter Heterocyclus bzw. als Cycloalkyl mit 1 oder mehreren Heteroatomen sei beispielsweise genannt: Piperidin, Pyrrolidin, Tetrahydrofuran, Morpholin, Piperazin, Hexahydroazepin sowie 2,6-Dimethyl morpholin, N-Phenyl-piperazin, Methoxymethylpyrrolidin, wobei die Verknüpfung mit einem dem Ring benachbarten Kohlenstoff über gegebenenfalls vorhandene Ringstickstoffe erfolgen kann.

Als Alkane, Alkene und Alkine für ***A*** seien beispielsweise genannt:
geradkettiges oder verzweiges Alkylen mit 1-8 C-Atomen wie: Methylen, Ethylen, Propylen, Butylen, Pentylen usw., 1-Methylethylen, 1-Ethylethylen, 1-Methylpropylen, 2-Mehtylpropylen, 1-Methylbutylen, 2-Methylbutylen, 1-Ethylbutylen, 2-Ethylbutylen, 1-Methylpentylen, 2-Methylpentylen, 3-Methylpentylen usw.
Geradgettiges oder verzweigtes Alkenylen und Alkinylen mit 2-8 C-Atomen sind Alkenylengruppen bzw. Alkinylengruppen mit Doppel- und Dreifachbindungen in allen möglichen Positionen sowie mit allen möglichen Methyl- oder Ethylsubstitutionen. In diesen Resten können jeweils ein oder zwei C-Atome gegen O, NH, NC₁₋₃-Alkyl oder N-C₁₋₃-Alkanoyl ausgetauscht sein, wobei die ausgetauschte Gruppe mindestens durch 2 C-Atome von Y getrennt ist.

Wenn zwei Reste orthoständig sind, können sie mit dem benachbarten Aromaten einen gemeinsamen Ring bilden Verbindungen. Verbindungen in denen N-, O- oder S-Atome an olefinische oder acetylenische Mehrfachbindungen gebunden sind, oder in denen mehrere N-, O-, S- oder Halogenatome an das gleiche aliphatische Kohlenstoffatom gebunden sind, oder in denen N-, O- oder S-Atome unmittelbar aneinander gebunden sind, sind ausgenommen, sofern diese Verknüpfungen nicht explizit, etwa in den im Anspruch genannten funktionellen Gruppen oder in Heteroaromaten definiert sind.

Bevorzugt sind die Benzimidazole bei denen
- ***R¹***: eine mono- oder bicyclische C₆₋₁₂Arylgruppe oder eine mono- oder bicyclische 5- 10 gliedrige Heteroarylgruppe mit 1-2 Heteroatomen ausgewählt aus der Gruppe bestehend aus N, S oder O bedeutet, wobei die genannte Aryl-oder Heteroarylgruppe mit bis zu drei der folgenden Substituenten unabhängig voneinander substituiert sein kann: F, Cl, Br, XOH, XOR⁴, XOCOR⁴, XOCONHR⁴, XOCOOR⁴, XCOR⁴, XCN, XCOOH, XCCOR⁴, XCONH₂, XCONR⁴R^{4'}, XCONHR⁴, XCONHOH, XCONHOR⁴, XCOSR⁴, XSR⁴, NO₂, XNHR⁴, XNR⁴R^{4'}, R⁴, wobei zwei Substituenten an ***R¹***, wenn sie zueinander orthoständig sind, so miteinander verknüpft sein können, daß sie gemeinsam Methandiylbisoxy, Ethan- 1,2-diylbisoxy, Propan-1,3-diyl, Butan-1,4-diyl bilden, bedeutet.

Bevorzugt sind auch Benzimidazole bei denen
- ***R²***: eine mono- oder bicyclische C₆₋₁₀-Arylgruppe oder eine mono- oder bicyclische 5- 10 gliedrige Heteroarylgruppe mit 1-2 Heteroatomen ausgewählt aus der Gruppe bestehend aus N, S oder O bedeutet, wobei die genannte Aryl-oder Heteroarylgruppe mit bis zu drei der folgenden Substituenten unabhängig voneinander substituiert sein kann: F, Cl, Br, XOH, XOR⁴, XOCOR⁴, XOCONHR⁴, XOCOOR⁴, XCOR⁴, XC(NOH)R⁴, XC(NOR⁴)R^{4'}, XC(NO(COR⁴))R^{4'}, XCOOH, XCOOR⁴, XCONH₂, XCONHR⁴, XCONR⁴R^{4'}, XCONHOH, XCONHOR⁴, XCOSR⁴, XSR⁴, XSOR⁴, XSO₂R⁴, SO₂NH₂, SO₂NHR⁴, SO₂NR⁴R^{4'}, NO₂, XNHR⁴, XNR⁴R^{4'}, XNHSO₂R⁴, XN(SO₂R⁴) SO₂R^{4'}, XNR⁴SO₂R^{4'}, R⁴, wobei zwei Substituenten an ***R²***, wenn sie zueinander orthoständig sind, so miteinander verknüpft sein können, daß sie gemeinsam Methandiylbisoxy, Ethan- 1,2-diylbisoxy, Propan-1,3-diyl, Butan-1,4-diyl bilden, bedeutet.

Ebenfalls bevorzugt sind Benzimidazole der allgemeinen Formel I, bei denen
- ***R³***: ein oder zwei Substituenten, die unabhängig voneinander: Wasserstoff, F, Cl, Br, XOH, XOR⁴, XOCOR⁴, XOCONHR⁴, XOCOOR⁴, XCOR⁴, XC(NOH)R⁴, XC(NOR⁴)R^{4'}, XC(NO(COR⁴))R^{4'} XCN, XSR⁴, XSOR⁴, XSO₂R⁴,SO₂NH₂, SO₂NHR⁴, SO₂NR⁴R^{4'}, NO₂, XNH₂, XNHR⁴, XNR⁴R^{4'}, XNHSO₂R⁴, XNR⁴SO₂R^{4'}, XN(SO₂R⁴) SO₂R^{4'}, XNHCOR⁴, XNHCOOR⁴, XNHCONHR⁴, oder R⁴ sein können, wobei zwei Substituenten ***R³***, wenn sie zueinander orthoständig sind, so miteinander verknüpft sein können, daß sie gemeinsam Methandiylbisoxy, Ethan-1,2-diylbisoxy, Propan- 1,3-diyl, Butan-1,4-diyil bilden, bedeutet.

Bevorzugt sind auch Benzimidazole der allgemeinen Formel I, bei denen
- **R⁴**: und **R^{4'}** unabhängig voneinander CF₃, C₂F₅, C₁₋₄-Alkyl, C₂₋₄-Alkenyl, C₂₋₄-Alkinyl, C₃₋₆- Cycloalkyl, (C₁₋₃-Alkyl-C₃₋₆-Cycloalkyl), Phenyl oder 5-6 gliedriges Heteroaryl mit 1-2 N-, S- oder O-Atomen, wobei die Phenyl- und Heteroarylgruppen mit ein oder zwei Substituenten aus der Gruppe bestehend aus F, Cl, Br, CH₃, C₂H₅, OCH₃, OC₂H₅, CF₃, C₂F₅ substituiert sein können. und weiterhin in einem 5-gliedrigen Cycloalkylring ein Ringglied ein N oder ein O sein kann und in einem 6-gliedrigen Cycloalkylring ein oder zwei Ringglieder N und/oder O sein können, wobei Ringstickstoffe gegenbenenfalls mit C₁₋₃-Alkyl oder C₁₋₃-Alkanoyl substituiert sein können, bedeuten.

Ebenfalls bevorzugt sind Benzimidazole der allgemeinen Formel I, bei denen
- ***R⁵***: und **R^{5'}** unabhängig voneinander C₁₋₆ Alkyl, wobei ein Kohlenstoffatom gegen O, NH, N C₁₋₃ Alkyl, N C₁₋₃ Alkanoyl ausgetauscht sein kann, C₃₋₇ Cycloalkyl-C₀₋₃ Alkyl, wobei in einem 5-gliedrigen Cycloalkylring ein Ringglied ein N oder ein O sein kann und in einem 6- oder 7-gliedrigen Cycloalkylring ein oder zwei Ringglieder N und/oder O sein können, wobei Ringstickstoff gegebenenfalls mit C₁₋₃ Alkyl oder C₁₋₃ Alkanoyl substituiert sein können, wobei der genannte C₁₋₆ Alkylteil mit einem der zuvor genannten Cycloalkyle oder auch einem 5-6 gliedrigen Heteroaromaten mit 1-2 Heteroatomen, ausgewählt aus N, S oder O, substituiert sein kann, wobei alle zuvor genannten Alkyl- und Cycloalkylteile mit bis zu zwei Substituenten aus CF₃, OH, O C₁₋₃ Alkyl, und die zuvor genannten Heteroarylgruppen mit ein oder zwei Substituenten aus F, Cl, CF₃, CH₃, C₂H₅, OCH₃, OC₂H₅ substituiert sein können oder R⁵ und R^{5'} gemeinsam mit dem Stickstoffatom einen 5-7 gliedrigen Heterocyclus bilden, der ein weiteres Sauerstoff-, Stickstoff- oder Schwefelatom enthalten kann und substituiert sein kann mit C₁₋₄-Alkyl, C₁₋₄-Alkoxy-C₀₋₂-alkyl, C₁₋₄- Alkoxy-carbonyl, Aminocarbonyl oder Phenyl, bedeuten.

Bevorzugt sind auch Benzimidazole der allgemeinen Formel I, bei denen
- ***A***: C₁₋₁₀ Alkandiyl, C₂₋₁₀ Alkendiyl, C₂₋₁₀ Alkindiyl, (C₀₋₅ Alkandiyl-C₃₋₇ Cycloalkandiyl- C₀₋₅ Alkandiyl), wobei in einem 5-gliedrigen Cycloalkylring ein Ringglied ein N oder ein O sein kann und in einem 6- oder 7-gliedrigen Cycloalkylring ein oder zwei Ringglieder N und/oder O sein können, wobei Ringstickstoffe gegebenenfalls mit C₁₋₃ Alkyl oder C₁₋₃ Alkanoyl substituiert sein können, wobei in den oben genannten aliphatischen Ketten ein Kohlenstoffatom oder zwei Kohlenstoffatome gegen O, NH, N C₁₋₃ Alkyl, N C₁₋₃ Alkanoyl ausgetauscht sein können, bedeutet.

Ebenfalls bevorzugt sind Benzimidazole der allgemeinen Formel I, bei denen
- ***B***: COOH, COOR⁵, CONH₂, CONHR⁵, CONR⁵R^{5'}, CONHOH, CONHOR⁵ oder Tetrazolyl, jeweils gebunden an ein Kohlenstoffatom der Gruppe ***A*** bedeutet.

Insbesondere bevorzugt ist B in der Bedeutung von COO R⁵, CONH₂, CONH R⁵, CON R⁵ R^{5'} oder.

Bevorzugt sind auch Benzimidazole der allgemeinen Formel I, bei denen
- ***X***: eine Bindung oder Methylen bedeutet.

Bevorzugt sind auch Benzimidazole der allgemeinen Formel I, bei denen
- ***Y***: O bedeutet. Insbesondere bedeuten R¹ und R² unabhängig voneinander Phenyl oder 5-6 gliedriges Heteroaryl, mit 1-2 Heteroatomen wie N-, O- oder S-Stome, die substituiert sein können mit F, Cl, Br, Cyano, C₁₋₄-Alkyl, C₁₋₄-Alkoxy, Methylendioxy, C₁₋₄-Alkylthio, NO₂, CF₃, NH₂, NH (C₁₋₃-Alkyl), N (C₁₋₃-Alkyl)₂. Insbesondere bevorzugt für R³ ist die Bedeutung H, F, Cl,, OH, C₁₋₄-Alkoxy, C₁₋₄-Alkyl, NO₂, NH₂, NH-C₁₋₄-Alkanoyl NH-SO₂-Benzyl oder NH-SO₂-Phenyl, wobei der Phenylrest substituiert sein kann mit F, Cl, Br, C₁₋₄-Alkyl, C₁₋₄-Alkoxy, CF₃ oder Acetylamino.

Besonders bevorzugt sind die folgenden Benzimidazole:
[(1,2-Diphenyl-1*H*-benzimidazol-6-yl)oxy]essigsäureisopropylester
3-[(1,2-Diphenyl-1*H*-benzimidazol-6-yl)oxy]propansäuremethylester
2-[( 1,2-Diphenyl-1*H*-benzimidazol-6-yl)oxy]propansäuremethylester
4-[(1,2-Diphenyl-1*H*-benzimidazol-6-yl)oxy]butansäureisopropylester
5-[(1,2-Diphenyl-1*H*-benzimidazol-6-yl)oxy]pentansäureisopropylester
6-[(1,2-Diphenyl-1*H*-benzimidazol-6-yl)oxy]hexansäuremethylester
6-[(1,2-Diphenyl-1*H*-benzimidazol-6-yl)oxy]hexansäureisopropylester
6-[(1,2-Diphenyl-1*H*-benzimidazol-6-yl)oxy]hexanamid
*N*-Methoxy-6-[(1,2-diphenyl-1*H*-benzimidazol-6-yl)oxy]hexanamid
*N*-(Phenylmethoxy)-6-[(1,2-diphenyl-1*H*-benzimidazol-6-yl)oxy]hexanamid
N-*Hydroxy-6-[(1,2-diphenyl-1H-benzimidazol-6-yl)oxy]hexanamid*
7-[(1,2-Diphenyl-1*H-*benzimidazol-6-yl)oxy]heptansäuremethylester
6-[[1-(3-Nitrophenyl)-2-phenyl-1*H*-benzimidazol-6-yl]oxy]hexansäureisopropylester
6-[[2-Phenyl-1-[3-(trifluormethyl)phenyl]-1*H*-benzimidazol-6-yl]oxy]hexansäuremethylester
6-[[2-Phenyl-1-[3-(trifluormethyl)phenyl]-1*H*-benzimidazol-6-yl]oxy]hexansäure-isopropylester
6-[[1-(3-Cyanophenyl)-2-phenyl-1*H*-benzimidazol-6-yl]oxy]hexansäuremethylester
6-[[1-(3-Cyanophenyl)-2-phenyl-1*H*-benzimidazol-6-yl]oxy]hexansäureisopropylester
6-[[1-(3-Cyanophenyl)-2-phenyl-1*H*-benzimidazol-6-yl]oxy]hexansäure
6-[[1-(4-Cyanophenyl)-2-phenyl-1*H*-benzimidazol-6-yl]oxy]hexansäuremethylester
6-[[1-(4-Cyanophenyl)-2-phenyl-1*H*-benzimidazol-6-yl]oxy]hexansäureisopropylester
6-[[1-(3-Chlorphenyl)-2-phenyl-1*H*-benzimidazol-6-yl]oxy]hexansäuremethylester
6-[[1-(3-Chlorphenyl)-2-phenyl-1*H*-benzimidazol-6-yl]oxy]hexansäureisopropylester
6-[[1-(4-Chlorphenyl)-2-phenyl-1*H*-benzimidazol-6-yl]oxy]hexansäuremethylester
6-[[1-(4-Chlorphenyl)-2-phenyl-1*H*-benzimidazol-6-yl]oxy]hexansäureisopropylester
6-[[1-(3-Methylphenyl)-2-phenyl-1*H*-benzimidazol-6-yl]oxy]hexansäuremethylester
6-[[1-(3-Methylphenyl)-2-phenyl-1*H*-benzimidazol-6-yl]oxy]hexansäureisopropylester
6-[[1-(4-Methylphenyl)-2-phenyl-1*H*-benzimidazol-6-yl]oxy]hexansäuremethylester
6-[[1-(4-Methylphenyl)-2-phenyl-1*H*-benzimidazol-6-yl]oxy]hexansäureisopropylester
6-[[1-(3,4-Dimethylphenyl)-2-phenyl-1*H*-benzimidazol-6-yl]oxy]hexansäuremethylester
6-[[1-(3,5-Dimethylphenyl)-2-phenyl-1*H*-benzimidazol-6-yl]oxy]hexansäuremethylester
6-[[1-(3,5-Dimethylphenyl)-2-phenyl-1*H*-benzimidazol-6-yl]oxy]hexansäureisopropylester
6-[[1-(3-Methoxyphenyl)-2-phenyl-1*H*-benzimidazol-6-yl]oxy]hexansäuremethylester
6-[[1-(4-Methoxyphenyl)-2-phenyl-1*H*-benzimidazol-6-yl]oxy]hexansäuremethylester
6-[[1-(3,4-Dimethoxyphenyl)-2-phenyl-1*H*-benzimidazol-6-yl]oxy]hexansäuremethylester
6-[[1-[3,4-(Methylendioxy)phenyl]-2-phenyl-1*H*-benzimidazol-6-yl]oxy]hexansäuremethylester
6-[[1-[3,4-(Methylendioxy)phenyl]-2-phenyl-1*H*-benzimidazol-6-yl]oxy]hexansäure
6-[[2-Phenyl-1-(3,4,5-trimethoxyphenyl)-1*H*-benzimidazol-6-yl]oxy]hexansäuremethylester
6-[[2-Phenyl-1-(3,4,5-trimethoxyphenyl)-1*H*-benzimidazol-6-yl]oxy]hexansäure
6-[[2-Phenyl-1-(3,4,5-trimethoxyphenyl)-1*H*-benzimidazol-6-yl]oxy]hexan-säureisopropylester
6-[[1-[4-(*N*,*N-*Dimethylamino)phenyl]-2-phenyl-1*H*-benzimidazol-6-yl]oxy]hexansäuremethylester
6-[[1-[4-(*N*,*N-*Dimethylamino)phenyl]-2-phenyl-1*H*-benzimidazol-6-yl]oxy]hexansäure
6-[[1-Phenyl-2-[3-(trifluormethyl)phenyl]-1*H*-benzimidazol-6-yl]oxy]hexansäureisopropylester
6-[[2-(3-Chlorphenyl)-1-phenyl-1*H*-benzimidazol-6-yl]oxy]hexansäuremethylester
6-[[2-(3-Chlorphenyl)-1-phenyl-1*H*-benzimidazol-6-yl]oxy]hexansäureisopropylester
6-[[2-(4-Chlorphenyl)-1-phenyl-1*H*-benzimidazol-6-yl]oxy]hexansäuremethylester
6-[[2-(4-Chlorphenyl)-1-phenyl-1*H*-benzimidazol-6-yl]oxy]hexansäureisopropylester
6-[[2-(4-Methylphenyl)-1-phenyl-1*H*-benzimidazol-6-yl]oxy]hexansäuremethylester
6-[[2-(4-Methylphenyl)-1-phenyl-1*H*-benzimidazol-6-yl]oxy]hexansäureisopropylester
6-[[1-Phenyl-2-(4-pyridinyl)-1*H*-benzimidazol-6-yl]oxy]hexansäuremethylester
6-[(1,2-Diphenyl-5-nitro-1*H*-benzimidazol-6-yl)oxy]hexansäuremethylester
6-[(1,2-Diphenyl-5-nitro-1*H*-benzimidazol-6-yl)oxy]hexansäureisopropylester
6-[[5-[[(4-Bromphenyl)sulfonyl]amino]-1,2-diphenyl-1*H*-benzimidazol-6-yl]oxy]hexansäureisopropylester
6-[[5-[[(4-Chlorphenyl)sulfonyl]amino]-1,2-diphenyl-1*H*-benzimidazol-6-yl]oxy]hexansäuremethylester
6-[[5-[[(4-Chlorphenyl)sulfonyl]amino]-1,2-diphenyl-1*H*-benzimidazol-6-yl]oxy]hexansäureisopropylester
6-[[1,2-Diphenyl-5-[[(3-methylphenyl)sulfonyl]amino]-1*H*-benzimidazol-6-yl]oxy]hexansäureisopropylester
6-[[1,2-Diphenyl-5-[[(4-methylphenyl)sulfonyl]amino]-1*H*-benzimidazol-6-yl]oxy]hexansäureisopropylester
6-[[1,2-Diphenyl-5-[[(4-methoxyphenyl)sulfonyl]amino)-1*H*-benzimidazol-6-yl]oxy]hexansäureisopropylester
6-[[1,2-Diphenyl-5-[[[(4-trifluormethyl)phenyl]sulfonyl]amino]-1*H*-benzimidazol-6-yl]oxy]-hexansäureisopropylester
6-[[5-[[[4-(Acetylamino)phenyl]sulfonyl]amino]-1,2-diphenyl-1*H*-benzimidazol-6-yl]oxy]-hexansäureisopropylester
6-[[5-[[Bis(3-chlorphenyl)sulfonyl]amino]-1,2-diphenyl-1*H*-benzimidazol-6-yl]oxy]hexansäureisopropylester
6-[[1,2-Diphenyl-5-[(propylsulfonyl)amino]-1*H*-benzimidazol-6-yl]oxy]hexansäureisopropylester
6-[[5-[(Benzylsulfonyl)amino]-1,2-diphenyl-1*H*-benzimidazol-6-yl]oxy]hexansäure-isopropylester
2-[2-[(1,2-Diphenyl-1*H*-benzimidazol-6-yl)oxy]ethoxy]essigsäuremethylester
3-[2-[(1,2-Diphenyl-1*H*-benzimidazol-6-yl)oxy]ethoxy]propansäuremethylester
6-[[1-(3-Nitrophenyl)-2-phenyl-1*H*-benzimidazol-6-yl]oxy]hexansäureethylester
6-[[4-Acetyl-1-(4-methylphenyl)-2-phenyl-1*H*-benzimidazol-6-yl]oxy]hexansäuremethylester
6-[[1-(4-Methylphenyl)-2-phenyl-1*H*-benzimidazol-5-yl]oxy]hexansäuremethylester
6-[[2-Phenyl-1-[4-(thiomethyl)phenyl]-1*H*-benzimidazol-5-yl]oxy]hexansäuremethylester
6-[[2-Phenyl-1-[(4-thiomethyl)phenyl]-1*H*-benzimidazol-6-yl]oxy]hexansäuremethylester
6-[[2-Phenyl-1-(3-thienyl)-1*H*-benzimidazol-5-yl]oxy]hexansäuremethylester
6-[[2-Phenyl-1-(3-thienyl)-1*H*-benzimidazol-6-yl]oxy]hexansäuremethylester
4-[(1,2-Diphenyl-1*H*-benzimidazol-6-yl)oxy]butansäuremethylester
*N*-(Phenylmethoxy)-6-[[2-phenyl-1-(3,4,5-trimethoxyphenyl)-1*H*-benzimidazol-6-yl]oxy]-hexanamid
*N,N-*Dimethyl-6-[(1,2-diphenyl-1*H*-benzimidazol-6-yl)oxy]hexanamid *N-*Isospropyl-6-[(1,2-diphenyl-1*H*-benzimidazol-6-yl)oxy]hexanamid
6-[(1,2-Diphenyl-1*H*-benzimidazol-6-yl)oxy]-1-pyrrolidin-1-ylhexan-1-on
5-[[5-[[(4-Chlorphenyl)sulfonyl]amino]-1,2-diphenyl-1*H*-benzimidazol-6-yl]oxy]pentansäure-methylester
6-[[5-[[(4-Chlorphenyl)sulfonyl]amino]-1-(4-methylphenyl)-2-phenyl-1*H*-benzimidazol-6-yl]oxy]hexansäuremethylester
6-[[5-[[(4-Chlorphenyl)sulfonyl]amino]-1-(4-methoxyphenyl)-2-phenyl-1*H*-benzimidazol-6-yl]oxy]hexansäuremethylester
6-[[4-(Acetyloxy)-1-(4-methylphenyl)-2-phenyl-1*H*-benzimidazol-6-yl]oxy]hexansäuremethylester
6-[[4-Hydroxy-1-(4-methylphenyl)-2-phenyl-1*H*-benzimidazol-6-yl]oxy]hexansäuremethylester
6-[[4-Hydroxy-1-(4-methylphenyl)-2-phenyl-1*H*-benzimidazol-6-yl]oxy]hexansäure
6-[[7-Methyl-1-(4-methylphenyl)-2-phenyl-1*H*-benzimidazol-6-yl]oxy]hexansäuremethylester
6-[[2-Phenyl-1-(3-pyridyl)-1*H*-benzimidazol-5-yl]oxy]hexansäuremethylester
6-[[2-Phenyl-1-(3-pyridyl)-1*H*-benzimidazol-6-yl]oxy]hexansäuremethylester
6-[[2-Phenyl-1-(4-pyridyl)-1*H*-benzimidazol-6-yl]oxy]hexansäuremethylester
6-[[2-(4-Fluor-phenyl)-1-phenyl-1*H*-benzimidazol-6-yl]oxy]hexansäuremethylester
6-[[2-(4-Methoxyphenyl)-1-phenyl-1*H*-benzimidazol-6-yl]oxy]hexansäuremethylester
6-[[2-(4-Bromphenyl)-1-phenyl-1*H*-benzimidazol-6-yl]oxy]hexansäuremethylester
6-[[2-[4-(Trifluormethyl)phenyl]-1-phenyl-1*H*-benzimidazol-6-yl]oxy]hexansäuremethylester
6-[[1-Phenyl-2-(benzothien-2-yl)-1*H*-benzimidazol-6-yl]oxy]hexansäuremethylester
6-[[1-Phenyl-2-(benzothien-2-yl)-1*H*-benzimidazol-6-yl]oxy]hexansäure
6-[[5-Hydroxy-1-(4-methylphenyl)-2-phenyl-1*H*-benzimidazol-6-yl]oxy]hexansäure-isopropylester
6-[[5-Hydroxy-1-(4-methylphenyl)-2-phenyl-1*H*-benzimidazol-6-yl]oxy]hexansäure
6-[[5-Methoxy-1-(4-methylphenyl)-2-phenyl-1*H*-benzimidazol-6-yl]oxy]hexansäure-isopropylester
6-[[5-Hydroxy-1-(4-methylphenyl)-2-phenyl-1*H*-benzimidazol-6-yl]oxy]hexansäuremethylester
6-[[5-Methoxy-1-(4-methylphenyl)-2-phenyl-1*H*-benzimidazol-6-yl]oxy]hexansäuremethylester
6-[[5-[[(4-Chlorphenyl)sulfonyl]amino]-1-(3,4-dimethylphenyl)-2-phenyl-1*H*-benzimidazol-6-yl]oxy]hexansäuremethylester benzimidazol-6-yl]oxy]hexansäuremethylester
6-[[5-[[(4-Chlorphenyl)sulfonyl]amino]-2-(4-fluorphenyl)-1-(4-methoxyphenyl)-1*H*-benzimidazol-6-yl]oxy]hexansäuremethylester
6-[[5-[[(4-Chlorphenyl)sulfonyl]amino]-1-(4-methoxyphenyl)-2-(4-methoxyphenyl)-1*H-*benzimidazol-6-yl]oxy]hexansäuremethylester
4-[[5-[[(4-Chlorphenyl)sulfonyl]amino]-1-(4-methoxyphenyl)-2-phenyl-1*H*-benzimidazol-6-yl]oxy]butansäuremethylester
5-[[5-[[(4-Chlorphenyl)sulfonyl]amino]-1-(4-methoxyphenyl)-2-phenyl-1*H*-benzimidazol-6-yl]oxy]pentansäuremethylester
5-[[5-[[(4-Chlorphenyl)sulfonyl]amino]-1,2-diphenyl-1*H*-benzimidazol-6-yl]oxy]pentansäure-methylester
6-[[5-[[(4-(Trifluormethyl)phenyl)sulfonyl]amino]-1-(4-methoxyphenyl)-2-phenyl-1*H-*benzimidazol-6-yl]oxy]hexansäuremethylester
6-[[5-[[(4-Chlorphenyl)sulfonyl]methylamino]-1-(4-methoxyphenyl)-2-phenyl-1*H-*benzimidazol-6-yl]oxy]hexansäuremethylester
6-[[1-(Indan-5-yl)-2-phenyl-1*H*-benzimidazol-6-yl]oxy]hexansäuremethylester
6-[[1-(Indan-5-yl)-2-phenyl-1*H*-benzimidazol-6-yl]oxy]hexansäure
6-[[1-(3-Fluorphenyl)-2-phenyl-1*H*-benzimidazol-6-yl]oxy]hexansäuremethylester
6-[[2-(4-Nitrophenyl)-1-phenyl-1*H*-benzimidazol-6-yl]oxy]hexansäuremethylester
6-[[1-Phenyl-2-(3-pyridinyl)-1*H*-benzimidazol-6-yl]oxy]hexansäuremethylester
*N*-(Cyclopropylmethoxy)-6-[(1,2-diphenyl-1*H*-benzimidazol-6-yl)oxy]hexanamid
*N*-Isobutoxy-6-[(1,2-diphenyl-1*H*-benzimidazol-6-yl)oxy]hexanamid
*N*-(Cyclopropylmethoxy)-6-[2-phenyl-1-(3,4,5-trimethoxyphenyl)-1H -benzimidazol-6-yl)oxy]-hexanamid
*N*-Isobutoxy-6-[2-phenyl-1-(3,4,5-trimethoxyphenyl)-1H-benzimidazol-6-yl)oxy]hexanamid
*N*-(2-Methoxyethyl)-6-[(1,2-diphenyl-1*H*-benzimidazol-6-yl)oxy]hexanamid
*N*-(3-Methoxypropyl)-6-[(1,2-diphenyl-1*H*-benzimidazol-6-yt)oxy]hexanamid
*N*-Isobutyl-6-[(1,2-diphenyl-1*H*-benzimidazol-6-yl)oxy]hexanamid
6-[(1,2-Diphenyl-1*H*-benzimidazol-6-yl)oxy]-1-morpholin-1-ylhexan-1-on
*N*,*N*-Di(-2-methoxyethyl)-6-[(1,2-diphenyl-1*H*-benzimidazol-6-yl)oxy]hexanamid
*N*-Isopentyl-6-[(1,2-diphenyl-1*H*-benzimidazol-6-yl)oxyl)hexanamid
*N*-(Pyridin-2-yl)-6-[(1,2-diphenyl-1*H*-benzimidazol-6-yl)oxy]hexanamid
*N*-(Pyridin-3-yl)-6-[(1,2-diphenyl-1*H*-benzimidazol-6-yl)oxy]hexanamid
*N*-Isopropyl-6-[[1-(3,4-dimethylphenyl)-2-phenyl-1*H*-benzimidazol-6-yl]oxy]hexanamid
*N*,*N*-Dimethyl-6-[[1-(3,4-dimethyiphenyl)-2-phenyl-1*H*-benzimidazol-6-yl]oxy]hexanamid
*N*,*N*-Diethyl-6-[[1-(3,4-dimethytphenyl)-2-phenyl-1*H*-benzimidazol-6-yl]oxy]hexanamid
*N*-Isobutyl-6-[[1-(3,4-dimethylphenyl)-2-phenyl-1*H*-benzimidazol-6-yl]oxy]hexanamid
*N*-Cyclopropyl-6-[[1-(3,4-dimethylphenyl)-2-phenyl-1*H*-benzimidazol-6-yl]oxy]hexanamid
*N*-Cyclobutyl-6-[[1-(3,4-dimethylphenyl)-2-phenyl-1*H*-benzimidazol-6-yl]oxy]hexanamid
*N*-tert-Butyl-6-[[1-(3,4-dimethylphenyl)-2-phenyl-1*H*-benzimidazol-6-yl]oxy]hexanamid
(*R*)-6-[[1-(3,4-Dimethylphenyl)-2-phenyl-1*H*-benzimidazol-6-yl]oxy]1-(2-methoxymethyl)-pyrrolidin-1-ylhexan-1-on
*N*-(3-Imidazol-1-yl-propyl)-6-[[1-(3,4-dimethylphenyl)-2-phenyl-1*H*-benzimidazol-6-yl]oxy]hexanamid
*N*-(2-Pyridin-2-ylethyl)-6-gl-(3,4-dimethylphenyl)-2-phenyl-1*H*-benzimidazol-6-yl]oxy]hexanamid
*N*-(3-Methoxypropyl)-6-[[1-(indan-5-yl)-2-phenyl-1*H*-benzimidazol-6-yl]oxy]heptanamid
6-[[1-(4-Methylphenyl)-2-(3-pyridyl)-1*H*-benzimidazol-6-yl]oxy]hexansäuremethylester
6-[[1-(4-Methylphenyl)-2-(4-pyridyl)-1*H*-benzimidazol-6-yl]oxy]hexansäuremethylester
6-[[1-(4-Methylphenyl)-2-(2-thienyl)-1*H*-benzimidazol-6-yl]oxy]hexansäuremethylester
6-[[1-(4-Methylphenyl)-2-(3-thienyl)-1*H*-benzimidazol-6-yl]oxy]hexansäuremethylester
6-[[2-(3-Indolyl)-1-(4-methylphenyl)-1*H*-benzimidazol-6-yl]oxy]hexansäuremethylester
6-[[1-(4-Methylphenyl)-2-(2-fury1)-1*H*-benzimidazol-6-yl]oxy]hexansäuremethylester
6-[[1-(4-Methylphenyl)-2-(3-furyl)-1*H*-benzimidazol-6-yl]oxy]hexansäuremethylester
6-[[1-(4-Methylphenyl)-2-(5-methyl-2-thienyl)-1*H*-benzimidazol-6-yl]oxy]hexansäuremethylester
6-[[1-(4-Methytphenyl]-2-(3-methyl-2-thienyl)-1*H*-benzimidazol-6-yl]oxy]hexansäuremethylester

Die emndüngsgemäßen Benzimidazol-Derivate hemmen die Aktivierung der Mikroglia und können daher verwendet werden zur Herstellung eines Arzneimittels zur Behandlung oder Verhütung von Krankheiten, die mit einer Mikroglia assozüert sind. Unter Mikroglia werden hier die Makrophagen des Gehirns verstanden.
Diese Wirkung ist überraschend, da bisher Benzimidazel-Derivate nur für die Behandlung von Thrombosen und Arteriosklerose (EP0531883, WO9507263. EP0104727, WO97/12613), Zystitis (WO97/33873) und Krankheiten, die mit einem ß-Amyloid-Peptid (US5,552,426) und einer verstärkten Aktivierung von Ca-Kanälen (EP520200) assozüert sind, beschrieben worden sind, aber ein Effekt auf Mikroglia nicht bekannt ist.

Die Erfindung betrifft auch Verwendung eines Benzimidazols der allgemeinen Formel II 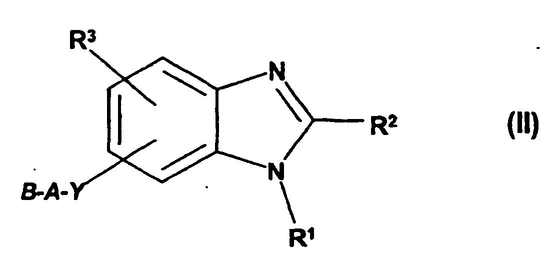 worin
- R': eine mono- oder bicyclische C₆₋₁₂-Arylgruppe oder eine mono- oder bicyclische 5- 10 gliedrige Heteroarylgruppe mit 1-4 Heteroatomen ausgewählt aus der Gruppe bestehend aus N, S oder O, wobei die genannte Aryl-oder Heteroarylgruppe mit bis zu drei der folgenden Substituenten unabhängig voneinander substituiert sein kann: F, Cl, Br, I, C(NH)NH₂, C(NH)NHR⁴, C(NH)NR⁴R⁴', C(NR⁴)NH₂, C(NR⁴)NHR⁴, C(NR⁴)NR⁴R⁴, XOH, XOR⁴, XOCOR⁴, XOCONHR⁴, XOCOOR⁴, XCOR⁴, XC(NOH)R⁴, XC(NOR⁴)R⁴', XC(NO(COR⁴))R⁴, XCN, XCOOH, XCOOR⁴, XCONH₂. XCONR⁴R⁴, XCONHR⁴, XCONHOH, XCONHOR⁴, XCOSR⁴, XSR⁴, X50R⁴, XSO₂R⁴, SO₂NH₂, SO₂NHR⁴,SO₂NR⁴R⁴, NO₂, XNH₂, XNHR⁴, XN⁴R⁴', XNHSO₂R⁴, XN(SO₂R⁴)(SO₂R^{4'}), XNR⁴SO₂R⁴', XNHCOR⁴, XNHCOOR⁴, XNHCONHR⁴, Tetrahydro-2,5-dioxopyrrol-1-yl, 2,5-Dihydro-2,5-dioxopyrrol-1-yl, 2,7-Dihydro-2,7-dioxoisoindol-1-yl, R⁴, wobei zwei Substituenten an *R*¹, wenn sie zueinander orthoständig sind, so miteinander verknüpft sein können, daß sie gemeinsam Methandiylbisoxy, Ethan-1,2-diylbisoxy, Propan-1,3-diyl, Butan-1,4- diyl bilden.
- ***R***²: eine mono- oder bicyclische C₆₋₁₀-10-Arylgruppe oder eine mono- oder bicyclische 5- 10 gliedrige Heteroarylgruppe mit 1-4 Heteroatomen ausgewählt aus der Gruppe bestehend aus N, S oder O, wobei die genannte Aryl-oder Heteroarylgruppe mit bis zu drei der folgenden Substituenten unabhängig voneinander substituiert sein kann: F, Cl, Br, I, C(NH)NH₂, C(NH)NHR⁴, C(NH)NR⁴R⁴, C(NR⁴)NH₂, C(NR⁴)NHR⁴, C(NR⁴)NR⁴R⁴, XOH, XOR⁴, XOCOR⁴, XOCONHR⁴, XOCOOR⁴, XCOR⁴; XC(NOH)R⁴, XC(NOR⁴)R⁴', XC(NO(COR⁴))R^{4'}, XCN, XCOOH, XCOOR⁴, XCONH₂, XOCNR⁴R⁴, XCONHR⁴, XCONHOH, XCONHOR⁴, XCOSR⁴ XSR⁴; XSOR⁴, XSO₂R⁴, SO₂NH₂, SO₂NHR,SO₂NR⁴R⁴, NO₂, XNH₂, XNHR⁴, XNR⁴R⁴, XNHSO₂N⁴, XN(SO₂R⁴SO₂R⁴), XNR⁴SO₂R⁴, XNHCOR⁴,XNHCOOR⁴, XNHCONHR⁴, Tetrahydro2,5-dioxopyrrol-1-yl, 2,5-Dihydro-2,5-dioxopyrrol-1-yl, 2,7-Dihydro-2,7-dioxoisoindol-1-yl, R⁴, wobei zwei Substituenten an *R*⁴, wenn sie zueinander orthoständig sind, so miteinander verknüpft sein können, daß sie gemeinsam Methandiylbisoxy, Ethan-1,2-diylbisoxy, Propan-1,3-diyl, Butan-1,4- diyl bilden,
- ***R***³ für: ein oder zwei Substituenten steht, die unabhängig voneinander: Wasserstoff. F, Cl, Br, I, XOH, XOR⁴, XOCOR⁴, XOCONHR⁴, XOCOOR⁴, XCOR⁴, XC(NOH)R⁴. XC(NOR⁴)R⁴', XC(NO(COR⁴))R^{4'}, XCN, XCOOOH, XCOOR⁴. XCONH₂, XCONHR⁴, XCONR⁴R⁴', XCONHOH, XCONHOR⁴, XCOSR⁴, XSR⁴, XSOR⁴, XSo₂R⁴,SO₂NH₂, SO₂NHR⁴, SO₂NR⁴R^{4'}, NO₂, XNH₂, XNHR⁴, XNR⁴R⁴', XNHSO₂R⁴, XNR⁴SO₂R^{4'}, XN(SO₂R⁴)(SO₂R⁴), XNHCOR⁴. XNHCCOOR⁴, XNHCONHR⁴, Tetrahydro-2,5-diaxopyrrol-yl-yl, 2,5-Dihydro-2,5-dioxopyrrol-1-yl, 2,7-Dihydro-2,7-dioxoisoindol-1-yl, R**⁴**, wobei zwei Substituenten ***R***³, wenn sie zueinander orthoständig sind, so miteinander verknüpft sein können, daß sie gemeinsam Methandiylbisoxy, Ethan-1,2-diylbisaxy, Propan-1,3-diyl, Guten-1,4- diyl bilden,
- ***R***⁴: und R^{4'} unabhängig voneinander C₁₋₄ Perfluoralkyl, C₁₋₆ Alkyl, C₂₋₆ Alkenyl, C₂₋₆ Alkenyl, C₃₋₇ Cycloalkyl. (C₁₋₃ Alkyl-C₃₋₇ Cycloalkyl), C₁₋₃ Alkyl-C₆₋₁₀-aryl, C₁₋₃ Alkyl 5- 10 gliedrige Heteroaryl mit 1-4 N-, S- oder O-Atomen C₆₋₁₀-Aryl oder 5-10 gliedriges Heteroaryl mit 1-4 N-, S- oder O-atome, wobei die C₆₋₁₀-Aryl- und Heteroarylgruppen mit ein oder zwei Substituenten aus der Gruppe bestehend aus F. Cl. Br. CH₃. C₂H₅. NO₂. OCH₃, OC₂H_{5,} CF₃, C₂F₅ substituiert sein können oder auch eine annelierte Methandiylbisoxy, Ethan-1,2-diylbisaxygruppe tragen können, wobei in einem 5-gliedrigen Cycloalkylring ein Ringglied ein N oder ein O sein kann und in einem 6- oder 7-gliedrigen Cycloalkylring ein oder zwei Ringglieder N und/oder O sein können, wobei Ringstickstoffe gegebenenfalls mit C₁₋₃ Alkyl oder C₁₋₃Alkanoyl substituiert sein können,
- ***R***⁵: und **R**⁵ unabhängig voneinander Wasserstoff C₁₋₈ Alkyl, C₂₋₆ Alkenyl, C₂₋₆ Alkinyl, wobei ein Kohlenstoffatom gegen O, S, SO. SO₂, NH. N C₁₋₃Alkyl oder N C₁₋₃ Alkanoyl ausgetauscht sein kann, C₃₋₇ Cycloalkyl-C₀₋₃ Alkyl, wobei in einem 5-gliedrigen Cycloalkylring ein Ringglied ein N oder ein O sein kann und in einem 6- oder 7-gliedrigen Cycloalkylring ein oder zwei Ringglieder N und/oder O sein können, wobei Ringstickstoffe gegebenenfalls mit C₁₋₃ Alkyl oder C₁₋₃ Alkanol substituiert sein können , C₆₋₁₀-Aryl oder 5-10 gliedriges Heteroaryl mit 1-4 Heteroatomen aus N, S und O, wobei die genannten Alkyl-, Alkenyl,- und Alkinylketten mit einem der zuvor genannten Cycloalkyle, Aryle oder Heteroaryle substituiert sein können, wobei alle zuvor genannten Alkyl- und Cycloalkylreste mit bis zu zwei Substituenten aus CF₃. C₂F₅, OH, O C₁₋₃ Alkyl, NH2, NAH C₁₋₃ Alkyl, NH C₁₋₃ Alkanoyl, N(C₁₋₃ Alkyl)₂, N(C₁₋₃ Alkyl)(C₁₋₃ Alkanoyl). COOH, CONH₂, COO C₁₋₃ Alkyl und alle zuvor genannten Aryl- und Heteroarylgruppen mit ein oder zwei Substituenten aus der Gruppe bestehend aus F, Cl, Br, CH₃, C₂H_{5,} NO₂, OCH₃, OC₂H₅, CF₃, C₂F₅ substituiert sein können oder auch eine annetierte Methandiylbisoxy, Ethan-1,2-diylbisoxygruppe tragen können, oder R⁵ und R⁵ gemeinsam mit dem Stickstoffatom einen 5-7 gliedrigen Heterocyclus bilden, der ein weiteres Sauerstoff-, Stickstoff- oder Schwefelatom enthalten kann und substituiert sein kann mit C₁₋₄-Alkyl, C₁₋₄-Alkoxy-C₀.₂-alkyl, C₁₋₄-Alkaxy-carbanyl, Aminocarbonyl oder Phenyl, bedeuten.
- ***A***: C₁₋₁₀ Alkandiyl, C₂₋₁₀ Alkendiyl, C₂₋₁₀ Alkindiyl, (C₀₋₅ Alkandiyl-C₃₋₇ Cycloalkandiyl- C₀₋₅ Alkandiyl), (C₀₋₅ Alkandiylarylen -C₀₋₅ Alkandiyl), (C₀₋₅ Alkandiyl-Heteroarylen- C₀₋₅ Alkandiyl), wobei die Aryl- und Heteraaryigruppen mit ein oder zwei Substituenten aus F, Cl, Br, CH₃, C₂H₅, NO₂, OCH₃, OC₂H₅, CF₃, C₂F₅ substituiert sein können, wobei in einem 5-gliedrigen Cycloalkylring ein Ringglied ein N oder ein O sein kann und in einem 6- oder 7-gliedrigen Cycloalkylring ein oder zwei Ringglieder N und/oder O sein können, wobei Ringstickstoffe gegebenenfalls mit C₁₋₃-Alkyl oder C₁₋₃-Alkanoyl substituiert sein können, wobei in den genannten aliphatischen Ketten ein Kohlenstoffatom oder zwei Kohlenstoffatome, gegen O, NH, NR⁴, NCOR⁴, NSO₂R⁴ ausgetauscht sein können, und wobei Alkyl- oder Cycloalkygruppen mit bis zu zwei Substituenten aus F, OH, OR⁴, OCOR⁴, =O, NH², NR⁴R⁴', NHCOR⁴, NHCOOR⁴, NHCONHR⁴, NHSO₂R⁴ SH, SR⁴ substituiert sein können,
- ***B***: Wasserstoff, OH, OCOR⁵, OCONHR⁵, OCOOR⁵, COR⁵, C(NOH)R⁵, C(NOR⁵)R^{5'}, C(NO(COR⁵))R⁵', COOH, COOR⁵, CONH₂, CONHNH₂, CONHR⁵, CONR⁵R^{5'}, CONHOH, CONHOR⁵, SO₃H, SO₂NH₂, SO₂NHR-, SO₂NR⁵R⁵', PO₃H, PO(OH)(OR⁵), PO(OR⁵)(OR⁵), PO(OH)(NHR⁵), PO(NHR⁵)(NHR^{5'}), Tetrazolyl, jeweils gebunden an ein Kohlenstoffatom der Gruppe ***A***, oder die gesamte Gruppe ***Y-A-B*** N(SO2R⁴)(SO₂R^{4'}) oder NHSO₂R⁴,
- ***X***: eine Bindung, CH₂, (CH₂)₂, CH(CH₃). (CH₂)₃. CH(CH₂CH₃), CH(CH₃)CH₂, CH₂CH(CH₃).
- ***Y***: eine Bindung, O, S, SO, SO₂, NH, NR⁴,NCOR⁴, NSO₂R⁴,
bedeuten, zur Herstellung eines Arzneimittels zur Behandlung oder Verhütung von Krankheiten, die mit einer Mikroglia-Aktivierung assoziiert sind.

Die allgemeine Formel II umfaßt neben den neuen Verbindungen der allgemeinen Formel 1 auch bekannte Verbindungen (EP 0 531 883, DE 4330959). Die erfindungsgemäßen Verbindungen der allgemeinen Formel II hemmen die Aktivierung der Mikroglia-Aktivierung. Diese Wirkung ist, auch für die bekannten Verbindungen, neu.

Bevorzugt ist die Verwendung von Verbindungen der allgemeinen Formel II wobei
- ***R¹***: eine mono- oder bicyclische Arylgruppe oder eine mono- oder bicyclische 5-10 gliedrige Heteroarylgruppe mit 1-2 Heteroatomen ausgewählt aus der Gruppe bestehend aus N, S oder O bedeutet, wobei die genannte Aryl-oder Heteroarylgruppe mit bis zu drei der folgenden Substituenten unabhängig voneinander substituiert sein kann: F, Cl, Br. XOH, XOR⁴, XOCOR⁴. XOCONHR⁴, XOCOOR⁴, XCOR⁴, XCN, XCOOH, XCOOR⁴, XCONH₂, XCONR⁴R⁴', XCONHR⁴, XCONHOH, XCONHOR⁴, XCOSR⁴, XSR⁴; NO₂, XNHR⁴_{.} XNR⁴R^{4'}, R⁴, wobei zwei Substituenten an ***R***¹, wenn sie zueinander orthoständig sind, so miteinander verknüpft sein können, daß sie gemeinsam Ntethandiylbisoxy, Ethan-1,2- diylbisoxy, Propan-1,3-diyl, Butan-1,4-diyl bilden.

Ebenfalls bevorzugt ist die Verwendung von Verbindungen der allgemeinen Formel II wobei
- ***R***²: eine mono- oder bicyclische Arylgruppe oder eine mono- oder bicyclische 5-10 gliedrige Heteroarylgruppe mit 1-2 Heteroatomen ausgewählt aus der Gruppe bestehend aus N, S oder O bedeuten, wobei die genannte Aryl-oder Heteroarylgruppe mit bis zu drei der folgenden Substituenten unabhängig voneinander substituiert sein kann: F, Cl, Br, XOH, XOR⁴, XOCOR⁴, XQCONHR⁴, XOCOOR⁴XCOR⁴. XC(NOH)A⁴, XC(NOR⁴)R⁴', XC(NO(COR⁴))R^{4'}, XCN, XCOOH, XCOOR⁴, XCONH₂, XCONR⁴R^{4'}, XCONHR⁴, XCONHOH, XCONHOR⁴, XCOSR⁴, XSR⁴, XSOR⁴, XSO₂R⁴, SO₂NH₂, SO₂NHR⁴, SO₂R⁴R⁴, NO₂, XNH₂, XNHR⁴, XNCN⁴R^{4'}, XNHSO₂R⁴, XN(SO₂R⁴(SO₂R^{4'}), XNR⁴SO₂R⁴, XNHCOR⁴, XNHCOOR⁴, XNHCONHR⁴, R⁴, wobei zwei Substituenten an R², wenn sie zueinander orthoständig sind, so iteinander verknüpft sein können, daß sie gemeinsam Methandiytbisoxy, Eihan-1,2- diylbisoxy, Propan=1,3-diyl, Butan-1,4-diyl bilden.

Ebenfalls bevorzugt ist die Verwendung von Verbindungen der allgemeinen Formel II wobei
- ***R***³: für ein oder zwei Substituenten steht, die unabhängig voneinander: Wasserstoff,F, Cl, Br, XOH, XOR⁴, XOCOR⁴, XOCONHR⁴, XOCOOR⁴, XCOR⁴, XC(NOH)R⁴, XC(NOR⁴)R^{4'}, XC(NO(COR⁴))R^{4'}, XCN, XSR⁴, XSOR⁴, XSa₂R⁴,SO₂NH₂, SO₂NHR⁴, SO₂NR₄R⁴,NO₂, XNH₂, XNHR⁴, XNR⁴R^{4'}, XNHSO₂R⁴, XNR⁴SO₂R^{4'}, XN(SO₂R⁴) (SO₂R⁴), XNHCOR⁴, XNHCOOR⁴, XNHCONHR⁴ oder R⁴ bedeuten, wobei zwei Substituenten ***R***³, wenn sie zueinander orthoständig sind, so miteinander verknüpft sein können, daß sie gemeinsam Methandiylbisoxy, Ethan-1.2-diylbisoxy, Propan-1,3-diyl. Buten-1.4- diyl bilden,

Ebenfalls bevorzugt ist die Verwendung von Verbindungen der allgemeinen Formel 11 wobei
- ***R***⁴: und ***R***^{4'} unabhängig voneinander CF₃, C₂F₅, C₁₋₄ Alkyl, C₂₋₄ Alkenyl, C₂₋₄ Alkinyl, C₃₋₆ Cycloalkyl, (C₁₋₃ Alkyl-C₃₋₆ Cycloalkyl), C₁₋₃ Alkylaryl, C₁₋₃ Alkylheteroaryl, monocyclisches Aryl oder 5-6 gliedriges Heteroaryl mit 1-2 N-, S- oder O-Atomen, wobei die Aryl- und Heteroarylgruppen mit ein oder zwei Substituenten aus der Gruppe bestehend aus F, Cl, Br, CH₃, C₂H₅, NO₂, OCH₃, OC₂H₅, CF₃, C₂F₅ substituiert sein können oder auch eine annelierte Methandiylbisoxy- oder Ethan- 1,2-diylbisoxygruppe tragen können, und weiterhin in einem 5-gliedrigen Cycloalkylring ein Ringglied ein N oder ein O sein kann und in einem 6-gliedrigen Cycloalkyläng ein oder zwei Ringglieder N und/oder O sein können, wobei Ringstickstoffe gegebenenfalls mit C₁₋₃ Alkyl oder C₁₋₃Alkanoyl substituiert sein können,

Ebenfalls bevorzugt ist die Verwendung von Verbindungen der allgemeinen Formel II wobei
- ***R***⁵: und ***R***^{5'} unabhängig voneinander C₁₋₆ Alkyl, wobei ein Kohlenstoffatom gegen O, NH, N C₁₋₃ Alkyl, N C₁₋₃ Alkanoyl ausgetauscht sein kann C₃₋₇ Cycloalkyl-C₀₋₃ Alkyl, wobei in einem 5-gliedrigen Cycloalkylring ein Ringglied ein N oder ein O sein kann und in einem 6- oder 7-gliedrigen Cycloalkylring ein oder zwei Ringglieder N und/oder O sein können, wobei Ringstickstoffe gegebenenfalls mit C₁₋₃ Alkyl oder C₁₋₃ Alkanoyl substituiert sein können, wobei der genannte C₁₋₆ Alkylteil mit einem der zuvor genannten Cycloalkyl oder auch einem 5-6 gliedrigen Heteroaromaten mit 1-2 Heteroatomen ausgewählt aus der Gruppe bestehend aus N, S oder O, substituiert sein kann, wobei alle zuvor genannten Alkyl- und CyGoalkylteile mit bis zu zwei Substituenten aus CF₃, OH, O C₁₋₃ Alkyl, und die zuvor genannten Heteroarylgruppen mit ein oder zwei Substituenten aus F, Cl, CF₃, CH₃, C₂H₅, OCH₃, OC₂H₅ substituiert sein können, oder R⁵ und R^{5'} gemeinsam mit dem Stickstoffatom einen 5-7 gliedrigen Heterocyclus bilden, der ein weiteres Sauerstoff-, Stickstoff- oder Schwefelatom enthalten kann und substituiert sein kann mit C₁₋₄-Alkyl, C₁₋₄-Alkoxy-C₀₋₂-alkyl, C₁₋₄- Alkoxy-carbonyl, Aminocarbonyl oder Phenyl, bedeutet.

Ebenfalls bevorzugt ist die Verwendung von Verbindungen der allgemeinen Formel II wobei
- ***A***: C₁₋₁₀ Alkandiyl, C₂₋₁₀ Alkendiyl, C₂₋₁₀ Alkindiyl, (C₀₋₅ Alkandiyl-C₃₋₇ Cycloalkandiyl- C₀₋₅ Alkandiyl) oder (C₀₋₅ Alkandiyl-Heteroarylen-C₀₋₅ Alkandiyl),bedeutet, wobei eine gegebenenfalls vorhandene Heteroarylgruppe mit ein oder zwei Substituenten aus F, Cl, Br, CH₃, C₂H₅, NO₂, OCH₃, OC₂H₅, CF₃, C₂F₅ substituiert sein kann, und weiterhin in einem 5-gliedrigen Cycloalkylring ein Ringglied ein N oder ein O sein kann und in einem 6- oder 7-gliedrigen Cycloalkylring ein oder zwei Ringglieder N und/oder O sein können, wobei Ringstickstoffe gegebenenfalls mit C₁₋₃ Alkyl oder C₁₋₃ Alkanoyl substituiert sein können, wobei in einer aliphatischen Kette ein Kohlenstoffatom oder zwei Kohlenstoffatome, gegen O, NH, N C₁₋₃ Alkyl, N C₁₋₃ Alkanoyl, NSO₂ C₁₋₃ Alkyl ausgetauscht sein können, und wobei Alkyl- oder Cycloalkylteile mit bis zu zwei F Atomen oder einem der Substituenten aus OH, O C₁₋₃ Alkyl, O C₁₋₃ Alkanoyl, =O, NH₂, NH C₁₋₃ Alkyl, N (C₁₋ 3 Alkyl)₂, NH C₁₋₃ Alkanoyl, N (C₁₋₃ Alkyl) (C₁₋₃ Alkanoyl), NHCOO C₁₋₃ Alkyl, NHCONH C₁₋₃ Alkyl, NHSO₂ C₁₋₃ Alkyl, SH, S C₁₋₃ Alkyl substituiert sein können,

Ebenfalls bevorzugt ist die Verwendung von Verbindungen der allgemeinen Formel II wobei
- ***B***: Wasserstoff, OH, OCOR⁵, OCONHR⁵, OCOOR⁵, COOH, COOR⁵, CONH₂, CONHR⁵, CONR⁵R⁵, CONHOH, CONHOR⁵ oder Tetrazolyl, jeweils gebunden an ein Kohlenstoffatom der Gruppe A bedeutet.

Ebenfalls bevorzugt ist die Verwendung von Verbindungen der allgemeinen Formel **II** wobei
- ***X***: eine Bindung oder CH₂ bedeutet.

Ebenfalls bevorzugt ist die Verwendung von Verbindungen der allgemeinen Formel wobei
- ***Y***: eine Bindung, O, S. NH, NR⁴,NCOR⁴ oder NSO₂R⁴ bedeutet.

In Beispiel 307 ist beschrieben, wie die Hemmung der Mikroglia-Aktivierung gemessen werden kann. Die Aktivierung der Mikroglia kann dabei durch verschiedene Stimuli erfolgen, wie z.B.Aß-Peptid (β-Amyloid, Araujo, D.M. and Cotman, C.M. Brain Res. 569, 141-145 (1992)), Prion-Protein, Zytokine oder durch Zellfragmente (Combs, C.K. et al. (1999) J. Neurosci., 19, 928-939, Wood, P.L.(1998) Neuroinflammation: Mechanisms and Management , Humana Press). Beispielsweise zeigt die Verbindung des Beispiel 49 6-[[1-(4-Methylphenyl)-2-phenyl-1*H*-benzimidazol-6-yl]oxy]hexansäureisopropylester eine Hemmung von IC₅₀ = 0,75µM.

Die Stimulierung mit dem Aß-Peptid entspricht der pathophysiologischen Situation bei der Alzheimerschen Krankheit In diesem Test zeigten bei Stimulierung mit dem Aß-Peptid die erfindungsgemäßen Substanzen eine Hemmung der Mikroglia-Aklvierung. Die Hemmung der Mikroglia-Aktivierung durch die erfindungsgemäßen Substanzen führt zu einer starken Reduktion der Cytokinproduktion und -sekretion, z.B. von II1ß und TNFa (gemessen durch ELISA und mRNA-Expressionsanalyse), und zu einer verminderten Sekretion von reaktivem Sauerstoff/Stickstoff-Intermediaten. Es werden also gleich mehrere Entzündungsfaktoren gehemmt.

Die *in vivo* Wirksamkeit der erfindungsgemäßen Substanzen wurde in einem MCAO-Modelt in Ratten gezeigt. Dieses Modell simuliert den Zustand eines Schlaganfalls. Die erfindungsgemäßen Substanzen reduzieren die Mikroglia-Aktivierung, die bei akuten Himlesionen in den Gehimen der Tiere auftritt.

Die Erfindung betrifft auch die Verwendung der erfindungsgemäßen Verbindungen der allgemeinen Formel I und der allgemeinen Formel II zur Herstellung eines Arzneimittels zur Behandlung oder Verhütung von Krankheiten, die mit einer Mikroglia-Aktiviemng assoziiert sind. Beispiele für solche Krankheiten sind AIDS-Demenz, Amyotrophe Lateralsklerose, Creuzfeld-Jacob-Krankheit, Down-Syndrom, diffuse Lewy-Körperchen-Krankheit, Chorea Huntington, Leukenzephalopathie, multiple Sklerose, Parkinsonsche Krankheit, Picksche Krankheit, Alzheimersche Krankheit, Schlaganfall, Temporal-Lappen-Epilepsie und Tumoren.

Ferner betrifft die Erfindung pharmazeutische Mittel, die eine oder mehrere erfindungsgemäße Verbindungen der allgemeinen Formel I und einen oder mehrere Trägerstoffe enthalten. Die pharmazeutischen Mittel bzw. Zusammensetzungen der Erfindung werden mit den üblichen festen oder flüssigen Trägerstoffen oder Verdünnungsmitteln und den üblichen pharmazeutischen und technischen Hilfsstoffen entsprechend der gewünschten Applikationsart mit einer geeigneten Dosierung in an sich bekannterweise hergestellt Die bevorzugten Zubereitungen bestehen in einer Darreichungsform, die zur oralen, enteralen oder parenteralen Applikation geeignet ist Solche Darreichungsformen sind beispielsweise Tabletten, Filmtabletten, Dragees, Pillen, Kapseln, Pulver oder Depotformen sowie Suppositorien. Entsprechende Tabletten können beispielsweise durch Mischen des Wirkstoffs mit bekannten Hilfsstoffen, beispielweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelatine, Gleitmittel wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielweise Polyvinylpyrrolidon oder Schellack, Gummiarabicum, Talk, Titanoxid oder Zucker, hergestellt werden. Dabei kann auch die Drageehülle aus mehreren Schichten bestehen, wobei die oben bei den Tabletten erwähnten Hilfsstoffe verwendet werden können. Wirkstoffe enthaltende Kapseln können beispielsweise hergestellt werden, indem man den Wirkstoff mit einem inerten Träger wie Milchzucker oder Sorbit mischt und in Gelatinekapseln einkapselt.
Die erfindungsgemäßen Substanzen können auch in geeigneten Lösungen wie beispielsweise physiologischer Kochsalzlösung, als Infusions- oder Injektionslösung zur Anwendung kommen.
Für die parenterale Applikation sind insbesondere ölige Lösungen, wie zum Beispiel Lösungen in Sesamöl, Rizinusöl und Baumwollsamenöl, geeignet. Zur Erhöhung der Löslichkeit können Lösungsvermittler, wie zum Beispiel Benzylabenzoat oder Benzylalkohol, zugesetzt werden.
Es ist auch möglich, die erfindungsgemäßen Substanzen in ein Transdermales System einzuarbeiten und sie damit transdermal zu applizieren.
Die Dosierung der erfindungsgemäßen Substanzen der allgemeinen Formel I und der allgemeinen Formel II wird vom behandelnden Arzt bestimmt und hängt unter anderem von der verabreichten Substanz, dem Verabreichungsweg, der zu behandelnden Erkrankung und von der Schwere der Erkrankung ab. Die tägliche Dosis beträgt nicht mehr als 1000 mg, vorzugsweise nicht mehr als 100 mg, wobei die Dosis als einmal zu verabreichende Einzeldosis oder unterteilt in 2 oder mehrere Tagesdosen gegeben werden kann.

Die Verbindungen der Formel I können als Tautomere, Stereoisomere oder geometrische Isomere vorlieben. Die Erfindung umfaßt auch alle möglichen Isomeren wie E- und Z-Isomere, S- und R-Enantiomere, Diastereomere, Razemate und Gemische derselben einschließlich der tautomeren Verbindungen.
Die Isomerengemische können nach üblichen Methoden wie beispielsweise Kristallisation, Chromatographie oder Salzbildung in die Enantiomeren bzw. EIZ-Isomeren aufgetrennt werden.
Die Herstellung der erfindungsgemäßen Verbindungen erfolgt in Analogie zu bekannten Verfahren, die beispielsweise in EP 531 883 beschrieben sind. Soweit die Herstellung der Ausgangsverbindungen nicht beschrieben wird, sind die Ausgangsverbindungen bekannt und käuflich oder deren Herstellung erfolgt analog nach den hier beschriebenen Verfahren. Nachfolgend wird die Herstellung einiger Vorstufen, Zwischenprodukte und Produkte exemplarisch beschrieben.

Bei der Herstellung der erfindungsgemäßen Substanzen bedient man sich beispielsweise folgender Verfahren:

### Allgemeine Arbeitsvorschrift 1:

### Reduktion Nitrogruppen, Hydrierung olefinischer Doppelbindungen und hydrogenolytische Spaltung von Benzylethern

Die zu reduzierende Verbindung wird in Ethylacetat, Tetrahydrofuran, Methanol oder Ethanol oder Gemischen der Lösungsmittel gelöst und an 2-5% (bezogen auf die Nitroverbindung) Palladium auf Kohle (10%) bei normalem Druck hydriert. Nach Ende der Wasserstoffaufnahme wird abgesaugt, der Rückstand mit Ethylacetat oder Methanol oder Ethanol gewaschen und das Filtrat im Vakuum eingeengt. Das Rohprodukt wird in der Regel ohne weitere Reinigung umgesetzt.

### Allgemeine Arbeitsvorschrift 2:

### Reduktion Nitrogruppen

9.2 g Eisen(II)sulfat werden in 30 ml Wasser und 9 ml Ammoniaklösung vorgelegt. Dazu wird eine Lösung aus 3.6 mmol der Nitroverbindung in 100 ml Ethanol getropft und die Suspension für 1 h bei 70°C intensiv gerührt. Anschließend läßt man absitzen, filtriert vom Feststoff ab, engt das Filtrat weitgehend ein, versetzt mit Wasser und extrahiert dreimal mit Ethylacetat. Die vereinigten Extrakte werden über Natriumsulfat getrocknet und im Vakuum eingeengt. Die Aminoverbindung wird als Rohprodukt weiterverarbeitet.

### Allgemeine Arbeitsvorschrift 3:

### Cyclisierung zu Benzimidazolen mit Orthoestern

10 mmol eines 1,2-Diaminobenzolderivats werden in 25 ml Ethanol gelöst. Dazu tropft man 47 ml einer 0.8 M etherischen HCI-Lösung, rührt für 30 min und engt dann im Vakuum zur Trockene ein. Der Rückstand wird in 230 ml Methanol aufgenommen und mit 6 ml Trimethylorthobenzoat oder der entsprechenden Menge eines anderen Orthoesters versetzt. Man erhitzt für 2-8 h zum Rückfluß, gießt nach dem Erkalten auf ges. Natriumhydrogencarbonatlösung, extrahiert dreimal mit Ethylacetat, trocknet die vereinigten Extrakte über Natriumsulfat und engt im Vakuum ein. Der Rückstand wird durch Kristallisation oder Säulenchromatographie an Kieselgel gereinigt.

### Allgemeine Arbeitsvorschrift 4:

### Cyclisierung zu Benzimidazolen mit Iminoesterhydrochloriden

1.2 mmol eines 1,2-Diaminobenzolderivats werden in 5 ml Tetrahydrofuran gelöst, mit 1.5 mmol eines Benzimidathydrochlorids versetzt und die Mischung für 15 h gerührt. Der Ansatz wird mit ges. Natriumhydrogencarbonatlösung versetzt, mit Wasser verdünnt und dreimal mit Ethylacetat extrahiert. Die vereinigten organischen Phasen werden dreimal mit 1 N wäßriger Salzsäure und einmal mit ges. Natriumchloridlösung gewaschen, über Natriumsulfat getrocknet, über eine Fritte mit Kieselgel filtriert und die Lösung zur Trockene eingeengt. Das Produkt kristallisiert aus Diisopropylether.

### Allgemeine Arbeitsvorschrift 5:

### Cyclisierung zu Benzimidazolen via Carbonsäureanilide

4.7 mmol eines 1,2-Diaminobenzolderivats werden in 20 ml Dichlormethan gelöst, mit 14 mmol Triethylamin und langsam mit 6 mmol Carbonsäurechtorid versetzt und die Mischung für 15 h gerührt. Der Ansatz wird mit ges. Natriumhydrogencarbonatlösung versetzt, mit Wasser verdünnt und zweimal mit Dichlormethan extrahiert. Die vereinigten organischen Phasen werden mit Wasser und mit ges. Natriumchloridlösung gewaschen, über Natriumsulfat getrocknet und im Vakuum eingeengt. Das zurückbleibende rohe Carbonsäureanilid wird in einem 9:1-Gemisch aus Methanol und konz. Salzsäure aufgenommen und für 1 h zum Rückfluß erhitzt. Das Reaktionsgemisch wird nach dem Erkalten langsam in ges. Natriumhydrogencarbonatlösung eingetragen, mit Wasser verdünnt und dreimal mit Dichlormethan extrahiert. Die vereinigten organischen Phasen werden mit ges. Natriumchloridlösung gewaschen, über Natriumsulfat getrocknet und im Vakuum eingeengt. Der Rückstand wird falls erforderlich durch Kristallisation oder Säulenchromatographie an Kieselgel gereinigt.

### Allgemeine Arbeitsvorschrift 6:

### Etherspaltung mit Bromwasserstoffsäure

5 g Arylmethylether werden mit 160 ml 48%iger wäßriger HBr versetzt und für 1-5 h zum Rückfluß erhitzt. Nach dem Erkalten wird filtriert. Der Rückstand wird in Ethylacetat aufgenommen, und dreimal mit ges. Natriumhydrogencarbonatlösung extrahiert. Nach Trocknung über über Natriumsulfat wird im Vakuum eingeengt. Der Rückstand wird falls erforderlich durch Kristallisation oder Säulenchromatographie an Kieselgel gereinigt.

### Allgemeine Arbeitsvorschrift 7:

### Etherspaltung mit Bortribromid

1.86 mmol Arylmethyether werden in 18 ml Dichlormethan gelöst und bei -35°C langsam mit 7.4 ml einer 1 M Lösung von Bortribromid in Dichlormethan versetzt. Man beläßt für 12-15 h bei -30°C, versetzt dann mit ges. Natriumhydrogencarbonatlösung, extrahiert dreimal mit Dichlormethan, trocknet die vereinigten Extrakte über Natriumsutfat und engt im Vakuum ein. Der Rückstand wird falls erforderlich durch Säulenchromatographie an Kieselgel gereinigt.

### Allgemeine Arbeitsvorschrift 8:

### Alkylierung von Hydroxybenzimidazolderivaten und Phenolderivaten mit Alkylhalogeniden

Eine Lösung von 1.85 mmol des Hydroxybenzimidazolderivats in 12 ml N,N Dimethylformamid wird mit 1.85 mmol Caesiumcarbonat, und 2.24 mmol Alkylbromid oder Alkyljodid versetzt. Bei Verwendung der Alkylbromide werden optional 1.85 mmol Natriumjodid zugesetzt. Man rührt für 12-96 h, gießt dann auf Wasser, nimmt mit Ethylacetat auf, wäscht die organische Phase viermal mit Wasser, trocknet die über Natriumsulfat und engt im Vakuum ein.
Alternativ zu dieser wäßrigen Aufarbeitung kann man das Reaktionsgemisch mit Dichlormethan versetzen, von den ausfallenden Salzen durch Filtration trennen und das Filtrat im Vakuum einengen.
Unabhängig von der Aufarbeitungsmethode wird der Rückstand durch Kristallisation oder Säulenchromatographie an Kieselgel gereinigt.

### Allgemeine Arbeitsvorschrift 9:

### Verseifung von Carbonsäurealkylestern

0.77 mmol des Carbonsäurealkylesters werden in 5 ml Methanol und 5 ml Tetrahydrofuran gelöst und mit 5 ml einer 0.5 N wäßrigen Lithium- oder Natriumhydroxidlösung versetzt. Nach 2-12 h Rühren wird im Vakuum weitestgehend eingeengt, durch Zusatz von wäßriger Salzsäure neutralisiert und mit Ethylacetat extrahiert. Man trocknet über Natriumsulfat und engt im Vakuum ein. Der Rückstand wird falls erforderlich durch Säulenchromatographie an Kieselgel gereinigt.

### Allgemeine Arbeitsvorschrift 10:

### Veresterung von Carbonsäuren

0.2 mmol Carbonsäure werden in 1 ml primärem oder sekundärem Alkohol gelöst, mit zwei Tropfen konz. Schwefelsäure versetzt und 12 h bei 60°C gerührt. Der Ansatz wird dann mit ges. Kaliumhydrogencarbonatlösung versetzt, mit Wasser verdünnt und dreimal mit Ethylacetat extrahiert. Nach Waschen der vereinigten Extrakte mit ges. Natriumchloridlösung und Trocknung über Natriumsulfat wird im Vakuum eingeengt und der Rückstand aus Diisopropylether kristallisiert.

### Allgemeine Arbeitsvorschrift 11:

### Reduktion von Carbonsäurealkylestern mit Lithiumaluminiumhydrid

0.15 mmol Carbonsäureester werden in Tetrahydrofuran gelöst und mit 0.09 mmol Lithiumaluminiumhydrid versetzt. Man läßt für 1-48 h rühren, versetzt mit Wasser und extrahiert dreimal mit Dichlormethan. Nach Trocknung der vereinigten organischen Phasen über Natriumsulfat engt man im Vakuum ein. Der Rückstand wird falls erforderlich durch Kristallisation oder durch Säulenchromatographie an Kieselgel gereinigt.

### Allgemeine Arbeitsvorschrift 12:

### Wittigreaktion von Benzimidazolcarbaldehyden mit (ω-Carboxy-alkyl)triphenylphosphoniumbromiden und Veresterung mit Methanol

2 mmol des (ω-Carboxyalkyl)triphenylphosphoniumbromids werden in 2.5 ml Dimethylsulfoxid und 2.5 ml Tetrahydrofuran bei 0°C mit 4 mmol Kalium-*tert*-butylat versetzt und für 30 min bei T>10°C gerührt. Anschließend setzt man eine Lösung aus 0.67 mmol des Aldehyds in 2 ml Tetrahydrofuran zu und rührt für 3 h bei 20°C. Der Ansatz wird dann mit ges. Ammoniumchloridlösung versetzt, mit Wasser verdünnt und dreimal mit Ethylacetat extrahiert. Nach Trocknung der vereinigten organischen Phasen über Natriumsulfat wird im Vakuum eingeengt. Der Rückstand wird in 15 ml Methanol gelöst, mit zwei Tropfen konz. Schwefelsäure versetzt und 48-72 h stehen gelassen. Nach Einengen i. Vak wird der Rückstand durch Säulenchromatographie an Kieselgel gereinigt.

### Allgemeine Arbeitsvorschrift 13:

**Umsetzung von Aminobenzimidazolen mit Alkyl- und Arylsulfonsäurehalogeniden** 47 µmol Aminobenzimidazolderivat werden in 0.5 ml Dichlormethan gelöst, mit 51 µmol Triethylamin und 51 µmol Alkyl- oder Arylsulfonsäurehalogenid versetzt und die Reaktionsmischung für 2-15 h gerührt. Zur Aufarbeitung gibt man ges.
Natriumhydrogencarbonatlösung zu, extrahiert dreimal mit Dichlormethan, wäscht die vereinigten organischen Phasen mit Wasser, trocknet über Natriumsulfat und engt im Vakuum ein. Der Rückstand wird durch Kristallisation oder durch Säulenchromatographie an Kieselgel gereinigt.

### Allgemeine Arbeitsvorschrift 14:

### Kupfervermittelte O- oder N-Arylierung von Benzimidazolen

5 mmol eines N-unsubstituierten Benzimidazolderivats oder eines N-arylsubstituierten Hydroxybenzimidazolderivats werden in 20 ml Dichlormethan gelöst. Man gibt 10 mmol einer Arylboronsäure, 5 mmol wasserfreies Kupfer(II)acetat, 10 mmol Pyridin oder Triethylamin und ca. 2.5 g Molekularsieb (4) zu, rührt für 48-72 h unter Feuchtigkeitsausschluß, setzt dann Kieselgel zu, engt im Vakuum zur Trockene ein und reinigt das zurückbleibende Pulver durch Chromatographie an Kieselgel. Regioisomere N-Arylierungsprodukte werden falls erforderlich mittels HPLC getrennt.

### Allgemeine Arbeitsvorschrift 15:

### Basenkatalysierte N-Substitution von Benzimidazolen

5 mmol eines N-unsubstituierten Benzimidazolderivats werden in 20 ml Dimethylacetamid gelöst. Man gibt 25 mmol Natriumhydrid und 20 mmol eines elektronenarmen Aryl-oder Heteroaryl-Halogenids zu und errhitzt für 48-72 h unter Feuchtigkeitsausschluß zum Rückfluss, setzt dann Kieselgel zu, engt im Vakuum zur Trockene ein und reinigt das zurückbleibende Pulver durch Chromatographie an Kieselgel. Regioisomere N-Arylierungsprodukte werden falls erforderlich mittels HPLC getrennt.

### Allgemeine Arbeitsvorschrift 16:

### Cyclisierung zu Benzimidazolen mit Aldehyden

1 mmol eines 1,2-Diaminobenzolderivats werden in 3 ml Nitrobenzol gelöst. Dazu gibt man 1 mmol eines Aryl- bzw. Heteroarylaldehyds. Man erhitzt für 2-6 h auf 150 °C und lässt erkalten. Der Rückstand wird ohne weitere Aufarbeitung direkt durch Säulenchromatographie an Kieselgel gereinigt.

### Allgemeine Arbeitsvorschrift 17:

### Überführung von Carbonsäuren in Carbonsäureamide

0.25 mmol einer Carbonsäure werden in 3 ml *N,N*-Dimethylformamid gelöst, mit 0.38 mmol eines primären oder sekundären Amins, 0.5 mmol Triethylamin und 0.25 mmol Diphenylphosphorylazid versetzt und die Mischung für 2 d gerührt. Zur Aufarbeitung gibt man Wasser zu, extrahiert dreimal mit Ethylacetat, wäscht die vereinigten organischen Phasen mit Wasser, trocknet über Natriumsulfat und engt im Vakuum ein. Der Rückstand wird durch Säulenchromatographie an Kieselgel gereinigt.

### Allgemeine Arbeitsvorschrift 18:

### Überführung von Carbonsäureestern in Carbonsäureamide

0.36 mmol eines Amins werden in 3 ml Toluol gelöst und unter Kühlung im Eisbad tropfenweise mit 0.18 ml einer 2 M Lösung von Trimethyaluminium in Toluol versetzt. Man versetzt mit einer Lösung aus 0.33 mmol des Carbonsäuremethylesters in 3 ml Toluol und rührt 2-8 h bei 95°C. Zur Aufarbeitung gibt man nach dem Erkalten Wasser zu, extrahiert dreimal mit Ethylacetat, wäscht die vereinigten organischen Phasen mit ges.

Natriumchloridlösung, trocknet über Natriumsulfat und engt im Vakuum ein. Der Rückstand wird durch Säulenchromatographie an Kieselgel gereinigt.

### Beispiel 1

### [(1,2-Diphenyl-1H-benzimidazol-6-yl)oxy]essigsäureisopropylester

wurde durch Umsetzung von 1,2-Diphenyl-6-hydroxy-1*H*-benzimidazol (Benincori, T.; Sannicolo, F.; J.Heterocycl.Chem.; 25; 1988; 1029-1033) mit Bromessigsäureisopropylester gemäß der allgemeinen Arbeitsvorschrift 8 erhalten. Fp. 137-138°C

### Beispiel 2

### [(1,2-Diphenyl-1H-benzimidazol-6-yl)oxy]ethan-1-ol

wurde wurde durch Umsetzung von [(1,2-Diphenyl-1*H*-benzimidazol-6-yl)oxy]essig-säuremethylester (DE 4330959) gemäß der allgemeinen Arbeitsvorschrift 11 erhalten. ¹H-NMR (D₆-DMSO): δ = 3.72 ppm t (J = 7.5 Hz, 2H); 4.02 t (J = 7.5 Hz, 2H); 6.72 d (J = 2 Hz, 1H); 7.10 dd (J = 8, 2 Hz, 1H); 7.38-7.68 m (10H); 7.76 d (J = 8 Hz, 1H).

### Beispiel 3

### 3-[(1,2-Diphenyl-1H-benzimidazol-6-yl)oxy]propan-1-ol

0.5 g 1,2-Diphenyl-6-hydroxy-1*H*-benzimidazol wurden gemäß der allgemeinen Arbeitsvorschrift 8 mit 3-(Brompropoxy)-*tert*-butyldimethylsilan umgesetzt. Nach Chromatographie an Kieselgel nahm man in 2.5 ml Methanol auf, setzte 0.4 ml konz. Salzsäure zu und ließ für 2 h bei 20°C rühren. Man goss auf ges. wässrige Natriumhydrogencarbonatlösung, extrahierte dreimal mit Ethylacetat, wusch die vereinigten Extrakte mit ges. wäßriger Natriumchloridlösung, trocknete über Natriumsulfat und engte im Vakuum ein.
Fp. 191-193°C

### Beispiel 4

### 3-[(1,2-Diphenyl-1H-benzimidazol-6-yl)oxy]propansäure

100 mg 3-[(1,2-Diphenyl-1*H*-benzimidazol-6-yl)oxy]propan-1-ol wurden in 2.5 ml Aceton vorgelegt und bei -15°C mit 0.15 ml einer Lösung von Jones-Reagenz (hergestellt aus 0.27 g Chrom(VI)oxid, 1 ml Wasser und 0.23 ml konz. Schwefelsäure) versetzt. Nach 3.5 h Rühren bei -15°C wurde durch Zusatz von Isopropanol gequencht. Man verdünnte mit Wasser, extrahierte dreimal mit Dichlormethan, trocknete die vereinigten organischen Phasen über Natriumsulfat und engte im Vakuum ein. Der Rückstand wurde durch Chromatographie an Kieselgel gereinigt. ¹H-NMR (D₆-DMSO): δ = 2.60 ppm t (J = 7.5 Hz, 2H); 4.15 t (J = 7.5 Hz, 2H); 6.64 d (J = 2 Hz, 1H); 6.95 dd (J = 8, 2 Hz, 1H); 7.30-7.61 m (10H); 7.69 d (J = 8 Hz, 1 H).

### Beispiel 5

### 3-[(1,2-Diphenyl-1H-benzimidazol-6-yl)oxy]propansäuremethylester

45 mg 3-[(1,2-Diphenyl-1*H*-benzimidazol-6-yl)oxy]propansäure wurden in 0.5 ml *N,N-*Dimethylformamid gelöst und mit 41 mg Caesiumcarbonat und 10 µl Methyljodid versetzt. Man ließ 2 d rühren, verdünnte mit Dichlormethan, filtrierte, engte das Filtrat im Vakuum ein und chromatographierte den Rückstand an Kieselgel.
Fp. 120-121 °C

### Beispiel 6

### 2-[(1,2-Diphenyl-1H-benzimidazol-6-yl)oxy]propansäuremethylester

wurde durch Umsetzung von 1,2-Diphenyl-6-hydroxy-1*H*-benzimidazol mit 2-Brompropansäuremethylester gemäß der allgemeinen Arbeitsvorschrift 8 erhalten.
Fp. 132-135°C

### Beispiel 7

### 4-[(1,2-Diphenyl-1H-benzimidazol-6-yl)oxy]butansäureisopropylester

wurde durch Umsetzung von 1,2-Diphenyl-6-hydroxy-1*H*-benzimidazol mit 4-Brombutansäureisopropylester gemäß der allgemeinen Arbeitsvorschrift 8 erhalten.
Fp. 89-91 °C

### Beispiel 8

### 4-[(1,2-Diphenyl-1H-benzimidazol-6-yl)oxy]butan-1-ol

wurde durch Umsetzung von 4-[(1,2-Diphenyl-1*H*-benzimidazol-6-yl)oxy]butan-säuremethylester gemäß der allgemeinen Arbeitsvorschrift 11 erhalten.
Fp. 159-160°C

### Beispiel 9

### Essigsäure-[4-[(1,2-Diphenyl-1H-benzimidazol-6-yl)oxy]but-1-yl]ester

50 mg 4-[(1,2-Diphenyl-1*H*-benzimidazol-6-yl)oxy]butan-1-ol wurden in 1 ml Dichlormethan gelöst, mit 0.34 ml Pyridin und 20 µl Acetylchlorid versetzt und 15 h gerührt. Man versetzte mit ges. Natriumhydrogencarbonatlösung, verdünnte mit Wasser, extrahierte zweimal mit Dichlormethan, wusch die vereinigten organischen Phasen mit ges. Natriumchloridlösung, trocknete über Natriumsulfat und engte im Vakuum ein. Der Rückstand wurde mittels Dickschichtchromatograpie gereinigt.
Fp. 68-69°C

### Beispiel 10

### Pivalinsäure-[4-[(1,2-Diphenyl-1H-benzimidazol-6-yl)oxy]but-1-yl]-ester

wurde analog zu der in Beispiel 9 angegebenen Vorschrift aus 50 mg 4-[(1,2-Diphenyl-1*H-*benzimidazol-6-yl)oxy]butan-1-ol, 0.34 ml Pyridin und 22 µl Trimethylessigsäurechlorid dargestellt.
Fp. 104-106°C

### Beispiel 11

### 4-[(1,2-Diphenyl-1H-benzimidazol-6-yl)oxy]butyl-N-methylcarbamat

100 mg 4-[(1,2-Diphenyl-1*H*-benzimidazol-6-yl)oxy]butan-1-ol wurden in 4 ml Dichlormethan gelöst, mit 0.1 ml Triethylamin und 20 mg Methylisocyanat versetzt und 15 h gerührt. Man gab weitere 0.1 ml Triethylamin und 20 mg Methylisocyanat zu, ließ für 20 h rühren und engte dann im Vakuum ein. Der Rückstand wurde mittels Chromatographie an Kieselgel gereinigt.
Fp. 124-126°C

### Beispiel 12

### 5-[(1,2-Diphenyl-1H-benzimidazol-6-yl)oxy]pentansäureisopropylester 994

wurde durch Umsetzung von 1,2-Diphenyl-6-hydroxy-1*H*-benzimidazol mit 5-Brompentansäureisopropylester gemäß der allgemeinen Arbeitsvorschrift 8 erhalten.
Fp. 91-92°C

### Beispiel 13

### 6-[(1,2-Diphenyl-1H-benzimidazol-6-yl)oxy]hexansäuremethylester

wurde durch Umsetzung von 1,2-Diphenyl-6-hydroxy-1*H*-benzimidazol mit 6-Bromhexansäuremethylester gemäß der allgemeinen Arbeitsvorschrift 8 erhalten.
¹H-NMR (CDCl₃): δ = 1.44-1.56 m (2H); 1.64-1.85 m (4H); 2.33 t (J = 7.5 Hz, 2H); 3.66 s (3H); 3.93 t (J = 7.5 Hz, 2H); 6.70 d (J = 2 Hz, 1H); 6.96 dd (J = 8, 2 Hz, 1 H); 7.22-7.38 m (5H); 7.43-7.58 m (5H); 7.76 d (J = 8 Hz, 1 H).

### Beispiel 14

### 6-[(1,2-Diphenyl-1H-benzimidazol-6-yl)oxy]hexansäureisopropylester

wurde durch Umsetzung von 1,2-Diphenyl-6-hydroxy-1*H*-benzimidazol mit 6-Bromhexansäureisopropylester gemäß der allgemeinen Arbeitsvorschrift 8 erhalten.
¹H-NMR (CDCl₃): δ = 1.22 ppm d (J = 7.5 Hz , 6H); 1.43-1.56 m (2H); 1.62-1.87 m (4H); 2.30 t (J = 7.5 Hz, 2H); 3.93 t (J = 7.5 Hz, 2H); 5.01 sp (J = 7.5 Hz, 1H); 6.69 d (J = 2 Hz, 1 H); 6.96 dd (J = 8, 2 Hz, 1 H); 7.25-7.38 m (5H); 7.43-7.55 m (5H); 7.75 d (J = 8 Hz, 1H).

### Beispiel 15

### 6-[(1,2-Diphenyl-1H-benzimidazol-6-yl)oxy]hexansäure

wurde durch Umsetzung von 6-[(1,2-Diphenyl-1*H*-benzimidazol-6-yl)oxy]hexansäuremethylester gemäß der allgemeinen Arbeitsvorschrift 9 erhalten.
¹H-NMR (D₆-DMSO): δ = 1.35-1.49 ppm m (2H); 1.50-1.63 m (2H); 1.65-1.77 m (2H); 2.23 t (J = 7.5 Hz, 2H); 3.92 t (J = 7.5 Hz, 2H); 6.62 d (J = 2 Hz, 1H); 6.95 dd (J = 10, 2 Hz, 1H); 7.30-7.62 m (10H); 7.68 d (J = 10 Hz, 1H).

### Beispiel 16

### 6-[(1,2-Diphenyl-1H-benzimidazol-6-yl)oxy]hexan-1-ol

wurde durch Umsetzung von 6-[(1,2-Diphenyl-1*H*-benzimidazol-6-yl)oxy]hexansäuremethylester gemäß der allgemeinen Arbeitsvorschrift 11 erhalten.
¹H-NMR (CDCl₃): δ = 1.35-1.85 ppm m (8H); 3.67 t (J = 7.5 Hz, 2H); 3.98 t (J = 7.5 Hz, 2H); 6.70 d (J = 2 Hz, 1H); 6.98 dd (J = 8, 2 Hz, 1 H); 7.24-7.38 m (5H); 7.45-7.58 m (5H); 7.75 d (J = 8 Hz, 1H).

### Beispiel 17

### 6-[(1,2-Diphenyl-1H-benzimidazol-6-yl)oxy]hexanamid

### a) 6-[(1,2-Diphenyl-1H-benzimidazol-6-yl)oxy]hexannitril

wurde durch Umsetzung von 1,2-Diphenyl-6-hydroxy-1*H*-benzimidazol mit 6-Bromhexannitril gemäß der allgemeinen Arbeitsvorschrift 8 erhalten.
Fp. 108-112°C

### 6-[(1,2-Diphenyl-1H-benzimidazol-6-yl)oxy]hexanamid

Zu einer Lösung aus 50 mg 6-[(1,2-Diphenyl-1*H*-benzimidazol-6-yl)oxy]hexannitril in 1 ml Methanol gab man 18 mg Kaliumcarbonat und 40 µl 30%ige Wasserstoffsuperoxidlösung und ließ für 24 h rühren. Man rührte dann eiskalte wässrige Natriumthiosulfatlösung ein und extrahierte dreimal mit Ethylacetat. Nach Trocknen über Natriumsulfat wurde im Vakuum eingeengt und der Rückstand an Kieselgel chromatographiert.
¹H-NMR (CDCl₃): δ = 1.48-1.60 ppm m (2H); 1.65-1.87 m (4H); 2.25 t (J = 7.5 Hz, 2H); 3.94 t (J = 7.5 Hz, 2H); 5.30-5.53 breit (2H); 6.68 d (J = 2 Hz, 1H); 6.95 dd (J = 8, 2 Hz, 1 H); 7.23-7.38 m (5H); 7.42-7.58 m (5H); 7.75 d (J = 8 Hz, 1H).

### Beispiel 18

### N-Methoxy-6-[(1,2-diphenyl-1H-benzimidazol-6-yl)oxy]hexanamid

100 mg 6-[(1,2-Diphenyl-1*H*-benzimidazol-6-yl)oxy]hexansäure wurden in 2 ml Tetrahydrofuran gelöst, mit 39 mg Carbonyldiimidazol versetzt, für 30 min bei 20°C gerührt und dann für 30 min zum Rückfluß erhitzt. Bei 20°C gab man dann 21 mg O-Methylhydroxylaminhydrochlorid zu und ließ für 18 h rühren. Das Reaktionsgemisch wurde mit Ethylacetat verdünnt und mit 2 N wäßriger Salzsäure und ges.
Kaliumhydrogencarbonatlösung gewaschen. Nach Trocknung über Natriumsulfat wurde im Vakuum eingeengt und der Rückstand an Kieselgel chromatographiert.
Fp. 144-145°C

### Beispiel 19

### N-(Phenylmethoxy)-6-[(1,2-diphenyl-1H-benzimidazol-6-yl)oxy]hexanamid

wurde durch Umsetzung von 6-[(1,2-Diphenyl-1*H*-benzimidazol-6-yl)oxy]hexansäure mit O-Benzylhydroxylaminhydrochlorid gemäß der in Beispiel 18 angegebenen Arbeitsvorschrift erhalten.
Fp. 144°C

### Beispiel 20

### N-Hydroxy-6-[(1,2-diphenyl-1H-benzimidazol-6-yl)oxy]hexanamid

23 mg *N*-(Phenylmethoxy)-6-[(1,2-diphenyl-1*H*-benzimidazol-6-yl)oxy]hexanamid wurden in 4 ml Ethanol gelöst, mit 15 mg Palladium auf Kohle (10%) versetzt und unter einer Wasserstoffatmosphäre für 3 h gerührt. Nach Abtrennung vom Katalysator wurde im Vakuum eingeengt und der Rückstand aus Diethylether kristallisiert.
Fp. 83-85°C

### Beispiel 21

### 7-[(1,2-Diphenyl-1H-benzimidazol-6-yl)oxy]heptansäuremethylester

wurde durch Umsetzung von 1,2-Diphenyl-6-hydroxy-1*H*-benzimidazol mit 7-Bromheptansäuremethylester gemäß der allgemeinen Arbeitsvorschrift 8 erhalten.
Fp. 77-80°C

### Beispiel 22

### 7-[(1,2-Diphenyl-1H-benzimidazol-6-yl)oxy]heptansäure

wurde durch Umsetzung von 7-[(1,2-Diphenyl-1*H*-benzimidazol-6-yl)oxy]hep-tansäuremethylester gemäß der allgemeinen Arbeitsvorschrift 9 erhalten.
Fp. 142-145°C

### Beispiel 23

### 7-[(1,2-Diphenyl-1H-benzimidazol-6-yl)oxy]heptansäureisopropylester

wurde durch Umsetzung von 7-[(1,2-Diphenyl-1*H*-benzimidazol-6-yl)oxy]heptansäure mit Isopropanol gemäß der allgemeinen Arbeitsvorschrift 10 erhalten.
Fp. 98-100°C

### Beispiel 24

### 6-[[1-(3-Nitrophenyl)-2-phenyl-1H-benzimidazol-6-yl]oxy]hexansäureisopropylester

wurde durch Umsetzung von 6-Hydroxy-1-(3-nitrophenyl)-2-phenyl-1*H*-benzimidazol (DE 4330959) mit 6-Bromhexansäureisopropylester gemäß der allgemeinen Arbeitsvorschrift 8 erhalten.
Fp. 111-113°C

### Beispiel 25

### 6-[[2-Phenyl-1-[3-(trifluormethyl)phenyl]-1H-benzimidazol-6-yl]oxy]hexansäure-methylester

### a) (5-Methoxy-2-nitrophenyl)[(3-trifluormethyl)phenyl]amin

2 g 3-Fluor-4-nitroanisol und 16 ml 3-(Trifluormethyl)anilin wurden für 72 h bei 140°C gerührt. Der Ansatz wurde anschließend mit Ethylacetat verdünnt, zehnmal mit 4 N wäßriger Salzsäure und einmal mit ges. Natriumchloridlösung gewaschen, über Natriumsulfat getrocknet, im Vakuum eingeengt und der Rückstand an Kieselgel chromatographiert.
¹H-NMR (CDCl₃): δ = 3.78 ppm s (3H); 6.42 dd (J = 8, 2 Hz, 1 H); 6.60 d (J = 2 Hz, 1 H); 7.45-7.60 m (4H); 8.22 d (J = 8 Hz, 1H); 9.78 s (breit)(1H).

### b) 6-Methoxy-2-phenyl-1-[(3-trifluormetyl)phenyl]-1H-benzimidazol

wurde durch Hydrierung von (5-Methoxy-2-nitrophenyl)[(3-trifluormethyl)phenyl]amin gemäß der allgemeinen Arbeitsvorschrift 1 und anschließende Cyclisierung mit Triethylorthobenzoat gemäß der allgemeinen Arbeitsvorschrift 3 erhalten.
Fp. 135-137°C

### c) 6-Hydroxy-2-phenyl-1-[(3-trifluormetyl)phenyl]-1H-benzimidazol

wurde durch Umsetzung von 6-Methoxy-2-phenyl-1-[(3-trifluormethyl)phenyl]-1*H-*benzimidazol gemäß der allgemeinen Arbeitsvorschrift 6 erhalten.
¹H-NMR (D₆-DMSO): δ = 6.56 ppm d (J = 2 Hz, 1 H); 6.82 dd (J = 8, 2 Hz, 1 H); 7.32-7.50 m (5H); 7.60 d (J = 8 Hz, 1H); 7.70-7.95 m (4H); 9.48 s (breit)(1H).

### 6-[[2-Phenyl-1-[3-(trifluormethyl)phenyl]-1H-benzimidazol-6-yl]oxy]hexansäure-methylester

wurde durch Umsetzung von 6-Hydroxy-2-phenyl-1-[(3-trifluormethyl)phenyl]-1*H-*benzimidazol mit 6-Bromhexansäuremethylester gemäß der allgemeinen Arbeitsvorschrift 8 erhalten.
Fp. 106-108°C

### Beispiel 26

### 6-[[2-Phenyl-1-[3-(trifluormethyl)phenyl]-1H-benzimidazol-6-yl]oxy]hexansäure-isopropylester

wurde durch Umsetzung von 6-Hydroxy-2-phenyl-1-[(3-trifluomethyl)phenyl]-1*H-*benzimidazol mit 6-Bromhexansäureisopropylester gemäß der allgemeinen Arbeitsvorschrift 8 erhalten.
Fp. 113-115°C

### Beispiel 27

### 6-[[2-Phenyl-1-[3-(trifluormethyl)phenyl]-1H-benzimidazol-6-yl]oxy]hexansäure wurde

durch Umsetzung von 6-[[2-Phenyl-1-[3-(trifluormethyl)phenyl]-1*H*-benzimidazol-6-yl]oxy]hexansäuremethylester gemäß der allgemeinen Arbeitsvorschrift 9 erhalten.
Fp. 156-158°C

### Beispiel 28

### 6-[[2-Phenyl-1-[3-(trifluormethyl)phenyl]-1H-benzimidazol-6-yl]oxy]hexan-1-ol

wurde durch Umsetzung von 6-[[2-Phenyl-1-[3-(trifluormethyl)phenyl]-1*H*-benzimidazol-6-yl]oxy]hexansäuremethylester gemäß der allgemeinen Arbeitsvorschrift 11 erhalten.
Fp. 143-145°C

### Beispiel 29

### 6-[[1-(3-Cyanophenyl)-2-phenyl-1H-benzimidazol-6-yl]oxy]hexansäuremethylestera)

### a) 3-(5-Methoxy-2-nitrophenyl)aminobenzonitril

2 g 3-Fluor-4-nitroanisol und 15 ml 3-Aminobenzonitril wurden für 65 h bei 140°C gerührt. Der Ansatz wurde anschließend mit Ethylacetat verdünnt, dreimal mit Wasser und einmal mit ges. Natriumchloridlösung gewaschen, über Natriumsulfat getrocknet, im Vakuum eingeengt und der Rückstzand an Kieselgel chromatographiert. Fp. 157-158°C

### b) 6-Methoxy1-(3-cyanophenyl)-2-phenyl-1H-benzimidazol

wurde durch Hydrierung von 3-(5-Methoxy-2-nitrophenyl)aminobenzonitril gemäß der allgemeinen Arbeitsvorschrift 1 und anschließende Cyclisierung mit Triethylorthobenzoat gemäß der allgemeinen Arbeitsvorschrift 3 erhalten. Bei der Cyclisierung wurde abweichend von der allgemeinen Arbeitsvorschrift Tetrahydrofuran als Lösungsmittel verwendet.
Fp. 185-191°C (Zers.)

### c) 6-Hydroxy-1-(3-cyanophenyl)-2-phenyl-1H-benzimidazol

wurde durch Umsetzung von 6-Methoxy1-(3-cyanophenyl)-2-phenyl-1*H*-benzimidazol gemäß der allgemeinen Arbeitsvorschrift 7 verhalten.
Fp. 216-218°C

### 6-[[1-(3-Cyanophenyl)-2-phenyl-1H-benzimidazol-6-yl]oxy]hexansäuremethylester

wurde durch Umsetzung von 6-Hydroxy-1-(3-cyanophenyl)-2-phenyl-1*H*-benzimidazol mit 6-Bromhexansäuremethylester gemäß der allgemeinen Arbeitsvorschrift 8 erhalten.
Fp. 115-118°C

### Beispiel 30

### 6-[[1-(3-Cyanophenyl)-2-phenyl-1H-benzimidazol-6-ylloxy]hexansäureisopropylester

wurde durch Umsetzung von 6-Hydroxy-1-(3-cyanophenyl)-2-phenyl-1*H*-benzimidazol mit 6-Bromhexansäureisopropylester gemäß der allgemeinen Arbeitsvorschrift 8 erhalten.
Fp. 101-102°C

### Beispiel 31

### 6-[[1-(3-Cyanophenyl)-2-phenyl-1H-benzimidazol-6-yl]oxy]hexansäure

wurde durch Umsetzung von 6-[[1-(3-Cyanophenyl)-2-phenyl-1*H*-benzimidazol-6-yl]oxy]hexansäuremethylester gemäß der allgemeinen Arbeitsvorschrift 9 erhalten.
Fp. 99-101°C

### Beispiel 32

### 6-[[1-(4-Cyanophenyl)-2-phenyl-1H-benzimidazol-6-yl]oxy]hexansäuremethylester

### a) 4-(5-Methoxy-2-nitrophenyl)aminobenzonitril

2 g 3-Fluor-4-nitroanisol und 15 ml 4-Aminobenzonitril wurden für 22 h bei 140°C gerührt. Der Ansatz wurde anschließend mit Ethylacetat verdünnt, dreimal mit 2 N wäßriger Salzsäure, dreimal mit Wasser und einmal mit ges. Natriumchloridlösung gewaschen, über Natriumsulfat getrocknet, im Vakuum eingeengt und der Rückstand an Kieselgel chromatographiert.
¹H-NMR (CDCl₃): δ = 3.70 ppm s (3H); 6.38 dd (J = 8, 2 Hz, 1 H); 6.68 d (J = 2 Hz, 1 H); 7.27 d (J = 8 Hz, 2H); 7.54 d (J = 8 Hz, 2H); 8.08 d (J = 8 Hz, 1H); 9.60 s (breit)(1H).

### b) 6-Methoxy1-(4-cyanophenyl)-2-phenyl-1H-benzimidazol

wurde durch Hydrierung von 4-(5-Methoxy-2-nitrophenyl)aminobenzonitril gemäß der allgemeinen Arbeitsvorschrift 1 und anschließende Cyclisierung mit Triethylorthobenzoat gemäß der allgemeinen Arbeitsvorschrift 3 erhalten. Bei der Cyclisierung wurde abweichend von der allgemeinen Arbeitsvorschrift Tetrahydrofuran als Lösungsmittel verwendet.
¹H-NMR (CDCl₃): δ = 3.82 ppm s (3H); 6.72 d (J = 2 Hz, 1 H); 7.00 dd (J = 8, 2 Hz, 1H); 7.30-7.49 m (7H); 7.78 d (J = 8 Hz, 1 H); 7.81 d (J = 8 Hz, 2H).

### c) 6-Hydroxy-1-(4-cyanophenyl)-2-phenyl-1H-benzimidazol

wurde durch Umsetzung von 6-Methoxy-1-(4-cyanophenyl)-2-phenyl-1*H*-benzimidazol gemäß der allgemeinen Arbeitsvorschrift 7 erhalten.
Fp. 266-268°C

### 6-[[1-(4-Cyanophenyl)-2-phenyl-1H-benzimidazol-6-yl]oxy]hexansäuremethylester

wurde durch Umsetzung von 6-Hydroxy-1-(4-cyanophenyl)-2-phenyl-1*H*-benzimidazol mit 6-Bromhexansäuremethylester gemäß der allgemeinen Arbeitsvorschrift 8 erhalten.
Fp. 145-148°C

### Beispiel 33

### 6-[[1-(4-Cyanophenyl)-2-phenyl-1H-benzimidazol-6-yl]oxy]hexansäureisopropylester

wurde durch Umsetzung von 6-Hydroxy-1-(4-cyanophenyl)-2-phenyl-1*H*-benzimidazol mit 6-Bromhexansäureisopropylester gemäß der allgemeinen Arbeitsvorschrift 8 erhalten.
Fp. 102-103°C

### Beispiel 34

### 6-[[1-(3-Chlorphenyl)-2-phenyl-1H-benzimidazol-6-yl]oxy]hexansäuremethylester

### a) 1-(3-Chlorphenyl)-6-methoxy-2-phenyl-1H-benzimidazol

wurde durch Reduktion von (3-Chlorphenyl)-(5-methoxy-2-nitrophenyl)amin (Belton, Mc Inemey; Proc.R.Ir.Acad.Sect.B; 69; 1970; 21,27) gemäß der allgemeinen Arbeitsvorschrift 2 und anschließende Cyclisierung mit Triethylorthobenzoat gemäß der allgemeinen Arbeitsvorschrift 3 erhalten.
Fp. 140-143°C

### b) 1-(3-Chlorphenyl)-6-hydroxy-2-phenyl-1H-benzimidazol

wurde durch Umsetzung von 6-Methoxy-1-(3-chlorphenyl)-2-phenyl-1*H*-benzimidazol gemäß der allgemeinen Arbeitsvorschrift 6 erhalten.
Fp. 210-214°C

### 6-[[1-(3-Chlorphenyl)-2-phenyl-1H-benzimidazol-6-yl]oxy]hexansäuremethylester

wurde durch Umsetzung von 1-(3-Chlorphenyl)-6-hydroxy-2-phenyl-1*H*-benzimidazol mit 6-Bromhexansäuremethylester gemäß der allgemeinen Arbeitsvorschrift 8 erhalten.
Fp. 101-105°C

### Beispiel 35

### 6-[[1-(3-Chlorphenyl)-2-phenyl-1H-benzimidazol-6-yl]oxy]hexansäureisopropylester

wurde durch Umsetzung von 1-(3-Chlorphenyl)-6-hydroxy-2-phenyl-1*H*-benzimidazol mit 6-Bromhexansäureisopropylester gemäß der allgemeinen Arbeitsvorschrift 8 erhalten.
Fp. 107-112°C

### Beispiel 36

### 6-[[1-(3-Chlorphenyl)-2-phenyl-1H-benzimidazol-6-yl]oxylhexansäure

wurde durch Umsetzung von 6-[[1-(3-Chlorphenyl)-2-phenyl-1*H*-benzimidazol-6-yl]oxy]hexansäuremethylester gemäß der allgemeinen Arbeitsvorschrift 9 erhalten. ¹H-NMR (D₆-DMSO): δ = 1.36-1.78 ppm m (6H); 2.24 t (J = 7.5 Hz, 2H); 3.96 t (J = 7.5 Hz, 2H); 6.68 d (J = 2 Hz, 1 H); 6.97 dd (J = 8, 2 Hz, 1 H); 7.32-7.65 m (9H); 7.69 d (J = 8 Hz, 1H).

### Beispiel 37

**6-[[1-(3-Chlorphenyl)-2-phenyl-1*H*-benzimidazol-6-yl]oxy]hexan-1-ol**

wurde durch Umsetzung von 6-[[1-(3-Chlorphenyl)-2-phenyl-1*H*-benzimidazol-6-yl]oxy]hexansäuremethylester gemäß der allgemeinen Arbeitsvorschrift 11 erhalten. ¹H-NMR (CDCl₃): δ = 1.38-1.88 ppm m (8H); 3.67 t (J = 7.5 Hz, 2H); 3.96 t (J = 7.5 Hz, 2H); 6.70 d (J = 2 Hz, 1 H); 6.97 dd (J = 8, 2 Hz, 1 H); 7.18 ddd (J = 8, 2, 2 Hz, 1 H); 7.25-7.55 m (8H); 7.76 d (J = 8 Hz, 1 H).

### Beispiel 38

### 6-[[1-(4-Chlorphenyl)-2-phenyl-1H-benzimidazol-6-yl]oxy]hexansäuremethylester

### a) 1-(4-Chlorphenyl)-6-methoxy-2-phenyl-1H-benzimidazol

wurde durch Reduktion von (4-Chlorphenyl)-(5-methoxy-2-nitrophenyl)amin (Kottenhahn et al.; J.Org.Chem.; 28; 1963; 3114,3118) gemäß der allgemeinen Arbeitsvorschrift 2 und anschließende Cyclisierung mit Triethylorthobenzoat gemäß der allgemeinen Arbeitsvorschrift 3 erhalten.
¹H-NMR (CDCl₃): δ = 3.82 ppm s (3H); 6.67 d (J = 2 Hz, 1H); 6.97 dd (J = 8, 2 Hz, 1 H); 7.22-7.40 m (5H); 7.46-7.55 m (4H); 7.77 d (J = 8 Hz, 1H).

### b) 1-(4-Chlorphenyl)-6-hydroxy-2-phenyl-1H-benzimidazol

wurde durch Umsetzung von 6-Methoxy-1-(4-chlorphenyl)-2-phenyl-1*H*-benzimidazol gemäß der allgemeinen Arbeitsvorschrift 6 erhalten.
¹H-NMR (D₆-DMSO): δ = 6.60 ppm d (J = 2 Hz, 1 H); 6.87 dd (J = 8, 2 Hz, 1 H); 7.40-7.56 m (7H); 7.64 d (J = 8 Hz, 1H); 7.70 d (J = 8 Hz, 2H); 9.50 s (breit)(1H).

### 6-[[1-(4-Chlorphenyl)-2-phenyl-1H-benzimidazol-6-yl]oxy]hexansäuremethylester

wurde durch Umsetzung von 1-(4-Chlorphenyl)-6-hydroxy-2-phenyl-1*H*-benzimidazol mit 6-Bromhexansäuremethylester gemäß der allgemeinen Arbeitsvorschrift 8 erhalten.
Fp. 100-104°C

### Beispiel 39

### 6-[[1-(4-Chlorphenyl)-2-phenyl-1H-benzimidazol-6-yl]oxy]hexansäureisopropylester

wurde durch Umsetzung von 1-(4-Chlorphenyl)-6-hydroxy-2-phenyl-1*H*-benzimidazol mit 6-Bromhexansäureisopropylester gemäß der allgemeinen Arbeitsvorschrift 8 erhalten.
Fp. 83-88°C

### Beispiel 40

### 6-[[1-(4-Chlorphenyl)-2-phenyl-1H-benzimidazol-6-yl]oxy]hexansäure

wurde durch Umsetzung von 6-[[1-(4-Chlorphenyl)-2-phenyl-1*H*-benzimidazol-6-yl]oxy]hexansäuremethylester gemäß der allgemeinen Arbeitsvorschrift 9 erhalten. ¹H-NMR (D₆-DMSO): δ = 1.35-1.78 ppm m (6H); 2.25 t (J = 7.5 Hz, 2H); 3.94 t (J = 7.5 Hz, 2H); 6.68 d (J = 2 Hz, 1H); 6.95 dd (J = 8, 2 Hz, 1H); 7.33-7.54 m (7H); 7.63 d (J = 8 Hz, 2 H); 7.69 d (J = 8 Hz, 1 H).

### Beispiel 41

### 6-[[1-(4-Chlorphenyl)-2-phenyl-1H-benzimidazol-6-yl]oxy]hexan-1-ol

wurde durch Umsetzung von 6-[[1-(4-Chlorphenyl)-2-phenyl-1*H*-benzimidazol-6-yl]oxy]hexansäuremethylester gemäß der allgemeinen Arbeitsvorschrift 11 erhalten.
Fp. 115-120°C

### Beispiel 42

### 6-[[1-(2-Methylphenyl)-2-phenyl-1H-benzimidazol-6-yl]oxy]hexansäuremethylester

### a) 5-Chlor-2-nitrophenyl-o-tolylamin

Zu einer Lösung aus 10 g 1-Chlor-3,4-dinitrobenzol in 50 ml Ethanol gab man 81 ml *o-*Toluidin und erhitzte für 72 h zum Rückfluß. Man engte im Vakuum ein und nahm den Rückstand in Ethylacetat und 2 N wäßriger Salzsäure auf. Die organische Phase wurde noch dreimal mit 2 N wäßriger Salzsäure extrahiert, über Natriumsulfat getrocknet und im Vakuum eingeengt. Man reinigte den Rückstand durch Chromatographie an Kieselgel. ¹H-NMR (CDCl₃): δ = 2.28 ppm s (3H); 6.70 dd (J = 10, 2 Hz, 1 H); 6.80 d (J = 2 Hz, 1H); 7.22-7.40 m (4H); 8.18 d (J = 10 Hz, 1H); 9.40 s (breit)(1H).

### b) 5-Methoxy-2-nitrophenyl-o-tolylamin

Zu einer Lösung aus 1 g Natrium in 20 ml Methanol gab man 1 g 5-Chlor-2-nitrophenyl-o-tolylamin und erhitzte für 72 h zum Rückfluß. Anschließend wird auf 0°C abgekühlt und das kristalline Produkt abgesaugt.
¹H-NMR (CDCl₃): δ = 2.30 ppm s (3H); 3.72 s (3H); 6.19 d (J = 2 Hz, 1H); 6.32 dd (J = 10, 2 Hz, 1H); 7.20-7.40 m (4H); 8.20 d (J = 10 Hz, 1H); 9.62 s (breit)(1H).

### c) 6-Methoxy-1-(2-methylphenyl)-2-phenyl-1H-benzimidazol

wurde durch Umsetzung von 5-Methoxy-2-nitrophenyl-o-tolylamin gemäß der allgemeinen Arbeitsvorschrift 1 und anschließende Cyclisierung mit Triethylorthobenzoat gemäß der allgemeinen Arbeitsvorschrift 3 erhalten.
¹H-NMR (CDCl₃): δ = 1.93 ppm s (3H); 3.78 s (3H); 6.42 d (J = 2 Hz, 1 H); 6.97 dd (J = 8, 2 Hz, 1 H); 7.22-7.48 m (7H); 7.57 dd (J = 8, 1 Hz, 2H); 7.78 d (J = 8 Hz, 1 H).

### 6-[[1-(2-Methylphenyl)-2-phenyl-1H-benzimidazol-6-yl]oxy]hexansäuremethylester

6-Methoxy-1-(2-methylphenyl)-2-phenyl-1*H*-benzimidazol wurde gemäß der allgemeinen Arbeitsvorschrift 6 umgesetzt. Das Rohprodukt wurde mit 6-Bromhexansäuremethylester gemäß der allgemeinen Arbeitsvorschrift 8 umgesetzt.
¹H-NMR (CDCl₃): δ = 1.43-1.58 ppm m (2H); 1.62-1.84 m (4H); 1.93 s (3H); 2.34 t (J = 7.5 Hz, 2H); 3.68 s (3H); 3.90 t (J = 7.5 Hz, 2H); 6.42 d (J = 2 Hz, 1 H); 6.96 dd (J = 8, 2 Hz, 1 H); 7.22-7.48 m (7H); 7.56 dd (J = 8, 1.5 Hz, 2H); 7.76 d (J = 8 Hz, 1 H).

### Beispiel 43

### 6-[[1-(2-Methylphenyl)-2-phenyl-1H-benzimidazol-6-yl]oxy]hexansäure

wurde durch Umsetzung 6-[[1-(2-Methylphenyl)-2-phenyl-1*H*-benzimidazol-6-yl]oxy]hexansäuremethylester gemäß der allgemeinen Arbeitsvorschrift 9 erhalten.
Fp. 198-200°C

### Beispiel 44

### 6-[[1-(3-Methylphenyl)-2-phenyl-1H-benzimidazol-6-yl]oxy]hexansäuremethylester

### a) 5-Chlor-2-nitrophenyl-m-tolylamin

Zu einer Lösung aus 50 g 1-Chlor-3,4-dinitrobenzol in 250 ml Ethanol gab man 81 ml *m-*Toluidin und ließ die Lösung für 72 h stehen. Das Rektionsgemisch wurde filtriert und das Kristallisat mit kaltem Ethanol und 2 N wäßriger Salzsäure gewaschen. Man reinigte durch Chromatographie an Kieselgel.
¹H-NMR (CDCl₃): δ = 2.40 ppm s (3H); 6.72 dd (J = 10, 2 Hz, 1 H); 7.04-7.13 m (3H); 7.14 d (J = 2 Hz, 1 H); 7.32 t (J = 10 Hz, 1 H); 8.18 d (J = 10 Hz, 1 H); 9.52 s (breit)(1 H).

### b) 5-Methoxy-2-nitrophenyl-m-tolylamin

Zu einer Lösung aus 9 g Natrium in 670 ml Methanol gab man 39 g 5-Chlor-2-nitrophenyl-*m*-tolylamin und erhitzte für 72 h zum Rückfluß. Anschließend wird auf 0°C abgekühlt und das kristalline Produkt abgesaugt.
¹H-NMR (CDCl₃): δ = 2.40 ppm s (3H); 3.73 s (3H); 6.33 dd (J = 10, 2 Hz, 1 H); 6.58 d (J = 2 Hz, 1H); 7.03-7.15 m (3H); 7.31 t (J = 10 Hz, 1 H); 8.19 d (J = 10 Hz, 1H); 9.72 s (breit)(1 H).

### c) 6-Methoxy-1-(3-methylphenyl)-2-phenyl-1H-benzimidazol

wurde durch Umsetzung von 5-Methoxy-2-nitrophenyl-*m*-tolylamin gemäß der allgemeinen Arbeitsvorschrift 1 und anschließende Cyclisierung mit Triethylorthobenzoat gemäß der allgemeinen Arbeitsvorschrift 3 erhalten.
¹H-NMR (CDCl₃): δ = 2.42 ppm s (3H); 3.81 s (3H); 6.69 d (J = 2 Hz, 1 H); 7.03 dd (J = 8, 2 Hz, 1H); 7.10-7.18 m (2H); 7.30-7.48 m (5H); 7.62 dd (J = 8, 1 Hz, 2H); 7.89 d (J = 8 Hz, 1 H).

### d) 6-Hydroxy-1-(3-methylphenyl)-2-phenyl-1H-benzimidazol

wurde durch Umsetzung von 6-Methoxy-1-(3-methylphenyl)-2-phenyl-1*H*-benzimidazol gemäß der allgemeinen Arbeitsvorschrift 6 erhalten.
¹H-NMR (D₆-DMSO): δ = 2.34 ppm s (3H); 6.52 d (J = 2 Hz, 1H); 6.80 dd (J = 8, 2 Hz, 1 H); 7.15 d (J = 8 Hz, 1 H); 7.28 s (breit)(1 H); 7.32-7.55 m (7H); 7.59 d (J = 8 Hz, 1H); 9.37 s (breit)(1 H).

### 6-[[1-(3-Methylphenyl)-2-phenyl-1H-benzimidazol-6-yl]oxy]hexansäuremethylester

wurde durch Umsetzung von 6-Hydroxy-1-(3-methylphenyl)-2-phenyl-1*H*-benzimidazol mit 6-Bromhexansäuremethylester gemäß der allgemeinen Arbeitsvorschrift 8 erhalten.
¹H-NMR (CDCl₃): δ = 1.44-1.58 ppm m (2H); 1.64-1.85 m (4H); 2.35 t (J = 7.5 Hz, 2H); 2.40 s (3H); 3.68 s (3H); 3.95 t (J = 7.5 Hz, 2H); 6.70 d (J = 2 Hz, 1 H); 6.96 dd (J = 8, 2 Hz, 1 H); 7.10 d (J = 8 Hz, 1H); 7.16 s (breit)(2H); 7.25-7.43 m (4H); 7.55 dd (J = 8, 1 Hz, 2H); 7.77 d (J=8Hz, 1H).

### Beispiel 45

### 6-[[1-(3-Methylphenyl)-2-phenyl-1H-benzimidazol-6-yl]oxy]hexansäureisopropylester

wurde durch Umsetzung von 6-Hydroxy-1-(3-methylphenyl)-2-phenyl-1*H*-benzimidazol mit 6-Bromhexansäureisopropylester gemäß der allgemeinen Arbeitsvorschrift 8 erhalten. ¹H-NMR (CDCl₃): δ = 1.22 ppm d (J = 8 Hz, 6H); 1.44-1.56 m (2H, CH₂); 1.64-1.84 m (4H, CH₂); 2.30 t (J = 7.5 Hz, 2H); 2.41 s (3H); 3.95 t (J = 7.5 Hz, 2H); 5.00 sp (J = 8 Hz, 1H); 6.68 d (J = 2 Hz, 1H); 6.96 dd (J = 8,2 Hz, 1H); 7.10 d (J = 8 Hz, 1 H); 7.14 s (breit)(1 H); 7.25-7.41 m (4H); 7.54 dd (J = 8, 1 Hz, 2H); 7.75 d (J = 8 Hz, 1H).

### Beispiel 46

### 6-[[1-(3-Methylphenyl)-2-phenyl-1H-benzimidazol-6-yl]oxy]hexansäure

wurde durch Umsetzung von 6-[[1-(3-Methylphenyl)-2-phenyl-1*H*-benzimidazol-6-yl]oxy]hexansäuremethylester gemäß der allgemeinen Arbeitsvorschrift 9 erhalten. ¹H-NMR (D₆-DMSO): δ = 1.38-1.80 ppm m (6H); 2.23 t (J = 7.5 Hz, 2H); 3.84-3.93 m (2H); 6.60 d (J = 2 Hz, 1H); 6.87 d (breit)(J = 8 Hz, 1 H); 7.15 d (J = 8 Hz, 2H); 7.20-7.32 m (4H); 7.42-7.50 m (2H); 7.59 d (J = 8 Hz, 1H); 7.77 d (J = 8 Hz, 1H).

### Beispiel 47

### 6-[[1-(3-Methylphenyl)-2-phenyl-1H-benzimidazol-6-yl]oxy]hexan-1-ol

wurde durch Umsetzung von 6-[[1-(3-Methylphenyl)-2-phenyl-1*H*-benzimidazol-6-yl]oxy]hexansäuremethylester gemäß der allgemeinen Arbeitsvorschrift 11 erhalten. ¹H-NMR (CDCl₃): δ = 1.40-1.85 m (8H); 2.40 s (3H); 3.68 t (J = 7.5 Hz, 2H); 3.96 t (J = 7.5 Hz, 2H); 6.69 d (J = 1.5 Hz, 1 H); 6.96 dd (J = 8,1.5 Hz, 1H); 7.10 d (J = 8 Hz, 1 H); 7.13 s (breit)(1 H); 7.25-7.42 m (5H); 7.54 dd (J = 8, 1 Hz, 2H); 7.76 d (J = 8 Hz, 1 H).

### Beispiel 48

### 6-[[1-(4-Methylphenyl)-2-phenyl-1H-benzimidazol-6-yl]oxy]hexansäuremethylester

### a) 5-Chlor-2-nitrophenyl-p-tolylamin

wurde analog zu 5-Chlor-2-nitrophenyl-*m*-tolylamin aus 1-Chlor-3,4-dinitrobenzol und *p-*Toluidin hergestellt. Man reinigte durch Kristallisation.
¹H-NMR (CDCl₃): δ = 2.40 ppm s (3H); 6.70 dd (J = 10, 2 Hz, 1H); 7.08 d (J = 2 Hz, 1H); 7.16 d (J = 10 Hz, 2H); 7.28 d (J = 10 Hz, 2H); 8.18 d (J = 10 Hz, 1H); 9.50 s (breit)(1H).

### b) 5-Methoxy-2-nitrophenyl-p-tolylamin

wurde analog zu 5-Methoxy-2-nitrophenyl-*m*-tolylamin aus 5-Chlor-2-nitrophenyl)-*p-*tolylamin und Natriummethanolat hergestellt.
¹H-NMR (CDCl₃): δ = 2.39 ppm s (3H); 3.72 s (3H); 6.31 dd (J = 10, 2 Hz, 1 H); 6.50 d (J = 2 Hz, 1 H); 7.19 d (J = 10 Hz, 2H); 7.25 d (J = 10 Hz, 2H); 8.19 d (J = 10 Hz, 1 H); 9.70 s (breit)(1 H).

### c) 6-Methoxy-1-(4-methylphenyl)-2-phenyl-1H-benzimidazol

wurde durch Umsetzung von 5-Methoxy-2-nitrophenyl-*p*-tolylamin gemäß der allgemeinen Arbeitsvorschrift 1 und anschließende Cyclisierung mit Triethylorthobenzoat gemäß der allgemeinen Arbeitsvorschrift 3 erhalten.
¹H-NMR (CDCl₃): δ = 2.49 ppm s (3H); 3.80 s (3H); 6.69 d (J = 2 Hz, 1H); 6.97 dd (J = 8, 2 Hz, 1H); 7.20 d (breit)(J = 8 Hz, 2H); 7.25-7.36 m (5H); 7.53 dd (J = 8, 1 Hz, 2H); 7.76 d (J = 8Hz, 1H).

### d) 6-Hydroxy-1-(4-methylphenyl)-2-phenyl-1H-benzimidazol

wurde durch Umsetzung von 6-Methoxy-1-(4-methylphenyl)-2-phenyl-1*H*-benzimidazol gemäß der allgemeinen Arbeitsvorschrift 6 erhalten.
¹H-NMR (D₆-DMSO): δ = 2.40 ppm s (3H); 6.50 d (J = 2 Hz, 1 H); 6.80 dd (J = 8, 2 Hz, 1 H); 7.28 d (J = 8 Hz, 2H); 7.32-7.43 m ( 5H); 7.46-7.52 m (2H); 7.56 d (J = 8 Hz, 1H); 9.28 s (breit)(1 H).

### 6-[[1-(4-Methylphenyl)-2-phenyl-1H-benzimidazol-6-yl]oxy]hexansäuremethylester

wurde durch Umsetzung von 6-Hydroxy-1-(4-methylphenyl)-2-phenyl-1*H*-benzimidazol mit 6-Bromhexansäuremethylester gemäß der allgemeinen Arbeitsvorschrift 8 erhalten. ¹H-NMR (CDCl₃): δ = 1.44-1.58 ppm m (2H); 1.62-1.86 m (4H); 2.34 t (J = 7.5 Hz, 2H); 2.48 s (3H); 3.68 s (3H); 3.94 t (J = 7.5 Hz, 2H); 6.69 d (J = 2 Hz, 1 H); 6.96 dd (J = 8, 2 Hz, 1 H); 7.19 d (J = 8 Hz, 2H); 7.28-7.38 m (5H); 7.55 dd (J = 8, 1 Hz, 2H); 7.75 d (J = 8 Hz, 1H).

### Beispiel 49

### 6-[[1-(4-Methylphenyl)-2-phenyl-1H-benzimidazol-6-yl]oxy]hexansäureisopropylester

wurde durch Umsetzung von 6-Hydroxy-1-(4-methylphenyl)-2-phenyl-1*H*-benzimidazol mit 6-Bromhexansäureisopropylester gemäß der allgemeinen Arbeitsvorschrift 8 erhalten. ¹H-NMR (CDCl₃): δ = 1.22 ppm d (J = 7.5 Hz, 6H); 1.44-1.56 m (2H); 1.62-1.85 m (4H); 2.30 t (J = 7.5 Hz, 2H); 2.47 s (3H); 3.93 t (J = 7.5 Hz, 2H); 5.01 sp (J = 7.5 Hz, 1 H); 6.68 d (J = 2 Hz, 1 H); 6.96 dd (J = 8, 2 Hz, 1 H); 7.20 d (J = 8 Hz, 2H); 7.26-7.36 m (5H); 7.55 dd (J = 8, 1 Hz, 2H); 7.75 d (J = 8 Hz, 1 H).

### Beispiel 50

### 6-[[1-(4-Methylphenyl)-2-phenyl-1H-benzimidazol-6-yl]oxy]hexansäure

wurde durch Umsetzung von 6-[[1-(4-Methylphenyl)-2-phenyl-1*H*-benzimidazol-6-yl]oxy]hexansäuremethylester gemäß der allgemeinen Arbeitsvorschrift 9 erhalten.
Fp. 186-190°C

### Beispiel 51

### 6-[[1-(4-Methylphenyl)-2-phenyl-1H-benzimidazol-6-yl]oxy]hexan-1-ol

wurde durch Umsetzung von 6-[[1-(4-Methylphenyl)-2-phenyl-1*H*-benzimidazol-6-yl]oxy]hexansäuremethylester gemäß der allgemeinen Arbeitsvorschrift 11 erhalten. ¹H-NMR (CDCl₃): δ = 1.38-1.80 m (8H); 2.47 s (3H); 3.65 t (J = 7.5 Hz, 2H); 3.93 t (J = 7.5 Hz, 2H); 6.68 d (J = 2 Hz, 1H); 6.97 dd (J = 8, 2 Hz, 1 H); 7.18 d (J = 8 Hz, 2H); 7.24-7.37 m (5H); 7.54 dd (J = 8, 1 Hz, 2H); 7.75 d (J = 8 Hz, 1H).

### Beispiel 52

### 6-[[1-(3,4-Dimethylphenyl)-2-phenyl-1H-benzimidazol-6-yl]oxy]hexansäuremethylester

### a) 3-(3,4-Dimethylphenyl)amino-4-nitrophenol

3 g 3-Fluor-4-nitrophenol und 6.9 g 3,4-Dimethylanilin wurden vermischt und für 2 h bei 150°C gerührt. Nach dem Erkalten wurde in Dichlormethan gelöst und sechsmal mit 1 N wäßriger Salzsäure extrahiert. Die organische Phase wurde verworfen, und die vereinigten wäßrigen Phasen wurden dreimal mit Chloroform extrahiert. Die vereinigten Extrakte wurden über Natriumsulfat getrocknet, im Vakuum eingeengt und der Rückstand an Kieselgel chromatographiert.
¹H-NMR (CDCl₃/D₆-DMSO): δ = 2.18 ppm s (6H); 6.13 dd (J = 8, 2 Hz, 1H); 6.36 d (J = 2 Hz, 1 H); 6.90-7.00 m (2H); 7.09 d (J = 8 Hz, 1 H); 7.93 d (J = 8 Hz, 1H); 9.50 s (breit)(1 H); 10.19 s (breit)(1H). b) 6-[3-(3,4-Dimethylphenyl)amino-4-nitrophenyl]oxyhexansäuremethylester wurde durch Umsetzung von 3-(3,4-Dimethylphenyl)amino-4-nitrophenol mit 6-Bromhexansäuremethylester gemäß der allgemeinen Arbeitsvorschrift 8 erhalten.
¹H-NMR (CDCl₃): δ = 1.38-1.52 ppm m (2H); 1.59-1.80 m (4H); 2.30 s (6H); 2.33 t (J = 7.5 Hz, 2H); 3.68 s (3H); 3.87 t (J = 7.5 Hz, 2H); 6.28 dd (J = 8, 2 Hz, 1 H); 6.48 d (J = 2 Hz, 1 H); 7.04 d (J = 8 Hz, 1 H); 7.06 s (breit)(1H); 7.18 d (J = 8 Hz, 1 H); 8.17 d (J = 8 Hz, 1 H); 9.71 s (breit) (, 1 H).

### 6-[[1-(3,4-Dimethylphenyl)-2-phenyl-1H-benzimidazol-6-yl]oxy]hexansäuremethylester

wurde durch Umsetzung von 6-[3-(3,4-Dimethylphenyl)amino-4-nitrophenyl]oxyhexansäure-methylester gemäß der allgemeinen Arbeitsvorschrift 1 und anschließende Cyclisierung mit Triethylorthobenzoat gemäß der allgemeinen Arbeitsvorschrift 3 erhalten.
¹H-NMR (CDCl₃): δ = 1.44-1.56 ppm m (2H); 1.62-1.84 m (4H); 2.30 s (3H); 2.33 t (J = 7.5 Hz, 2H); 2.34 s (3H); 3.68 s (3H); 3.93 t (J = 7.5 Hz, 2H); 6.67 d (J = 2 Hz, 1H); 6.94 dd (J = 8, 2 Hz, 1H); 7.03 dd (J = 8, 1.5 Hz, 1 H); 7.09 s (breit)(1H); 7.22-7.35 m (4H); 7.57 dd (J = 8, 1.5 Hz, 2H); 7.76 d (J = 8 Hz, 1 H).

### Beispiel 53

### 6-[[1-(3,4-Dimethylphenyl)-2-phenyl-1H-benzimidazol-6-yl]oxy]hexansäure

wurde durch Umsetzung von 6-[[1-(3,4-Dimethylphenyl)-2-phenyl-1*H*-benzimidazol-6-yl]-oxy]hexansäuremethylester gemäß der allgemeinen Arbeitsvorschrift 9 erhalten.
Fp. 158-161 °C

### Beispiel 54

### 6-[[1-(3,5-Dimethylphenyl)-2-phenyl-1H-benzimidazol-6-yl]oxy]hexansäuremethylester

### a) 3-(3,5-Dimethylphenyl)amino-4-nitrophenol

5.4 g 3-Fluor-4-nitrophenol und 4.3 ml 3,5-Dimethylanilin wurden vermischt und für 6 h bei 120°C gerührt. Nach dem Erkalten wurde in Ethylacetat und Wasser aufgenommen und dreimal mit 1 N wäßriger Salzsäure extrahiert. Die vereinigten wäßrigen Phasen wurden dreimal mit Ethylacetat extrahiert. Die vereinigten organischen Phasen wurden über Natriumsulfat getrocknet, im Vakuum eingeengt und der Rückstand kristallisiert.
¹H-NMR (D₆-DMSO): δ = 2.30 ppm s (6H); 6.28 dd (J = 8, 2 Hz, 1H); 6.49 d (J = 2 Hz, 1H); 6.52 d (J = 2 Hz, 1 H); 6.90 s (breit)(1H); 6.98 s (breit)(2H); 8.04 d (J = 8 Hz, 1H); 9.51 s (breit) (1H). b) 6-[3-(3,5-Dimethylphenyl)amino-4-nitrophenyl]oxyhexansäuremethylester wurde durch Umsetzung von 3-(3,5-Dimethylphenyl)amino-4-nitrophenol mit 6-Bromhexansäuremethylester gemäß der allgemeinen Arbeitsvorschrift 8 erhalten.
¹H-NMR (CDCl₃): δ = 1.40-1.52 ppm m (2H); 1.60-1.80 m (4H); 2.30 t (J = 7.5 Hz, 2H) 2.32 s (6H); 3.68 s (3H); 3.88 t (J = 7.5 Hz, 2H); 6.30 dd (J = 8, 2 Hz, 1 H); 6.52 d (J = 2 Hz, 1 H); 6.88 s (breit)(1H); 6.91 s (breit)(2H); 8.17 d (J = 8 Hz, 1H); 9.69 s (breit) (1 H).

### 6-[3-(3,5-Dimethylphenyl)amino-4-nitrophenyl]oxyhexansäuremethylester

wurde durch Umsetzung von 6-[3-(3,5-Dimethylphenyl)amino-4-nitrophenyl]oxyhexansäure-methylester gemäß der allgemeinen Arbeitsvorschrift 1 und anschließende Cyclisierung mit Triethylorthobenzoat gemäß der allgemeinen Arbeitsvorschrift 3 erhalten.
Fp. 124-126°C

### Beispiel 55

### 6-[[1-(3,5-Dimethylphenyl)-2-phenyl-1H-benzimidazol-6-yl]oxy]hexansäure

wurde durch Umsetzung von 6-[3-(3,5-Dimethylphenyl)amino-4-nitrophenyl]oxyhexansäure-methylester gemäß der allgemeinen Arbeitsvorschrift 9.
Fp. 162-164°C

### Beispiel 56

### 6-[[1-(3,5-Dimethylphenyl)-2-phenyl-1H-benzimidazol-6-yl]oxy]hexansäure-isopropylester

wurde durch Umsetzung von 6-[3-(3,5-Dimethylphenyl)amino-4-nitrophenyl]oxyhexansäure mit Isopropanol gemäß der allgemeinen Arbeitsvorschrift 10.
Fp. 98-101°C

### Beispiel 57

### 6-[[1-(3-Methoxyphenyl)-2-phenyl-1H-benzimidazol-6-yl]oxy]hexansäuremethylester

### a) 3-(3-Methoxyphenyl)amino-4-nitrophenol

4 g 3-Fluor-4-nitrophenol und 9.4 g *m*-Anisidin wurden vermischt und für 2.5 h bei 150°C gerührt. Nach dem Erkalten wurde in Dichlormethan gelöst und dreimal mit 1 N wäßriger Salzsäure extrahiert. Die organische Phase wurde über Natriumsulfat getrocknet, im Vakuum eingeengt und der Rückstand an Kieselgel chromatographiert.
¹H-NMR (CDCl₃): δ = 3.83 ppm s (3H); 6.30 dd (J = 10, 2 Hz, 1H); 6.57 d (J = 2 Hz, 1H); 6.70-6.84 m (2H); 6.89 d (breit)(J = 10 Hz, 1 H); 7.32 t (J = 10 Hz, 1H); 8.19 d (J = 10 Hz, 1H); 9.68 s (breit)(1H); 9.69 s (breit).

### b) 6-[3-(3-Methoxyphenyl)amino-4-nitrophenyl]oxyhexansäuremethylester

wurde durch Umsetzung von 3-(3-Methoxyphenyl)amino-4-nitrophenol mit 6-Bromhexansäuremethylester gemäß der allgemeinen Arbeitsvorschrift 8 erhalten. ¹H-NMR (CDCl₃): δ = 1.42-1.58 ppm m (2H); 1.60-1.93 m (4H); 2.34 t (J = 7.5 Hz, 2H); 3.68 s (3H); 3.80 s (3H); 4.03 t (J = 7.5 Hz, 2H); 6.32 dd (J = 10, 2 Hz, 1H); 6.59 d (J = 2 Hz, 1 H); 6.68-6.84 m (2H); 6.90 d (breit)(J = 8 Hz, 1 H); 7.32 t (J = 8 Hz, 1 H); 8.19 d (J = 10 Hz, 1 H); 9.70 s (breit)(1H).
6-[[1-(3-Methoxyphenyl)-2-phenyl-1*H*-benzimidazol-6-yl]oxy]hexansäuremethylester wurde durch Umsetzung von 6-[3-(3-Methoxyphenyl)amino-4-nitrophenyl]oxyhexansäure-methylester gemäß der allgemeinen Arbeitsvorschrift 1 und anschließende Cyclisierung mit Triethylorthobenzoat gemäß der allgemeinen Arbeitsvorschrift 3 erhalten.
¹H-NMR (CDCl₃): δ = 1.44-1.58 ppm m (2H); 1.62-1.86 m (4H); 2.34 t (J = 7.5 Hz, 2H); 3.68 s (3H); 3.78 s (3H); 3.95 t (J = 7.5 Hz, 2H); 6.71 d (J = 1.5 Hz, 1 H); 6.83 dd (J = 1.5, 1.5 Hz, 1H); 6.90 dd (J = 8, 1.5 Hz, 1 H); 6.94 dd (J = 8, 1.5 Hz, 1H); 7.01 dd (J = 8, 1.5 Hz, 1 H); 7.27-7.36 m (3H); 7.40 t (J = 8 Hz, 1 H); 7.56 dd (J = 8, 2 Hz, 2H); 7.74 d (J = 8 Hz, 1H).

### Beispiel 58

### 6-[[1-(3-Methoxyphenyl)-2-phenyl-1H-benzimidazol-6-yl]oxy]hexansäure

wurde durch Umsetzung von 6-[[1-(3-Methoxyphenyl)-2-phenyl-1*H*-benzimidazol-6-yl]oxy]hexansäuremethylester gemäß der allgemeinen Arbeitsvorschrift 9 erhalten.
Fp. 149-152°C

### Beispiel 59

### 6-[[1-(4-Methoxyphenyl)-2-phenyl-1H-benzimidazol-6-yl]oxy]hexansäuremethylester

### a) 3-(4-Methoxyphenyl)amino-4-nitrophenol

0.16 g 3-Fluor-4-nitrophenol und 0.37 g *p*-Anisidin wurden vermischt und für 1.5 h bei 150°C gerührt. Nach dem Erkalten wurde in Dichlormethan gelöst, zweimal mit 1 N wäßriger Salzsäure und einmal mit ges. Natriumchloridlösung extrahiert. Die organische Phase wurde über Natriumsulfat getrocknet, und im Vakuum eingeengt.
¹H-NMR (CDCl₃/D₆-DMSO): δ = 3.57 ppm s (3H); 6.06 dd (J = 10, 2 Hz, 1H); 6.18 d (J = 2 Hz, 1H); 6.77 d (J = 10 Hz, 2H); 7.03 d (J = 10 Hz, 2H); 7.89 d (J = 10 Hz, 1H); 9.40 s (breit)(1H); 9.80 s (breit).

### b) 6-[3-(4-Methoxyphenyl)amino-4-nitrophenyl]oxyhexansäuremethylester

**wurde durch Umsetzung von 3-(4-Methoxyphenyl)amino-4-nitrophenol mit 6-**Bromhexansäuremethylester gemäß der allgemeinen Arbeitsvorschrift 8 erhalten. ¹H-NMR (CDCl₃): δ = 1.38-1.50 ppm m (2H); 1.60-1.80 m (4H); 2.33 t (J = 7.5 Hz, 2H); 3.67 s (3H); 3.85 t (J = 7.5 Hz, 2H); 3.88 s (3H); 6.29 dd (J = 10, 1.5 Hz, 1H); 6.30 d (J = 1.5 Hz, 1H); 6.98 d (J = 10 Hz, 2H); 7.20 d (J = 10 Hz, 2H); 8.18 d (J = 10 Hz, 1H); 9.63 s (breit)(1 H).
6-[[1-(4-Methoxyphenyl)-2-phenyl-1*H*-benzimidazol-6-yl]oxy]hexansäuremethylester wurde durch Umsetzung von 6-[3-(4-Methoxyphenyl)amino-4-nitrophenyl]oxyhexansäure-methylester gemäß der allgemeinen Arbeitsvorschrift 1 und anschließende Cyclisierung mit Triethylorthobenzoat gemäß der allgemeinen Arbeitsvorschrift 3 erhalten.
Fp. 98-102°C

### Beispiel 60

### 6-[[1-(4-Methoxyphenyl)-2-phenyl-1H-benzimidazol-6-yl]oxy]hexansäure

wurde durch Umsetzung von 6-[[1-(4-Methoxyphenyl)-2-phenyl-1*H*-benzimidazol-6-yl]oxy]hexansäuremethylester gemäß der allgemeinen Arbeitsvorschrift 9 erhalten.
Fp. 160-165°C

### Beispiel 61

### 6-[[1-(3,4-Dimethoxyphenyl)-2-phenyl-1H-benzimidazol-6-yl]oxy]hexansäure-methylester

### a) 3-(3,4-Dimethoxyphenyl)amino-4-nitrophenol

3 g 3-Fluor-4-nitrophenol und 8.8 g 3,4-Dimethoxyanilin wurden vermischt und für 2 h bei 150°C gerührt. Nach dem Erkalten wurde in Dichlormethan gelöst und zweimal mit 1 N wäßriger Salzsäure extrahiert. Die wäßrige Phase wurde zweimal mit Chloroform extrahiert und die vereinigten Chloroformextrakte über Natriumsulfat getrocknet und im Vakuum eingeengt.
¹H-NMR (D₆-DMSO): δ = 3.75 ppm s (3H); 3.78 s (3H); 6.25 dd (J = 10, 2 Hz, 1 H); 6.35 d (J = 2 Hz, 1H); 6.88 dd (J = 8, 1.5 Hz, 1H); 6.98 d (J = 1.5 Hz, 1H); 7.05 d (J = 8 Hz, 1H); 8.04 d (J = 10 Hz, 1H); 9.52 s (breit)(1H); 10.72 s (breit).

### b) 6-[3-(3,4-Dimethoxyphenyl)amino-4-nitrophenyl]oxyhexansäuremethylester

wurde durch Umsetzung von 3-(3,4-Dimethoxyphenyl)amino-4-nitrophenol mit 6-Bromhexansäuremethylester gemäß der allgemeinen Arbeitsvorschrift 8 erhalten. ¹H-NMR (CDCl₃): δ = 1.40-1.52 ppm m (2H); 1.60-1.80 m (4H); 2.32 t (J = 7.5 Hz, 2H); 3.68 s (3H); 3.85 t (J = 7.5 Hz, 2H); 3.88 s (3H); 3.93 s (3H); 6.29 dd (J = 10, 1.5 Hz, 1 H); 6.33 d (J = 1.5 Hz, 1H); 6.80 d (J = 1.5 Hz, 1 H); 6.87 dd (J = 10, 1.5 Hz, 1 H); 6.92 d (J = 10 Hz, 1H); 8.18 d (J = 10 Hz, 1H); 9.68 s (breit)(1 H).

### 6-[[1-(3,4-Dimethoxyphenyl)-2-phenyl-1H-benzimidazol-6-yl]oxy]hexansäure-methylester

wurde durch Umsetzung von 6-[3-(3,4-Dimethoxyphenyl)amino-4-nitrophenyl]oxyhexan-säuremethylester gemäß der allgemeinen Arbeitsvorschrift 1 und anschließende Cyclisierung mit Triethylorthobenzoat gemäß der allgemeinen Arbeitsvorschrift 3 erhalten. Fp. 116-118°C

### Beispiel 62

### 6-[[1-(3,4-Dimethoxyphenyl)-2-phenyl-1H-benzimidazol-6-yl]oxy]hexansäure

wurde durch Umsetzung von 6-[[1-(3,4-Dimethoxyphenyl)-2-phenyl-1*H*-benzimidazol-6-yl]oxy]hexansäuremethylester gemäß der allgemeinen Arbeitsvorschrift 9 erhalten.
Fp. 158-161 °C

### Beispiel 63

### 6-[[1-[3,4-(Methylendioxy)phenyl]-2-phenyl-1H-benzimidazol-6-yl]oxy]hexansäure-methylester

### a) 3-(3,4-Methylendioxyphenyl)amino-4-nitrophenol

0.86 g 3-Fluor-4-nitrophenol und 2.25 g 3,4-Methylendioxyanilin wurden vermischt und für 5 h bei 120°C gerührt. Das Rohgemisch wurde an Kieselgel chromatographiert.
¹H-NMR (D₆-DMSO): δ = 6.02 ppm s (2H); 6.25 dd (J = 10, 2 Hz, 1H); 6.33 d (J = 2 Hz, 1H); 6.72 dd (J = 8, 1.5 Hz, 1H); 6.87 d (J = 1.5 Hz, 1H); 7.05 d (J = 10 Hz, 1H); 8.18 d (J = 10 Hz, 1 H); 9.52 s (breit)(1H). b) 6-[3-(3,4-Methylendioxyphenyl)amino-4-nitrophenyl]oxyhexansäuremethylester wurde durch Umsetzung von 3-(3,4-Methylendioxyphenyl)amino-4-nitrophenol mit 6-Bromhexansäuremethylester gemäß der allgemeinen Arbeitsvorschrift 8 erhalten.
Fp. 108-111°C

### 6-[[1-(3,4-Methylendioxyphenyl)-2-phenyl-1H-benzimidazol-6-yl]oxy]hexansäure-methylester

wurde durch Umsetzung von 6-[3-(3,4-Methylendioxyphenyl)amino-4-nitrophenyl]oxyhexan-säuremethylester gemäß der allgemeinen Arbeitsvorschrift 1 und anschließende Cyclisierung mit Triethylorthobenzoat gemäß der allgemeinen Arbeitsvorschrift 3 erhalten.
¹H-NMR (CDCl₃): δ = 1.43-1.55 ppm m (2H); 1.65-1.82 m (4H); 2.35 t (J = 7.5 Hz, 2H); 3.68 s (3H); 3.95 t (J = 7.5 Hz, 2H); 6.10 s (2H); 6.65 d (J = 1.5 Hz, 1 H); 6.72-6.83 m (2H); 6.90 d (J = 10 Hz, 1H); 6.93 dd (J = 10, 1.5 Hz, 1H); 7.29-7.38 m (3H); 7.52-7.62 m (2H); 7.72 d (J = 10 Hz, 1H).

### Beispiel 64

### 6-[[1-[3,4-(Methylendioxy)phenyl]-2-phenyl-1H-benzimidazol-6-yl]oxy]hexansäure

wurde durch Umsetzung von 6-[[1-(3,4-Methylendioxyphenyl)-2-phenyl-1*H*-benzimidazol-6-yl]oxy]hexansäuremethylester gemäß der allgemeinen Arbeitsvorschrift 9 erhalten.
Fp. 130°C

### Beispiel 65

### 6-[[2-Phenyl-1-(3,4,5-trimethoxyphenyl)-1H-benzimidazol-6-yl]oxy]hexansäure-methylester

### a) 3-(3,4,5-Trimethoxyphenyl)amino-4-nitrophenol

3.7 g 3-Fluor-4-nitrophenol und 4.76 g 3,4,5-Trimethoxyanilin wurden vermischt und für 10 h bei 100°C gerührt. Nach dem Erkalten wurde in Ethylacetat und Wasser aufgenommen und dreimal mit Ethylacetat extrahiert. Die vereinigten organischen Phasen wurden dreimal mit 1 N wäßriger Salzsäure und einmal mit ges. Natriumchloridlösung extrahiert, über Natriumsulfat getrocknet und im Vakuum weitestgehend eingeengt. Das Produkt wurde mit Diisopropylether digeriert.
¹H-NMR (D₆-DMSO): δ = 3.70 ppm s (3H); 3.80 s (6H); 6.28 dd (J = 10, 2 Hz, 1H); 6.53 d (J = 2 Hz, 1H); 6.70 s (2H); 8.05 d (J = 10 Hz, 1H); 9.50 s (breit)(1H); 10.71 s (breit). b) 6-[4-Nitro-3-[(3,4,5-Trimethoxyphenyl)amino]phenyl]oxyhexansäuremethylester wurde durch Umsetzung von 4-Nitro-3-[(3,4,5-Trimethoxyphenyl)amino]phenol mit 6-Bromhexansäuremethylester gemäß der allgemeinen Arbeitsvorschrift 8 erhalten. ¹H-NMR (CDCl₃): δ = 1.40-1.53 ppm m (2H); 1.60-1.82 m (4H); 2.32 t (J = 7.5 Hz, 2H); 3.67 s (3H); 3.85 s (6H); 3.88 t (J = 7.5 Hz, 2H); 3.90 s (3H); 6.30 dd (J = 10, 1.5 Hz, 1 H); 6.50 d (J = 1.5 Hz, 1 H); 6.52 s (2H); 8.18 d (J = 10 Hz, 1H); 9.68 s (breit)(1 H).

### 6-[[2-Phenyl-1-(3,4,5-trimethoxyphenyl)-1H-benzimidazol-6-yl]oxy]hexansäure-methylester

wurde durch Umsetzung von 6-[4-Nitro-3-[(3,4,5-Trimethoxyphenyl)amino]phenyl]oxy-hexansäuremethylester gemäß der allgemeinen Arbeitsvorschrift 1 und anschließende Cyclisierung mit Triethylorthobenzoat gemäß der allgemeinen Arbeitsvorschrift 3 erhalten. Fp. 128-130°C

### Beispiel 66

### 6-[[2-Phenyl-1-(3,4,5-trimethoxyphenyl)-1H-benzimidazol-6-yl]oxy]hexansäure

wurde durch Umsetzung von 6-[[2-Phenyl-1-(3,4,5-trimethoxyphenyl)-1*H*-benzimidazol-6-yl]oxy]hexansäuremethylester gemäß der allgemeinen Arbeitsvorschrift 9 erhalten.
Fp. 198-201°C

### Beispiel 67

### 6-[[2-Phenyl-1-(3,4,5-trimethoxyphenyl)-1H-benzimidazol-6-yl]oxy]hexan-säureisopropylester

wurde durch Umsetzung von 6-[[2-Phenyl-1-(3,4,5-trimethoxyphenyl)-1*H*-benzimidazol-6-yl]oxy]hexansäure mit Isopropanol gemäß der allgemeinen Arbeitsvorschrift 10 erhalten. Fp. 98-101°C

### Beispiel 68

### 6-[[1-[4-(N,N-Dimethylamino)phenyl]-2-phenyl-1H-benzimidazol-6-yl]oxy]hexansäure-methylester

### a) N,N-Dimethyl-N'-(5-chlor-2-nitrophenyl)benzol-1,4-diamin

wurde analog zu 5-Chlor-2-nitrophenyl-*m*-tolylamin aus 1-Chlor-3,4-dinitrobenzol und *N,N-*Dimethyl-*p*-phenylendiamin hergestellt.
¹H-NMR (CDCl₃): δ = 3.01 ppm s (6H); 6.63 dd (J = 10, 2 Hz, 1 H); 6.80 d (breit)(J = 10 Hz, 2H); 6.97 d (J = 2 Hz, 1H); 7.14 d (J = 10 Hz, 2H); 8.14 d (J = 10 Hz, 1H); 9.42 s (breit)(1 H).

### b) N,N-Dimethyl-N'-(5-methoxy-2-nitrophenyl)benzol-1,4-diamin

24.9 g *N,N*-Dimethyl-*N'*-(5-chlor-2-nitrophenyl)benzol-1,4-diamin wurden zu einer Lösung aus 8 g Natrium in 200 ml Methanol gegeben und das Gemisch in einem Autoklaven für 9 h auf 120°C erhitzt. Nach dem Erkalten wurde vom kristallinen Produkt abgesaugt.
¹H-NMR (CDCl₃): δ = 3.00 ppm s (6H); 3.70 s (3H); 6.25 dd (J = 10, 2 Hz, 1 H); 6.34 d (J = 2 Hz, 1H); 6.78 d (J = 10 Hz, 2H); 7.14 d (J = 10 Hz, 2H); 8.16 d (J = 10 Hz, 1H); 9.67 s (breit)(1 H).

### c) 6-Methoxy-1-[4-(N,N-dimethylamino)phenyl]-2-phenyl-1H-benzimidazol

wurde durch Umsetzung von *N,N*-Dimethyl-*N'*-(5-methoxy-2-nitrophenyl)benzol-1,4-diamin gemäß der allgemeinen Arbeitsvorschrift 1, anschließende Umsetzung des rohen Diamins mit Trimethylorthobenzoat gemäß der allgemeinen Arbeitsvorschrift 3 und nachfolgendes Erhitzen des Rohprodukts mit 6 N wäßriger Salzsäure für 1 h zum Rückfluß dargestellt. Nach Alkalisieren des Reaktionsgemisches mit wäßriger Natronlauge wurde mit Ethylacetat extrahiert, über Natriumsulfat getrocknet und im Vakuum eingeengt.
¹H-NMR (CDCl₃): δ = 3.04 ppm s (6H); 3.80 s (3H); 6.68 d (J = 2 Hz, 1 H); 6.78 d (J = 10 Hz, 2H); 6.95 dd (J = 10, 2 Hz, 1H); 7.17 d (J = 10 Hz, 2H); 7.25-7.33 m (3H); 7.56-7.64 m (2H); 7.74 d (J = 10 Hz, 1 H).

### d) 6-Hydroxy-1-[4-(N,N-dimethylamino)phenyl]-2-phenyl-1H-benzimidazol

wurde durch Umsetzung von 6-Methoxy-1-[4-(*N*,*N*-dimethylamino)phenyl]-2-phenyl-1*H-*benzimidazol gemäß der allgemeinen Arbeitsvorschrift 6 erhalten.
¹H-NMR (D₆-DMSO): δ = 2.98 ppm s (6H); 6.48 d (J = 2 Hz, 1H); 6.78 dd (J = 10, 2 Hz, 1H); 6.83 d (J = 10 Hz, 2H); 7.17 d (J = 10 Hz, 2H); 7.30-7.38 m (3H); 7.50-7.57 m (3H); 9.32 s (breit)(1 H).

### 6-[[1-[4-(N,N-Dimethylamino)phenyl]-2-phenyl-1H-benzimidazol-6-yl]oxy]hexansäure-methylester

wurde durch Umsetzung von 6-Hydroxy-1-[4-(*N*,*N*-dimethylamino)phenyl]-2-phenyl-1*H-*benzimidazol mit 6-Bromhexansäuremethylester gemäß der allgemeinen Arbeitsvorschrift 8 erhalten.
¹H-NMR (CDCl₃): δ = 1.43-1.57 ppm m (2H); 1.64-1.85 m (4H); 2.33 t (J = 7.5 Hz, 2H); 3.05 s (6H); 3.67 s (3H); 3.93 t (J = 7.5 Hz, 2H); 6.65 d (J = 2 Hz, 1 H); 6.76 d (J = 10 Hz, 2H); 6.93 dd (J = 10, 2 Hz, 1H); 7.14 d (J = 10 Hz, 2H); 7.23-7.27 m (3H); 7.62 dd (J = 10, 1.5 Hz, 2H); 7.74 d (J = 10 Hz, 1 H).

### Beispiel 69

### 6-[[1-[4-(N,N-Dimethylamino)phenyl]-2-phenyl-1H-benzimidazol-6-yl]oxy]hexansäure

wurde durch Umsetzung von 6-[[1-[4-(*N*,*N*-Dimethylamino)phenyl]-2-phenyl-1*H-*benzimidazol-6-yl]oxy]hexansäuremethylester gemäß der allgemeinen Arbeitsvorschrift 9 erhalten.
Fp. 210-213°C

### Beispiel 70

### 6-[[1-(4-Biphenyl)-2-phenyl-1H-benzimidazol-6-yl]oxy]hexansäuremethylester

### a) 5-Chlor-2-nitrophenyl-4-biphenylamin

wurde analog zu 5-Chlor-2-nitrophenyl-m-tolylamin aus 1-Chlor-3,4-dinitrobenzol und 4-Biphenylamin hergestellt. Man reinigte durch Chromatographie an Kieselgel.
¹H-NMR (CDCl₃): δ = 6.76 dd (J = 10, 2 Hz, 1H); 7.26 d (J = 2 Hz, 1H); 7.35 d (J = 8 Hz, 1 H); 7.32-7.52 m (4H); 7.60-7.72 m (4H); 8.19 d (J = 10 Hz, 1 H); 9.60 s (breit)(1 H).

### b) 5-Methoxy-2-nitrophenyl-4-biphenylamin

wurde analog zu 5-Methoxy-2-nitrophenyl-m-tolylamin aus 5-Chlor-2-nitrophenyl-4-biphenylamin und Natriummethanolat hergestellt
Fp. 150-154°C

### b) 1-(4-Biphenyl)-6-methoxy-2-phenyl-1H-benzimidazol

wurde durch Umsetzung von 5-Methoxy-2-nitrophenyl-4-biphenylamin gemäß der allgemeinen Arbeitsvorschrift 1 und anschließende Cyclisierung mit Triethylorthobenzoat gemäß der allgemeinen Arbeitsvorschrift 3 erhalten.
Fp. 140-144°C

### c) 1-(4-Biphenyl)-6-hydroxy-2-phenyl-1H-benzimidazol

wurde durch Umsetzung von 1-(4-Biphenyl)-6-methoxy-2-phenyl-1*H*-benzimidazol gemäß der allgemeinen Arbeitsvorschrift 6 erhalten.
Fp. 312°C

### 6-[[1-(4-Biphenyl)-2-phenyl-1H-benzimidazol-6-yl]oxyjhexansäuremethylester

wurde durch Umsetzung von 1-(4-Biphenyl)-6-hydroxy-2-phenyl-1*H*-benzimidazol mit 6-Bromhexansäuremethylester gemäß der allgemeinen Arbeitsvorschrift 8 erhalten.
Fp. 106-108°C

### Beispiel 71

### 6-[[1-(4-Biphenyl)-2-phenyl-1H-benzimidazol-6-yl]oxy]hexansäure

wurde durch Umsetzung von 6-[[1-(4-Biphenyl)-2-phenyl-1*H*-benzimidazol-6-yl]oxy]hexansäuremethylester gemäß der allgemeinen Arbeitsvorschrift 9 erhalten.
¹H-NMR (D₆-DMSO): δ = 1.35-1.78 ppm m (6H); 2.20 t (J = 7.5 Hz, 2H); 3.96 m (2H); 6.72 d (J = 2 Hz, 1 H); 6.97 dd (J = 10, 2 Hz, 1 H); 7.32-7.58 m (10H); 7.69 d (J = 10 Hz, 1 H); 7.80 d (J = 8 Hz, 2H); 7.89 d (J = 10 Hz, 2H).

### Beispiel 72

### 6-[[1-(2-Naphtyl)-2-phenyl-1H-benzimidazol-6-yl]oxy]hexansäuremethylester

### a) 3-(2-Naphtylamino)-4-nitrophenol

3 g 3-Fluor-4-nitrophenol und 8.2 g 2-Naphtylamin wurden vermischt und für 8 h bei 180°C gerührt. Das Rohgemisch wurde in Chloroform aufgenommen und mit 2 N wäßriger Salzsäure gewaschen. Die organische Phase wurde über Natriumsulfat getrocknet und im Vakuum eingeengt. Der Rückstand wurde an Kieselgel chromatographiert.
¹H-NMR (D₆-DMSO): δ = 6.02 ppm s (2H); 6.25 dd (J = 10, 2 Hz, 1 H); 6.33 d (J = 2 Hz, 1 H); 6.72 dd (J = 8, 1.5 Hz, 1 H); 6.87 d (J = 1.5 Hz, 1H); 7.05 d (J = 10 Hz, 1H); 8.18 d (J = 10 Hz, 1 H); 9.52 s (breit)(1 H).

### b) 6-[3-(2-Naphtyl)amino-4-nitrophenyl]oxyhexansäuremethylester

wurde durch Umsetzung von 3-(2-Naphtylamino)-4-nitrophenol mit 6-Bromhexansäuremethylester gemäß der allgemeinen Arbeitsvorschrift 8 erhalten.
¹H-NMR (CDCl₃): δ = 1.35-1.49 ppm m (2H); 1.60-1.80 m (4H); 2.30 t (J = 7.5 Hz, 2H); 3.64 s (3H); 3.84 t (J = 7.5 Hz. 2H); 6.35 dd (J = 10, 2 Hz, 1H); 6.62 d (J = 2 Hz, 1H); 7.43 dd (J = 10, 2 Hz, 1 H); 7.48-7.57 m (2H); 7.75 d (J = 2 Hz, 1 H); 7.78-7.90 m (2H); 7.91 d (J = 10 Hz, 1 H); 8.21 d (J = 10 Hz, 1H); 9.92 s (breit)(1H).

### 6-[[1-(2-Naphtyl)-2-phenyl-1H-benzimidazol-6-yl]oxy]hexansäuremethylester

wurde durch Umsetzung von 6-[3-(2-Naphtylamino)-4-nitrophenyl]oxyhexansäure-methylester gemäß der allgemeinen Arbeitsvorschrift 1 und anschließende Cyclisierung mit Triethylorthobenzoat gemäß der allgemeinen Arbeitsvorschrift 3 erhalten. Fp. 111-114°C

### Beispiel 73

### 6-[[1-(2-Naphtyl)-2-phenyl-1H-benzimidazol-6-yl]oxy]hexansäure

wurde durch Umsetzung von 6-[[1-(2-Naphtyl)-2-phenyl-1*H*-benzimidazol-6-yl]oxy]hexansäuremethylester gemäß der allgemeinen Arbeitsvorschrift 9 erhalten.
Fp. 170-175°C

### Beispiel 74

### 6-[[1-(2-Fluorenyl)-2-phenyl-1H-benzimidazol-6-yl]oxy]hexansäuremethylester

### a) 3-(2-Fluorenylamino)-4-nitrophenol

2.17 g 3-Fluor-4-nitrophenol und 5 g 2-Aminofluoren wurden vermischt und für 9 h bei 140°C gerührt. Das Rohgemisch wurde in Ethylacetat und Wasser aufgenommen und mit 1 N wäßriger Salzsäure gewaschen. Die wäßrige Phase wurde mit Ethylacetat extrahiert und die vereinigten organischen Phasen wurden dreimal mit 2 N wäßriger Salzsäure gewaschen und einmal mit ges. Natriumchloridlösung gewaschen, über Natriumsulfat getrocknet und im Vakuum eingeengt. Der Rückstand wurde an Kieselgel chromatographiert.
¹H-NMR (D₆-DMSO): δ = 3.96 ppm s (2H); 6.30 dd (J = 10, 2 Hz, 1H); 6.52 d (J = 2 Hz, 1H); 7.28-7.45 m (3H); 7.57 s (breit)(1H); 7.60 d (J = 8 Hz, 1H); 7.92 d (J = 8 Hz, 1H); 7.98 d (J = 8 Hz, 1H); 8.10 d (J = 10 Hz, 1 H); 9.70 s (1 H); 10.80 s (breit)(1 H). b) 6-[3-(2-Fluorenylamino)-4-nitrophenyl]oxyhexansäuremethylester wurde durch Umsetzung von 3-(2-Fluorenylamino)-4-nitrophenol mit 6-Bromhexansäuremethylester gemäß der allgemeinen Arbeitsvorschrift 8 erhalten.
¹H-NMR (CDCl₃): δ = 1.38-1.50 ppm m (2H); 1.58-1.80 m (4H); 2.30 t (J = 7.5 Hz, 2H); 3.65 s (3H); 3.84 t (J = 7.5 Hz, 2H); 3.95 s (2H); 6.31 dd (J = 10, 2 Hz, 1 H); 6.53 d (J = 2 Hz, 1 H); 7.33 t (J = 8 Hz, 2H); 7.42 t (J = 8 Hz, 1 H); 7.47 s (1 H); 7.58 d (J = 8 Hz, 1 H); 7.80 d (J = 8 Hz, 1 H); 7.83 d (J = 8 Hz, 1 H); 8.21 d (J = 10 Hz, 1H); 9.87 s (breit)(1 H).

### 6-[[1-(2-Fluorenyl)-2-phenyl-1H-benzimidazol-6-yl]oxy]hexansäuremethylester

wurde durch Umsetzung von 6-[3-(2-Fluorenyl)amino)-4-nitrophenyl]oxyhexansäure-methylester gemäß der allgemeinen Arbeitsvorschrift 1 und anschließende Cyclisierung mit Triethylorthobenzoat gemäß der allgemeinen Arbeitsvorschrift 3 erhalten.
Fp. 125-128°C

### Beispiel 75

### 6-[[1-Phenyl-2-[3-(trifluormethyl)phenyl]-1H-benzimidazol-6-yl]oxy]hexansäure-methylester

### a) Ethyl-(3-trifluormethy)benzimidathydrochlorid

9.7 ml 3-(Trifluormethyl)benzonitril wurden in 12 ml Ethanol gelöst und die Lösung unter Kühlung im Eisbad mit HCl-Gas gesättigt. Nach 72 h wurde vom ausgefallenen Produkt abgesaugt. Das Produkt wurde mit Diethylether gewaschen.
Fp. 131-133°C (Zers.)

### b) 5-Methoxy-2-nitrophenyldiphenylamin

Eine Lösung aus 2 g 3-Fluor-4-nitroanisol in 16 ml Anilin wurde für 24 h bei 140°C gerührt. Nach dem Erkalten nahm man in Ethylacetat auf und extrahierte mit 2 N wäßriger Salzsäure. Die organische Phase wurde über Natriumsulfat getrocknet und im Vakuum eingeengt. Der Rückstand wurde an Kieselgel chromatographiert.
¹H-NMR (CDCl₃): δ = 3.72 ppm s (3H); 6.36 dd (J = 10, 2 Hz, 1H); 6.57 d (J = 2 Hz, 1H); 7.22-7.33 m (3H); 7.44 dd (J = 8, 8 Hz, 2H); 8.18 d (J = 10 Hz, 1H); 9.78 s (breit)(1 H).

### c) 4-Methoxy-N²-phenyl-o-phenylendiamin

wurde durch Umsetzung von 5-Methoxy-2-nitrophenyldiphenylamin gemäß der allgemeinen Arbeitsvorschrift 1 erhalten.
¹H-NMR (CDCl₃): δ = 3.42 ppm s (breit)(2H); 3.72 s (3H); 5.33 s (breit)(1H); 6.56 dd (J = 10, 2 Hz, 1 H); 6.76 d (J = 10 Hz, 1H); 6.79 d (J = 2 Hz, 1 H); 6.82-6.90 m (3H); 7.25 dd (J = 8, 8 Hz, 2H).

### d) 6-Methoxy-1-phenyl-2-[3-(trifluormethyl)phenyl]-1H-benzimidazol

wurde durch Umsetzung von 4-Methoxy-N²-phenyl-o-phenylendiamin mit Ethyl-(3-trifluormethy)benzimidathydrochlorid gemäß der allgemeinen Arbeitsvorschrift 4 erhalten. Fp. 138-140°C

### e) 6-Hydroxy-1-phenyl-2-[3-(trifluormethyl)phenyl]-1H-benzimidazol

wurde durch Umsetzung von 6-Methoxy-1-phenyl-2-[3-(trifluormethyl)phenyl]-1*H-*benzimidazol gemäß der allgemeinen Arbeitsvorschrift 7 erhalten.
¹H-NMR (D₆-DMSO): δ = 6.60 ppm d (J = 2 Hz, 1H); 6.99 dd (J = 10, 2 Hz, 1H); 7.50-7.89 m (10H).

### 6-[[1-Phenyl-2-[3-(trifluormethyl)phenyl]-1H-benzimidazol-6-yl]oxy]hexansäure-methylester

wurde durch Umsetzung von 6-Hydroxy-1-phenyl-2-(3-(trifluormethyl)phenyl]-1*H*-benzimidazol mit 6-Bromhexansäuremethylester gemäß der allgemeinen Arbeitsvorschrift 8 erhalten.
Fp. 68-70°C

### Beispiel 76

### 6-[[1-Phenyl-2-[3-(trifluormethyl)phenyl]-1H-benzimidazol-6-yl]oxy]hexansäure-isopropylester

wurde durch Umsetzung von 6-Hydroxy-1-phenyl-2-[3-(trifluormethyl)phenyl]-1*H*-benzimidazol mit 6-Bromhexansäureisopropylester gemäß der allgemeinen Arbeitsvorschrift 8 erhalten.
Fp. 96-98°C

### Beispiel 77

### 6-[[1-Phenyl-2-[3-(trifluormethyl)phenyl])-1H-benzimidazol-6-yl]oxy]hexansäure

wurde durch Umsetzung von 6-[[1-Phenyl-2-[3-(trifluormethyl)phenyl]-1*H*-benzimidazol-6-yl]oxy]hexansäuremethylester gemäß der allgemeinen Arbeitsvorschrift 9 erhalten. ¹H-NMR (D₆-DMSO): δ = 1.38-1.80 ppm m (6H); 2.27 t (J = 7.5 Hz, 2H); 3.98 t (J = 7.5 Hz, 2H); 6.70 d (J = 2 Hz, 1H); 7.02 dd (J = 10, 2 Hz, 1H); 7.48-7.88 m (9H); 7.77 d (J = 10 Hz, 1H); 11.94 s (breit)(1H).

### Beispiel 78

### 6-[[1-Phenyl-2-[3-(trifluormethyl)phenyl]-1H-benzimidazol-6-yl]oxy]hexan-1-ol

wurde durch Umsetzung von 6-[[1-Phenyl-2-[3-(trifluormethyl)phenyl]-1*H*-benzimidazol-6-yl]oxy]hexansäuremethylester gemäß der allgemeinen Arbeitsvorschrift 11 erhalten. ¹H-NMR (CDCl₃): δ = 1.38-1.68 ppm m (6H); 1.75-1.87 m (2H); 3.60-3.72 m (2H); 3.94 t (J = 7.5 Hz, 2H); 6.69 d (J = 2 Hz, 1 H); 6.99 dd (J = 10, 2 Hz, 1H); 7.25-7.35 m (2H); 7.40 dd (J = 8,8 Hz; 1 H); 7.50-7.61 m (4H); 7.68 d (breit)(J = 8 Hz, 1 H); 7.78 d (J = 10 Hz, 1H); 7.83 s (breit)(1H).

### Beispiel 79

### 6-[[2-(3-Chlorphenyl)-1-pheny-1H-benzimidazol-6-yl]oxy]hexansäuremethytester

### a) 2-(3-Chlorphenyl)-6-methoxy-1-phenyl-1H-benzimidazol

wurde durch Umsetzung von 4-Methoxy-N²-phenyl-o-phenylendiamin mit Ethyl-3-chlorbenzimidathydrochlorid (hergestellt nach: DeWolfe und Augustine; J. Org. Chem.; 30; 699) gemäß der allgemeinen Arbeitsvorschrift 4 erhalten.
Fp. 149-151°C

### b) 2-(3-Chlorphenyl)-6-hydroxy-1-phenyl-1H-benzimidazol

wurde durch Umsetzung von 2-(3-Chlorphenyl)-6-methoxy-1-phenyl-1H-benzimidazol gemäß der allgemeinen Arbeitsvorschrift 7 erhalten.
Fp. 199-202°C

### 6-[[2-(3-Chlorphenyl)-1-phenyl-1H-benzimidazol-6-yl]oxy]hexansäuremethylester

wurde durch Umsetzung von 2-(3-Chlorphenyl)-6-hydroxy-1-phenyl-1*H*-benzimidazol mit 6-Bromhexansäuremethylester gemäß der allgemeinen Arbeitsvorschrift 8 erhalten.
Fp. 69-72°C

### Beispiel 80

### 6-[[2-(3-Chlorphenyl)-1-phenyl-1H-benzimidazol-6-yl]oxy]hexansäureisopropylester

wurde durch Umsetzung von 2-(3-Chlorphenyl)-6-hydroxy-1-phenyl-1*H*-benzimidazol mit 6-Bromhexansäureisopropylester gemäß der allgemeinen Arbeitsvorschrift 8 erhalten.
Fp. 98-100°C

### Beispiel 81

### 6-[[2-(3-Chlorphenyl)-1-phenyl-1H-benzimidazol-6-yl]oxy]hexansäure

wurde durch Umsetzung von 6-[[2-(3-Chlorphenyl)-1-phenyl-1*H*-benzimidazol-6-yl]oxy]hexansäuremethylester gemäß der allgemeinen Arbeitsvorschrift 9 erhalten.
Fp. 137-140°C

### Beispiel 82

### 6-[[2-(3-Chlorphenyl)-1-phenyl-1H-benzimidazol-6-yl]oxy]hexan-1-ol

wurde durch Umsetzung von 6-[[2-(3-Chlorphenyl)-1-phenyl-1*H*-benzimidazol-6-yl]oxy]hexansäuremethylester gemäß der allgemeinen Arbeitsvorschrift 11 erhalten. ¹H-NMR (CDCl₃): δ = 1.40-1.70 ppm m (6H); 1.75-1.86 m (2H); 3.67 t (J = 7.5 Hz, 2H); 3.93 t (J = 7.5 Hz, 2H); 6.69 d (J = 2 Hz, 1H); 6.99 dd (J = 10, 2 Hz, 1 H); 7.20 dd (J = 8,8 Hz; 1 H); 7.26-7.38 m (4H); 7.47-7.58 m (3H); 7.60 dd (J = 2, 2 Hz, 1 H); 7.76 d (J = 10 Hz, 1 H).

### Beispiel 83

### 6-[[2-(4-Chlorphenyl)-1-phenyl-1H-benzimidazol-6-yl]oxy]hexansäuremethylester

### a) Ethyl-4-chlorbenzimidathydrochlorid

10 g 4-Chlorbenzonitril wurden in 12 ml Ethanol suspendiert und durch Zusatz von Diethylether gelöst. Unter Kühlung im Eisbad wurde mit HCl-Gas gesättigt. Nach 72 h wurde vom ausgefallenen Produkt abgesaugt. Das Produkt wurde mit Diethylether gewaschen.
Fp. 173-174°C (Zers.)

### a) 2-(4-Chlorphenyl)-6-methoxy-1-phenyl-1H-benzimidazol

wurde durch Umsetzung von 4-Methoxy-N²-phenyl-o-phenylendiamin mit Ethyl-4-chlorbenzimidathydrochlorid gemäß der allgemeinen Arbeitsvorschrift 4 erhalten.
Fp. 162-164°C

### b) 2-(4-Chlorphenyl)-6-hydroxy-1-phenyl-1H-benzimidazol

wurde durch Umsetzung von 2-(4-Chlorphenyl)-6-methoxy-1-phenyl-1*H*-benzimidazol gemäß der allgemeinen Arbeitsvorschrift 7 erhalten.
Fp. 246-250°C

### 6-[[2-(4-Chlorphenyl)-1-phenyl-1H-benzimidazol-6-yl]oxy]hexansäuremethylester

wurde durch Umsetzung von 2-(4-Chlorphenyl)-6-hydroxy-1-phenyl-1*H*-benzimidazol mit 6-Bromhexansäuremethylester gemäß der allgemeinen Arbeitsvorschrift 8 erhalten.
Fp. 86-87°C

### Beispiel 84

### 6-[[2-(4-Chlorphenyl)-1-phenyl-1H-benzimidazol-6-yl]oxy]hexansäureisopropylester

wurde durch Umsetzung von 2-(4-Chlorphenyl)-6-hydroxy-1-phenyl-1*H*-benzimidazol mit 6-Bromhexansäureisopropylester gemäß der allgemeinen Arbeitsvorschrift 8 erhalten.
Fp. 124-126°C

### Beispiel 85

### 6-[[2-(4-Chlorphenyl)-1-phenyl-1H-benzimidazol-6-yl]oxy]hexansäure

wurde durch Umsetzung von 6-[[2-(4-Chlorphenyl)-1-phenyl-1*H*-benzimidazol-6-yl]oxy]hexansäuremethylester gemäß der allgemeinen Arbeitsvorschrift 9 erhalten. ¹H-NMR (D₆-DMSO): δ = 1.35-1.48 ppm m (2H); 1.50-1.62 m (2H); 1.64-1.77 m (2H); 2.23 t (J = 7.5 Hz, 2H); 3.91 t (J = 7.5 Hz, 2H); 6.64 d (J = 2 Hz, 1H); 6.96 dd (J = 10, 2 Hz, 1 H); 7.38-7.50 m (6H); 7.52-7.65 m (3H); 7.70 d (J = 10 Hz, 1 H).

### Beispiel 86

### 6-[[2-(4-Chlorphenyl)-1-phenyl-1H-benzimidazol-6-yl]oxy]hexan-1-ol

wurde durch Umsetzung von 6-[[2-(4-Chlorphenyl)-1-phenyl-1*H*-benzimidazol-6-yl]oxy]hexansäuremethylester gemäß der allgemeinen Arbeitsvorschrift 11 erhalten.
¹H-NMR (CDCl₃): δ = 1.38-1.68 ppm m (6H); 1.74-1.85 m (2H); 3.67 t (breit)(J = 7.5 Hz, 2H); 3.94 t (J = 7.5 Hz, 2H); 6.68 d (J = 2 Hz, 1H); 6.98 dd (J = 10, 2 Hz, 1H); 7.22-7.35 m (5H); 7.47 d (J = 8 Hz; 2H); 7.49-7.59 m (2H); 7.73 d (J = 10 Hz, 1H).

### Beispiel 87

### 6-[[2-(3-Methylphenyl)-1-phenyl-1H-benzimidazol-6-yl]oxy]hexansäuremethylester

### a) 6-Methoxy-2-(3-methylphenyl)-1-phenyl-1H-benzimidazol

wurde durch Umsetzung von 4-Methoxy-N²-phenyl-o-phenylendiamin mit Ethyl-3-methylbenzimidathydrochlorid (hergestellt nach: DeWolfe und Augustine; J. Org. Chem.; 30; 699) gemäß der allgemeinen Arbeitsvorschrift 4 erhalten.
Fp. 156-158°C

### b) 6-Hydroxy-2-(3-methylphenyl)-1-phenyl-1H-benzimidazol

wurde durch Umsetzung von 6-Methoxy-2-(3-methylphenyl)-1-phenyl-1*H*-benzimidazol gemäß der allgemeinen Arbeitsvorschrift 7 erhalten.
¹H-NMR (D₆-DMSO): δ = 2.23 ppm s (3H); 6.52 d (J = 2 Hz, 1 H); 6.80 dd (J = 10, 2 Hz, 1 H); 7.18 s (breit)(3H); 7.35-7.52 m (3H); 7.50-7.63 m (4H); 9.28 s (breit)(1 H).

### 6-[[2-(3-Methylphenyl)-1-phenyl-1H-benzimidazol-6-yl]oxy]hexansäuremethylester

wurde durch Umsetzung von 6-Hydroxy-2-(3-methylphenyl)-1-phenyl-1*H*-benzimidazol mit 6-Bromhexansäuremethylester gemäß der allgemeinen Arbeitsvorschrift 8 erhalten.
Fp. 82-84°C

### Beispiel 88

### 6-[[2-(3-Methylphenyl)-1-phenyl-1H-benzimidazol-6-yl]oxy]hexansäureisopropylester

wurde durch Umsetzung von 6-Hydroxy-2-(3-methylphenyl)-1-phenyl-1*H*-benzimidazol mit 6-Bromhexansäureisopropylester gemäß der allgemeinen Arbeitsvorschrift 8 erhalten. ¹H-NMR (CDCl₃): δ = 1.22 ppm d (J = 7.5 Hz, 6H); 1.38-1.56 m (2H); 1.62-1.85 m (4H); 2.30 t (J = 7.5 Hz, 2H); 2.30 s (3H); 3.93 t (J = 7.5 Hz, 2H); 5.00 sp (J = 7.5 Hz, 1 H); 6.68 d (J = 2 Hz, 1 H); 6.95 dd (J = 10, 2 Hz, 1 H); 7.13 s (breit)(3H); 7.31 dd (J = 8, 2 Hz, 2H); 7.42-7.57 m (4H); 7.76 d (J = 10 Hz, 1H).

### Beispiel 89

### 6-[[2-(3-Methylphenyl)-1-phenyl-1H-benzimidazol-6-yl]oxy]hexansäure

wurde durch Umsetzung von 6-[[2-(3-Methylphenyl)-1-phenyl-1*H*-benzimidazol-6-yl]oxy]hexansäuremethylester gemäß der allgemeinen Arbeitsvorschrift 9 erhalten. ¹H-NMR (D₆-DMSO): δ = 1.35-1.49 ppm m (2H); 1.50-1.63 m (2H); 1.64-1.78 m (2H); 2.22 t (J = 7.5 Hz, 2H); 2.24 s (3H); 3.92 t (J = 7.5 Hz, 2H); 6.62 d (J = 2 Hz, 1 H); 6.95 dd (J = 10, 2 Hz, 1H); 7.18 s (breit)(3H); 7.37-7.42 m (3H); 7.51-7.65 m (3H); 7.67 d (J = 10 Hz, 1H); 11.90 s (breit)(1H).

### Beispiel 90

### 6-[[2-(3-Methylphenyl)-1-phenyl-1H-benzimidazol-6-yl]oxy]hexan-1-ol

wurde durch Umsetzung von 6-[[2-(3-Methylphenyl)-1-phenyl-1*H*-benzimidazol-6-yl]oxy]hexansäuremethylester gemäß der allgemeinen Arbeitsvorschrift 9 erhalten.
Fp. 92-94°C

### Beispiel 91

### 6-[[2-(4-Methylphenyl)-1-phenyl-1H-benzimidazol-6-yl]oxy]hexansäuremethylester

### a) 6-Methoxy-2-(4-methylphenyl)-1-phenyl-1H-benzimidazol

wurde durch Umsetzung von 4-Methoxy-N²-phenyl-o-phenylendiamin mit Ethyl-4-methylbenzimidathydrochlorid (hergestellt nach: DeWolfe und Augustine; J. Org. Chem.; 30; 699) gemäß der allgemeinen Arbeitsvorschrift 4 erhalten.
Fp. 150-152°C

### b) 6-Hydroxy-2-(4-methylphenyl)-1-phenyl-1H-benzimidazol

wurde durch Umsetzung von 6-Methoxy-2-(4-methylphenyl)-1-phenyl-1*H*-benzimidazol gemäß der allgemeinen Arbeitsvorschrift 7 erhalten.
Fp. 257-264°C

### 6-[[2-(4-Methylphenyl)-1-phenyl-1H-benzimidazol-6-yl]oxy]hexansäuremethylester

wurde durch Umsetzung von 6-Hydroxy-2-(4-methylphenyl)-1-phenyl-1*H*-benzimidazol mit 6-Bromhexansäuremethylester gemäß der allgemeinen Arbeitsvorschrift 8 erhalten.
Fp. 99-102°C

### Beispiel 92

### 6-[[2-(4-Methylphenyl)-1-phenyl-1H-benzimidazol-6-yl]oxy]hexansäureisopropylester

wurde durch Umsetzung von 6-Hydroxy-2-(4-methylphenyl)-1-phenyl-1*H*-benzimidazol mit 6-Bromhexansäureisopropylester gemäß der allgemeinen Arbeitsvorschrift 8 erhalten.
Fp. 107-109°C

### Beispiel 93

### 6-[[2-(4-Methylphenyl)-1-phenyl-1H-benzimidazol-6-yl)oxy]hexansäure

wurde durch Umsetzung von 6-[[2-(4-Methylphenyl)-1-phenyl-1*H*-benzimidazol-6-yl]oxy]hexansäuremethylester gemäß der allgemeinen Arbeitsvorschrift 9 erhalten. ¹H-NMR (D₆-DMSO): δ = 1.33-1.49 ppm m (2H); 1.50-1.62 m (2H); 1.64-1.77 m (2H); 2.22 t (J = 7.5 Hz, 2H); 2.30 s (3H); 3.90 t (J = 7.5 Hz, 2H); 6.62 d (J = 2 Hz, 1 H); 6.94 dd (J = 10, 2 Hz, 1 H); 7.15 d (J = 8 Hz, 2H); 7.36 d (J = 8 Hz, 2H); 7.40 dd (J = 8, 1.5 Hz, 2H); 7.52-7.62 m (3H); 7.68 d (J = 10 Hz, 1 H).

### Beispiel 94

### 6-[[2-(4-Methylphenyl)-1-phenyl-1H-benzimidazol-6-yl]oxy]hexan-1-ol

wurde durch Umsetzung von 6-[[2-(4-Methylphenyl)-1-phenyl-1*H*-benzimidazol-6-yl]oxy]hexansäuremethylester gemäß der allgemeinen Arbeitsvorschrift 11 erhalten.
Fp. 150-152°C

### Beispiel 95

### 6-[[1-Phenyl-2-(4-pyridinyl)-1H-benzimldazol-6-yl]oxy]hexansäuremethylester

### a) 6-Methoxy-1-phenyl-2-(4-pyridinyl)-1H-benzimidazol

0.4 g 4-Methoxy-N²-phenyl-o-phenylendiamin wurden in 8 ml *N*,*N*-Dimethylformamid gelöst und die Lösung mit 0.7 g Ethyl-2-ethoxy-1,2-dihydrochinolin-1-carboxylat und 0.34 g Isonicotinsäure versetzt. Man rührte für 16 h bei 100°C, versetzte nach dem Abkühlen mit Wasser, extrahierte dreimal mit Ethylacetat, wusch die vereinigten organischen Phasen mit ges. Natriumchloridlösung, trocknete über Natriumsulfat und engte im Vakuum ein. Nach chromatographischer Reinigung an Kieselgel nahm man das Amid in 5 ml 6 N wäßriger Salzsäure auf und erhitzte für 3 h zum Rückfluß. Nach dem Abkühlen rührte man in ges. Natriumhydrogencarbonatlösung ein, extrahierte dreimal mit Ethylacetat, wusch die vereinigten Extrakte mit ges. Natriumchloridlösung, trocknete über Natriumsulfat und engte im Vakuum ein.
¹H-NMR (CDCl₃): δ = 3.80 ppm s (3H); 6.66 ppm d (J = 2 Hz, 1H); 7.02 dd (J = 10, 2 Hz, 1 H); 7.32-7.38 m (2H); 7.42 dd (J = 8, 1.5 Hz, 2H); 7.54-7.62 m (3H); 7.79 d (J = 10 Hz, 1 H); 8.53 d (breit)(J = 6 Hz, 2H).

### b) 6-Hydroxy-1-phenyl-2-(4-pyridinyl)-1H-benzimidazol

wurde durch Umsetzung von 6-Methoxy-1-phenyl-2-(4-pyridinyl)-1*H*-benzimidazol gemäß der allgemeinen Arbeitsvorschrift 7 dargestellt.
¹H-NMR (CD₃OD): δ = 6.52 ppm d (J = 2 Hz, 1H); 6.82 dd (J = 10, 2 Hz, 1 H); 7.28-7.33 m (2H); 7.39 dd (J = 8, 1.5 Hz, 2H); 7.49-7.57 m (4H); 8.40 d (breit)(J = 6 Hz, 2H).

### 6-[[1-Phenyl-2-(4-pyridinyl)-1H-benzimidazol-6-yl]oxy]hexansäuremethylester

wurde durch Umsetzung von 6-Hydroxy-1-phenyl-2-(4-pyridinyl)-1*H*-benzimidazol gemäß der allgemeinen Arbeitsvorschrift 8 dargestellt.
Fp. 100-103°C

### Beispiel 96

### 6-[[-Phenyl-2-(4-pyridinyl)-1H-benzimidazol-6-yl]oxy]hexansäure

wurde durch Umsetzung von 6-[[1-Phenyl-2-(4-pyridinyl)-1*H*-benzimidazol-6-yl]oxy]hexansäuremethylester gemäß der allgemeinen Arbeitsvorschrift 9 dargestellt.
Fp. 160-162°C

### Beispiel 97

### 6-[(1,2-Diphenyl-5-nitro-1H-benzimidazol-6-yl)oxy]hexansäuremethylester

### a) 1,2-Diphenyl-6-hydroxy-5-nitro-1H-benzimidazol

### b) 1,2-Diphenyl-6-hydroxy-7-nitro-1H-benzimidazol

### c) 1,2-Diphenyl-6-hydroxy-5,7-dinitro-1H-benzimidazol

**5 g 1,2-Diphenyl-6-hydroxy-1*H*-benzimidazol wurden in 45 ml Eisessig gelöst und bei 10-15°C tropfenweise mit einer Lösung aus 1.67 g Kaliumnitrit in 15 ml Wasser versetzt. Man ließ 2 h im Eisbad und dann 2 h bei 20°C rühren, engte das Reaktionsgemisch im Vakuum ein und reinigte durch Chromatographie an Kieselgel.**
a) ¹H-NMR (CDCl₃): δ = 6.83 ppm s (1H); 7.25-7.44 m (5H); 7.52-7.60 m (5H); 8.66 s (1H); 10.78 s (1H).
b) ¹H-NMR (D₆-DMSO): δ = 7.05 ppm d (J = 10Hz, 1H); 7.30-7.53 m (10H); 7.82 d (J = 10Hz, 1 H); 10.83 s (1 H).
c) **¹H-NMR (D₆-DMSO): δ = 7.32-7.58 ppm m (10H); 8.67 s (1H).**

### 6-[(1,2-Diphenyl-5-nitro-1H-benzimidazol-6-yl)oxy] hexansäuremethylester

wurde durch Umsetzung von 1,2-Diphenyl-6-hydroxy-5-nitro-1*H*-benzimidazol mit 6-Bromhexansäuremethylester gemäß der allgemeinen Arbeitsvorschrift 8 erhalten.
Fp. 123°C

### Beispiel 98

### 6-[(1,2-Diphenyl-5-nitro-1H-benzimidazol-6-yl)oxy]hexansäureisopropylester

wurde durch Umsetzung von 1,2-Diphenyl-6-hydroxy-5-nitro-1*H*-benzimidazol mit 6-Bromhexansäureisopropylester gemäß der allgemeinen Arbeitsvorschrift 8 erhalten.
Fp. 115-117°C

### Beispiel 99

### 6-[(1,2-Diphenyl-7-nitro-1H-benzimidazol-6-yl)oxy]hexansäuremethylester

wurde durch Umsetzung von 1,2-Diphenyl-6-hydroxy-7-nitro-1*H*-benzimidazol mit 6-Bromhexansäuremethylester gemäß der allgemeinen Arbeitsvorschrift 8 erhalten.
Fp. 110-112°C

### Beispiel 100

### 6-[(1,2-Diphenyl-7-nitro-1H-benzimidazol-6-yl)oxy]hexansäureisopropylester

wurde durch Umsetzung von 1,2-Diphenyl-6-hydroxy-5-nitro-1*H*-benzimidazol mit 6-Bromhexansäureisopropylester gemäß der allgemeinen Arbeitsvorschrift 8 erhalten.
Fp. 88°C

### Beispiel 101

### 6-[(7-Amino-1,2-diphenyl-1H-benzimidazol-6-yl)oxy]hexansäuremethylester

340 mg 6-[(1,2-Diphenyl-7-nitro-1*H*-benzimidazol-6-yl)oxy]hexansäuremethylester wurden in Ethanol mit Raneynickel in einem Autoklaven bei 50°C und bei normalem Druck hydriert. Nach beendeter Wasserstoffaufnahme wurde vom Katalysator abfiltriert und im Vakuum eingeengt.
Fp. 113-115°C

### Beispiel 102

### 6-[(7-Amino-1,2-diphenyl-1H-benzimidazol-6-yl)oxy]hexansäureisopropylester

erhielt man analog zu der in Beispiel 101 angegebenen Vorschrift durch Umsetzung von 6-[(1,2-Diphenyl-7-nitro-1*H*-benzimidazol-6-yl)oxy]hexansäureisopropylester.
¹H-NMR (CDCl₃): δ = 1.22 ppm d (J = 7.5 Hz, 6H); 1.43-1.88 m (6H); 2.30 t (J = 7.5 Hz, 2H); 4.04 t (J = 7.5 Hz, 2H); 5.00 sp (J = 7.5 Hz, 1 H); 6.97 d (J = 7.5 Hz, 1H); 7.20-7.33 m (4H); 7.42-7.53m (7H).

### Beispiel 103

### 6-[(5,7-Dinitro-1,2-diphenyl-1H-benzimidazol-6-yl)oxy]hexansäuremethylester

wurde durch Umsetzung von 5,7-Dinitro-1,2-diphenyl-6-hydroxy-1*H*-benzimidazol mit 6-Bromhexansäuremethylester gemäß der allgemeinen Arbeitsvorschrift 8 erhalten.
Fp. 88-91 °C

### Beispiel 104

### 6-[(5,7-Dinitro-1,2-diphenyl-1H-benzimidazol-6-yl)oxy]hexansäureisopropylester

wurde durch Umsetzung von 5,7-Dinitro-1,2-diphenyl-6-hydroxy-1*H*-benzimidazol mit 6-Bromhexansäureisopropylester gemäß der allgemeinen Arbeitsvorschrift 8 erhalten.
Fp. 92-93°C

### Beispiel 105

### 6-[[5-(Acetylamino)-1,2-diphenyl-1H-benzimidazol-6-yl]oxy]hexansäuremethylester

### a) 5-Fluor-2,4-dinitrophenol

0.41 g 1,3-Difluor-4,6-dinitrobenzol wurden in 8 ml 0.5 N wäßriger Natronlauge gelöst und für 2 h zum Rückfluß erhitzt. Nach dem Erkalten wurde mit Wasser verdünnt und dreimal mit Diethylether extrahiert. Die wäßrige Phase wurde durch Zusatz von 1 N Salzsäure sauer gestellt und mit Diethylether extrahiert. Die organische Phase wurde über Natriumsulfat getrocknet und im Vakuum eingeengt.
¹H-NMR (CDCl₃): δ = 7.10 ppm d (J = 12 Hz, 1H); 9.03 d (J = 8 Hz. 1H); 11.10 s (1H).

### b) 2,4-Dinitro-5-hydroxydiphenylamin

Zur Suspension aus 50 mg 5-Fluor-2,4-dinitrophenol in 0.5 ml Ethanol gab man 100 µl Anilin, rührte für 30 min und ließ dann für 15 h stehen. Man saugte ab, wusch den Feststoff mit 1 N wäßriger Salzsäure und trocknete im Vakuum.
¹H-NMR (CDCl₃): δ = 6.58 ppm s (1H); 7.31 d (J = 10 Hz, 2H); 7.39 dd (J = 10, 10 Hz, 1H); 7.51 dd (J = 10,10 Hz, 2H); 9.20 s (1H); 9.90 s (breit)(1H); 10.97 s (breit)(1H).

### c) Essigsäure-(2,4-dinitro-5-phenylamino)phenylester

Zu 275 mg 2,4-Dinitro-5-hydroxydiphenylamin in 1 ml Pyridin gab man 0.11 ml
Essigsäureanhydrid und ließ 30 min im Eisbad und dann noch 1h bei 20°C rühren. Nach Verdünnen mit Ethylacetat wurde dreimal mit eiskalter 1 N wäßriger Salzsäure, einmal mit ges. Kaliumhydrogencarbonatlösung und einmal mit ges. Natriumchloridlösung gewaschen, über Natriumsulfat getrocknet und im Vakuum eingeengt.
¹H-NMR (CDCl₃): δ = 2.34 ppm s (3H); 6.80 s (1H); 7.32 d (J = 10 Hz, 2H); 7.40 dd (J = 10, 10 Hz, 1H); 7.52 dd (J = 10,10 Hz, 2H); 9.21 s (1H); 9.95 s (breit)(1H).

### d) Essigsäure-(1,2-diphenyl-6-hydroxy-1H-benzimidazol-5-yl)amid

wurde durch Umsetzung von Essigsäure-(2,4-dinitro-5-phenylamino)phenylester gemäß der allgemeinen Arbeitsvorschrift 1 und nachfolgende Umsetzung mit Trimethyorthobenzoat gemäß der allgemeinen Arbeitsvorschrift 3 erhalten.
¹H-NMR (CDCl₃): δ = 2.26 ppm s (3H); 6.88 s (1H); 7.22-7.36 m (5H); 7.42-7.53 m (5H); 7.61 s (1H); 8.43 s (breit)(1H).

### 6-[[5-(Acetylamino)-1,2-diphenyl-1H-benzimldazol-6-yl]oxy]hexansäuremethylester

wurde durch Umsetzung von Essigsäure-(1,2-diphenyl-6-hydroxy-1*H*-benzimidazol-5-yl)amid mit 6-Bromhexansäuremethylester gemäß der allgemeinen Arbeitsvorschrift 8 erhalten.
Fp. 128-130°C

### Beispiel 106

6-[(5-Amino-1,2-diphenyl-1*H*-benzimidazol-6-yl)oxy]hexansäureisopropylester wurde durch Umsetzung von 6-[(1,2-Diphenyl-5-nitro-1*H*-benzimidazol-6-yl)oxy]hexansäureisopropylester gemäß der allgemeinen Arbeitsvorschrift 1 erhalten.
¹H-NMR (CDCl₃): δ = 1.23 ppm d (J = 7.5 Hz, 6H); 1.47-1.90 m (6H); 2.32 t (J = 7.5 Hz, 2H); 3.95 t (J = 7.5 Hz, 2H); 5.02 sp (J = 7.5 Hz, 1H); 6.60 s (1H); 7.20 s (1 H); 7.22-7.33 m (5H); 7.43-7.58m (5H).

### Beispiel 107

### 6-[[5-[[(4-Bromphenyl)sulfonyl]amino]-1,2-diphenyl-1H-benzimidazol-6-yl]oxy]hexan-säureisopropylester

6-[(5-Amino-1,2-diphenyl-1*H*-benzimidazol-6-yl)oxy]hexansäureisopropylester wurde gemäß der allgemeinen Arbeitsvorschrift 13 mit 4-Brombenzolsulfonsäurechlorid umgesetzt.
Fp. 173-175°C

### Beispiel 108

### 6-[(5-Amino-1,2-diphenyl-1H-benzimidazol-6-yl)oxy]hexansäuremethylester

wurde durch Umsetzung von 6-[(1,2-Diphenyl-5-nitro-1*H*-benzimidazol-6-yl)oxy]hexansäuremethylester gemäß der allgemeinen Arbeitsvorschrift 1 erhalten.
¹H-NMR (CDCl₃): δ = 1.48-1.88 ppm (6H); 2.36 t (J = 7.5 Hz, 2H, CH₂=CO); 3.67 s (3H); 3.94 t (J = 7.5 Hz, 2H); 6.60 s (1H); 7.21 s (1H); 7.22-7.35 m (5H); 7.43-7.59m (5H).

### Beispiel 109

### 6-[[5-[[(4-Chlorphenyl)sulfonyl]amino]-1,2-diphenyl-1H-benzimldazol-6-yl]oxy]hexan-säuremethylester

6-[(5-Amino-1,2-diphenyl-1*H*-benzimidazol-6-yl)oxy]hexansäuremethylester wurde gemäß der allgemeinen Arbeitsvorschrift 13 mit 4-Chlorbenzolsulfonsäurechlorid umgesetzt.
Fp. 157-159°C

### Beispiel 110

### 6-[[5-[[(4-Chlorphenyl)sulfonyl]amino]-1,2-diphenyl-1H-benzimidazol-6-yl]oxy]hexan-säureisopropylester

6-[(5-Amino-1,2-diphenyl-1*H*-benzimidazol-6-yl)oxy]hexansäureisopropylester wurde gemäß der allgemeinen Arbeitsvorschrift 13 mit 4-Chlorbenzolsulfonsäurechlorid umgesetzt Fp. 158-159°C

### Beispiel 111

### 6-[[5-[[(4-Chlorphenyl)sulfonyl]amino]-1,2-diphenyl-1H-benzimidazol-6-yl]oxy]hexan-säure

wurde durch Umsetzung von 6-[[5-[[(4-Chlorphenyl)sulfonyl]amino]-1,2-diphenyl-1*H-*benzimidazol-6-yl]oxy]hexansäuremethylester gemäß der allgemeinen Arbeitsvorschrift 9 erhalten.
Fp. 201-203°C

### Beispiel 112

### 6-[[1,2-Diphenyl-5-[[(3-methylphenyl)sulfonyl]amino]-1H-benzimidazol-6-yl]oxy]-hexansäureisopropylester

6-[(5-Amino-1,2-diphenyl-1*H*-benzimidazol-6-yl)oxy]hexansäureisopropylester wurde gemäß der allgemeinen Arbeitsvorschrift 13 mit 3-Methylbenzolsulfonsäurechlorid umgesetzt.
Fp. 149-151°C

### Beispiel 113

### 6-[[1,2-Diphenyl-5-[[(4-methylphenyl)sulfonyl]amino]-1H-benzimidazol-6-yl]oxy]-hexansäureisopropylester

6-[(5-Amino-1,2-diphenyl-1*H*-benzimidazol-6-yl)oxy]hexansäureisopropylester wurde gemäß der allgemeinen Arbeitsvorschrift 13 mit 4-Methylbenzolsulfonsäurechlorid umgesetzt.
Fp. 139-141°C

### Beispiel 114

### 6-[[1,2-Diphenyl-5-[[(4-methoxyphenyl)sulfonyl]amino]-1H-benzimidazol-6-yl]oxy]-hexansäureisopropylester

6-[(5-Amino-1,2-diphenyl-1*H*-benzimidazol-6-yl)oxy]hexansäureisopropylester wurde gemäß der allgemeinen Arbeitsvorschrift 13 mit 4-Methoxybenzolsulfonsäurechlorid umgesetzt.
¹H-NMR (CDCl₃): δ = 1.25 ppm d (J = 7.5 Hz, 6H); 1.35-1.45 m (2H); 1.59-1.73 m (4H); 2.30 t (J = 7.5 Hz, 2H); 3.72 t (J = 7.5 Hz, 2H); 3.80 s (3H); 5.02 sp (J = 7.5 Hz, 1 H); 6.50 s (1H); 6.85 d (J = 10 Hz, 2H); 6.99 s (1H); 7.25-7.35 m (5H); 7.45-7.52 m (5H); 7.74 d (J = 10 Hz, 2H); 7.99 s (1H).

### Beispiel 115

### 6-[[1,2-Diphenyl-5-[[[(4-trifluormethyl]phenyl]sulfonyl]amino]-1H-benzimidazol-6-yl]oxy]hexansäureisopropylester

6-[(5-Amino-1,2-diphenyl-1*H*-benzimidazol-6-yl)oxy]hexansäureisopropylester wurde gemäß der allgemeinen Arbeitsvorschrift 13 mit 4-(Trifluormethyl)benzolsulfonsäurechlorid umgesetzt.
Fp. 170-171°C

### Beispiel 116

### 6-[[5-[[[4-(Acetylamino)phenyl]sulfonyl]amino]-1,2-diphenyl-1H-benzimidazol-6-yl]oxy]hexansäureisopropylester

6-[(5-Amino-1,2-diphenyl-1*H*-benzimidazol-6-yl)oxy]hexansäureisopropylester wurde gemäß der allgemeinen Arbeitsvorschrift 13 mit 4-(Acetylamino)benzolsulfonsäurechlorid umgesetzt.
Fp. 100-102°C

### Beispiel 117

### 6-[[5-[[Bis(3-chlorphenyl)sulfonyl]amino]-1,2-diphenyl-1H-benzimidazol-6-yl]oxy]-hexansäureisopropylester

6-[(5-Amino-1,2-diphenyl-1*H*-benzimidazol-6-yl)oxy]hexansäureisopropylester wurde gemäß der allgemeinen Arbeitsvorschrift 13 mit 3-Chlorbenzolsulfonsäurechlorid umgesetzt.
Fp. 163-167°C

### Beispiel 118

### 6-[[1,2-Diphenyl-5-[(propylsulfonyl)amino]-1H-benzimidazol-6-yl]oxy]hexansäure-isopropylester

6-[(5-Amino-1,2-diphenyl-1*H*-benzimidazol-6-yl)oxy]hexansäureisopropylester wurde gemäß der allgemeinen Arbeitsvorschrift 13 mit Propansulfonsäurechlorid umgesetzt.
Fp. 126-128°C

### Beispiel 119

### 6-[[5-[(Benzylsulfonyl)amino]-1,2-diphenyl-1H-benzimidazol-6-yl]oxy]hexansäure-isopropylester

6-[(5-Amino-1,2-diphenyl-1*H*-benzimidazol-6-yl)oxy]hexansäureisopropylester wurde gemäß der allgemeinen Arbeitsvorschrift 13 mit Benzolmethansulfonsäurechlorid umgesetzt.
Fp. 137-138°C

### Beispiel 120

### 4-[(1,2-Diphenyl-1H-benzimidazol-6-yl)oxy]methylbenzoesäuremethylester

wurde duch Umsetzung von 1,2-Diphenyl-6-hydroxy-1*H*-benzimidazol mit 4-(Brommethyl)-benzoesäuremethylester gemäß der allgemeinen Arbeitsvorschrift 8 dargestellt.
Fp. 180-184°C

### Beispiel 121

### 4-[(1,2-Diphenyl-1H-benzimidazol-6-yl)oxy]methylbenzoesäure

wurde durch Umsetzung von 4-[(1,2-Diphenyl-1*H*-benzimidazol-6-yl)oxy]methylbenzoe-säuremethylester gemäß der allgemeinen Arbeitsvorschrift 9 dargestellt.
¹H-NMR (D₆-DMSO): δ = 5.12 ppm s (2H); 6.76 d (J = 2 Hz, 1H); 7.04 dd (J = 10, 2 Hz, 1H); 7.30-7.63 m (12H); 7.70 d (J = 10 Hz, 1H); 7.89 d (J = 8 Hz, 2H).

### Beispiel 122

### 4-[[1-(3-Methylphenyl)-2-phenyl-1H-benzimidazol-6-yl]oxy]methylbenzoe-säuremethylester

wurde durch Umsetzung von 6-Hydroxy-1-(3-methylphenyl)-2-phenyl-1*H*-benzimidazol mit 4-(Brommethyl)benzoesäuremethylester gemäß der allgemeinen Arbeitsvorschrift 8 dargestellt.
Fp. 138-142°C

### Beispiel 123

### 4-[[1-(4-Methylphenyl)-2-phenyl-1H-benzimidazol-6-yl]oxy]methylbenzoe-säuremethylester

wurde durch Umsetzung von 6-Hydroxy-1-(4-methylphenyl)-2-phenyl-1*H*-benzimidazol mit 4-(Brommethyl)benzoesäuremethylester gemäß der allgemeinen Arbeitsvorschrift 8 dargestellt.
Fp. 145-148°C

### Beispiel 124

### 2-[2-[(1,2-Diphenyl-1H-benzimidazol-6-yl)oxy]ethoxy]essigsäure-tert-butylester

0.2 g [(1,2-Diphenyl-1*H*-benzimidazol-6-yl)oxy]ethan-1-ol wurden in 1.7 ml Toluol und 0.7 ml Tetrahydrofuran suspendiert. Dazu gab man 0.1 ml Bromessigsäure-*tert*-butylester und 13 mg Tetrabutylammoniumhydrogensulfat und 1.45 ml 32%ige Natronlauge und ließ für 48 h rühren. Man gab weitere 0.1 ml Bromessigsäure-*tert* butylester und 13 mg Tetrabutylammoniumhydrogensulfat zu und ließ das Gemisch für 48 h im Ultraschallbad. Anschließend wurde mit Wasser verdünnt und dreimal mit Toluol extrahiert. Die vereinigten organischen Phasen wurden mit Wasser und ges. Natriumchloridlösung gewaschen, über Natriumsulfat getrocknet und im Vakuum eingeengt. Der Rückstand wurde an Kieselgel chromatographiert.
¹H-NMR (CDCl₃): δ = 1.43 ppm s (9H); 3.91 t (J = 6 Hz, 2H); 4.10 s (2H); 4.17 t (J = 6 Hz, 2H); 6.75 d (J = 2 Hz, 1H); 7.00 dd (J = 10, 2 Hz, 1H); 7.24-7.36 m (5H); 7.45-7.56 m (5H); 7.76 d (J = 10 Hz, 1 H).

### Beispiel 125

### 2-[2-[(1,2-Diphenyl-1H-benzimidazol-6-yl)oxy]ethoxy]essigsäure

*5*0 mg 2-[2-[(1,2-Diphenyl-1*H*-benzimidazol-6-yl)oxy]ethoxy]essigsäure-*tert*-butylester wurden in 0.5 ml Trifluoressigsäure gelöst und für 48 h gerührt. Anschließend wurde mit Wasser verdünnt und dreimal mit Ethylacetat extrahiert. Die vereinigten organischen Phasen wurden mit ges. Natriumchloridlösung gewaschen, über Natriumsulfat getrocknet und im Vakuum eingeengt. Der Rückstand wurde an Kieselgel chromatographiert.
Fp. 134-136°C

### Beispiel 126

### 2-[2-[(1,2-Diphenyl-1H-benzimidazol-6-yl)oxy]ethoxy]essigsäuremethylester

35 mg 2-[2-[(1,2-Diphenyl-1*H*-benzimidazol-6-yl)oxy]ethoxy]essigsäure wurden in 0.4 ml *N,N*-Dimethylformamid gelöst und mit 29 mg Caesiumcarbonat, und 50 µl Methyljodid versetzt. Man rührte für 20 h engte dann im Vakuum ein und chromtographierte an Kieselgel.
¹H-NMR (CDCl₃): δ = 3.73 ppm s (3H); 3.93 t (J = 6 Hz, 2H); 4.18 t (J = 6 Hz, 2H); 4.25 s (2H); 6.73 d (J = 2 Hz, 1H); 7.00 dd (J = 10, 2 Hz, 1 H); 7.25-7.42 m (5H); 7.46-7.58 m (5H); 7.77 d (J = 10Hz, 1H).

### Beispiel 127

### 3-[2-[(1,2-Diphenyl-1H-benzimidazol-6-yl)oxy]ethoxy]propansäure-tert-butylester

0.2 g [(1,2-Diphenyl-1*H*-benzimidazol-6-yl)oxy]ethan-1-ol wurden in 1.7 ml Toluol und 0.7 ml Tetrahydrofuran suspendiert. Dazu gab man 60 µl Acrylsäure-*tert*-butylester, 13 mg Tetrabutylammoniumhydrogensulfat und 1.45 ml 32%ige Natronlauge und ließ für 48 h rühren. Man gab weitere 60 µl Acrylsäure-*tert*-butylester und 13 mg Tetrabutylammoniumhydrogensulfat zu und ließ das Gemisch für 48 h im Ultraschallbad. Anschließend wurde mit Wasser verdünnt und dreimal mit Toluol extrahiert. Die vereinigten organischen Phasen wurden mit Wasser und ges. Natriumchloridlösung gewaschen, über Natriumsulfat getrocknet und im Vakuum eingeengt. Der Rückstand wurde an Kieselgel chromatographiert.
¹H-NMR (CDCl₃): δ = 1.45 ppm s (9H); 2.52 t (J = 8 Hz, 2H); 3.73-3.84 m (4H); 4.10 t (J = 6 Hz, 2H); 6.72 d (J = 2 Hz, 1 H); 6.99 dd (J = 10, 2 Hz, 1 H); 7.22-7.38 m (5H); 7.45-7.57 m (5H); 7.75 d (J = 10 Hz, 1 H).

### Beispiel 128

### 3-[2-[(1,2-Diphenyl-1H-benzimidazol-6-yl)oxy]ethoxy]propansäure

50 mg 3-[2-[(1-2-Diphenyl-1*H*-benzimidazol-6-yl)oxy]ethoxy]propansäure-*tert*-butylester wurden in 0.5 ml Trifluoressigsure gelöst und für 15 h gerührt. Anschließend wurde mit Wasser verdünnt und dreimal mit Ethylacetat extrahiert. Die vereinigten organischen Phasen wurden mit ges. Natriumchloridlösung gewaschen, über Natriumsulfat getrocknet und im Vakuum eingeengt. Der Rückstand wurde an Kieselgel chromatographiert. ¹H-NMR (D₆-DMSO): δ = 2.26 ppm t (J = 8 Hz, 2H); 3.60-3.70 m (4H); 3.98-4.06 m (2H); 6.65 d (J = 2 Hz, 1 H); 6.94 dd (J = 10, 2 Hz, 1H); 7.30-7.62 m (10H); 7.68 d (J = 10 Hz, 1H).

### Beispiel 129

### 3-[2-[(1,2-Diphenyl-1H-benzimidazol-6-yl)oxy]ethoxy]propansäuremethylester

35 mg 3-[2-[(1,2-Diphenyl-1*H*-benzimidazol-6-yl)oxy]ethoxy]propansäure wurden in 0.4 ml *N,N*-Dimethylformamid gelöst, mit 28 mg Caesiumcarbonat, und 50 µl Methyljodid versetzt und für 30 h gerührt. Anschließend wurde mit Wasser verdünnt und dreimal mit Ethylacetat extrahiert. Die vereinigten organischen Phasen wurden mit ges. Natriumchloridlösung gewaschen, über Natriumsulfat getrocknet und im Vakuum eingeengt. Der Rückstand wurde an Kieselgel chromatographiert.
Fp. 91-93°C

### Beispiel 130

### 3-[3-[(1,2-Diphenyl-1H-benzimidazol-6-yl)oxy]propoxy]propansäure-tert-butylester

0.2 g 3-[(1,2-Diphenyl-1*H*-benzimidazol-6-yl)oxy]propan-1-ol wurden in 1.7 ml Toluol und 0.7 ml Tetrahydrofuran suspendiert. Dazu gab man 60 µl Acrylsäure-*tert*-butylester, 13 mg Tetrabutylammoniumhydrogensulfat und 1.47 ml 32%ige Natronlauge und ließ für 48 h rühren. Man gab weitere 60 µl Acrylsäure-*tert*-butylester und 13 mg Tetrabutylammoniumhydrogensulfat zu und ließ das Gemisch für 48 h im Ultraschallbad. Anschließend wurde mit Wasser verdünnt und dreimal mit Toluol extrahiert. Die vereinigten organischen Phasen wurden mit Wasser und ges. Natriumchloridlösung gewaschen, über Natriumsulfat getrocknet und im Vakuum eingeengt. Der Rückstand wurde an Kieselgel chromatographiert.
Fp. 95-98°C

### Beispiel 131

### (E/Z)-5-(1,2-Diphenyl-1H-benzimidazol-6-yl)pent-4-ensäuremethylester

### a) 1,2-Diphenyl-6-methyl-1H-benzimldazol

5.1 g 5-Methyl-2-nitrodiphenylamin wurden in 55 ml Ethanol gemäß der allgemeinen Arbeitsvorschrift 1 hydriert. Das Rohprodukt wurde gemäß der allgemeinen Arbeitsvorschrift 3 mit Trimethylorthobenzoat umgesetzt.
Fp. 134-136°C

### b) 1,2-Diphenyl-1H-benzimidazol-6-carbaldehyd

1 g 1,2-Diphenyl-6-methyl-1*H*-benzimidazol wurden in 31 ml 40%iger Schwefelsäure suspendiert und mit 13.5 g Cerammoniumnitrat versetzt. Man ließ 2.5 h bei 80°C rühren, kühlte auf 20°C ab und rührte vorsichtig in ges. wäßrige Natriumhydrogencarbonatlösung ein. Das Gemisch wurde dreimal mit Ethylacetat extrahiert, die vereinigten Extrakte wurden mit ges. wäßriger Natriumchloridlösung gewaschen, über Natriumsulfatlösung getrocknet und im Vakuum zur Trockene eingeengt. Der Rückstand wurde an Kieselgel chromatographiert.
¹H-NMR (CDCl₃): δ = 7.30-7.42 ppm m (5H); 7.50-7.66 m (5H); 7.81 d (J = 2 Hz, 1H); 7.89 dd (J = 8, 2 Hz, 1H); 8.00 d (J = 8 Hz, 1 H); 10.05 s (1 H).

### (E/Z)-5-(1,2-Diphenyl-1H-benzimidazol-6-yl)pent-4-ensäuremethylester

wurde durch Umsetzung von 1,2-Diphenyl-1*H*-benzimidazol-6-carbaldehyd gemäß der allgemeinen Arbeitsvorschrift 12 mit 3-Carboxypropyltriphenylphosphoniumbromid erhalten. ¹H-NMR (CDCl₃): δ = 2.40-2.71 ppm m (4H); 3.68 (3.66) je s (3H); 5.56-5.64 (6.12-6.22) je m (1H); 6.50 d (J = 18 Hz, 1 H); 6.58 d (breit) (J = 12 Hz, 1 H); 7.12 (7.15) je s (breit)(1H); 7.25-7.40 m (6H); 7.45-7.62 m (5H); 7.80 (7.83) je d (J = 8 Hz, 1 H).

### Beispiel 132

### E-5-(1,2-Diphenyl-1H-benzimidazol-6-yl)pent-4-ensäure

wurde durch Umsetzung von (E/Z)-5-(1,2-Diphenyl-1*H*-benzimidazol-6-yl)pent-4-ensäuremethylester gemäß der allgemeinen Arbeitsvorschrift 9 erhalten.
¹H-NMR (CD₃OD): δ = 2.26-2.43 ppm m (4H); 6.10-6.21 m (1H); 6.45 d (J = 18 Hz, 1 H); 7.08 s (1H); 7.22-7.52 m (11 H); 7. 59 d (J = 8 Hz, 1H).

### Beispiel 133

### 5-(1,2-Diphenyl-1H-benzimidazol-6-yl)pentansäuremethylester

wurde durch Umsetzung von (E/Z)-5-(1,2-Diphenyl-1*H*-benzimidazol-6-yl)pent-4-ensäuremethylester gemäß der allgemeinen Arbeitsvorschrift 1 erhalten.
¹H-NMR (CDCl₃): δ = 1.63-1.72 ppm m (4H); 2.30-2.39 m (2H); 2.68-2.77 m (2H); 3.65 s (3H); 7.04 s (breit)(1H); 7.17 dd (J = 8, 2 Hz, 1H); 7.25-7.38 m (5H); 7.45-7.60 m (5H); 7.79 d (J = 8 Hz, 1H).

### Beispiel 134 5-(1,2-Diphenyl-1H-benzimidazol-6-yl)pentansäure

wurde durch Umsetzung von 5-(1,2-Diphenyl-1*H*-benzimidazol-6-yl)pentansäuremethylester gemäß der allgemeinen Arbeitsvorschrift 9 erhalten.
Fp. 192-193°C

### Beispiel 135 (E/Z)-6-(1,2-Diphenyl-1H-benzimidazol-6-yl)hex-5-ensäuremethylester

wurde durch Umsetzung von 1,2-Diphenyl-1*H*-benzimidazol-6-carbaldehyd gemäß der allgemeinen Arbeitsvorschrift 12 mit 4-Carboxybutylltriphenylphosphoniumbromid erhalten. ¹H-NMR (CDCl₃): δ = 1.72-1.88 ppm m (2H); 2.20-2.42 m (4H); 3.65 (3.67) je s (3H, CH₃); 5.57-5.68 (6.10-6.20) je m (1H); 6.48 d (J = 18 Hz, 1 H); 6.56 d (breit) (J = 12 Hz, 1H); 7.12 (7.16) je s (breit)(1H); 7.25-7.38 m (6H); 7.45-7.60 m (5H); 7.80 (7.84) je d (J = 8 Hz, 1 H).

### Beispiel 136 (E/Z)-6-(1,2-Diphenyl-1H-benzimidazol-6-yl)hex-5-ensäure

wurde durch Umsetzung von (E/Z)-6-(1,2-Diphenyl-1*H*-benzimidazol-6-yl)hex-5-ensäuremethylester gemäß der allgemeinen Arbeitsvorschrift 9 erhalten.
¹H-NMR (CDCl₃): δ = 1.74-1.89 ppm m (2H); 2.22-2.43 m (4H); 5.58-5.68 (6.10-6.22) je m (1H); 6.47 d (J = 18 Hz, 1 H); 6.55 d (breit) (J = 12 Hz, 1 H); 7.11 (7.14) je s (breit)(1H); 7.25-7.40 m (6H); 7.48-7.59 m (5H); 7.80 (7.85) je d (J = 8 Hz, 1 H).

### Beispiel 137 6-(1,2-Diphenyl-1H-benzimidazol-6-yl)hexansäuremethylester

wurde durch Umsetzung von (E/Z)-6-(1,2-Diphenyl-1*H*-benzimidazol-6-yl)hex-5-ensäuremethylester gemäß der allgemeinen Arbeitsvorschrift 1 erhalten.
¹H-NMR (CDCl₃): δ = 1.32-1.43 ppm m (2H); 1.62-1.74 m (4H); 2.31 t (J = 7.5 Hz, 2H); 2.72 t (J = 7.5 Hz, 2H); 3.56 s (3H); 7.02 s (breit)( 1 H); 7.18 dd (J = 8, 2 Hz, 1 H); 7.27-7.38 m (5H); 7.45-7.60 m (5H); 7.80 d (J = 8 Hz, 1 H).

### Beispiel 138

### 6-(1,2-Diphenyl-1H-benzimidazol-6-yl)hexansäure

wurde durch Umsetzung von 6-(1,2-Diphenyl-1*H*-benzimidazol-6-yl)hexansäuremethylester gemäß der allgemeinen Arbeitsvorschrift 9 erhalten.
¹H-NMR (CDCl₃): δ = 1.30-1.45 ppm m (2H); 1.54-1.74 m (4H); 2.32 t (J = 7.5 Hz, 2H); 2.70 t (J = 7.5 Hz, 2H); 7.02 s (breit)(1H); 7.20 dd (J = 8, 2 Hz, 1 H); 7.25-7.38 m (5H); 7.42-7.60 m (5H); 7.81 d (J = 8 Hz, 1H).

### Beispiel 139 (E/Z)-7-(1,2-Diphenyl-1H-benzimidazol-6-yl)hept-6-ensäuremethylester

wurde durch Umsetzung von 1,2-Diphenyl-1*H-*benzimidazol-6-carbaldehyd gemäß der allgemeinen Arbeitsvorschrift 12 mit 5-Carboxypentyltriphenylphosphoniumbromid erhalten.
¹H-NMR (CDCl₃): δ = 1.43-1.55 ppm m (2H); 1.58-1.72 m (2H); 2.18-2.38 m (4H); 3.65 (3.66) je s (3H, CH₃); 5.58-5.68 (6.12-6.22) je m (1H); 6.45 d (J = 18 Hz, 1H); 6.54 d (breit) (J = 12 Hz, 1H); 7.12 (7.14) je s (breit)(1H); 7.26-7.40 m (6H); 7.48-7.60 m (5H); 7.80 (7.83) je d (J = 8 Hz, 1H).

### Beispiel 140

### (E/Z)-7-(1,2-Diphenyl-1H-benzimidazol-6-yl)hept-6-ensäure

wurde durch Umsetzung von (E/Z)-7-(1,2-Diphenyl-1*H*-benzimidazol-6-yl)hept-6-ensäuremethylester gemäß der allgemeinen Arbeitsvorschrift 9 erhalten.
¹H-NMR (D₆-DMSO: δ = 1.40-1.60 ppm m (4H); 2.14-2.28 m (4H); 6.18-6.30 m (1H); 6.50 d (J = 18 Hz, 1H); 7.07 (7.12) je s (breit)(1H); 7.32-7.64 m (11 H); 7.70 (7.78) je d (J = 8 Hz, 1H); 12.00 s (breit)(1 H).

### Beispiel 141

### 7-(1,2-Diphenyt-1H-benzimidazol-6-yl)heptansäuremethylester

wurde durch Umsetzung von (E/Z)-7-(1,2-Diphenyl-1*H*-benzimidazol-6-yl)hept-6-ensäuremethylester gemäß der allgemeinen Arbeitsvorschrift 1 erhalten.
¹H-NMR (CDCl₃): δ = 1.30-1.42 ppm m (4H); 1.55-1.70 m (4H); 2.30 t (J = 7.5 Hz, 2H); 2.68 t (J = 7.5 Hz, 2H); 3.56 s (3H); 7.02 s (breit)(1H); 7.18 dd (J = 8, 2 Hz, 1 H); 7.28-7.35 m (5H); 7.45-7.58 m (5H); 7.79 d (J = 8 Hz, 1H).

### Beispiel 142 7-(1,2-Diphenyl-1H-benzimidazol-6-yl)heptansäure

wurde durch Umsetzung von 7-(1,2-Diphenyl-1*H*-benzimidazol-6-yl)heptansäu-remethylester gemäß der allgemeinen Arbeitsvorschrift 9 erhalten.
Fp. 99-103°C

### Beispiel 143

### N-(1,2-Diphenyl-1H-benzimidazol-5-yl)benzolsulfonamid

### Beispiel 144 N-(Phenylsulfonyl)-N-(1,2-diphenyl-1H-benzimidazol-5-yl)benzolsulfonamid

### a) 5-Amino-1,2-diphenyl-1H-benzimidazol

2,4-Diaminodiphenylamin wird gemäß der allgemeinen Arbeitsvorschrift 3 mit Trimethylorthobenzoat umgesetzt.
¹H-NMR (CDCl₃): δ= 6.70 ppm dd (J = 7.5, 2 Hz, 1 H); 7.06 d (J = 7.5 Hz, 1H); 7.18 d (J = 2 Hz, 1H); 7.28-7.60 m (10H).

5-Amino-1,2-diphenyl-1*H*-benzimidazol wurde gemäß der allgemeinen Arbeitsvorschrift 13 mit Benzolsulfonsäurechlorid umgesetzt.
143: Fp. 196-205°C
144: ¹H-NMR (CDCl₃): δ = 6.94 ppm dd (J = 7.5, 2 Hz, 1H); 7.20 d (J = 2 Hz, 1H); 7.26-8.04 m (21 H).

### Beispiel 145 3-Chlor-N-(1,2-Diphenyl-1H-benzimidazol-5-yl)benzolsulfonamid

### Beispiel 146 N-[(3-Chlorphenyl)sulfonyl]-N-(1,2-diphenyl-1H-benzimidazol-5-yl)-(3-chlorbenzol)sulfonamid

5-Amino-1,2-diphenyl-1*H*-benzimidazol wurde gemäß der allgemeinen Arbeitsvorschrift 13 mit 3-Chlorbenzolsulfonsäurechlorid umgesetzt.
**145:** Fp. 160-162°C
**146:** ¹H-NMR (CDCl₃): δ = 6.93 ppm dd (J = 7.5, 2 Hz, 1H); 7.25 d (J = 2 Hz, 1 H); 7.28-7.57 m (13H); 7.66 d (breit)(2H); 7.90 d (breit)(2H); 8.00 d (breit)(2H).

### Beispiel 147 4-Chlor-N-(1,2-Diphenyl-1H-benzimidazol-5-yl)benzolsulfonamid

5-Amino-1,2-diphenyl-1*H*-benzimidazol wurde gemäß der allgemeinen Arbeitsvorschrift 13 mit 4-Chlorbenzolsulfonsäurechlorid umgesetzt.
¹H-NMR (CDCl₃): δ = 6.86 ppm s (breit)(1H); 7.11 d (J = 7.5, 2 Hz, 1H); 7.17 d (J = 2 Hz, 1H); 7.25-7.55 m (12H); 7.70 d (J = 10 Hz, 2H).

### Beispiel 148 4-Brom-N-(1,2-Diphenyl-1H-benzimidazol-5-yl)benzolsulfonamid

### Beispiel 149 N-(4-Bromphenylsulfonyl)-N-(1,2-diphenyl-1H-benzimidazol-5-yl)-4-brom-benzolsulfonamid

5-Amino-1,2-diphenyl-1*H*-benzimidazol wurde gemäß der allgemeinen Arbeitsvorschrift 13 mit 4-Brombenzolsuffonsäurechlorid umgesetzt.
**148:** Fp. 135-139°C
**149:** ¹H-NMR (CDCl₃): δ = 6.90 ppm dd (J = 7.5, 2 Hz, 1 H); 7.23 d (J = 2 Hz, 1 H); 7.28-7.43 m (11H); 7.72 d (J = 10 Hz, 2H); 7.86 d (J = 10 Hz, 2H).

### Beispiel 150 4-(Trifluormethyl)-N-(1,2-Diphenyl-1H-benzimidazol-5-yl)benzolsulfonamid

### Beispiel 151 N-(1,2-Diphenyl-1H-benzimidazol-5-yl)-N-[(3-trifluormethyl)phenylsulfon-yl]-(3-trifluormethyl)benzolsulfonamid

5-Amino-1,2-diphenyl-1*H*-benzimidazol wurde gemäß der allgemeinen Arbeitsvorschrift 13 mit (3-Trifluormethyl) benzolsulfonsäurechlorid umgesetzt.
**150:** Fp. 116-121°C
**151:** Fp. 238-241 °C

### Beispiel 152 3-Methyl-N-(1,2-Diphenyl-1H-benzimidazol-5-yl)benzolsulfonamid

### Beispiel 153 N-(1,2-Diphenyl-1H-benzimidazol-5-yl)-N-(3-methylphenylsulfonyl)-3-methylbenzolsulfonamid

5-Amino-1,2-diphenyl-1*H*-benzimidazol wurde gemäß der allgemeinen Arbeitsvorschrift 13 mit 3-Methylbenzolsulfonsäurechlorid umgesetzt.
**152:** Fp. 192-195°C
**153:** Fp. 173-176°C

### Beispiel 154 4-Methy-N-(1,2-Diphenyl-1H-benzimidazol-5-yl)benzolsulfonamid

### Beispiel 155 N-(1,2-Diphenyl-1H-benzimidazol-5-yl)-N-(4-methylphenylsulfonyl)-4-methylbenzolsulfonamid

5-Amino-1,2-diphenyl-1*H*-benzimidazol wurde gemäß der allgemeinen Arbeitsvorschrift 13 mit 4-Methylbenzolsulfonsäurechlorid umgesetzt.
**154:** ¹H-NMR (CDCl₃): δ = 2.38 ppm s (3H); 6.77 s (breit)(1H); 7.14-7.55 m (14H); 7.66 d (J = 10 Hz, 2H).
**155:** Fp. 234-236°C

### Beispiel 156

### 4-Methoxy-N-(1,2-Diphenyl-1H-benzimidazol-5-yl)benzolsulfonamid

### Beispiel N-(1,2-Diphenyl-1H-benzimidazol-5-yl)-N-(4-methoxyphenylsulfonyl)-4-methoxybenzolsulfonamid

5-Amino-1,2-diphenyl-1*H*-benzimidazol wurde gemäß der allgemeinen Arbeitsvorschrift 13 mit 4-Methoxybenzolsulfonsäurechlorid umgesetzt.
**156:** ¹H-NMR (CDCl₃): δ = 3.82 ppm s (3H); 6.78 s (breit)(1H, H-4); 6.88 d (J = 7.5 Hz, 1 H); 7.14 d (J = 1.5 Hz, 1H); 7.28-7.55 m (12H); 7.72 d (J = 8 Hz, 2H).
**157:** ¹H-NMR (CDCl₃): δ = 3.90 ppm s (6H); 6.93 dd (J = 7.5, 2 Hz, 1 H); 7.00 d (J = 10 Hz, 4H); 7.06 d (J = 2 Hz, 1 H); 7.30-7.58 m (11 H); 7.93 d (J = 10 Hz, 4H).

### Beispiel 158 N-(1,2-Diphenyl-1H-benzimidazol-5-yl)propansulfonamid

### Beispiel 159 N-(1,2-Diphenyl-1H-benzimidazol-5-yl)-N-(propylsulfonyl)-propansulfon-amid

5-Amino-1,2-diphenyl-1*H*-benzimidazol wurde gemäß der allgemeinen Arbeitsvorschrift 13 mit Propanbenzolsulfonsäurechlorid umgesetzt.
158: **¹H-NMR (CDCl₃/D₆-DMSO): δ = 0.80 ppm t (J = 7.5 Hz, 3H); 1.65 m (2H); 2.82 m (2H);** 6.95 d (J = 7.5 Hz, 1H); 7.08 dd (J = 7.5, 2 Hz, 1 H); 7.10-7.40 m (10H); 7.61 d (J = 2 Hz, 1 H); 9.05 s (breit)(1H, NH).
**159:** ¹H-NMR (CDCl₃): δ = 1.08 ppm t (J = 7.5 Hz, 3H); 1.12 t (J = 7.5 Hz, 3H); 2.00 m (4H); 3.60 m (4H); 7.25-7.63 m (13H).

### Beispiel 160

### N-(1,2-Diphenyl-1H-benzimidazol-5-yl)benzolmethansulfonamid

5-Amino-1,2-diphenyl-1*H*-benzimidazol wurde gemäß der allgemeinen Arbeitsvorschrift 13 mit Benzolmethansulfonsäurechlorid umgesetzt.
Fp. 185-188°C

### Beispiel 161 6-[(1,2-Diphenyl-1H-benzimidazol-5-yl]amino]hexansäuremethylester

### Beispiel 162

### 6-[N-(1,2-Diphenyl-1H-benzimidazol-5-yl)-N-[(5-methoxycarbonyl)pentyl]amino]hexan-säuremethylester

Zu einer Lösung von 285 mg 5-Amino-1,2-diphenyl-1*H*-benzimidazol in 5 ml Methanol gab man 207 mg 6-Bromhexansäuremethylester, 138 mg Kaliumcarbonat und 150 mg Natriumjodid und ließ für 3 d bei 20°C rühren. Man versetzte mit Wasser, extrahierte dreimal mit Ethylacetat, trocknete die vereinigten organischen Phasen über Natriumsulfat und engte im Vakuum ein. Den Rückstand chromatographierte man an Kieselgel.
**161:** Fp. 109-113°C
**162:** ¹H-NMR (CDCl₃): δ = 1.30-1.43 m (4H); 1.53-1.73 m (8H); 2.32 t (J = 7.5 Hz, 4H); 3.30 t (J = 7.5 Hz, 4H); 3.68 s (6H); 6.75 dd (J = 10, 2 Hz, 1H); 7.10 d (J = 10 Hz, 1H); 7.14 d (J = 2 Hz, 1H); 7.23-7.35 m (5H); 7.42-7.58 m (5H).

### Beispiel 163 6-[[1,2-Diphenyl-1H-benzimidazol-5-yl]amino]hexansäure

wurde durch Umsetzung von 6-[[1,2-Diphenyl-1*H*-benzimidazol-5-yl]amino]hexansäuremethylester gemäß der allgemeinen Arbeitsvorschrift 9 erhalten.
¹H-NMR (D₆-DMSO): δ = 1.35-1.50 ppm m (2H); 1.50-1.68 m (4H); 2.23 t (J = 7.5 Hz, 2H); 3.05 t (J = 7.5 Hz, 2H); 6.67 dd (J = 10, 2 Hz, 1H); 6.80 d (J = 2 Hz, 1H); 6.92 d (J = 10 Hz, 1H); 7.30-7.40 m (4H); 7.45-7.62 m (6H).

### Beispiel 164

### 6-[[2-Phenyl-1-[4-(phenylmethoxy)phenyl]-1H-benzimidazol-6-yl]oxy]hexansäure-methylester

### a) (5-Hydroxy-2-nitrophenyl)[(4-(phenylmethoxy)phenyl]amin

1g 3-Fluor-4-nitrophenol und 3,8 g 4-Benzyloxyanilin wurden für 6,5 h bei 150°C gerührt. Der Ansatz wurde dann mit Dichlormethan verdünnt. Nach zweimaliger Extraktion mit 1 N wäßriger Salzsäure und waschen mit Wasser wurde zweimal mit 2 N wäßriger Natranlauge extahiert. Die basische Wasserphase wurde mit Ethylacetat und 1 N wäßriger Salzsäure versetzt. Nach Phasentrennung wurde die organische Phase mehrfach mit 1 N wäßriger Salzsäure extrahiert. Nach dem Waschen der organischen Phase mit ges. Natriumchloridlösung wurde über Natriumsulfat getrocknet, im Vakuum eingeengt und der Rückstand an Kieselgel chromatographiert.
¹H-NMR (D₆-DMSO): δ = 5.14 ppm s (2H); 6,23 m (2H); 7.10 d (J = 8 Hz, 2H); 7.26 d (J = 8 Hz, 2H); 7.32-7.52 m (5H); 8.03 d (J = 8 Hz, 1H); 9.52 s (1H); 10.71 s (1H).

### b) 6-[[4-Nitro-3-[[4-(phenylmethoxy)henyl]amino]phenyl]oxy]hexansäuremethylester wurde durch Umsetzung von (5-Hydroxy-2-nitrophenyl)[(4-(phenylmethoxy)phenyl]amin mit

6-Bromhexansäuremethylester gemäß der allgemeinen Arbeitsvorschrift 8 erhalten. ¹H-NMR (CDCl₃: δ = 1.37-1.50 m (2H); 1.59-1.80 m (4H); 2.33 t (J = 7.5 Hz, 2H); 3.67 s (3H); 3.83 t (J = 7.5 Hz, 2H); 5.12 s (2H); 6.24-6.33 m (2H); 7.04 d (J = 8 Hz, 2H); 7.21 d (J = 8 Hz, 2H); 7.32-7.50 m (5H); 8.17 d (J = 8 Hz, 1H); 9.66 s (1H).

### 6-[[2-Phenyl-1-[4-(phenylmethoxy)phenyl]-1H-benzimidazol-6-yl]oxy]hexansäure-methylester

wurde durch Reduktion von 6-[[4-Nitro-3-[[4-(phenylmethoxy)phenyl]amino]phenyl]-oxy]hexansäuremethylester gemäß der allgemeinen Arbeitsvorschrift 2 und anschließende Cyclisierung mit Trimethylorthobenzoat gemäß der allgemeinen Arbeitsvorschrift 3 erhalten.
¹H-NMR (CDCl₃: δ = 1.43-1.58 m (2H): 1.65-1.86 m (4H); 2.35 t (J = 7.5 Hz, 2H); 3.67 s (3H); 3.94 t (J = 7.5 Hz, 2H); 5.14 s (2H); 6.64 d (J = 2 Hz, 1H); 6.95 dd (J = 8,2 Hz, 1H); 7.11 d (J = 8 Hz, 2H); 7.18-7.61 m (12H); 7.74 d (J = 8 Hz, 1H).

### Beispiel 165

### 6-[[2-Phenyl-1-[4-(phenylmethoxy)phenyl]-1H-benzimidazol-6-yl]oxy]hexansäure

6-[[2-Phenyl-1-[4-(phenylmethoxy)phenyl]-1*H*-benzimidazol-6-yl)oxy]hexansäuremethylester wurde gemäß der allgemeinen Arbeitsvorschrift 9 umgesetzt.
¹H-NMR (D₆-DMSO): δ = 1.36-1.62 m (4H); 1.65-1.78 m (2H); 2.22 t (J = 7.5 Hz, 2H); 3.92 t (J = 7.5 Hz, 2H); 5.18 s (2H); 6.59 d (J = 2 Hz, 1H); 6.92 dd (J = 8, 2 Hz, 1H); 7.20 d (J = 8 Hz, 2H); 7.30-7.54 m (12H); 7.66 d (J = 8 Hz, 1H).

### Beispiel 166

### 6-[[1-(4-Hydroxyphenyl)-2-phenyl-1H-benzimidazol-6-yl]oxy]hexansäure

6-[[2-Phenyl-1-[4-(phenylmethoxy)phenyl]-1*H*-benzimidazol-6-yl]oxy]hexansäure wurde gemäß der allgemeinen Arbeitsvorschrift 1 umgesetzt.
¹H-NMR (D₆-DMSO): δ = 1.37-1.79 m (6H); 2.22 t (J = 7.5 Hz, 2H); 3.92 t (J = 7.5 Hz, 2H); 6.60 d (J = 2 Hz. 1H), 6.91 dd (J = 8, 2 Hz, 1H); 6.94 d (J = 8 Hz, 2H); 7.20 d (J = 8 Hz, 2H); 7.36 m (3H); 7.52 m (2H); 7.63 d (J = 8 Hz, 1H).

### Beispiel 167

### 6-[[2-Phenyl-1-(3-(phenylmethoxy)phenyl]-1H-benzimidazol-6-yl]oxy]hexansäure-methylester

### a) (5-Hydroxy-2-nitrophenyl)[(3-(phenylmethoxy)phenyl]amin

1 g 3-Fluor-4-nitrophenol und 3,81 g 3-Benzyloxyanilin wurden 22 h bei 150°C gerührt. Anschließend wurde in wenig Dichlormethan aufgenommen und direkt an Kieselgel chromatographiert.
¹H-NMR (CDCl₃): δ = 5.10 ppm s (2H); 5.82 s (br) (1H); 6.27 dd (J = 8, 2 Hz, 1H); 6.48 d (J = 2Hz, 1H); 6.86 m (3H); 7.28-7.48 m (5H); 8.15 d (J = 8 Hz, 1H); 9.52 s (br) (1H); 10.71 s (1H).

### b) 6-[[4-Nitro-3-[[3gphenylmethoxy)phenyl]amino]phenyl]oxy]hexansäure-methylester

wurde durch Umsetzung von (5-Hydroxy-2-nitrophenyl)[(3-(phenylmethoxy)phenyl]amin mit 6-Bromhexansäuremethylester gemäß der allgemeinen Arbeitsvorschrift 8 erhalten. ¹H-NMR (CDCl₃): δ = 1.40-1.53 m (2H); 1.61-1.82 m (4H); 2.34 t (J = 7.5 Hz, 2H); 3.67 s (3H); 3.88 t (J = 7.5 Hz. 2H); 5.10 s (2H); 6.33 dd (J = 8, 2 Hz, 1H); 6.58 d (J = 2Hz, 1H); 6.83-6.96 m (3H); 7.28-7.49 m (5H); 8.17 d (J = 8 Hz, 1H); 9.74 s (br).

### 6-[[2-Phenyl-1-[3-(phenylmethoxy)phenyl)-1H-benzimidazol-6-yl]oxy]hexansäure-methylester

wurde durch Reduktion von 6-[[4-Nitro-3-[[3-(phenylmethoxy)phenyl]amino]-phenyl]oxy]hexansäuremethylester gemäß der allgemeinen Arbeitsvorschrift 2 und anschließende Cyclisierung mit Trimethylorthobenzoat gemäß der allgemeinen Arbeitsvorschrift 3 erhalten.
¹H-NMR (CDCl₃): δ = 1.45-1.60 m (2H); 1.66-1.88 m (4H); 2.35 t (J = 7.5 Hz, 2H); 3.68 s (3H); 3.93 t (J = 7.5 Hz, 2H); 5.02 s (2H); 6.69 d (J = 2 Hz, 1H); 6.90 m (2H); 6.97 dd (J = 8, 2 Hz, 1H); 7.11 ddd (J = 8, 2, 2 Hz, 1H); 7.28-7.46 m (9H); 7.78 d (J = 8 Hz, 1H).

### Beispiel 168

### 6-[[2-Phenyl-1-[3-(phenylmethoxy)phenyl]-1H-benzimidazol-6-yl]oxy]hexansäure

### 6-[[2-Phenyl-1-[3-(phenylmethoxy)phenyl]-1H-benzimidazol-6-yl]oxy]hexansäuremethylester wurde gemäß der allgemeinen Arbeitsvorschrift 9 umgesetzt.

¹H-NMR (CDCl₃): δ = 1.49-1.62 m (2H); 1.67-1.88 m (4H); 2.39 t (J = 7.5 Hz, 2H); 3.93 t (J = 7.5 Hz, 2H); 5.03 s (2H); 6.68 d (J = 2 Hz, 1H); 6.91 m (3H); 6.98 dd (J = 8, 2 Hz, 1H); 7.12 ddd (J = 8, 2, 2 Hz, 1H): 7.29-7.47 m (8H); 7.57 d (J = 8 Hz, 2H); 7.81 d (J = 8 Hz, 1H).

### Beispiel 169

### 6-[[1-(3-Hydroxyphenyl)-2-phenyl-1H-benzimidazol-6-yl]oxy]hexansäure

### 6-[[2-Phenyl-1-[3-(phenylmethoxy)phenyl]-1H-benzimidazol-6-yl]oxy]hexansäure wurde gemäß der allgemeinen Arbeitsvorschrift 1 umgesetzt.

¹H-NMR (D₆-DMSO): δ = 1.39-1.80 m (6H); 2.23 t (J = 7.5 Hz, 2H); 3.94 t (J = 7.5 Hz, 2H); 6.57 d (J = 2 Hz, 1H); 6.74 dd (J = 2, 2 Hz, 1H); 6.84 dd (J = 8, 2 Hz, 1H); 6.94 m (2H); 7.38 m (4H); 7.53 m (2H); 7.66 d (J = 8 Hz, 1H).

### Beispiel 170

### 6-[[1-(3-Hydroxyphenyl)-2-phenyl-1H-benzimidazol-6-yl]oxy]hexansäuremethylester

### 6-[[2-Phenyl-1-[3-(phenylmethoxy)phenyl]-1H-benzimidazol-6-yl]oxy]hexansäuremethylester wurde gemäß der allgemeinen Arbeitsvorschrift 1 umgesetzt.

¹H-NMR (D₆-DMSO): δ = 1.38-1.80 m (6H); 2.32 t (J = 7.5 Hz, 2H); 3.59 s (3H); 3.94 t (J = 7.5 Hz, 2H); 6.66 d (J = 2 Hz, 1H): 6.74 dd (J = 2, 2 Hz, 1H): 6.83 dd (J = 8, 2 Hz, 1H): 6.93 dd (J = 8, 2 Hz, 2H); 7.38 m (4H); 7.54 m (2H); 7.67 d (J = 8 Hz, 1H).

### Beispiel 171

### 6-[[1-(3-Nitrophenyl)-2-phenyl-1H-benzimidazol-6-yl)oxy]hexansäureethylester

wurde durch Umsetzung von 6-Hxdroxy-1-(3-nitrophenyl)-2-phenylbenzimidazol (DE 4330959) mit 6-Bromhexansäureethylester gemäß der allgemeinen Arbeitsvorschrift 8 erhalten.
Fp. 104-106°C

### Beispiel 172

### 6-[[4-Brom-1-(4-methylphenyl)-2-phenyl-1H-benzimidazol-6-yl]oxy]hexan-säuremethylester

### a) 4-Brom-6-methoxy-2-phenyl-1H-benzimidazol

36,6 g 4-Amino-3-bromo-5-nitroanisol (J. Chem. Soc. 1966, 1769) wurden in 750 ml Ethanol vorgelegt und mit 19,8 g Eisenpulver und 126 ml Essigsäure versetzt. Nachdem 2,5 h bei 55 °C gerührt wurde, wurde mit 350 ml Dichlormethan versetzt und mit 2 N Natronlauge basisch gestellt. Nach Filtration über Celite wurde mit Wasser und gesättigter Kochsalzlösung gewaschen und eingeengt. Das so erhaltene rohe Phenylendiamin wurde gemäß der allgemeinen Arbeitsvorschrift 3 mit Trimethylorthobenzoat umgesetzt.
Fp. 203-205°C

### b) 4-Brom-6-methoxy-1-(4-methylphenyl)-2-phenyl-1H-benzimidazol

2,5 g 4-Brom-6-methoxy-2-phenyl-1*H*-benzimidazol und 2,24 g 4-(Methylbenzol)boronsäure wurden mit 1,5 g wasserfreiem Kupfer(11)acetat und circa 3 g Molsieb in 35 ml Pyridin 7 h bei 100°C gerührt. Nach Zusatz von Dichlormethan und Celite wurde eingeengt und an Kieselgel mit einem Hexan/Ethylacetat-Gemisch chromatographiert.
Fp. 209-210°C

### c) 4-Brom-6-hydroxy-1-(4-methylphenyl)-2-phenyl-1H-benzimidazol

1,2 g 4-Brom-6-methoxy-1-(4-methylphenyl)-2-phenyl-1*H*-benzimidazol, 6 ml Essigsäure und 6 ml wäßrige Bromwaserstoffsäure (62%-ig) werden 5,5 h gekocht. Anschließend wird mit Wasser gefällt und der Niederschlag abgesaugt. Dieser wurde anschließend zwischen Ethylacetat und 2 N Natronlauge verteilt. Nach Waschen der ogenischen Phase mit Wasser wurde eingeengt.
Fp. 136-137°C

**6-[[4-Brom-1-(4-methylphenyl)-2-phenyl-1*H*-benzimidazol-6-yl]oxy]hexan-säuremethylester**

wurde durch Umsetzung von 4-Brom-6-hydroxy-1-(4-methylphenyl)-2-phenyl-1*H-*benzimidazol mit 6-Bromhexansäuremethylester gemäß der allgemeinen Arbeitsvorschrift 8 erhalten.
Fp. 136°C

### Beispiel 173

### 6-[[4-Acetyl-1-(4-methylphenyl)-2-phenyl-1H-benzimidazol-6-yl]oxy]hexan-säuremethylester

0,5 g 4-Brom-6-hydroxy-1-(4-methylphenyl)-2-phenyl-1*H*-benzimidazol, 0,37 ml (α-Ethoxyvinyl)tributylzinn und 140 mg Dichlorobis(triphenylphosphin)palladium wurden in 10 ml Toluol 18 h bei 100°C gerührt. Nach Abkühlung wurde 0,25 h mit 2 N wäßriger Salzsäure gerührt. Nach Phasentrennung wurde die organische Phase mit Wasser geaschen und eingeengt. Der Rückstand wurde an Kieselgel mit einem Hexan/Ethylacetat-Gemisch chromatographiert.
Fp. 114-115°C

### Beispiel 174

### 6-[[1-(4-Methylphenyl)-2-phenyl-1H-benzimidazol-5-yl]oxy]hexansäuremethylester

### a) 5-Methoxy-1-(4-methylphenyl)-2-phenyl-1H-benzimidazol

16,8 g 5-Methoxy-2-phenyl-1*H*-benzimidazol (Bull. Sci. Fac. Chim. Ind. Bologna, 11 1953, 42) und 20.4 g 4-(Methylbenzol)boronsäure werden gemäß der allgemeinen Arbeitsvorschrift 14 umgesetzt.
¹H-NMR (CDCl₃): δ = 2.45 s (3H); 3.91 s (3H); 6.90 dd (J = 8, 2 Hz, 1H); 7.12 d (J = 8 Hz, 1H); 7.18 d (J = 8 Hz, 2H); 7.25-7.38 m (6H); 7.57 m (2H).
Außerdem wurde 6-Methoxy-1-(4-methylphenyl)-2-phenyl-1*H*-benzimidazol erhalten.

### b) 5-Hydroxy-1-(4-methylphenyl)-2-phenyl-1H-benzimidazol

wurde aus 5-Methoxy-1-(4-methylphenyl)-2-phenyl-1*H*-benzimidazol gemäß der allgemeinen Arbeitsvorschrift 6 erhalten.
Fp. 270°C

### 6-[[1-(4-Methylphenyl)-2-phenyl-1H-benzimidazol-5-yl]oxy]hexansäuremethylester

wurde aus 5-Hydroxy-1-(4-methylphenyl)-2-phenyl-1*H*-benzimidazol durch Umsetzung mit 6-Bromhexansäuremethylester gemäß der allgemeinen Arbeitsvorschrift 8 erhalten. ¹H-NMR (CDCl₃): Δ = 1.48-1.92 m (6H); 2.38 t (J = 7.5 Hz, 2H); 2,46 s (3H); 3.69 s (3H); 4.06 t (J = 7.5 Hz, 2H); 6.89 dd (J = 8, 2 Hz, 1H); 7.11 d (J = 8 Hz, 1H); 7.18 d (J = 8 Hz, 2H); 7.24-7.37 m (6H); 7.57 m (2H).

### Beispiel 175

### 6-[[1-(4-Methylphenyl)-2-phenyl-1H-benzimidazol-5-yl]oxy]hexansäure

wurde aus 6-[[1-(4-Methylphenyl)-2-phenyl-1*H*-benzimidazol-5-yl]oxy]hexansäuremethylester gemäß der allgemeinen Arbeitsvorschrift 9 erhalten.
¹H-NMR (D₆-DMSO): δ = 1.41-1.67 m (4H); 1.70-1.83 m (2H); 2.26 t (J = 7.5 Hz, 2H); 2.43 s (3H); 4.05 t (J = 7.5 Hz. 2H); 6.90 dd (J = 8, 2 Hz, 1H); 7.04 d (J = 8 Hz, 1H); 7.23-7.40 m (8H); 7.52 m (2H); 11.92 s (br.) (1H).

### Beispiel 176

### 6-[[2-Phenyl-1-[4-(thiomethyl)phenyll-1H-benzimidazol-5-yl]oxy]hexansäure-methylester

### a) 6-[[2-Phenyl]-1H-benzimidazol-5-yl]oxylhexansäuremethylester

4,84 g 2-Phenyl-5-hydroxy-1*H*-Benzimidazol (Izv. Akad. Nauk. SSSR Ser. Chim. 8 1990. 1888) wurde durch Umsetzung mit 6-Bromhexansäuremethylester gemäß der allgemeinen Arbeitsvorschrift 8 erhalten.
¹H-NMR (CDCl₃): δ = 1.43-1.58 m (2H); 1.64-1.87 m (4H); 2.37 t (J = 7.5 Hz. 2H); 3.69 s (3H); 3.94 t (J = 7.5 Hz, 2H); 6.87 dd (J = 8, 2 Hz, 1H); 7.02 s (br.); 7.40-7.57 m (4H); 8.05 m (2H).

### 6-[[2-Phenyl-1-[4-(thiomethyl)phenyl)-1H-benzimidazol-5-yl)oxy]hexansäure-methylester

wurde durch Umsetzung von 6-[[2-Phenyl]-1*H*-benzimidazol-5-yl]oxy]hexansäuremethylester mit 4-(Thiomethylbenzol)boronsäure gemäß der allgemeinen Arbeitsvorschrift 14 erhalten.
¹H-NMR (CDCl₃): δ = 1.48-1.61 m (2H); 1.66-1.92 m (4H); 2.36 t (J = 7.5 Hz, 2H); 2.54 s (3H); 3.68 s (3H); 4.05 t (J = 7.5 Hz, 2H); 6.90 dd (J = 8, 2 Hz, 1H); 7.11 d (J = 8 Hz, 1H); 7.22 d (J = 8 Hz, 2H); 7.27-7.49 m (6H); 7.57 m (2H).

### Beispiel 177

### 6-[[2-Phenyl-1-[(4-thiomethyl)phenyl]-1H-benzimidazol-6-yl]oxy]hexansäuremethylester

wurde durch Umsetzung von 6-[[2-Phenyl]-1*H*-benzimidazol-5-yl]oxy)hexansäuremethylester mit 4-(Thiomethylbenzol)boronsäure gemäß der allgemeinen Arbeitsvorschrift 14 erhalten.
¹H-NMR (CDCl₃): δ = 1.45-1.57 m (2H); 1.62-1.86 m (4H); 2.44 t(J = 7.5 Hz, 2H); 2.56 s (3H); 3.66 s (3H); 3.93 t (J = 7.5 Hz, 2H); 6.66 d (J = 2 Hz, 1H); 6.96 dd (J = 8. 2 Hz, 1H); 7.18-7.39 m (7H); 7.54 m (2H); 7.73 d (J = 8 Hz, 1H).

### Beispiel 178 6-([2-Phenyl-1-(3-thienyl)-1H-benzimidazol-5-yl]oxy]hexansäuremethylester

wurde durch Umsetzung von 6-[[2-Phenyl]-1*H*-benzimidazol-5-ylJoxy]hexansäuremethylester mit Thiophen-3-boronsäure gemäß der allgemeinen Arbeitsvorschrift 14 erhalten.
¹H-NMR (CDCl₃): δ = 1.48-1.62 m (2H); 1.66-1.92 m (4H); 2.47 t (J = 7.5 Hz, 2H); 3.68 s (3H); 4.04 t (J = 7.5 Hz, 2H); 6.93 dd (J = 8, 2 Hz, 1H); 6.98 dd (J = 5, 1Hz, 1H); 7.18 d (J = 8 Hz, 1H); 7.28 dd (J = 3, 1Hz, 1H); 7.30-7.40 m (4H); 7.46 dd (J = 5, 3 Hz, 1H); 7.60 m (2H).

### Beispiel 179 6-[[2-Phenyl-1-(3-thienyl)-1H-benzimidazol-6-yl]oxy]hexansäuremethylester

wurde durch Umsetzung von 6-[[2-Phenyl]-1*H*-benzimidazol-5-yl]oxy]hexansäuremethylester mit Thiophen-3-boronsäure gemäß der allgemeinen Arbeitsvorschrift 14 erhalten.
¹H-NMR (CDCl₃): δ = 1.45-1.58 m (2H); 1.64-1.87 m (4H); 2.35 t (J = 7.5 Hz, 2H); 3.67 s (3H); 3.97 t (J = 7.5 Hz, 2H); 6.74 d (J = 2 Hz, 1H); 6.95 dd (J = 8, 2 Hz, 1H); 7.01 dd (J = 5, 1 Hz,1H); 7.29 dd (J = 3, 1 Hz, 1H); 7.30-7.38 m (4H); 7.47 dd (J = 5, 3 Hz, 1H); 7.58 m (2H); 7.73 d (J = 8 Hz, 1H).

### Beispiel 180

### 4-[3-[[1-(4-Methylphenyl)-2-phenyl-1H-benzimidazol-6-yl]oxy]phenoxy]butansäuremethylester

### a) 6-(3-Methoxyphenoxy)-1-(4-methytphenyl)-2-pheny)-1H-benzimidazol

wurde aus 6-Hydroxy-1-(4-methylphenyl)-2-phenyl-1*H*-benzimidazol und 3-Methoxybenzolboronsäure gemäß der allgemeinen Arbeitsvorschrift 14 erhalten.
Fp. 120-122°C

### b) 3-[[1-(4-Methylphenyl)-2-phenyl-1H-benzimidazol-6-yl]oxy]phenol

wurde durch Umsetzung von 6-(3-Methoxyphenoxy)-1-(4-methylphenyl)-2-phenyl-1*H*-benzimidazol gemäß der allgemeinen Arbeitsvorschrift 6 unter Zusatz von 10 mol-% Hexadecyltributylphosphoniumbromid erhalten.
Fp. 252-253°C

### 4-[3-[[1-(4-Methylphenyl)-2-phenyl-1H-benzimidazol-6-yl]oxy]phenoxy]butansäure-methylester

wurde durch Umsetzung von 3-[[1-(4-Methylphenyl)-2-phenyl-1*H*-benzimidazol-6-yl]oxy]phenol mit 4-Brombuttersäuremethylester gemäß der allgemeinen Arbeitsvorschrift 8 erhalten.
¹H-NMR (CDCl₃): δ = 2.00-2.13 m (2H); 2.43 s (3H); 2.50 t (J = 7.5 Hz, 2H); 3.67 s (3H); 3.93 t (J = 7.5 Hz, 2H); 6.44-6.62 m (3H); 6.95 d (J = 2 Hz, 1H); 7.06 dd (J = 8, 2 Hz, 1H); 7.12-7.22 m (3H); 7.25-7.39 m (5H); 7.59 m (2H); 7.87 d (J = 8 Hz, 1H).

### Beispiel 181

### 4-[4-[[1-(4-Methylphenyl)-2-phenyl-1H-benzimidazol-6-yl]oxy]phenoxy]butansäuremethylester

### a) 6-(4-Methoxyphenoxy)-1-(4-methylphenyl)-2-phenyl-1H-benzimidazol

wurde aus 6-Hydroxy-1-(4-methylphenyl)-2-phenyl-1*H*-benzimidazol und 4-Methoxybenzolboronsäure gemäß der allgemeinen Arbeitsvorschrift 14 erhalten.
¹H-NMR (CDCl₃): δ = 2.44 s (3H); 3.79 s (3H); 6.82-6.98 m (5H); 7.01 dd (J = 8, 2 Hz, 1H); 7.17 d (J = 8 Hz, 2H); 7.25-7.41 m (5H); 7.57 m (2H); 7.82 d (J = 8 Hz, 1H).

### b) 4-[[1-(4-Methylphenyl)-2-phenyl-1H-benzimidazol-6-yl]oxy]phenol

wurde durch Umsetzung von 6-(3-Methoxyphenoxy)-1-(4-methylphenyl)-2-phenyl-1*H*-benzimidazol gemäß der allgemeinen Arbeitsvorschrift 6 unter Zusatz von 10 mol-% Hexadecyltributylphosphoniumbromid erhalten.
¹H-NMR (D₆-DMSO): δ = 2.38 s (3H); 6.61 d (J = 2 Hz, 1H); 6.74 d (J = 8 Hz, 2H); 6.86 d (J = 8 Hz, 2H); 6.91-7.01 m (2H); 7.22-7.41 m (6H); 7.49 m (2H); 7.75 d (J = 8 Hz, 1H); 9.32 s (1H).

### 4-[4-[[1-(4-Methylphenyl)-2-phenyl-1H-benzimidazol-6-yl]oxy]phenoxy]butansäuremethylester

wurde durch Umsetzung von 4-[[1-(4-Methylphenyl)-2-phenyl-1*H*-benzimidazol-6-yl]oxy]phenol mit 4-Brombuttersäuremethylester gemäß der allgemeinen Arbeitsvorschrift 8 erhalten.
¹H-NMR (CDCl₃): δ = 2.03-2.16 m (2H); 2.42 s (3H); 2.53 t (J = 7.5 Hz, 2H); 3.69 s (3H); 3.97 t (J = 7.5 Hz, 2H); 6.78-6.94 m (5H); 6.99 dd (J = 8, 2 Hz, 1H); 7.16 d (J = 8, Hz, 2H); 7.24-7.38 m (5H); 7.57 m (2H); 7.79 d (J = 8 Hz, 1H).

### Beispiel 182

### [4-[[1-(4-Methylphenyl)-2-phenyl-1H-benzimidazol-6-yl]oxy]phenoxy]essigsäuremethylester

wurde durch Umsetzung von 4-[[1-(4-Methylphenyl)-2-phenyl-1*H*-benzimidazol-6-yl]oxy]phenol mit Bromesigsäuremethylester gemäß der allgemeinen Arbeitsvorschrift 8 erhalten.
¹H-NMR (CDCl₃): δ = 2.43 s (3H); 3.82 s (3H); 4.61 s (2H); 6.78-6.96 m (5H); 7.00 dd (J = 8, 2 Hz, 1H); 7.14 d (J = 8, Hz, 2H); 7.23-7.38 m (5H); 7.56 m (2H); 7.80 d (J = 8 Hz, 1H).

### Beispiel 183

### 4-[(1,2-Diphenyl-1H-benzimidazol-6-yl)oxy]butansäuremethylester

wurde durch Umsetzung von 1,2-Diphenyl-6-hydroxy-1*H*-benzimidazol mit 4-Brombutansäuremethylester gemäß der allgemeinen Arbeitsvorschrift 8 erhalten.
Fp. 107-110°C

### Beispiel 184

### 6-[[2-Phenyl-1-(3-pyridyl)-1H-benzimidazol-5-yl]oxy]hexansäuremethylester

wurde durch Umsetzung von 6-[[2-Phenyl]-1*H*-benzimidazol-5-yl]oxy]hexansäuremethylester mit Pyridin-3-boronsäure gemäß der allgemeinen Arbeitsvorschrift 14 erhalten. MS (EI): 415 (Molekülionpeak)

### Beispiel 185

### 6-[[2-Phenyl-1-(3-pyridyl)-1H-benzimidazol-6-yl]oxy]hexansäuremethylester

wurde durch Umsetzung von 6-[[2-Phenyl]-1*H*-benzimidazol-5-yl]oxy]hexansäuremethylester mit Pyridin-3-boronsäure gemäß der allgemeinen Arbeitsvorschrift 14 erhalten. MS (EI): 415 (Molekülionpeak)

### Beispiel 186

### 6-[[2-Phenyl-1-(2-pyridyl)-1H-benzimidazol-5-yl]oxy]hexansäure

wurde durch Umsetzung von 6-[[2-Phenyl]-1*H*-benzimidazol-5-yl]oxy]hexansäuremethylester mit 2-Fluor-Pyridin gemäß der allgemeinen Arbeitsvorschrift 15 erhalten.
MS (EI): 401 (Molekülionpeak)

### Beispiel 187

### 6-[[2-Phenyl-1-(2-pyridyl)-1H-benzimldazol-6-yl]oxy]hexansäure

wurde durch Umsetzung von 6-[[2-Phenyl]-1*H*-benzimidazol-5-yl]oxy]hexansäuremethylester mit 2-Fluor-Pyridin gemäß der allgemeinen Arbeitsvorschrift 15 erhalten.
MS (EI): 401 (Molekülionpeak)

### Beispiel 188

### 6-[[2-Phenyl-1-(4-pyridyl)-1H-benzimidazol-6-yl]oxy]hexansäuremethylester

wurde durch Umsetzung von 6-[[2-Phenyl]-1*H*-benzimidazol-5-yl]oxy]hexansäuremethylester mit Pyridin-4-boronsäure gemäß der allgemeinen Arbeitsvorschrift 14 erhalten.
MS (EI): 415 (Molekülionpeak)

### Beispiel 189

### 6-[[2-(4-Fluorphenyl)-1-phenyl-1H-benzimidazol-6-yl]oxy]hexansäuremethylester

wurde durch Umsetzung von 6-[[4-Amino-3-(phenylamino)phenyl]oxy]hexansäuremethylester mit 4-Fluorbenzoylchlorid gemäß der allgemeinen Arbeitsvorschrift 5 erhalten. MS (EI): 432 (Molekülionpeak)

### Beispiel 190

### 6-[[2-(4-Methoxyphenyl)-1-phenyl-1H-benzimidazol-6-yl]oxy]hexansäuremethylester

wurde durch Umsetzung von 6-[[4-Amino-3-(phenylamino)phenyl]oxy]hexansäuremethylester mit 4-Methoxybenzoylchlorid gemäß der allgemeinen Arbeitsvorschrift 5 erhalten.
MS (EI): 444 (Molekülionpeak)

### Beispiel 191

### 6-[[2-(3-Fluorphenyl)-1-phenyl-1H-benzimidazol-6-yl]oxy]hexansäuremethylester

wurde durch Umsetzung von 6-[[4-Amino-3-(phenylamino)phenyl]oxy]hexansäuremethylester mit 3-Fluorbenzoylchlorid gemäß der allgemeinen Arbeitsvorschrift 5 erhalten. MS (EI): 432 (Molekülionpeak)

### Beispiel 192

### 6-[[2-(4-Bromphenyl)-1-phenyl-1H-benzimidazol-6-yl]oxy]hexansäuremethylester

wurde durch Umsetzung von 6-[[-Amino-3-(phenylamino)phenyl]oxy]hexansäuremethylester mit 4-Brombenzoylchlorid gemäß der allgemeinen Arbeitsvorschrift 5 erhalten. MS (EI): 492/494 (Molekülionpeaks)

### Beispiel 193

### 6-[[2-(4-(Trifluormethyl)phenyl]-1-phenyl-1H-benzimidazol-6-yl]oxy]hexansäuremethylester

wurde durch Umsetzung von 6-[[4-Amino-3-(phenylamino)phenyl]oxy]hexansäuremethylester mit 4-(Trifluormethyl)benzoylchiorid gemäß der allgemeinen Arbeitsvorschrift 5 erhalten.
MS (EI): 482 (Molekülionpeak)

### Beispiel 194

### 6-[[2-(4-Fluorphenyl)-1-phenyl-1H-benzimidazol-6-yl]oxy]hexansäure

wurde durch Umsetzung von 6-[[2-(4-Fluorphenyl)-1-phenyl-1*H*-benzimidazol-6-yl]oxy]hexansäuremethylester gemäß der allgemeinen Arbeitsvorschrift 9 erhalten.
MS (EI): 418 (Molekülionpeak)

### Beispiel 195

### 6-[[1-Phenyl-2-(benzothien-2-yl)-1H-benzimidazol-6-yl]oxy]hexansäuremethylester

wurde durch Umsetzung von 6-[[4-Amino-3-(phenylamino)phenyl]oxy]hexansäuremethylester mit Benzothiophen-2-carbonsäurechlorid gemäß der allgemeinen Arbeitsvorschrift 5 erhalten.
Fp. 129-130°C

### Beispiel 196

### 6-[[1-Phenyl-2-(benzothien-2-yl)-1H-benzimidazol-6-yl]oxy]hexansäure wurde gemäß der allgemeinen Arbeitsvorschrift 9 dargestellt.

Fp. 340°C (Zers.)

### Beispiel 197

### 6-[[5-Hydroxy-1-(4-methylphenyl)-2-phenyl-1H-benzimidazol-6-yl]oxy]hexansäureisopropylester

### Beispiel 198

### 6-[[6-Hydroxy-1-(4-methylphenyl)-2-phenyl-1H-benzimidazol-5-yl]oxy]hexansäureisopropylester

4,5-Dimethoxy-1,2-dinitrobenzol wurde gemäß der allgemeinen Arbeitsvorschrift 1 zur Diaminoverbindung hydriert, die als Rohprodukt gemäß der allgemeinen Arbeitsvorschrift 3 mit Trimethylorthobenzoat zu 5,6-Dimethoxy-2-phenyl-1*H*-benzimidazol (Fp.131-133°C) cyclisiert wurde. Dieses Benzimidazolderivat wurde gemäß der allgemeinen Arbeitsvorschrift 14 mit 4-Methylphenylboronsäure zu 5,6-Dimethoxy-1-(4-methylphenyl)-2-phenyl-1*H*-benzimidazol (Fp. 145-148°C) umgesetzt. Nach Etherspaltung mit Bromwasserstoffsäure gemäß der allgemeinen Arbeitsvorschrift 6 zum 5,6-Dihydroxy-1-(4-methylphenyl)-2-phenyl-1*H*-benzimidazol (¹H-NMR des Hydrobromids (D₆-DMSO): δ = 2.42 ppm s (3H); 6.68 s (1H); 7.22 s (1H); 7.40-7-62 m (10H)) wurde gemäß der allgemeinen Arbeitsvorschrift 8 mit 6-Bromhexansäureisopropylester alkyliert. Man erhielt 6-[[5-Hydroxy-1-(4-methylphenyl)-2-phenyl-1*H*-benzimidazol-6-yl]oxy]hexansäureisopropylester
Fp. 137-139°C
und 6-[[6-Hydroxy-1-(4-methylphenyl)-2-phenyl-1*H*-benzimidazol-5-yl]oxy]hexansäure-isopropylester
Fp. 177-178°C.

### Beispiel 199

### 6-[[5-Hydroxy-1-(4-methylphenyl)-2-phenyl-1H-benzimidazol-6-yl)oxy]hexansäure wurde gemäß der allgemeinen Arbeitsvorschrift 9 dargestellt.

Fp. 245-248°C

### Beispiel 200

### 6-[[6-Hydroxy-1-(4-methylphenyl)-2-phenyl-1H-benzimidazol-5-yl]oxy]hexansäure wurde gemäß der allgemeinen Arbeitsvorschrift 9 dargestellt.

Fp. 182-184°C

### Beispiel 201

### 6-[[5-Methoxy-1-(4-methylphenyl)-2-phenyl-1H-benzimidazol-6-yl]oxy]hexansäure-isopropylester

6-[[5-Hydroxy-1-(4-methylphenyl)-2-phenyl-1*H*-benzimidazol-6-yl]oxy]hexansäure-isopropylester wurde gemäß der allgemeinen Arbeitsvorschrift 8 mit Methyljodid methyliert.
Fp. 89-91 °C

### Beispiel 202

### 6-[[5-Methoxy-1-(4-methylphenyl)-2-phenyl-1H-benzimidazol-6-yl]oxy]hexansäure wurde gemäß der allgemeinen Arbeitsvorschrift 9 dargestellt.

Fp. 184-186°C

### Beispiel 203

### 6-[[5-Hydroxy-1-(4-methylphenyl)-2-phenyl-1H-benzimidazol-6-yl]oxy]hexansäuremethylester

### Beispiel 204 6-[[6-Hydroxy-1-(4-methylphenyl)-2-phenyl-1H-benzimidazol-5-yl]oxy]-hexansäuremethylester

wurden analog zu den Isopropylestem durch Alkylierung von 5,6-Dihydroxy-1-(4-methylphenyl)-2-phenyl-1*H*-benzimidazol gemäß der allgemeinen Arbeitsvorschrift 8 mit 6-Bromhexansäuremethylester dargestellt. Man erhielt 6-[[5-Hydroxy-1-(4-methylphenyl)-2-phenyl-1*H*-benzimidazol-6-yl]oxy]hexansäuremethylester
¹H-NMR (CDCl₃): δ = 1.45-1.58 ppm m (2H); 1.65-1.90 m (4H); 2.37 t (J = 7.5 Hz, 2H); 2.48 s (3H); 3.68 s (3H); 3.98 t (J = 7.5 Hz, 2H); 5.68 s (breit) (1H, OH); 6.62 s (1H); 7.18 d (J = 8 Hz, 2H); 7.22-7.38 m (5H); 7.40 s (1H); 7.53 dd (J = 8, 1Hz, 2H)
und 6-[[6-Hydroxy-1-(4-methylphenyl)-2-phenyl-1*H*-benzimidazol-5-yl]oxy]hexansäuremethylester.
Fp. 141-143°C

### Beispiel 205

### 6-[[5-Methoxy-1-(4-methylphenyl)-2-phenyl-1H-benzimidazol-6-yl]oxy]hexansäuremethylester

40 mg 6-[[5-Methoxy-1-(4-methylphenyl)-2-phenyl-1*H*-benzimidazol-6-yl]oxy]hexansäure wurden in 2 ml Methanol gelöst, mit 1 Tropfen konz. Schwefelsäure versetzt und das Gemisch für 2 h gerührt. Man versetzte mit ges. Kaliumhydrogencarbonatlösung, verdünnte mit Wasser, extrahierte mit Ethylacetat, trocknete die Extrakte über Natriumsulfat und engte im Vakuum ein. Der Rückstand kristallisierte aus Diisopropylether.
Fp. 81-82°C

### Beispiel 206 6-[[6-Methoxy-1-(4-methylphenyl)-2-phenyl-1H-benzimidazol-5-yl]oxy]-hexansäuremethylester

6-[[6-Hydroxy-1-(4-methylphenyl)-2-phenyl-1*H*-benzimidazol-5-yl]oxy]hexansäuremethylester wurde gemäß der allgemeinen Arbeitsvorschrift 8 mit Methyljodid methyliert. Fp. 108-110°C

### Beispiel 207

### 6-[[6-Methoxy-1-(4-methylphenyl)-2-phenyl-1H-benzimidazol-5-yl]oxy]hexansäure wurde gemäß der allgemeinen Arbeitsvorschrift 9 dargestellt.

Fp. 182-184°C

### Beispiel 208 6-[(5-Amino-1-(3,4-dimethylphenyl)-2-phenyl-1H-benzimidazol-6-yl)oxy]hexansäuremethylester

### a) 3-[(3,4-Dimethylphenyl)amino]-4,6-dinirtrophenol

Zu einer Suspension aus 4 g 4,6-Dinitro-3-fluorphenol (J. Org. Chem. 1991, 5958) in 100 ml Ethanol gab man 6.6 g 3,4-Dimethylanilin und rührte für 7 d bei 40°C. Nach dem Abkühlen wurde abgesaugt und der Rückstand aus Ethanol umkristallisiert.
¹H-NMR (CDCl₃): δ = 2.20 ppm s (6H); 6.43 s (1H); 6.90-7.0 m (2H); 7.14 d (J = 8 Hz, 1H); 9.08 s (1H); 9.70 s (breit) (1H); 10.2-10.6 (1H)

### b) 6-[[3-[(3,4-Dimethylphenyl)amino]-4,6-dinitrophenyl]oxy]hexansäuremethylester

5 g 3-[(3,4-Dimethylphenyl)amino]-4,6-dinitrophenol wurden analog zur allgemeinen Arbeitsvorschrift 8 mit 6-Bromhexansäuremethylester bei 70°C O-alkyliert.
¹H-NMR (CDCl₃): δ = 1.45-1.88 ppm m (6H); 2.30 s (6H); 2.33 t (J = 7.5 Hz, 2H); 3.68 s (3H); 3.88 t (J = 7.5 Hz, 2H); 6.45 s (1H); 7.00-7.08 m (2H); 7.25 d (J = 8 Hz, 1H); 9.03 s (1H); 9.89 s (breit) (1H)

### c) 6-[(5-Amino-1-(3,4-dimethylphenyl)-2-phenyl-1H-benzimidazol-6-yl)oxy]hexansäuremethylester

2.45 g 6-[[3-[(3,4-Dimethylphenyl)amino]-4,6-dinitrophenyl]oxy]hexansäuremethylester wurden gemäß der allgemeinen Arbeitsvorschrift 1 in Methanol hydriert. 500 mg des Rohprodukts wurden gemäß der allgemeinen Arbeitsvorschrift 4 mit Benzimidathydrochlorid umgesetzt. Abweichend von der allgemeinen Arbeitsvorschrift 4 wurde das Rohprodukt nach Aufnehmen im Lösungsmittel nicht mit wäßriger Salzsäure gewaschen.
¹H-NMR (CDCl₃): δ = 1.48-1.58 ppm m (2H); 1.62-1.78 m (2H); 1.78-1.90 m (2H); 2.30 s (3H); 2.38 s (3H); 2.38 t (J = 7.5 Hz, 2H); 3.67 s (3H); 3.93 t (J = 7.5 Hz, 2H); 6.56 s (1H); 6.98-7.08 m (2H); 7.18 s (1H); 7.20-7.32 m (4H); 7.52 dd (J = 8 Hz u. 2 Hz, 2H)

### Beispiel 209

### 6-[[5-[[(4-Chlorphenyl)sulfonyl]amino]-1-(3,4-dimethylphenyl)-2-phenyl-1H-benzimidazol-6-yl]oxy]hexansäuremethylester

6-[(5-Amino-1-(3,4-dimethylphenyl)-2-phenyl-1*H*-benzimidazol-6-yl)oxy]hexansäuremethylester wurde gemäß der allgemeinen Arbeitsvorschrift 13 mit 4-Chlorbenzolsulfonsäurechlorid umgesetzt.
Fp. 186-191 °C

### Beispiel 210

### 6-[(5-Amino-2-(4-fluorphenyl)-1-(4-methoxyphenyl)-1H-benzimidazol-6-yl)oxy]hexansäuremethylester

wurde analog zu 6-[(5-Amino-1-(3,4-dimethylphenyl)-2-phenyl-1*H*-benzimidazol-6-yl)oxy)-hexansäuremethylester dargestellt.
MS (EI): 477 (Molekülionpeak)

### Beispiel 211

### 6-[(5-Amino-1-(4-methoxyphenyl)-2-(4-methoxyphenyl)-1H-benzimidazol-6-yl)oxy]-hexansäuremethylester

wurde analog zu 6-[(5-Amino-1-(3,4-dimethylphenyl)-2-phenyl-1*H*-benzimidazol-6-yl)oxy]-hexansäuremethylester dargestellt.
MS (EI): 489 (Molekülionpeak)

### Beispiel 212

### 6-[[5-[[(4-Chlorphenyl)sulfonyl]amino]-2-(4-fluorphenyl)-1-(4-methoxyphenyl)-1H-benzimidazol-6-yl]oxy]hexansäuremethylester

6-[(5-Amino-2-(4-fluorphenyl)-1-(4-methoxyphenyl)-1*H*-benzimidazol-6-yl)oxy]hexansäuremethylester wurde gemäß der allgemeinen Arbeitsvorschrift 13 mit 4-Chlorbenzolsulfonsäurechlorid umgesetzt.
Fp. 180-182°C

### Beispiel 213

### 6-[[5-[[(4-Chlorphenyl)sulfonyl]amino]-1-(4-methoxyphenyl)-2-(4-methoxyphenyl)-1H-benzimidazol-6-yl]oxy]hexansäuremethylester

6-[(5-Amino-1-(4-methoxyphenyl)-2-(4-methoxyphenyl)-1*H*-benzimidazol-6-yl)oxy]hexansäuremethylester wurde gemäß der allgemeinen Arbeitsvorschrift 13 mit 4-Chlorbenzolsulfonsäurechlorid umgesetzt.
Fp. 169-171 °C

### Beispiel 214

### 4-[(5-Amino-1-(4-methoxyphenyl)-2-phenyl-1H-benzimidazol-6-yl)oxy]butansäuremethylester

wurde analog zu 6-[(5-Amino-1-(3,4-dimethylphenyl)-2-phenyl-1*H*-benzimidazol-6-yl)oxy]-hexansäuremethylester dargestellt.
¹H-NMR (CDCl₃): δ = 2.17 ppm tt (J=8 u. 8 Hz, 2H); 2.52 t (J=8 Hz, 2H); 3.68 s (3H); 3.90 s (3H); 3.98 t (J = 7.5 Hz, 2H); 6.54 s (1H); 7.0 d (J=12 Hz, 2H); 7.18-7.35 m (6H); 7.50-7.58 m (2H)

### Beispiel 215

### 4-[[5-[[(4-Chlorphenyl)sulfonyl]amino]-1-(4-methoxyphenyl)-2-phenyl-1H-benzimidazol-6-yl]oxy]butansäuremethylester

4-[(5-Amino-1-(4-methoxyphenyl)-2-phenyl-1*H*-benzimidazol-6-yl)oxy]butansäure-methylester wurde gemäß der allgemeinen Arbeitsvorschrift 13 mit 4-Chlorbenzolsulfonsäurechlorid umgesetzt.
MS (EI): 605 (Molekülionpeak)

### Beispiel 216

### 5-[(5-Amino-1-(4-methoxyphenyl)-2-phenyl-1H-benzimidazol-6-yl)oxy]pentansäuremethylester

wurde analog zu 6-[(5-Amino-1-(3,4-dimethylphenyl)-2-phenyl-1*H*-benzimidazol-6-yl)oxy]-hexansäuremethylester dargestellt.
¹H-NMR (CDCl₃): δ = 1.78-1.89 ppm m (4H); 2.32 t (J=8 Hz, 2H); 3.68 s (3H); 3.88 s (3H); 3.92 t (J = 7.5 Hz, 2H); 6.53 s (1H); 7.0 d (J=12 Hz, 2H); 7.18-7.36 m (6H); 7.48-7.58 m (2H)

### Beispiel 217

### 5-[[5-[[(4-Chlorphenyl)sulfonyl]amino]-1-(4-methoxyphenyl)-2-phenyl-1H-benzimidazol-6-yl)oxy)pentansäuremethylester

5-[(5-Amino-1-(4-methoxyphenyl)-2-phenyl-1*H*-benzimidazol-6-yl)oxy]pentansäure-methylester wurde gemäß der allgemeinen Arbeitsvorschrift 13 mit 4-Chlorbenzolsulfonsäurechlorid umgesetzt.
MS (EI): 619 (Molekülionpeak)

### Beispiel 218

### 6-[(5-Amino-1-(4-methoxyphenyl)-2-phenyl-1H-benzimidazol-6-yl)oxy]hexansäure-methylester

wurde analog zu 6-[(5-Amino-1-(3,4-dimethylphenyl)-2-phenyl-1*H*-benzimidazol-6-yl)oxy]-hexansäuremethylester dargestellt.
Fp. 129-131 °C

### Beispiel 219

### 6-[[5-[[(4-Chlorphenyl)sulfonyl]amino]-1-(4-methoxyphenyl)-2-phenyl-1H-benzimidazol-6-yl]oxy]hexansäuremethylester

6-[(5-Amino-1-(4-methoxyphenyl)-2-phenyl-1*H*-benzimidazol-6-yl)oxy]hexansäuremethylester wurde gemäß der allgemeinen Arbeitsvorschrift 13 mit 4-Chlorbenzolsulfonsäurechlorid umgesetzt.
Fp. 168-170°C

### Beispiel 220

### 6-[[5-[[(4-Chlorphenyl)sulfonyl]amino]-1-(4-methoxyphenyl)-2-phenyl-1H-benzimidazol-6-yl]oxy]hexansäure

wurde gemäß der allgemeinen Arbeitsvorschrift 9 dargestellt.
Fp. 181-182°C

### Beispiel 221

### 6-[(5-Amino-1-(4-methylphenyl)-2-phenyl-1H-benzimidazol-6-yl)oxy]hexansäuremethylester

wurde analog zu 6-[(5-Amino-1-(3,4-dimethylphenyl)-2-phenyl-1*H*-benzimidazol-6-yl)oxy]-hexansäuremethylester dargestellt.
Fp. 105-107°C

### Beispiel 222

### 6-[[5-[[(4-Chlorphenyl)sulfonyl]amino]-1-(4-methylphenyl)-2-phenyl-1H-benzimidazol6-yl]oxy]hexansäuremethylester

6-[(5-Amino-1-(4-methylphenyl)-2-phenyl-1*H*-benzimidazol-6-yl)oxy]hexansäuremethylester wurde gemäß der allgemeinen Arbeitsvorschrift 13 mit 4-Chlorbenzolsulfonsäurechlorid umgesetzt.
Fp. 189-191°C

### Beispiel 223

### 6-[[5-[[(4-Chlorphenyl)sulfonyl]amino]-1-(4-methylphenyl)-2-phenyl-1H-benzimidazol6-yl]oxy]hexansäure

wurde gemäß der allgemeinen Arbeitsvorschrift 9 dargestellt.
Fp. 102-105°C

### Beispiel 224 5-[(5-Amino-1,2-diphenyl-1H-benzimidazol-6-yl)oxy]pentansäuremethylester

wurde analog zu 6-[(5-Amino-1-(3,4-dimethylphenyl)2-phenyl-1*H*-benzimidazol-6-yl)oxy]-hexansäuremethylester dargestellt.
¹H-NMR (CDCl₃): δ = 1.82-1.95 ppm m (4H); 2.39 t (J=8 Hz, 2H); 3.69 s (3H); 3.92-4.00 m (2H); 6.60 s (1H); 7.26-7.34 m (6H); 7.43-7.58 m (5H)

### Beispiel 225 5-[[5-[[(4-Chlorphenyl)sulfonyl]amino]-1,2-diphenyl-1H-benzimidazol-6-yl]oxy]pentansäuremethylester

5-[(5-Amino-1,2-diphenyl-1*H*-benzimidazol-6-yl)oxy]pentansäuremethylester wurde gemäß der allgemeinen Arbeitsvorschrift 13 mit 4-Chlorbenzolsulfonsäurechlorid umgesetzt.
Fp. 157-161 °C

### Beispiel 226 5-[[5-[[(4-Chlorphenyl)sulfonyl]amino]-1,2-diphenyl-1H-benzimidazol-6-yl]oxy]pentansäure

wurde gemäß der allgemeinen Arbeitsvorschrift 9 dargestellt.
Fp. 236-242°C

### Beispiel 227 6-[[5-[[(4-Fluorphenyl)sulfonyl]amino]-1-(4-methoxyphenyl)-2-phenyl-1H-benzimidazol-6-yl]oxy]hexansäurermethylester

6-[(5-Amino-1-(4-methoxyphenyl}-2-phenyl-1*H*-benzimidazol-6-yl)oxy]hexansäuremethylester wurde gemäß der allgemeinen Arbeitsvorschrift 13 mit 4-Fluorbenzolsulfonsäurechlorid umgesetzt.
MS (EI): 617 (Molekülionpeak)

### Beispiel 228

### 6-[[5-[[(4-(Trifluormethyl)phenyl)sulfonyl]amino]-1-(4-methoxyphenyl)-2-phenyl-1H-benzimidazol-6-yl]oxy]hexansäuremethylester

6-[(5-Amino-1-(4-methoxyphenyl)-2-phenyl-1*H*-benzimidazol-6-yl)oxy]hexansäuremethylester wurde gemäß der allgemeinen Arbeitsvorschrift 13 mit 4-(Trifluormethyl)benzolsulfonsäurechlorid umgesetzt.
MS (EI): 668 (Molekülionpeak)

### Beispiel 229

### 6-[[5-[[(4-Trifluorphenyl)sulfonyl]amino]-1-(4-methoxyphenyl)-2-phenyl-1H-benzimidazol-6-yl]oxy]hexansäure

wurde gemäß der allgemeinen Arbeitsvorschrift 9 dargestellt.
Fp. 190-192°C

### Beispiel 230 6-[[5-[[(4-Chlorphenyl)sulfonyl]methylamino]-1-(4-methoxyphenyl)-2-phenyl-1H-benzimidazol-6-yl]oxy]hexansäuremethylester

100 mg 6-[[5-[[(4-Chlorphenyl)sulfonyl]amino]-1-(4-methoxyphenyl)-2-phenyl-1*H-*benzimidazol-6-yl]oxy]hexansäuremethylester wurden in 3 ml Tetrahydrofuran gelöst. Dazu gab man bei 0°C 10 mg Natriumhydrid, ließ für 30 min rühren, tropfte dann 50 µl Methyljodid zu und ließ für weitere 60 min bei 0°C rühren. Man versetzte mit ges. Ammoniumchloridlösung, extrahierte dreimal mit Ethylacetat, wusch die organischen Phasen mit Wasser, trocknete über Natriumsulfat und engte im Vakuum ein. Den Rückstand chromatographierte man an Kieselgel.
Fp. 178-180°C

### Beispiel 231

### [[(4-Chlorphenyl)sulfonyl][1,2-diphenyl-1H-benzimidazol-5-yl]amino]essigsäuremethylester

100 mg 4-Chlor-N-(1,2-Diphenyl-1*H*-benzimidazol-5-yl)benzolsulfonamid wurden in 0.5 ml *N,N*-Dimethylformamid suspendiert, mit 8 mg Natriumhydrid versetzt und 30 min bei 20°C gerührt. Man setzte 50 mg Bromessigsäuremethylester zu, ließ 15 h rühren, versetzte mit Wasser, extrahierte dreimal mit Ethylacetat, trocknete die Extrakte über Natriumsulfat, engte im Vakuum ein und chromatographierte den Rückstand an Kieselgel.
¹H-NMR (CDCl₃): δ = 3.70 ppm s (3H); 4.52 s (2H); 7.20 d (J = 8 Hz, 1H); 7.26-7.58 m (14H); 7.70 d (J = 10Hz, 2H)

### Beispiel 232

### [[(4-Chlorphenyl)sulfonyl][1,2-diphenyl-1H-benzimidazol-5-yl]amino]essigsäure

wurde gemäß der allgemeinen Arbeitsvorschrift 9 dargestellt.
Fp. 248°C

### Beispiel 233

### 4-[[(4-Chlorphenyl)sulfonyl][1,2-diphenyl-1H-benzimidazol-5-yl]amino]butansäuremethylester

100 mg 4-Chlor-N-(1,2-Diphenyl-1*H*-benzimidazol-5-yl)benzotsulfonamid wurden in 0.5 ml *N,N*-Dimethylformamid suspendiert, mit 6 mg Natriumhydrid versetzt und 30 min bei 20°C gerührt. Man setzte 56 mg 4-Brombuttersäuremethylester zu, ließ 15 h rühren, versetzte mit Wasser, extrahierte dreimal mit Ethylacetat, trocknete die Extrakte über Natriumsulfat, engte im Vakuum ein und digerierte den Rückstand mit Düsopropylether.
Fp. 54-58°C

### Beispiel 234

### 4-[[(4-Chlorphenyl)sulfonyl][1,2-diphenyl-1H-benzimidazol-5-yl]amino]butansäure

wurde gemäß der allgemeinen Arbeitsvorschrift 9 dargestellt.
Fp. 249-254°C

### Beispiel 235

### 5-[[(4-Chlorphenyl)sulfonyl][1,2-diphenyl-1H-benzimidazol-5-yl]amino]pentansäuremethylester

100 mg 4-Chlor-N-(1,2-Diphenyl-1*H*-benzimidazol-5-yl)benzolsulfonamid wurden in 0.5 ml N,N-Dimethylformamid suspendiert, mit 8 mg Natriumhydrid versetzt und 30 min bei 20°C gerührt. Man setzte 60 mg 5-Brompentansäuremethylester zu, ließ 15 h rühren, versetzte mit Wasser, extrahierte dreimal mit Ethylacetat, trocknete die Extrakte über Natriumsulfat, engte im Vakuum ein und chromatographierte den Rückstand an Kieselgel.
¹H-NMR (CDCl₃): δ = 1.46-1.54 ppm m (2H); 1.62-1.78 m (2H); 2.30 t (J=8 Hz, 2H); 3.62 s (3H); 3.62 t (J=8 Hz, 2H); 7.12-7.53 m (17H)

### Beispiel 236

### 5-[[(4-Chlorphenyl)sulfonyl][1,2-diphenyl-1H-benzimidazol-5-yl]amino]pentansäure

wurde gemäß der allgemeinen Arbeitsvorschrift 9 dargestellt.
Fp. 123-127°C

### Beispiel 237

### 6-[[(4-Chlorphenyl)sulfonyl][1,2-diphenyl-1H-benzimidazol-5-yl]amino]hexansäuremethylester

6-[[1,2-Diphenyl-1*H*-benzimidazol-5-yl]amino]hexansäuremethylester wurde gemäß der allgemeinen Arbeitsvorschrift 13 mit 4-Chlorbenzolsulfonsäurechlorid umgesetzt.
MS (EI): 588 (Molekülionpeak)

### Beispiel 238

### 7-[[(4-Chlorphenyl)sulfonyl][1,2-diphenyl-1H-benzimidazol-5-yl]amino]heptansäuremethylester

100 mg 4-Chlor-N-(1,2-Diphenyl-1*H*-benzimidazol-5-yl)benzolsulfonamid wurden in 0.5 ml N,N-Dimethylformamid suspendiert, mit 8 mg Natriumhydrid versetzt und 30 min bei 20°C gerührt. Man setzte 70 mg 7-Bromheptansäuremethylester zu, ließ 15 h rühren, versetzte mit Wasser, extrahierte dreimal mit Ethylacetat, trocknete die Extrakte über Natriumsulfat, engte im Vakuum ein und chromatographierte den Rückstand an Kieselgel.
¹H-NMR (CDCl₃): δ = 1.26-1.64 ppm m (8H); 2.27 t (J=8 Hz, 2H); 3.60 t (J=8 Hz, 2H); 3.68 s (3H); 7.12 dd (J=10, 2 Hz, 1H); 7.22 d (J=10 Hz, 1H); 7.30-7.61 m (15H)

### Beispiel 239

### 7-[[(4-Chlorphenyl)sulfonyl][1,2-diphenyl-1H-benzimidazol-5-yl]amino]heptansäure

**wurde gemäß der allgemeinen Arbeitsvorschrift 9 dargestellt.**
Fp. 172-178°C

### Beispiel 240

### N-(1,2-Diphenyl-1H-benzimidazol-5-yl)-4-fluorbenzolsulfonamid

5-Amino-1,2-diphenyl-1*H*-benzimidazol wurde gemäß der allgemeinen Arbeitsvorschrift 13 mit 4-Fluorbenzolsulfonsäurechlorid umgesetzt.
Fp. 209-214°C

### Beispiel 241

### 6-[[(4-Fluorphenyl)sulfonyl][1,2-diphenyl-1H-benzimidazol-5-yl]amino]hexansäure-methylester

150 mg N-(1,2-Diphenyl-1H-benzimidazot-5-yl)-4-fluorbenzolsulfonamid wurden in 0.5 ml *N,N*-Dimethylformamid suspendiert, mit 12 mg Natriumhydrid versetzt und 30 min bei 20°C gerührt. Man setzte 98 mg 6-Bromhexansäuremethylester zu, ließ 15 h rühren, versetzte mit Wasser, extrahierte dreimal mit Ethylacetat, trocknete die Extrakte über Natriumsulfat, engte im Vakuum ein und chromatographierte den Rückstand an Kieselgel.
Fp. 128-134°C

### Beispiel 242

### 6-[[(4-Ftuorphenyl)sulfonyl][1,2-diphenyl-1H-benzimidazol-5-yl]amino]hexansäure

wurde gemäß der allgemeinen Arbeitsvorschrift 9 dargestellt.
Fp. 200-210°C

### Beispiel 243

### 6-[[[4-(Trifluormethyl)phenyl]sulfonyl][1,2-diphenyl-1H-benzimidazol-5-yl]-amino]hexansäuremethylester

150 mg 4-(Trifluormethyl)-N-(1,2-Diphenyl-1*H*-benzimidazol-5-yl)benzolsulfonamid wurden in 0.5 ml *N,N*-Dimethylformamid suspendiert, mit 11 mg Natriumhydrid versetzt und 30 min bei 20°C gerührt. Man setzte 88 mg 6-Bromhexansäuremethylester zu, ließ 15 h rühren, versetzte mit Wasser, extrahierte dreimal mit Ethylacetat, trocknete die Extrakte über Natriumsulfat, engte im Vakuum ein und digerierte den Rückstand mit Diisopropyether.
Fp. 159-161°C

### Beispiel 244

### 6-[[[4-(Trifluormethyl)phenyl]sulfonyl][1,2-diphenyl-1H-benzimidazol-5-yl]-amino]hexansäure

wurde gemäß der allgemeinen Arbeitsvorschrift 9 dargestellt.
Fp. 224-230°C

### Beispiel 245

### 4-Chlor-N-[1-(4-methoxyphenyl)-2-phenyl-1H-benzimidazol-5-yl]benzolsulfonamid

### a) (2,4-Dinitrophenyl)(4-methoxyphenyl)amin

1.43 g 4-(2,4-Dinitroanilino)phenol, 500 mg Kaliumcarbonat und 0.32 ml Methyljodid wurden in 5 ml *N,N*-Dimethylformamidfür 2 d bei 20°C gerührt. Man goß das Gemisch auf Wasser, extrahierte dreimal mit Ethylacetat, trocknete die Extrakte über Natriumsulfat, engte im Vakuum ein und chromatographierte den Rückstand an Kieselgel.
Fp. 117-127°C

### b) 5-Amino-1-(4-methoxyphenyl)-2-phenyl-1H-benzimidazol

(2,4-Dinitrophenyl)(4-methoxyphenyl)amin wurde gemäß der allgemeinen Arbeitsvorschrift 1 hydriert. Das Rohprodukt wurde gemäß der allgemeinen Arbeitsvorschrift 3 mit Trimethylorthobenzoat zum Benzimidazolderivat zyklisiert.
¹H-NMR (CDCl₃): δ = 3.88 ppm s (3H); 6.70 dd (J=12, 2 Hz, 1H); 6.95-7.06 m (4H); 7.18-7.38 m (7H); 7.53-7.65 m (2H)

### c) 4-Chlor-N-[1-(4-methoxyphenyl)-2-phenyl-1H-benzimidazol-5-yl]benzolsulfonamid

5-Amino-1-(4-methoxyphenyl)-2-phenyl-1*H*-benzimidazol wurde gemäß der allgemeinen Arbeitsvorschrift 13 mit 4-Chlorbenzolsulfonsäurechlorid umgesetzt.
Fp. 238-24°C

### Beispiel 246

### 6-[[(4-Chlorphenyl)sulfonyl][1-(4-methoxyphenyl)-2-phenyl-1H-benzimidazol-5-yl]amino]hexansäuremethylester

75 mg 4-Chlor-*N*-[1-(4-methoxyphenyl)-2-phenyl-1*H*-benzimidazol-5-yl]benzolsulfonamid wurden in 0.5 ml *N,N*-Dimethylformamid suspendiert, mit 6 mg Natriumhydrid versetzt und 30 min bei 20°C gerührt. Man setzte 44 mg 6-Bromhexansäuremethylester zu, ließ 15 h rühren, versetzte mit Wasser, extrahierte dreimal mit Ethylacetat, trocknete die Extrakte über Natriumsulfat, engte im Vakuum ein und chromatographierte den Rückstand an Kieselgel.
MS (EI): 617 (Molekülionpeak)

### Beispiel 247

### 6-[[(4-Chlorphenyl)sulfonyl][1-(4-methoxyphenyl)-2-phenyl-1H-benzimidazol-5-yl]amino]hexansäure

wurde gemäß der allgemeinen Arbeitsvorschrift 9 dargestellt.
Fp. 205-208°C

### Beispiel 248

### 2,2-Dimethyl-6-[(1,2-diphenyl-1H-benzimidazol-6-yl)oxy]hexanamid

### a) 2,2-Dimethyl-6-[(1,2-diphenyl-1H-benzimidazol-6-yl)oxy]hexannitril wurde durch

Umsetzung von 1,2-Diphenyl-6-hydroxy-1*H*-benzimidazol mit 6-Brom-1,1-dimethylhexannitril gemäß der allgemeinen Arbeitsvorschrift 8 erhalten.
Fp. 115-118°C

### b) 2,2-Dimethyl-6-[(1,2-diphenyl-1H-benzimidazol-6-yl)oxy]hexanamid

500 mg 2,2-Dimethyl-6-[(1,2-diphenyl-1*H*-benzimidazol-6-yl)oxy]hexannitril wurden für 2h in 5 ml 80%iger Schwefelsäure zum Rückfluß erhitzt. Nach dem Erkalten wurde vorsichtig auf Eiswasser gegeben, der pH-Wert mit Natronlauge auf pH 8 eingestellt, dreimal mit Ethylacetat extrahiert, die Extrakte über Natriumsulfat getrocknet, und im Vakuum eingeengt. Der Rückstand wurde an Kieselgel chromatographiert.
Fp. 115-118°C

### Beispiel 249

### 8-[(1,2-Diphenyl-1H-benzimidazol-6-yl)oxy)octansäuremethylester

wurde durch Umsetzung von 1,2-Diphenyl-6-hydroxy-1*H*-benzimidazol mit 8-Bromoctansäuremethylester gemäß der allgemeinen Arbeitsvorschrift 8 erhalten.
Fp. 92-95°C

### Beispiel 250

### 8-[(1,2-Diphenyl-1H-benzimidazol-6-yl)oxy]octansäure

wurde gemäß der allgemeinen Arbeitsvorschrift 9 dargestellt.
Fp. 136-140°C

### Beispiel 251

### 6-[[1-(Indan-5-yl)-2-phenyl-1H-benzimidazol-6-yl]oxy]hexansäuremethylester

wurde analog zu 6-[[1-(3,4-Dimethylphenyl)2-phenyl-1*H*-benzimidazol-6-yl]oxy]hexansäuremethylester dargestellt.
Fp. 81-85°C

### Beispiel 252

### 6-[[1-(Indan-5-yl)-2-phenyl-1H-benzimidazol-6-yl]oxy]hexansäure

wurde gemäß der allgemeinen Arbeitsvorschrift 9 dargestellt.
Fp. 176-180°C

### Beispiel 253

### 7-[[1-(lndan-5-yl)-2-phenyl-1H-benzimidazol-6-yl]oxy]heptansäuremethylester

wurde analog zu 6-[[1-(3,4-Dimethylphenyl)-2-phenyl-1*H*-benzimidazol-6-yl]oxy]hexansäuremethylester dargestellt.
Fp. 92-98°C

### Beispiel 254

### 7-[[1-Indan-5-yl)-2-phenyl-1H-benzimidazol-6-yl]oxy]heptansäure

wurde gemäß der allgemeinen Arbeitsvorschrift 9 dargestellt.
Fp. 175-.178°C

### Beispiel 255

### 6-[[1-(3-Fluorphenyl)-2-phenyl-1H-benzimidazol-6-yl]oxy]hexansäuremethylester

wurde analog zu 6-[[1-(3,4-Dimethylphenyl)-2-phenyl-1*H*-benzimidazol-6-yl]oxy]hexansäuremethylester dargestellt.
Fp. 104-106°C

### Beispiel 256

### 6-[[1-(3-Fluorphenyl)-2-phenyl-1H-benzimidazol-6-yl]oxy]hexansäure

wurde gemäß der allgemeinen Arbeitsvorschrift 9 dargestellt.
Fp. 149-151°C

### Beispiel 257

### 6-[[2-(4-Nitrophenyl)-1-phenyl-1H-benzimidazol-6-yl]oxy]hexansäuremethylester

### a) 6-Methoxy-2-(4-nitrophenyl)-1-phenyl-1H-benzimidazol

200 mg 4-Methoxy-N²-phenyl-*o*-phenylendiamin wurden in 5 ml *N,N*-Dimethylforamid gelöst, mit 346 mg EEDQ und 234 mg 4-Nitrobenzoesäure versetzt und die Mischung 5 h bei 100°C gerührt. Nach dem Abkühlen wurde mit Wasser versetzt. Der Niederschlag wurde abgesaugt und durch Säulenchromatographie gereinigt, in 6 N Salzsäure aufgenommen, und 2 h unter Rückfluß erhitzt. Nach dem Erkalten wurde in ges.

Kaliumhydrogencarbonatlösung eingetropft. Der Niederschlag wurde abgesaugt und getrocknet.
Fp. 189-191°C

### b) 6-Hydroxy-2-(4-nitrophenyl)-1-phenyl-1H-benzimidazol

wurde duch Umsetzung gemäß der allgemeinen Arbeitsvorschrift 6 erhalten.
¹H-NMR (D₆-DMSO): δ = 6.56 ppm d (J=2 Hz, 1H); 6.87 dd (J=10, 2 Hz, 1H); 7.46 dd (J=10, 2 Hz, 2H); 7.53-7.70 m (4H); 7.75 d (J=10 Hz, 2H); 8.20 d (J=10 Hz, 2H); 9.55 s (breit)(1H)

### c) 6-[[2-(4-Nitrophenyl)-1-phenyl-1H-benzimidazol-6-yl]oxy]hexansäuremethylester

wurde duch Umsetzung gemäß der allgemeinen Arbeitsvorschrift 8 erhalten.
¹H-NMR (CDCl₃): δ = 1.45-1.55 ppm m (2H); 1.62-1.84 m (4H); 2.33 t (J=8 Hz, 2H); 3.68 s (3H); 3.95 t (J=8 Hz, 2H); 6.67 d (J=2 Hz, 1H); 7.00 dd (J=10, 2 Hz, 1H); 7.28-7.38 m (2H); 7.52-7.60 m (3H); 7.71 d (J=10 Hz, 2H); 7.77 d (J=10 Hz, 1H); 8.13 d (J=10 Hz, 2H)

### Beispiel 258

### 6-[[2-(4-Nitrophenyl)-1-phenyl-1H-benzimidazol-6-yl]oxy]hexansäure

wurde gemäß der allgemeinen Arbeitsvorschrift 9 dargestellt.
Fp. 181-186°C

### Beispiel 259

### 6-[[1-Phenyl-2-(3-pyridinyl)-1H-benzimidazol-6-yl]oxy]hexansäuremethylester

wurde analog zu 6-[[1-Phenyl-2-(4-pyridinyl)-1*H*-benzimidazol-6-yl]oxy]hexansäuremethylester dargestellt.
Fp. 159-160°C

### Beispiel 260

### N-(Cyclopropylmethoxy)-6-[(1,2-diphenyl-1H-benzimidazol-6-yl)oxy]hexanamid

wurde gemäß der allgemeinen Arbeitsvorschrift 18 dargestellt.
MS (EI): 469 (Molekülionpeak)

### Beispiel 261

### N-Isobutoxy-6-[(1,2-dlphenyl-1H-benzimidazol-6-yl)oxy]hexanamid

wurde gemäß der allgemeinen Arbeitsvorschrift 18 dargestellt.
MS (EI): 471 (Molekülionpeak)

### Beispiel 262

### N-(Phenylmethoxy)-6-[2-phenyl-1-(3,4,5-trimethoxyphenyl)-1H-benzimidazol-6-yl)oxy]-hexanamid

Zu einer Lösung aus 17 mg Carbonyldümidazol in 1 ml Tetrahydrofuran gab man eine Lösung aus 50 mg 6-[2-Phenyl-1-(3,4,5-trimethoxyphenyl)-1H-benzimidazol-6-yl)oxy]hexansäure in 1 ml Tetrahydrofuran, rührte für 30 min bei 20°C und erhitzte für 30 min zum Rückfluß. Bei 20°C gab man 16 mg *O*-Benzylhydroxylamin Hydrochlorid zu und ließ 20 h rühren. Zur Aufarbeitung gab man Ethylacetat zu, extrahiert mit 2 N Salzsäure und ges. Natriumhydrogencarbonatlösung, trocknete über Natriumsulfat und engte im Vakuum ein. Der Rückstand wurde durch Säulenchromatographie an Kieselgel gereinigt.
Fp. 145-148°C

### Beispiel 263

### N-(Cyclopropylmethoxy)-6-[2-phenyl-1-(3,4,5-trimethoxyphenyl)-1H-benzimidazol-6-yl)oxy]hexanamid

wurde analog zu *N*-(Phenylmethoxy)-6-[2-phenyl-1-(3,4,5-trimethoxyphenyl)-1*H-*benzimidazol-6-yl)oxy]hexanamid dargestellt.
MS (EI): 559 (Molekülionpeak)

### Beispiel 264

### N-Isobutoxy-6-[2-phenyl-1-(3,4,5-trimethoxyphenyl)-1H-benzimidazol-6-yl)oxy]hexan-amid

wurde analog zu *N*-(Phenylmethoxy)-6-[2-phenyl-1-(3,4,5-trimethoxyphenyl)-1H-benzimidazol-6-yl)oxy]hexanamid dargestellt.
¹H-NMR (CDCl₃): δ = 0.94 ppm d(J=8 Hz, 6H); 1.48-2.03 m (7H); 2.05-2.18 m (2H); 3.60-3.72 m (2H); 3.76 s (6H); 3.90-4.00 m (2H); 3.96 s (3H); 6.50 s (2H); 6.72 d (J=2 Hz, 1H); 6.95 dd (J=10, 2 Hz, 1H); 7.28-7.38 m (3H); 7.55-7.62 m (2H); 7.74 d (J=10 Hz, 1H); 8.20 s (breit) (1H)

### Beispiel 265

### N-Isopropyl-6-[(1,2-diphenyl-1H-benzimidazol-6-yl)oxy]hexanamid

wurde gemäß der allgemeinen Arbeitsvorschrift 17 dargestellt.
Fp. 107-112°C

### Beispiel 266

### N,N-Dimethyl-6-[(1,2-diphenyl-1H-benzimidazol-6-yl)oxy]hexanamid

wurde gemäß der allgemeinen Arbeitsvorschrift 17 dargestellt.
Fp. 83-88°C

### Beispiel 267

### 6-[(1,2.Diphenyl-1H-benzimidazol-6yl)oxy]-1-pyrrolidin-1-ylhexan-1-on

wurde gemäß der allgemeinen Arbeitsvorschrift 17 dargestellt.
Fp. 84-88°C

### Beispiel 268

### N-(2-Methoxyethyl)-6-[(1,2-diphenyl-1H-benzimidazol-6-yl)oxy]hexanamid

wurde gemäß der allgemeinen Arbeitsvorschrift 17 dargestellt.
Fp. 63-68°C

### Beispiel 269

### N-(3-Methoxypropyl)-6-[(1,2-diphenyl-1H-benzimidazol-6-yl)oxy]hexanamid

wurde gemäß der allgemeinen Arbeitsvorschrift 18 dargestellt.
Fp. 84-91°C

### Beispiel 270

### N-isobutyl-6-[(1,2-diphenyl-1H-benzimidazol-6-yl)oxy]hexanamid

wurde gemäß der allgemeinen Arbeitsvorschrift 17 dargestellt.
¹H-NMR (CDCl₃): δ = 0.90 ppm d(J=8 Hz, 6H); 1.44-1.57 m (2H); 1.65-1.85 m (5H); 2.20 t (J=8 Hz, 2H); 3.08 t (J=8 Hz, 2H); 3.94 t (J=8 Hz, 2H); 6.68 d (J=2 Hz, 1H); 6.96 dd (J=10, 2 Hz, 1H); 7.25-7.38 m (5H); 7.45-7.58 m (5H); 7.75 d (J=10 Hz, 1H)

### Beispiel 271

### N-[(2,2-Dimethylamino)ethyl]-N-methyl-6-[(1,2-diphenyl-1H-benzimidazol-6-yl)-oxy]hexanamid

wurde gemäß der allgemeinen Arbeitsvorschrift 17 dargestellt.
¹H-NMR (CDCl₃) (Signale des Hauptrotameren): δ = 1.44-1.57 ppm m (2H); 1.64-1.84 m (4H); 2.30 s (6H); 2.34 t (J=8 Hz, 2H); 2.47 t (J=8 Hz. 2H); 3.00 s (3H); 3.50 t (J=8 Hz, 2H); 3.94 t (J=8Hz. 2H); 6.69 d (J=2 Hz. 1H); 6.96 dd (J=10, 2 Hz, 1H); 7.25-7.36 m (5H); 7.45-7.56 m (5H); 7.73 d (J=10 Hz, 1H)

### Beispiel 272

***N*-(2-Methoxyethyl)-*N*-methyl-6-[(1,2-diphenyl-1*H*-benzimidazol-6-yl)oxylhexanamid** wurde gemäß der allgemeinen Arbeitsvorschrift 17 dargestellt.
¹H-NMR (CDCl₃) (Signale des Hauptrotameren): δ = 1.43-1.58 ppm m (2H); 1.63-1.84 m (4H); 2.33 t (J=8 Hz, 2H); 3.07 s (3H); 3.32 s (3H); 3.47-3.58 m (4H); 3.95 t (J=8Hz, 2H); 6.70 d (J=2 Hz, 1H); 6.96 dd (J=10, 2 Hz, 1H); 7.25-7.35 m (5H); 7.45-7.55 m (5H); 7.75 d (J=10 Hz, 1H)

### Beispiel 273 6-[(1,2-Diphenyl)-1H-benzimidazol-6-yl)oxy]-1-morpholin-1-ylhexan-1-on

**wurde gemäß der allgemeinen Arbeitsvorschrift 17 dargestellt.**
¹H-NMR (CDCl₃): δ = 1.47-1.59 ppm m (2H); 1.63-1.88 m (4H); 2.34 t (J=8 Hz, 2H); 3.42-3.49 m (2H); 3.57-3.70 m (6H); 3.94 t (J=8Hz, 2H); 6.68 d (J=2 Hz. 1H); 6.96 dd (J=10, 2 Hz, 1H); 7.23-7.38 m (5H); 7.45-7.56 m (5H); 7.75 d (J=10 Hz, 1H)

### Beispiel 274

### N,N-Di(-2-methoxyethyl)-6-[(1,2-diphenyl-1H-benzimidazol-6-yl)oxy]hexanamid

wurde gemäß der allgemeinen Arbeitsvorschrift 18 dargestellt.
Fp. 88-98°C

### Beispiel 275

### N-Isopentyl-6-[(1,2.diphenyl-1H-benzimidazol-6-yl)oxy]hexanamid

wurde gemäß der allgemeinen Arbeitsvorschrift 18 dargestellt
Fp. 127-129°C

### Beispiel 276

### N-(Pyridin-2-yl)-6-[(1,2-diphenyl-1H-benzimidazol-6-yl)oxy]hexanamid

wurde gemäß der allgemeinen Arbeitsvorschrift 18 dargestellt.
Fp. 120-124°C

### Beispiel 277

### N-(Pyridin-3-yl)-6-[(1,2-diphenyl-1H-benzimidazol-6-yl)oxy]hexanamid

wurde gemäß der allgemeinen Arbeitsvorschrift 18 dargestellt.
Fp. 154°C

### Beispiel 278

### 6-[(1,2-Diphenyl-1H-benzimidazol-6-yl)oxy]-1-piperidin-1-ylhexan1-on

wurde gemäß der allgemeinen Arbeitsvorschrift 18 dargestellt.
Fp. 93-98°C

### Beispiel 279

### [6-[(1,2-Diphenyl-1H-benzimidazol-6-yl)oxy]-1-hexanoyl]piperidin-4-carbonamid

wurde gemäß der allgemeinen Arbeitsvorschrift 17 dargestellt.
Fp. 177-178°C

### Beispiel 280 [[6-[(1,2-Diphenyl-1H-benzimidazol-6-yl)oxy]-1-hexanoyl]methylamino]-essigsäureethylester

wurde gemäß der allgemeinen Arbeitsvorschrift 17 dargestellt.
¹H-NMR (CDCl₃) (Signale des Hauptrotameren): δ = 1.23 ppm t (J=8 Hz, 3H); 1.45-1.88 m (6H); 2.40 t (J=8 Hz, 2H); 3.08 s (3H); 3.93 t (J=8 Hz, 2H); 4.12 s (2H); 4.18 q (J=8 Hz, 2H); 6.70 d (J=2 Hz, 1H): 6.97 dd (J=10, 2 Hz, 1 H); 7.23-7.35 m (5H); 7.45-7.58 m (5H); 7.75 d (J=10 Hz, 1H)

### Beispiel 281

### 4-[[6-[(1,2-Diphenyl-1H-benzimidazol-6-yl)oxy]-1-hexanoyl]]piperazin-1-carbonsäureethylester

wurde gemäß der allgemeinen Arbeitsvorschrift 17 dargestellt.
¹H-NMR (CDCl₃): δ = 1.27 ppm t (J=8 Hz, 3H); 1.45-1.60 m (2H); 1.63-1.88 m (4H); 2.36 t (J=8 Hz, 2H); 3.40-3.53 m (6H); 3.56-3.64 m (2H); 3.93 t (J=8 Hz, 2H); 4.15 q (J=8 Hz, 2H); 6.69 d (J=2 Hz. 1 H); 6.96 dd (J=10, 2 Hz, 1 H); 7.23-7.38 m (5H); 7.45-7.56 m (5H); 7.76 d (J=10 Hz, 1 H)

### Beispiel 282

### N-Isopropyl-6-[[1-(3,4-dimethylphenyl)-2-phenyl-1H-benzimidazol-6-yl]oxy]hexanamid

wurde gemäß der allgemeinen Arbeitsvorschrift 18 dargestellt.
MS (EI): 469 (Molekülionpeak)

### Beispiel 283

### N,N-Dimethyl-6-[[1-(3,4-dimethylphenyl)-2-phenyl-1H-benzimidazol-6-yl]oxy]-hexanamid

wurde gemäß der allgemeinen Arbeitsvorschrift 18 dargestellt.
MS (EI): 455 (Molekülionpeak)

### Beispiel 284

### N,N-Diethyl-6-[[1-(3,4-dimethylphenyl)-2-phenyl-1H-benzimidazol-6-yl]oxy]hexanamid

wurde gemäß der allgemeinen Arbeitsvorschrift 18 dargestellt.
MS (EI): 483 (Molekülionpeak)

### Beispiel 285

### N-Isobutyl-6-[[1-(3,4-dimethylphenyl)-2-phenyl-1H-benzimidazol-6-yl]oxy]hexanamid

wurde gemäß der allgemeinen Arbeitsvorschrift 18 dargestellt.
¹H-NMR (CDCl₃): δ = 0.90 ppm d (J=8 Hz, 6H); 1.44-1.55 m (2H); 1.58-1.83 m (5H); 2.20 t (J=8 Hz, 2H); 2.30 s (3H); 2.35 s (3H); 3.09 t (J=8 Hz, 2H); 3.94 t (J=8 Hz, 2H); 6.63 d (J=2 Hz, 1H); 6.94 dd (J=10, 2 Hz, 1H); 7.02 dd (J=10, 2 Hz, 1H); 7.10 d (J=2 Hz, 1 H); 7.22-7.35 m (4H); 7.56 dd J=8 Hz u. 2 Hz, 2H); 7.73 d (J=10 Hz, 1H)

### Beispiel 286

### N-Cyclopropyl-6-[[1-(3,4-dimethylphenyl)-2-phenyl-1H-benzimidazol-6-yl]oxy]-hexanamid

wurde gemäß der allgemeinen Arbeitsvorschrift 18 dargestellt.
MS (EI): 467 (Molekülionpeak)

### Beispiel 287

### N-Cyclobutyl-6-[[1-(3,4-dimethylphenyl)-2-phenyl-1H-benzimidazol-6-yl]-oxy]hexanamid

wurde gemäß der allgemeinen Arbeitsvorschrift 18 dargestellt.
¹H-NMR (CDCl₃): δ = 1.42-1.55 ppm m (2H); 1.60-1.88 m (8H); 2.15 t (J=8 Hz, 2H); 2.28-2.40 m (2H); 2.30 s (3H); 2.35 s (3H); 3.93 t (J=8 Hz, 2H); 4.40 quintett (J= 8 Hz, 2H); 5.55 s (breit)(1 H); 6.63 d (J=2 Hz, 1 H); 6.92 dd (J=10,2 Hz, 1 H); 7.03 dd (J=10 Hz u. 2 Hz, 1 H); 7.08 d (J= 2 Hz, 1H); 7.20-7.36 m (4H); 7.57 dd (J=8, 2 Hz, 2H); 7.72 d (J=10 Hz, 1H)

### Beispiel 288

### N-tert-Butyl-6-[[1-(3,4-dimethylphenyl)-2-phenyl-1H-benzimidazol-6-yl]oxy]hexanamid

wurde gemäß der allgemeinen Arbeitsvorschrift 18 dargestellt.
¹H-NMR (CDCl₃): δ = 1.32 ppm s (9H); 1.42-1.55 m (2H); 1.62-1.82 m (4H); 2.10 t (J=8 Hz, 2H); 2.30 s (3H); 2.36 s (3H); 3.92 t (J=8 Hz, 2H); 5.23 s (breit)(1 H); 6.66 d (J=2 Hz. 1 H); 6.93 dd (J=10, 2 Hz, 1H); 7.02 dd (J=10 Hz u. 2 Hz, 1 H); 7.09 s (breit) (1H); 7.22-7.36 m (4H); 7.56 dd (J=8, 2 Hz, 2H); 7.73 d (J=10 Hz, 1 H)

### Beispiel 289

### (R)-6-[[1-(3,4-Dimethylphenyl)-2-phenyl-1H-benzimidazol-6-yl]oxy]1-(2-methoxy-methyl)pyrrolidin-1-ylhexan-1-on

wurde gemäß der allgemeinen Arbeitsvorschrift 18 dargestellt.
MS (EI): 467 (Molekülionpeak)

### Beispiel 290

### N-(3-Imidazol-1-yl-propyl)-6-[[1-(3,4-dimethylphenyl)-2-phenyl-1H-benzimidazol-6-yl]oxy]hexanamid

wurde gemäß der allgemeinen Arbeitsvorschrift 18 dargestellt.
¹H-NMR (CDCl₃): δ = 1.42-1.53 ppm m (2H); 1.62-2.02 m (6H); 2.17 t (J=8 Hz, 2H); 2.27 s (3H); 2.34 s (3H); 3.24 q (J=8 Hz, 2H); 3.92 t (J=8 Hz, 2H); 3.96 t (J=8 Hz, 2H); 5.68 s (breit)(1H); 6.63 d (J=2 Hz, 1H); 6.88-6.95 m (2H); 7.00 dd (J=10 Hz u. 2 Hz, 1H); 7.04-7.10 m (2H); 7.20-7.36 m (4H); 7.50 s (breit) (1H); 7.53 dd (J=8, 2 Hz, 2H); 7.72 d (J=10 Hz, 1H)

### Beispiel 291

### N-(2-Pyridin-2-ylethyl)-6-[[1-(3,4-dimethylphenyl)-2-pheny1-1H-benzimidazol-6-yl]oxy]hexanamid

wurde gemäß der allgemeinen Arbeitsvorschrift 18 dargestellt.
¹H-NMR (CDCl₃): δ = 1.38-1.52 ppm m (2H); 1.62-1.82 m (4H); 2.15 t (J=8 Hz, 2H); 2.30 s (3H); 2.35 s (3H); 2.96 t (J=8 Hz, 2H); 3.66 q (J=8 Hz, 2H); 3.90 t (J=8 Hz, 2H); 6.48 s (breit)(1 H); 6.65 d (J=2 Hz, 1 H); 6.92 dd (J=10, 2 Hz, 1 H); 7.00 dd (J=10 Hz u. 2 Hz, 1 H); 7.06-7.38 m (7H); 7.53-7.62 m (3H); 7.72 d (J=10 Hz, 1H); 8.50 d (breit)(J=6 Hz, 1H)

### Beispiel 292

### N,N-Dimethyl-6-[[2-(4-nitrophenyl)-1-phenyl-1H-benzimidazol-6-yl]oxy]hexanamid

wurde gemäß der allgemeinen Arbeitsvorschrift 18 dargestellt.
¹H-NMR (CDCl₃): δ = 1.46-1.58 ppm m (2H); 1.64-1.88 m (4H); 2.32 t (J=8 Hz, 2H); 2.93 s (3H); 3.00 s (3H); 3.96 t (J=8 Hz, 2H); 6.65 d (J=2 Hz, 1H); 7.00 dd (J=10, 2 Hz, 1H); 7.28-7.36 m (2H); 7.53-7.61 m (3H); 7.70 d (J=10 Hz, 2H); 7.76 d (J=8 Hz, 1 H); 8.13 d (J=8 Hz, 2H)

### Beispiel 293

### N-Isopropyl-6-[[2-(4-nitrophenyl)-1-phenyt-1H-benzimidazol-6-yl]oxylhexanamid

wurde gemäß der allgemeinen Arbeitsvorschrift 18 dargestellt.
Fp. 162-165°C

### Beispiel 294

### N-Isopentyl-6-[[2-(4-nitrophenyl)-1-phenyl-1H-benzimidazol-6-yl]oxy]hexanamid

wurde gemäß der allgemeinen Arbeitsvorschrift 18 dargestellt.
Fp. 148-154°C

### Beispiel 295

### N-(3-Methoxypropyl)-6-[[2-(4-nitrophenyl)-1-phenyl-1H-benzimidazol-6-yl]-oxy]hexanamid

wurde gemäß der allgemeinen Arbeitsvorschrift 18 dargestellt.
Fp. 104-110°C

### Beispiel 296

### N-(3-Methoxypropyl)-6-[[1-(indan-5-yl)-2-phenyl-1H-benzimidazol-6-yl]oxy]hexanamid

wurde gemäß der allgemeinen Arbeitsvorschrift 18 dargestellt.
¹H-NMR (CDCl₃): δ = 1.43-1.56 ppm m (2H); 1.62-1.85 m (6H); 2.10-2.23 m (4H); 2.95 t (J=10 Hz, 2H); 3.00 t (J= 10 Hz, 2H); 3.32 s (3H); 3.32-3.40 m (2H); 3.48 t (J=8 Hz, 2H); 3.93 t (J=8 Hz, 2H); 6.03 s (breit)(1H); 6.67 d (J=2 Hz, 1H); 6.93 dd (J=10, 2 Hz, 1 H); 7.03 dd (J=10, 2 Hz, 1H); 7.12 s (breit)(1H); 7.26-7.35 m (4H); 7.55 dd (J=10 Hz, 2H); 7.72 d (J=8 Hz, 1 H)

### Beispiel 297

### 6-[[1-(4-Methylphenyl)-2-(3-pyridyl)-1H-benzimidazol-6-yl]oxy]hexansäuremethylester

wurde durch Umsetzung aus 6-[[4-Amino-3-((4-methylphenyl)amino)phenyl]-oxy]hexansäuremethylester mit 3-Pyridylcarbaldehyd gemäß der allgemeinen Arbeitsvorschrift 16 erhalten.
MS (EI): 429 (Molekülionpeak)

### Beispiel 298

### 6-[[1-(4-Methylphenyl)-2-(4-pyridyl)-1H-benzimidazol-6-yl]oxy]hexansäuremethylester

wurde durch Umsetzung aus 6-[[4-Amino-3-((4-methylphenyl)amino)phenyl]-oxy]hexansäuremethylester mit 4-Pyridylcarbaldehyd gemäß der allgemeinen Arbeitsvorschrift 16 erhalten.
MS (EI): 429 (Molekülionpeak)

### Beispiel 299

### 6-[[1-(4-Methylphenyl)-2-(2-thienyl)-1 H-benzimidazol-6-yl]oxy]hexansäuremethylester

wurde durch Umsetzung aus 6-[[4-Amino-3-((4-methylphenyl)amino)phenyl]-oxy]hexansäuremethylester mit 2-Thienylcarbaldehyd gemäß der allgemeinen Arbeitsvorschrift 16 erhalten.
MS (EI): 434 (Molekülionpeak)

### Beispiel 300

### 6-[[1-(4-Methylphenyl)-2-(3-thienyl)-1H-benzimidazol-6-yl]oxy]hexansäuremethylester

wurde durch Umsetzung aus 6-[[4-Amino-3-((4-methylphenyl)amino)phenyl]-oxy]hexansäuremethylester mit 3-Thienylcarbaldehyd gemäß der allgemeinen Arbeitsvorschrift 16 erhalten.
MS (EI): 434 (Molekülionpeak)

### Beispiel 301

### 6-[[2-(3-Indolyl)-1-(4-methylphenyl)-1H-benzimidazol-6-yl]oxy]hexansäuremethylester

wurde durch Umsetzung aus 6-[[4-Amino-3-((4-methylphenyl)amino)phenyl]-oxy]hexansäuremethylester mit 3-Indolylcarbaldehyd gemäß der allgemeinen Arbeitsvorschrift 16 erhalten.
MS (EI): 467 (Molekülionpeak)

### Beispiel 302

### 6-[[1-(4-Methylphenyl)-2-(2-furyl)-1H-benzimidazol-6-yl]oxy]hexansäuremethylester

wurde durch Umsetzung aus 6-[[4-Amino-3-((4-methylphenyl)amino)phenyl]-oxy]hexansäuremethylester mit 2-Furylcarbaldehyd gemäß der allgemeinen Arbeitsvorschrift 16 erhalten.
MS (EI): 418 (Molekülionpeak)

### Beispiel 303

### 6-[[1-(4-Methylphenyl)-2-(3-furyl)-1H-benzimidazol-6-yl]oxy]hexansäuremethylester

wurde durch Umsetzung aus 6-[[4-Amino-3-((4-methylphenyl)amino)phenyl]-oxy]hexansäuremethylester mit 3-Furylcarbaldehyd gemäß der allgemeinen Arbeitsvorschrift 16 erhalten.
MS (EI): 418 (Molekülionpeak)

### Beispiel 304

### 6-[[1-(4-Methylphenyl)-2-(5-methyl-2-thienyl)-1H-benzimidazol-6-yl]oxy]hexansäuremethylester

wurde durch Umsetzung aus 6-[[4-Amino-3-((4-methylphenyl)amino)phenyl]-oxy]hexansäuremethylester mit 5-Methyl-2-thienyl-carbaldehyd gemäß der allgemeinen Arbeitsvorschrift 16 erhalten.
MS (EI): 448 (Molekülionpeak)

### Beispiel 305

### 6-[[1-(4-Methylphenyl)-2-(4-brom-2-thienyl)-1H-benzimidazol-6-yl]oxy]hexansäuremethylester

wurde durch Umsetzung aus 6-[[4-Amino-3-((4-methylphenyl)amino)phenyl]-oxy]hexansäuremethylester mit 4-Brom-2-thienylcarbaldehyd gemäß der allgemeinen Arbeitsvorschrift 16 erhalten.
MS (EI): 512/514 (Molekülionpeaks)

### Beispiel 306

### 6-[[1-(4-Methylphenyl)-2-(3-methyl-2-thienyl)-1H-benzimidazol-6-yl]oxy]hexan-säuremethylester

wurde durch Umsetzung aus 6-[[4-Amino-3-((4-methylphenyl)amino)phenyl]-oxy]hexansäuremethylester mit 3-Methyl-2-thienylcarbaldehyd gemäß der allgemeinen Arbeitsvorschrift 16 erhalten.
MS (EI): 448 (Molekülionpeak)

### Beispiel 307

### Hemmung der Mikroglia-Aktivierung

Zur *in vitro* Darstellung von Aß-aktivierten Mikroglia werden primäre Ratten-Mikroglia mit synthetischem Aß Peptid inkubiert:
Zur Simulierung von Aß-Ablagerungen wird synthetisches Aß Peptid auf 96-Loch Gewebekulturplatten eingetrocknet. Dazu wird eine Peptidstammlösung von 2mg/ml H₂O 1:50 in H₂O verdünnt. Zur Beschichtung der 96-Loch Platten werden 30µL dieser verdünnten Peptidlösung/Loch eingesetzt und über Nacht bei Raumtemperatur eingetrocknet.
Primäre Rattenmikroglia werden von gemischten Gliakulturen geerntet, die von P3 Rattenhimen gewonnen wurden. Zu Herstellung von gemischten Gliakulturen werden die Hirne von 3 Tage alten Ratten entnommen und von Hirnhäuten befreit. Die Zellvereinzelung wird durch Trypsinisierung erreicht (0,25 % Trypsinlösung, 15 Min 37°C)). Nach Abtrennung von nicht-verdauten Gewebefragmenten mit Hilfe eines 40µm Nylonnetzes werden die isolierten Zellen abzentrifugiert (800 rpm/10 Min). Das Zellpellet wird in Kulturmedium resuspendiert und in 100ml Gewebekulturflaschen überführt. (1 Hirn/ Gewebekulturflasche). Die Kultivierung der Zellen erfolgt über einen Zeitraum von 5-7 Tagen in Dulbeccos modified Eagle Medium (DMEM, mit Glutamin), supplementiert mit Penicillin (50 U/ml), Streptomycin (40µg/ml) und 10 % (v/v) fötalem Kälber Serum (FCS) bei 37°C und 5% CO₂. Während dieser Inkubation wird ein adhäsiver Zellrasen gebildet, der hauptsächlich aus Astrozyten besteht. Mikroglia proliferieren als nicht-oder schwachadhesive Zellen auf diesem und werden über Schüttelinkubation abgeerntet (420 Umdrehungen/Min, 1 Std).

Zur Aktivierung der Mikroglia durch Aß-Peptid werden 2,5 mal 10⁴ Mikroglia/Loch auf Aßbeschichtete Gewebekulturplatten ausgesät und über einen Zeitraum von 7 Tagen in DMEM (mit Glutamin), supplementiert mit Penicillin (50 U/ml), Streptomycin (40µg/ml) und 10 % (v/v) fötalem Kälber Serum (FCS) bei 37°C und 5% CO₂ inkubiert. Am Tag 5 erfolgt die Zugabe einer erfindungsgemäßen Verbindung in verschiedenen Konzentrationen (0,1, 0,3,1,3, und 10µM).
Zur Quantifizierung der Mikroglia-Reaktivität wird am Kultivierungstag 7 die metabolische Aktivität über die Reduktion von MTS (3-(4,5-dimethylthiazol-2-yl)-5-(3carboxymethoxyphenyl)-2-(sulfophenyl)2H-tetrazolium), Owen's Reagenz, Baltrop, J.A. et al. Bioorg. & Med. Chem. Lett 1, 6111 (1991)) gemessen. Die Prozent Inhibition bezieht sich auf eine nur mit DMSO behandelte Kontrolle. Die erfindungsgemäßen Verbindungen inhibieren die Mikroglia-Aktivierung

### Beispiel 308

### Zerebraler Hirninfarkt in der Ratte (MCAO-Modell)

Die erfindungsgemäßen Verbindungen wurden in einem Tiermodell für zerebrale Ischämie (Schlaganfall), dem MCAO (permanent middle cerebral artery occlusion) Modell, auf *in vivo* Aktivität hin getestet. Durch einseitigen Verschluß der mittleren Himarterie (MCA) wird ein Hirninfarkt ausgelöst, der auf der Unterversorgung des entsprechenden Hirnbereiches mit Sauerstoff und Nährstoffen beruht. Folge dieser Unterversorgung ist ein ausgeprägter Zelluntergang sowie, nachfolgend, eine starke Mikroglia-Aktivierung. Diese Mikroglia-Aktivierung erreicht allerdings erst nach mehreren Tagen ihr Maximum und kann über mehrere Wochen anhalten. Zur Testung der Substanzen wurden die erfindungsmäßigen Verbindungen 1-6 Tage nach Okklusion intraperitonal appliziert. Die Tiere wurden am Tag 7 perfundiert und getötet. Das Ausmaß der Mikroglia-Aktivierung wurde durch eine modifizierte immunhistochemische Methode gemessen. Dazu wurden Vibratom-Schnitte von fixierten Gehirnen mit Antikörper inkubiert, die den CR3 Komplement-Rezeptor bzw den MHCII Komplex auf aktivierten Mikroglia erkennen. Die Quantifizierung der primären Antikörperbindung erfolgte durch eine Enzym-gekoppeltes Detektionssystem.
Die Behandlung mit den erfindungsgemäßen Verbindungen führte zu einer signifikanten Reduktion der Mikroglia-Aktivierung in der vom Hirninfarkt betroffenen Himhemisphere. Die Reduktion betrug mindestens 20 % .

### Beispiel 309

### Aktivierung von Makrophagen

Zur Testung des Substanzen an Makrophagen/Monozyten wurden LPS-atkivierte THP-1 Zellen eingesetzt. Dazu wurden 2,5 x106 Zellen/ml in RPMI Medium (RPMI 1640 + 10%FCS) ausgesät. Die erfindungsmäßigen Verbindungen wurden in einer Konzentration von 5µM hinzugegeben und für 30 Minuten vorinkubiert. Die Stimulation der Zellen erfolgte über Nacht bei 37C mit 1µg/ml LPS . Danach wurde das Medium geerntet und die TNFα - Menge quantitativ bestimmt. Die Behandlung der Zellen mit den erfindungsmäßigen Substanzen führte zu einer Reduktion der TNFα Menge von mindestens 30%.

## Patentansprüche

1. Benzimidazole der allgemeinen Formel (I) 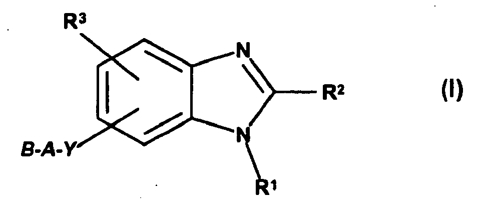 worin
***R¹*** eine mono- oder bicyclische C₆₋₁₂Arylgruppe oder eine mono- oder bicyclische 5-10 gliedrige Heteroarylgruppe mit 1-4 Heteroatomen ausgewählt aus der Gruppe bestehend aus N, S oder O, wobei die genannte Aryl-oder Heteroarylgruppe mit bis zu drei der folgenden Substituenten unabhängig voneinander substituiert sein kann: F, CI, Br, I, C(NH)NH₂, C(NH)NHR⁴, C(NH)NR⁴R^{4'}, C(NR⁴)NH₂, C(NR⁴)NHR^{4'}, C(NR⁴)NR⁴R^{4'}, XOH, XOR⁴, XOCOR⁴, XOCONHR⁴, XOCOOR⁴, XCOR⁴, XC(NOH)R⁴, XC(NOR⁴)R^{4'}, XC(NO(COR⁴))R^{4'} XCN, XCOOH, XCOOR⁴, XCONH₂, XCONR⁴R^{4'}, XCONHR⁴, XCONHOH, XCONHOR⁴, XCOSR⁴ XSR⁴, XSOR⁴ XSO₂R⁴, SO₂NH₂, SO₂NHR⁴, SO₂NR⁴R^{4'}, NO₂, XNH₂, XNHR⁴, XNR⁴R^{4'}, XNHSO₂R⁴, XN(SO₂R⁴) SO₂R^{4'}, XNR⁴SO₂R^{4'}, XNHCOR⁴, XNHCOOR⁴, XNHCONHR⁴, Tetrahydro-2,5-dioxopyrrol-1-yl, 2,5-Dihydro-2,5-dioxopyrrol- 1-yl, 2,7-Dihydro-2,7-dioxoisoindol-1-yl, R⁴, wobei zwei Substituenten an ***R¹***, wenn sie zueinander orthoständig sind, so miteinander verknüpft sein können, dass sie gemeinsam Methandiylbisoxy, Ethan-1,2-diylbisoxy, Propan-1,3-diyl, Butan-1,4-diyl bilden,
*R²* eine mono- oder bicyclische C₆₋₁₀- Arylgruppe oder eine mono- oder bicyclische 5-10 gliedrige Heteroarylgruppe mit 1-4 Heteroatomen ausgewählt aus der Gruppe bestehend aus N, S oder O, wobei die genannte Aryl-oder Heteroarylgruppe mit bis zu drei der folgenden Substituenten unabhängig voneinander substituiert sein kann: F, CI, Br, I, XOH, XOR⁴, XOCOR⁴, XOCONHR⁴, XOCOOR⁴, XCOR⁴, XC(NOH)R⁴, XC(NOR⁴)R^{4'}, XC(NO(COR⁴))R^{4'}, XCOOH, XCOOR⁴, XCONH², XCONHR⁴, XCONR⁴R^{4'}, XCONHOH, XCONHOR⁴, XCOSR⁴, XSR⁴, XSOR⁴, XSO₂R⁴, SO₂NH₂, SO₂NHR⁴, SO₂NR⁴R^{4'}, NO₂, XNHR⁴, XNR⁴R^{4'}, XNHSO₂R⁴, XN(SO₂R⁴) SO₂R^{4'} XNR⁴SO₂R^{4'}, Tetrahydro-2,5- dioxopyrrol-1-yl, 2,5-Dihydro-2,5-dioxopyrrol-1-yl, 2,7-Dihydro-2,7-dioxoisoindol-1-yl, R⁴, wobei zwei Substituenten an ***R²***, wenn sie zueinander orthoständig sind, so miteinander verknüpft sein können, dass sie gemeinsam Methandiylbisoxy, Ethan-1,2-diylbisoxy, Propan-1,3-diyl, Butan-1,4-diyl bilden,
***R³*** ein oder zwei Substituenten, die unabhängig voneinander: Wasserstoff, F, Cl, Br, I, XOH, XOR⁴, XOCOR⁴, XOCONHR⁴, XOCOOR⁴, XCOR⁴, XC(NOH)R⁴, XC(NOR⁴)R^{4'}, XC(NO(COR⁴))R^{4'}, XCN, XCOOH, XCOOR⁴, XCONH₂, XCONHR⁴, XCONR⁴R^{4'}, XCONHOH, XCONHOR⁴, XCOSR⁴, XSR⁴, XSOR⁴, XSO₂R⁴,SO₂NH₂, SO₂NHR⁴, SO₂NR⁴R^{4'}, NO₂, XNH₂, XNHR⁴, XNR⁴R^{4'}, XNHSO₂R⁴, XNR⁴SO₂R^{4'}, XN(SO₂R⁴)(SO₂R^{4'}), XNHCOR⁴, XNHCOOR⁴, XNHCONHR⁴, Tetrahydro-2,5-dioxopyrrol-1-yl, 2,5-Dihydro-2,5- dioxopyrrol-1-yl, 2,7-Dihydro-2,7-dioxoisoindol-1-yl, oder R⁴ sein können, wobei zwei Substituenten ***R³***, wenn sie zueinander orthoständig sind, so miteinander verknüpft sein können, dass sie gemeinsam Methandiylbisoxy, Ethan-1,2-diylbisoxy, Propan-1,3-diyl, Butan-1,4-diyl bilden,
***R⁴*** und **R^{4'}** unabhängig voneinander C₁₋₄ Perfluoralkyl, C₁₋₆ Alkyl, C₂₋₆ Alkenyl, C₂₋₆ Alkinyl, C₃₋₇ Cycloalkyl, (C₁₋₃ Alkyl-C₃₋₇ Cycloalkyl), C₁₋₃ Alkyl-C₆₋₁₀-aryl, C₁₋₃ Alkyl-5-10 gliedriges Heteroaryl mit 1-4 N-, S- oder O-Atomen, C₆₋₁₀-Aryl oder 5-10 gliedriges Heteroaryl mit 1-4 N-, S- oder O-Atomen, wobei die Aryl- und Heteroarylgruppen mit ein oder zwei Substituenten aus der Gruppe bestehend aus F, Cl, Br, CH₃, C₂H₅, NO₂, OCH₃, OC₂H₅, CF₃, C₂F₅ substituiert sein können oder auch eine annelierte Methandiylbisoxy, Ethan-1,2- diylbisoxygruppe tragen können, und weiterhin in einem 5-gliedrigen Cycloalkylring ein Ringglied ein N oder ein O sein kann und in einem 6- oder 7-gliedrigen Cycloalkylring ein oder zwei Ringglieder N und/oder O sein können, wobei Ringstickstoffe gegebenenfalls mit C₁₋₃ Alkyl oder C₁₋₃ Alkanoyl substituiert sein können,
***R⁵*** und **R^{5'}** unabhängig voneinander C₁₋₆ Alkyl, C₂₋₆ Alkenyl, C₂₋₆ Alkinyl, wobei ein Kohlenstoffatom gegen O, S, SO, SO₂, NH, N C₁₋₃ Alkyl oder N C₁₋₃ Alkanoyl ausgetauscht sein kann, C₃₋₇ Cycloalkyl-C₀₋₃ Alkyl, wobei in einem 5-gliedrigen Cycloalkylring ein Ringglied ein N oder ein O sein kann und in einem 6- oder 7-gliedrigen Cycloalkylring ein oder zwei Ringglieder N und/oder O sein können, wobei Ringstickstoffe gegebenenfalls mit C₁₋₃ Alkyl oder C₁₋₃ Alkanoyl substituiert sein können C₆₋₁₀-Aryl oder 5-10 gliedriges Heteroaryl mit 1-4 Heteroatomen aus N, S und O, wobei die genannten Alkyl-, Alkenyl- und Alkinylketten mit einem der zuvor genannten Cycloalkyle, Aryle oder Heteroaryle substituiert sein können, wobei alle zuvor genannten Alkyl- und Cycloalkylreste mit bis zu zwei Substituenten aus CF₃, C₂F₅, OH, O C₁₋₃ Alkyl, NH2, NH C₁₋₃ Alkyl, NH C₁₋₃ Alkanoyl, N (C₁₋₃ Alkyl)₂, N(C₁₋₃ Alkyl)(C₁₋₃ Alkanoyl), COOH, CONH₂, COO C₁₋₃ Alkyl und alle zuvor genannten Aryl- und Heteroarylgruppen mit ein oder.zwei Substituenten aus der Gruppe bestehend aus F, Cl, Br, CH₃, C₂H₅, NO₂, OCH₃, OC₂H₅, CF₃, C₂F₅ substituiert sein können oder auch eine annelierte Methandiylbisoxy, Ethan-1,2-diylbisoxygruppe tragen können, oder R⁵ und R^{5'} gemeinsam mit dem Stickstoffatom einen 5-7 gliedrigen Heterocyclus bilden, der ein weiteres Sauerstoff-, Stickstoff- oder Schwefelatom enthalten kann und substituiert sein kann mit C₁₋₄-Alkyl, C₋₄-Alkoxy-C₀₋₂-alkyl, C₁₋₄-Alkoxy-carbonyl, Aminocarbonyl oder Phenyl,
***A*** C₁₋₁₀ Alkandiyl, C₂₋₁₀ Alkendiyl, C₂₋₁₀ Alkindiyl, (C₀₋₅ Alkandiyl-C₃₋₇ Cycloalkandiyl-C₀₋₅ Alkandiyl), wobei in einem 5-gliedrigen Cycloalkylring ein Ringglied ein N oder ein O sein kann und in einem 6- oder 7-gliedrigen Cycloalkylring ein oder zwei Ringglieder N und/oder O sein können, wobei Ringstickstoffe gegebenenfalls mit C₁₋₃ Alkyl oder C₁₋₃ Alkanoyl substituiert sein können wobei in den oben genannten aliphatischen Ketten ein Kohlenstoffatom oder zwei Kohlenstoffatome gegen O, NH, N C₁₋₃ Alkyl, N C₁₋₃ Alkanoyl ausgetauscht sein können und wobei Alkyl- oder Cycloalkygruppen mit bis zu zwei Substituenten aus =O, OH, O C₁₋₃ Alkyl, NH2, NH C₁₋₃ Alkyl, NH C₁₋₃ Alkanoyl, N(C₁₋₃ Alkyl)₂, N(C₁₋₃ Alkyl)(C₁₋₃ Alkanoyl) substituiert sein können,
***B*** COOH, COOR⁵, CONH₂, CONHNH₂, CONHR⁵, CONR⁵R^{5'}, CONHOH, CONHOR⁵, SO₃H, SO₂NH₂, SO₂NHR⁵, SO₂NR⁵R^{5'}, PO₃H, PO(OH)(OR⁵), PO(OR⁵) (OR^{5'}), PO(OH)(NHR⁵), PO(NHR⁵) (NHR^{5'}), Tetrazolyl, jeweils gebunden an ein Kohlenstoffatom der Gruppe ***A*,** oder die gesamte Gruppe ***Y-A-B*** N(SO₂R⁴)(SO₂R^{4'}) oder NHSO₂R⁴,
***X*** eine Bindung, CH₂, (CH₂)₂. CH(CH₃), (CH₂)₃, CH(CH₂CH₃), CH(CH₃)CH₂, CH₂CH(CH₃),
***Y*** O, NH, NR⁴,NCOR⁴, NSO₂R⁴,
bedeuten,
mit der Maßgabe, dass,
A) falls Y NH, NR⁴, NCOR⁴ oder NSO₂R⁴ bedeutet,
a) und der Substituent R² einen stickstoffhaltigen gesättigten Heterocyclus enthält, dieser Heterocyclus nicht am Iminstickstoff mit H, Methyl, Ethyl, Propyl oder Isopropyl substituiert ist, oder
b) in gegebenenfalls vorhandenen Gruppen XNHR⁴ oder XNR⁴R^{4'} des Substituenten ***R²*** R⁴ und/oder R^{4'} nicht C₁₋₄-Alkyl bedeuten,
und dass
B) nicht gleichzeitig ***B*** COOH, SO₃H, PO₃H₂ oder Tetrazolyl und ***R¹*** und ***R²*** unabhängig voneinander C₅₋₈ Heteroaryl oder Phenyl bedeuten, wenn diese unabhängig voneinander unsubstituiert, einfach mit C₁₋₆ Alkyl, C₁₋₄ Perfluoralkyl, O C₁₋₆ Alkyl, O C₁₋₄ Perfluoralkyl, COOH, COO C₁₋₆ Alkyl, CO C₁₋₆ Alkyl, CONH₂, CONHR⁴, NO₂, NH₂, NHCOR⁴, NHSO₂R⁴ oder mit 1 oder 2 Halogenatomen aus der Gruppe F, Cl, Br, J substituiert sind, und
wobei die folgenden Verbindungen ausgeschlossen sind:
[(1,2-Diphenyl-1H-benzimidazol-6-yl)oxy]essigsäuremethylester,
5-[(1,2-DiphenyL-1H-benzimidazol-6-yl)oxy]pentansäuremethylester,
4-[(1,2-Diphenyl-1H-benzimidazol-6-yl)oxy]butansäureethylester,
5-[[1-(4-Nitrophenyl)-2-phenyl-1H-benzimidazol-6-yl]oxy]pentansäuremethylester,
6-[[1-(4-Nitrophenyl-2-phenyl-1H-benzimidazol-6-yl]oxy]hexansäuremethylester,
5-[[1-(4-Aminophenyl)-2-phenyl-1H-benzimidazol-6-yl]oxy]pentansäuremethylester,
5-[[1-[4-[[(4-Chlorphenyl)sulfonyl]amino]phenyl]-2-phenyl-1H-benzimidazol-6-yl]oxy]pentansäuremethylester,
5-[[1-[4-[(Acetyl)amino]phenyl]-2-phenyl-1H-benzimidazol-6-yl]oxy]pentansäuremethylester,
5-[[1-(3-Nitrophenyl)-2-phenyl-1H-benzimidazol-6-yl]oxy]pentansäuremethylester,
6-[[1-(3-Nitrophenyl)-2-phenyl-1H-benzimidazol-6-yl]oxy]hexansäuremethylester,
5-[[1-(3-Aminophenyl)-2-phenyl-1H-benzimidazol-6-yl]oxy]pentansäuremethylester,
5-[[1-[3-[[(4-Chlorphenyl)sulfonyl]amino]phenyl]-2-phenyl-1H-benzimidazol-6-yl]oxy]pentansäuremethylester,
5-[[1-[3-[(Acetyl)amino]phenyl]-2-phenyl-1H-benzimidazol-6-yl]oxy]pentansäuremethylester.

2. Benzimidazole nach Anspruch 1, **dadurch gekennzeichnet, dass**
***R¹*** eine mono- oder bicyclische C₆₋₁₂-Arylgruppe oder eine mono- oder bicyclische 5-10 gliedrige Heteroarylgruppe mit 1-2 Heteroatomen ausgewählt aus der Gruppe bestehend aus N, S oder O bedeutet, wobei die genannte Aryl-oder Heteroarylgruppe mit bis zu drei der folgenden Substituenten unabhängig voneinander substituiert sein kann: F, Cl, Br, XOH, XOR⁴, XOCOR⁴, XOCONHR⁴, XOCOOR⁴, XCOR⁴, XCN, XCOOH, XCOOR⁴; XCONH₂, XCONR⁴R^{4'}, XCONHR⁴, XCONHOH, XCONHOR⁴, XCOSR⁴, XSR⁴, NO₂, XNHR⁴, XNR⁴R^{4'}, R⁴, wobei zwei Substituenten an ***R¹***, wenn sie zueinander orthoständig sind, so miteinander verknüpft sein können, dass sie gemeinsam Methandiylbisoxy, Ethan-1,2-diylbisoxy, Propan-1,3-diyl, Butan-1,4-diyl bilden, bedeutet.

3. Benzimidazole nach Anspruch 1 oder 2, **dadurch gekennzeichnet, dass**
***R²*** eine mono- oder bicyclische C₆₋₁₀-Arylgruppe oder eine mono- oder bicyclische 5-10 gliedrige Heteroarylgruppe mit 1-2 Heteroatomen ausgewählt aus der Gruppe bestehend aus N, S oder O bedeutet, wobei die genannte Aryl-oder Heteroarylgruppe mit bis zu drei der folgenden Substituenten unabhängig voneinander substituiert sein kann: F, Cl, Br, XOH, XOR⁴, XOCOR⁴, XOCONHR⁴, XOCOOR⁴, XCOR⁴, XC(NCH)R⁴, XC(NOR⁴)R^{4'}, XC(NO(COR⁴))R^{4'}, XCOOH, XCOOR⁴, XCONH₂, XCONHR⁴, XCONR⁴R^{4'}, XCONHOH, XCONHOR⁴, XCOSR⁴, XSR⁴, XSOR⁴, XSO2R⁴, SP₂NH₂,SO₂NHR⁴, SO₂NR⁴R^{4'}, NO₂. XNHR⁴, XNR⁴R^{4'}, XNHSO₂R⁴, XN(SO₂R⁴) SO₂R^{4'}, XNR⁴SO₂R^{4'}, R⁴, wobei zwei Substituenten an *R²*, wenn sie zueinander orthoständig sind, so miteinander verknüpft sein können, dass sie gemeinsam Methandiylbisoxy, Ethan-1,2-diylbisoxy, Propan-1,3-diyl, Butan-1,4-diyl bilden, bedeutet.

4. Benzimidazole nach einem der Ansprüche 1-3, **dadurch gekennzeichnet, dass**
***R³*** ein oder zwei Substituenten, die unabhängig voneinander: Wasserstoff, F, Cl, Br, XOH, XOR⁴, XOCOR⁴, XOCONHR⁴, XOCOOR⁴, XCOR⁴, XC(NOH)R⁴, XC(NOR⁴)R^{4'}, XC(NO(COR⁴))R^{4'} XCN, XSR⁴, XSOR⁴, XSO₂R⁴, SO₂NH₂, SO₂NHR⁴, SO₂NR⁴R^{4'}, NO₂, XNH₂, XNHR⁴, XNR⁴N^{4'}, XNHSO₂R⁴, XNR⁴SO₂R^{4'}, XN(SO²R⁴) SO₂R^{4'}, XNHCOR⁴, XNHCOR⁴, XNNCONHR⁴, oder R⁴ sein können, wobei zwel Substituenten *R³*, wenn sie zueinander orthoständig sind, so miteinander verknüpft sein können, dass sie gemeinsam Methandiylbisoxy, Ethan-1,2-diylbisoxy, Propan-1,3-diyl, Butan-1,4-diyl bilden, bedeutet.

5. Benzimidazole nach einem der Ansprüche 1-4, **dadurch gekennzeichnet, dass**
R⁴ und R^{4'} unabhängig voneinander CF₃, C₂F₅, C₁₋₄Alkyl, C₂₋₄-Alkenyl, C₂₋₄-Alkinyl, C₃₋₈- Cycloalkyl, (C₁₋₃-Alkyl-C₃₋₆-Cycloalkyl), Phenyl oder 5-6 gliedriges Heteroaryl mit 1-2 N-, S- oder O-Atomen, wobei die Phenyl- und Heteroarylgruppen mit ein oder zwei Substituenten aus der Gruppe bestehend aus F, Cl, Br, CH₃, C₂H₅, OCH₃, OC₂H₅, CF₃, C₂F₅ substituiert sein können, und weiterhin in einem 5-gliedrigen Cycloalkylring ein Ringglied ein N oder ein O sein kann und in einem 6-gliedrigen Cycloalkylring ein oder zwei Ringglieder N und/oder O sein können, wobei Ringstickstoffe gegenbenenfalls mit C₁₋₃-Alkyl oder C₁₋₃-Alkanoyl substituiert sein können, bedeuten.

6. Benzimidazole nach einem der Ansprüche 1-5, **dadurch gekennzeichnet, dass**
***R⁵*** und **R^{5'}** unabhängig voneinander C₁₋₆ Alkyl, wobei ein Kohlenstoffatom gegen O, NH, N C₁- ₃ Alkyl, N C₁₋₃ Alkanoyl ausgetauscht sein kann, C₃₋₇ Cycloalkyl-C₀₋₃ Alkyl, wobei in einem 5-gliedrigen Cycloalkylring ein Ringglied ein N oder ein O sein kann und in einem 6- oder 7-gliedrigen Cycloalkylring ein oder zwei Ringglieder N und/oder O sein können, wobei Ringstickstoffe gegebenenfalls mit C₁₋₃ Alkyl oder C₁₋₃ Alkanoyl substituiert sein können, wobei der genannte C₁₋₆ Alkylteil mit einem der zuvor genannten Cycloalkyle oder auch einem 5-6 gliedrigen Heteroaromaten mit 1-2 Heteroatomen, ausgewählt aus N, S oder O, substituiert sein kann, wobei alle zuvor genannten Alkyl- und Cycloalkylteile mit bis zu zwei Substituenten aus CF₃, OH, O C₁₋₃ Alkyl, und die zuvor genannten Heteroarylgruppen mit ein oder zwei Substituenten aus F, Cl, CF₃, CH₃, C₂H₅, OCH₃, OC₂H₅ substituiert sein können oder R⁵ und R^{5'} gemeinsam mit dem Stickstoffatom einen 5-7 gliedrigen Heterocyclus bilden, der ein weiteres Sauerstoff-, Stickstoff- oder Schwefelatom enthalten kann und substituiert sein kann mit C₁₋₄-Alkyl, C₁₋₄-Alkoxy-C₀₋₂-alkyl, C₁₋₄-Alkoxy-carbonyl, Aminocarbonyl oder Phenyl, bedeuten.

7. Benzimidazole nach einem der Ansprüche 1-6, **dadurch gekennzeichnet, dass**
***A*** C₁₋₁₀ Alkandiyl, C₂₋₁₀ Alkendiyl, C₂₋₁₀ Alkindiyl, (C₀₋₅ Alkandiyl-C₃₋₇ Cycloalkandiyl-C₀₋₅ Alkandiyl), wobei in einem 5-gliedrigen Cycloalkylring ein Ringglied ein N oder ein O sein kann und in einem 6- oder 7-gliedrigen Cycloalkylring ein oder zwei Ringglieder N und/oder O sein können, wobei Ringstickstoffe gegebenenfalls mit C₁₋₃ Alkyl oder C₁₋₃ Alkanoyl substituiert sein können, wobei in den oben genannten aliphatischen Ketten ein Kohlenstoffatom oder zwei Kohlenstoffatome gegen O, NH, N C₁₋₃ Alkyl, N C₁₋₃ Alkanoyl ausgetauscht sein können, bedeutet.

8. Benzimidazole nach einem der Ansprüche 1-7, **dadurch gekennzeichnet, dass**
***B*** COOH, COOR⁵, CONH₂, CONHR⁵, CONR⁵R^{5'}, CONHOH, CONHOR⁵ oder Tetrazolyl, jeweils gebunden an ein Kohlenstoffatom der Gruppe A bedeutet.

9. Benzimidazole nach einem der Ansprüche 1-8, **dadurch gekennzeichnet, dass**
***X*** eine Bindung oder Methylen bedeutet.

10. Benzimidazole nach einem der Ansprüche 1-9, **dadurch gekennzeichnet, dass**
***Y*** O bedeutet.

11. Ein Benzimidazol nach einem der Ansprüche 1-10, ausgewählt aus der Gruppe bestehend aus
[(1,2-Diphenyl-1*H*-benzimidazol-6-yl)oxylessigsäureisopropylester
3-[(1,2-Diphenyl-1*H*-benzimidazol-6-yl)oxy]propansäuremethylester
2-[(1,2-Diphenyl-1*H-*benzimidazol-6-yl)oxylpropansäuremethylester
4-[(1,2-Diphenyl-1*H-*benzimidazol-6-yl)oxylbutansäureisopropylester
5-[(1,2-Diphenyl-1*H-*benzimidazol-6-yl)oxylpentansäureisopropylester
6-[(1,2-Diphenyl-1*H-*benzimidazol-6-yl)oxy]hexansäuremethylester
6-[(1,2-Diphenyl-1*H-*benzimidazol-6-yl)oxy]hexansäureisopropylester
6-[(1,2-Diphenyl-1*H-*benzimidazol-6-yl)oxy]hexanamid
*N*-Methoxy-6-[(1,2-diphenyl-1H-benzimidazol-6-yl)oxy]hexanamid
*N*-(Phenylmethoxy)-6-[(1,2diphenyl-1*H-*benzimidazol-6-yl)oxy]hexanamid
N*-Hydroxy-6-[(1,2-diphenyl-1H-benzimidazol-6yl)oxy]hexanamid*
7-[(1,2-Diphenyl-1*H-*benzimidazol-6-yl)oxy]heptansäuremethylester
6-[[1-(3-Nitrophenyl)-2-phenyl-1H-benzimidazol-6-yl]oxy]hexansäureisopropylester
6-[[2-Phenyl-1-[3-(trifluormethyl)phenyl]-1*H-*benzimidazol-6-yl]oxy]hexansäuremethylester
6-[[2-Phenyl-1-[3-(trifluormethyl)phenyl]-1*H*-benzimidazol-6-yl]oxy]hexansäureisopropylester
6-[[1-(3-Cyanophenyl)-2-phenyl-1*H*-benzimidazol-6-yl]oxy]hexansäuremethylester
6-[[1-(3-Cyanophenyl)-2-phenyl-1*H*-benzimidazol-6-yl]oxy]hexansäureisopropylester.
6-[[1-(3-Cyanophenyl)-2-phenyl-1*H-*benzimidazol-6-yl]oxy]hexansäure
6-[[1-(4-Cyanophenyl)-2-phenyl-1*H-*benzimidazol-6-yl]oxy]hexansäuremethylester
6-[[1-(4-Cyanophenyl)-2-phenyl-1*H-*benzimidazol-6-yl]oxy]hexansäureisopropylester
6-[[1-(3-Chlorphenyl)-2-phenyl-1*H-*benzimidazol-6-yl]oxy]hexansäuremethylester
6-[[1-(3-Chlorphenyl)-2-phenyl-1*H-*benzimidazol-6-yl]oxy]hexansäureisopropylester
6-[[1-(4-Chlorphenyl)-2-phenyl-1*H-*benzimidazol-6-yl]oxy]hexansäuremethylester
6-[[1-(4-Chlorphenyl)-2-phenyl-1*H-*benzimidazol-6-yl]oxy]hexansäureisopropylester
6-[[1-(3-Methylphenyl)-2-phenyl-1*H-*benzimidazol-6-yl]oxy]hexansäuremethylester
6-[[1-(3-Methylphenyl)-2-phenyl-1*H-*benzimidazol-6-yl]oxy]hexansäureisopropylester
6-[[1-(4-Methylphenyl-2-phenyl-1*H-*benzimidazol-6-yl]oxy]hexansäuremethylester
6-[[1-(4-Methylphenyl)-2-phenyl-1*H-*benzimidazol-6-yl]oxy]hexansäureisopropylester
6-[[1-(3,4-Dimethylphenyl)-2-phenyl-1*H-*benzimidazol-6-yl]oxy]hexansäuremethylester
6-[[1-(3,5-Dimethylphenyl)-2-phenyl-1*H-*benzimidazol-6-yl]oxy]hexansäuremethylester
6-[[1-(3,5-Dimethylphenyl)-2-phenyl-1*H-*benzimidazol-6-yl]oxy]hexansäureisopropylester
6-[[1-(3-Methoxyphenyl)2-phenyl-1*H-*benzimidazol-6-yl]oxy]hexansäuremethylester
6-[[1-(4-Methoxyphenyl)-2-phenyl-1*H-*benzimidazol-6-yl]oxy]hexansäuremethylester
6-[[1-(3,4-Dimethoxyphenyl)-2-phenyl-1*H-*benzimidazol-6-yl]oxy]hexansäuremethylester
6-[[1-[3,4-(Methylendioxy)phenyl]-2-phenyl-1*H-*benzimidazol-6-yl]oxy]hexansäuremethylester
6-[[1-[3,4-(Methylendioxy)phenyl]-2-phenyl-1*H*-benzimidazol-6-yl]oxy]hexansäure
6-[[2-Phenyl-1-(3,4,5-trimethoxyphenyl)-1*H-*benzimidazol-6-yl]oxy]hexansäuremethylester
6-[[2-Phenyl-1-(3,4,5-trimethoxyphenyl)-1*H-*benzimidazol-6-yl]oxy]hexansäure
6-[[2-Phenyl-1-(3,4,5-trimethoxyphenyl)-1*H-*benzimidazol-6-yl]oxy]hexansäureisopropylester
6-[[1-[4-(*N,N*-Dimethylamino)phenyl]-2-phenyl-1*H-*benzimidazol-6-yl]oxy]hexansäuremethylester
6-[[1-[4-(*N,N*-Dimethylamino)phenyl]-2-phenyl-1*H-*benzimidazol-6-yl]oxy]hexansäure
6-[[1-Phenyl-2-[3-(trifluormethyl)phenyl]-1*H-*benzimidazol-6-yl]oxy]hexansäureisopropylester
6-[[2-(3-Chlorphenyl)-1-phenyl-1*H-*benzimidazol-6-yl]oxy]hexansäuremethylester
6-[[2-(3-Chlorphenyl)-1-phenyl-1*H-*benzimidazol-6-yl]oxy]hexansäureisopropylester
6-[[2-(4-Chlorphenyl)-1-phenyl-1*H-*benzimidazol-6-yl]oxy]hexansäuremethylester
6-[[2-(4-Chlorphenyl)-1-phenyl-1*H-*benzimidazol-6-yl]oxy]hexansäureisopropylester
6-[[2-(4-Methylphenyl)-1-phenyl-1*H-*benzimidazol-6-yl]oxy]hexansäuremethylester
6-[[2-(4-Methylphenyl)-1-phenyl-1*H-*benzimidazol-6-ylloxy]hexansäureisopropylester
6-[[1-Phenyl-2-(4-pyddinyl)-1*H-*benzimidazol-6-yl]oxy]hexansäuremethylester
6-[(1,2-Diphenyl-5-nitro-1*H-*benzimidazol-6-yl)oxy]hexansäuremethylester
6-[(1,2-Diphenyl-5-nitro-1*H-*benzimidazol-6-yl)oxy]hexansäureisopropylester
6-[[5-[[(4-Bromphenyl)sulfonyl]amino]-1,2-diphenyl-1*H-*benzimidazol-6-yl]oxy]hexansäureisopropylester
6-[[5-[[(4-Chlorphenyl)sulfonyl]amino]-1,2-diphenyl-1*H-*benzimidazol-6-yl]oxy]hexansäuremethylester
6-[[5-[[(4-Chlorphenyl)sulfonyl]amino]-1,2-diphenyl-1*H-*benzimidazol-6-yl]oxy]hexansäureisopropylester
6-[[1,2-Diphenyl-5-[[(3-methylphenyl)sulfonyl]amino]-1*H-*benzimidazol-6-yl]oxy]hexansäureisopropylester
6-[[1,2-Diphenyl-5-[[(4-methylphehyl)sulfonyl]aminol-1*H-*benzimidazol-6-yl]oxy]hexansäureisopropylester
6-[[1,2-Diphenyl-5-[[(4-methoxyphenyl)sulfonyl]amino]-1*H-*benzimidazol-6-yl]oxy]hexansäureisopropylester
6-[[1,2-Diphenyl-5-[[[(4-trifluormethyl)phenyl]sulfonyl]amino]-1*H-*benzimidazol-6-yl]oxy]hexansäureisopropylester
6-[[5-[[[4-(Acetylamino)phenyl]sulfonyl]amino]-1,2-diphenyl-1*H-*benzimidazol-6-yl]oxy]hexansäureisopropylester
6-[[5-[[Bis(3-chlorphenyl)sulfonyl]amino]-1,2-diphenyl-1*H-*benzimidazol-fryl]oxy]hexansäureisopropylester
6-[[1,2-Diphenyl-5-[(propylsulfonyl)amino]-1*H-*benzimidazol-6-yl]oxy]hexansäureisopropylester
6-[[5-[(Benzylsulfonyl)amino]-1,2-diphenyl-1*H-*benzimidazol-6-yl]oxy]hexansäureisopropylester
2-[2-[(1,2-Diphenyl-1*H-*benzimidazol-6-yl)oxy]ethoxy]essigsäuremethylester
3-[2-[(1,2-Diphenyl-1*H-*benzimidazol-6-yl)oxy]ethoxy]propansäuremethylester
6-[[1-(3-Nitrophenyl)-2-phenyl-1*H-*benzimidazol-6-yl]oxy]hexansäureethlester
6-[[4-Acetyl-1-(4-methylphenyl)-2-phenyl-1*H-*benzimidazol-6-yl]oxy]hexansäuremethylester
6-[[1-(4-Methylphenyl)-2-phenyl-1*H-*benzimidazol-5-yl]oxy]hexansäuremethylester
6-[[2-Phenyl-1-[4-(thiomethyl)phenyl]-1*H-*benzimidazol-5-yl]oxy]hexansäuremethylester
6-[[2-Phenyl-1-[(4-thiomethyl)phenyl]-1*H-*benzimidazol-6-yl]oxy]hexansäuremethylester
B-[[2-Phenyl-1-(3-thienyl)-1*H-*benzimidazol-5-yl]oxy]hexansäuremethylester
6-[[2-Phenyl-1-(3-thienyl)-1*H-*benzimidazol-6-yl]oxy]hexansäuremethylester
4-[(1,2-Diphenyl-1*H-*benzimidazol-6-yl)oxy]butansäuremethylester
*N-*(Phenylmethoxy)-6-[[2-phenyl-1-(3,4,5-trimethoxyphenyl)-1*H-*benzimidazol-6-yl]oxy]hexanamid
*N*,*N-*Dimethyl-6-[(1,2-diphenyl-1*H-*benzimidazol-6-yl)oxy]hexanamid
*N-*Isospropyl-6-[(1,2-diphenyl-1*H-*benzimidazol-6-yl)oxy]hexanamid
6-[(1,2-Diphenyl-1*H-*benzimidazol-6-yl)-oxy]-1-pyrrolidin-1-ylhexan-1-on
5-[[5-[[(4-Chlorphenyl)suffonyl]amino]-1,2-diphenyl-1*H-*benzimidazol-6-yl]oxy]pentansäuremethylester
6-[[5-[[(4-Chlorphenyl)sulfonyl]amino]-1-(4-methylphenyl)-2-phenyl-1*H-*benzimidazol-6-yl]oxy]-hexansäuremethylester
6-[[5-[[(4-Chlorphenyl)sulfonyl]amino]-1-(4-methoxyphenyl)-2-phenyl-1*H-*benzimidazol-6-yl]oxy]-hexansäuremethylester
6-[[4-(Acetyloxy)-1-(4-methylphenyl)-2-phenyl-1*H-*benzimidazol-6-yl]oxy]hexansäuremethylester
6-[[4-Hydroxy-1-(4-methylphenyl)-2-phenyl-1*H-*benzimidazol-6-yl]oxy]hexansäuremethylester
6-[[4-Hydroxy-1-(4-methylphenyl)-2-phenyl-1*H-*benzimidazol-6-yl]oxy]hexansäure
6-[[7-Methyl-1-(4-methylphenyl)-2-phenyl-1*H-*benzimidazol-6-yl]oxy]hexansäuremethylester
6-[[2-Phenyl-1-(3-pyridyl)-1*H-*benzimidazol-5-yl]oxy]hexansäuremethylester
6-[[2-Phenyl-1-(3-pyridyl)-1*H-*benzimidazol-6-yl]oxy]hexansäuremethylester
6-[[2-Phenyl-1-(4-pyridyl)-1*H-*benzimidazol-6-yl]oxy]hexansäuremethylester
6-[[2-(4-Fluor-phenyl)-1-phenyl-1*H-*benzimidazol-6-yl]oxy]hexansäuremethylester
6-[[2-(4-Methoxyphenyl)-1-phenyl-1*H-*benzimidazol-6-yl]oxy]hexansäuremethylester
6-[[2-(4-Bromphenyl)-1-phenyl-1*H-*benzimizol-6-yl]oxy]hexansäuremethylester
6-[[2-[4-(Trifluormethyl)phenyl]-1-phenyl-1*H-*benzimidazol-6-yl]oxy]hexansäuremethylester
6-[[1-Phenyl-2-(benzothien-2-yl)-1*H-*benzimidazol-6-yl]oxy]hexansäuremethylester
6-[[1-Phenyl-2-(benzothien-2-yl)-1*H-*benzimidazol-6-yl]oxy]hexansäure
6-[[5-Hydroxy-1-(4-methylphenyl)-2-phenyl-1*H-*benzimidazol-6-yl]oxy]hexansäureisopropylester
6-[[5-Hydroxy-1-(4-methylphenyl)-2-phenyl-1*H-*benzimidazol-6-yl]oxy]hexansäure
6-[[5-Methoxy-1-(4-methylphenyl)-2-phenyl-1*H-*benzimidazol-6-yl]oxy]hexansäureisopropylester
6-[[5-Hydroxy-1-(4-methylphenyl)-2-phenyl-1*H-*benzimidazol-6-yl]oxy]hexansäuremethylester
6-[[5-Methoxy-l-(4-methylphenyl)-2-phenyl-1*H-*benzimidazol-6-yl]loxy]hexansäuremethylester
6-[[5-[[(4-Chlorphenyl)sulfonyl]amino]-1-(3,4-dimethylphenyl)-2-phenyl-1*H-*benzimidazol-6-yl]oxy]-hexansäuremethylester
6-[[5-[[(4-Chlorphenyl)sulfonyl]amino]-2-(4-fluorphenyl)-1-(4-methoxyphenyl)-1*H-*benzimidazol-6-yl]oxy]hexansäuremethylester
6-[[5-[[(4-Chlorphenyl)sulfonyl]amino]-1-(4-methoxyphenyl)-2-(4-methoxyphenyl)-1*H-*benzimidazol-6-yl]oxy]hexansäuremethylester
4-[[5-[[(4-Chlorphenyl)sulfonyl]amino]-1-(4-methoxyphenyl)-2-phenyl-1*H-*benzimidazol-6-yl]oxy]-butansäuremethylester
5-[[5-[[(4-Chlorphenyl)sulfonyl]amino]-1-(4-methoxyphenyl)-2-phenyl-1*H-*benzimidazol-6-yl]oxy]pentansäuremethylester
5-[[5-[[(4-Chlorphenyl)sulfonyl]amino]-1,2-diphenyl-1*H-*benzimidazol-6-yl]oxy]pentansäuremethylester
6-[[5-[[(4-(Trifluormethyl)phenyl)sulfonyl]amino]-1-(4-methoxyphenyl)-2-phenyl-1*H-*benzimidazol-6-yl]oxy]hexansäuremethylester
6-[[5-[[(4-Chlorphenyl)sulfonyl]methylamino]-1-(4-methoxyphenyl)-2-phenyl-1*H-*benzimidazol-6-yl]oxy]hexansäuremethylester
6-[[1-(Indan-5-yl)-2-phenyl-1*H-*benzimidazol-6-yl]oxy]hexansäuremethylester
6[[1-(Indan-5-yl)-2-phenyl-1*H-*benzimidazol-6-yl]oxy]hexansäure
6-[[1-(3-Fluorphenyl)-2-phenyl-1*H-*benzimidazol-6-yl]oxy]hexansäuremethylester
6-[[2-(4-Nitrophenyl)-1-phenyl-1*H-*benzimidazol-6-yl]oxy]hexansäuremethylester
6-[[1-Phenyl-2-(3-pyridinyl)-1*H-*benzimidazol-6-yl]oxylhexansäuremethylester
*N-*(Cyclopropylmethoxy)-6-[(1,2-diphenyl-1*H-*benzimidazol-6-yl)oxy]hexanamid
*N-*Isobutoxy-6-[(1,2-diphenyl-1*H-*benzimidazol-6-yl)oxy]hexanamid
*N-*(Cyclopropylmethoxy)-6-[2-phenyl-1-(3,4,5-trimethoxyphenyl)-1*H-*benzimidazol-6-yl)oxy]hexanamid
*N-*Isobutoxy-6-[2-phenyl-1-(3,4,5-trimethoxyphenyl)-1*H-*benzimidazol-6-yl)oxy]hexanamid
*N-*(2-Methoxyethyl)-6[(1,2-diphenyl-1*H-*benzimidazol-6yl)oxy]hexanamid
*N-*(3-Methoxypropyl-6-[(1,2-diphenyl-1*H-*benzimidazol-6-yl)oxy]hexanamid
*N-*Isobutyl-6-[(1,2-diphenyl-1*H-*benzimidazol-6-yl)oxy]hexanamid
6-[(1,2-Diphenyl-1*H-*benzimidazol-6-yl)oxy]-1-morpholin-1-ylhexan-1-on
*N*,*N-*Di(-2-methoxyethyl-6-[(1,2-diphenyl-1*H-*benzimidazol-6-yl)oxy]hexanamid
*N-*Isopentyl-6-[(1,2-diphenyl-1*H-*benzimidazol-6-yl)oxy]hexanamid
*N-*(Pyridin-2-yl)-6-[(1,2-diphenyl-1*H-*benzimidazol-6-yl)oxy]hexanamid
*N-*(Pyridin-3-yl)-6-[(1,2-diphenyl-1*H-*benzimidazol-6-yl)oxy]hexanamid
*N-*Isopropyl-6-[[1-(3,4-dimethylphenyl)-2-phenyl-1*H-*benzimidazol-6-yl]oxy]hexanamid
*N*,*N-*Dimethyl-6-[[1-(3,4-dimethylphenyl)-2-phenyl-1*H-*benzimidazol-6-yl]oxy]hexanamid
N,*N-*Diethyl-6-[[1-(3,4-dimethylphenyl)-2-phenyl-1*H-*benzimidazol-6-yl]oxy]hexanamid
*N-*Isobutyl-6-[[1-(3,4-dimethylphenyl)-2-phenyl-1*H-*benzimidazol-6-yl]oxy]hexanamid
*N-*Cyclopropyl-6-[[1-(3,4-dimethylphenyl)-2-phenyl-1*H-*benzimidazol-6yl]oxy]hexanamid
*N-*Cyclobutyl-6-[[1-(3,4-dimethylphenyl)-2-phenyl-1*H-*benzimidazol-6-yl]oxy]hexanamid
*N-tert-*Butyl-6-[[1-(3,4-dimethylphenyl)2-phenyl-1*H-*benzimidazol-6-yl]oxy]hexanamid
(*R*)-6-[[1-(3,4-Dimethylphenyl)-2-phenyl-1*H-*benzimidazol-6-yl]oxy]1-(2-methoxymethyl)pyrrolidin-1-ylhexan-1-on
*N-*(3-Imidazol-1-yl-propyl)-6-[[1-(3,4-dimethylphenyl)-2-phenyl-1*H-*benzimidazol-6-yl]oxy]hexanamid
*N-*(2-Pyridin-2-ylethyl)-6-[[1-(3,4-dimethylphenyl)-2-phenyl-1*H-*benzimidazol-6-yl]oxy]hexanamid
*N-*(3-Methoxypropyl)-6-[[1-(indan-5-yl)-2-phenyl-1*H-*benzimidazol-6-yl]oxy]heptanamid
6-[[1-(4-Methylphenyl)-2-(3-pyridyl)-1*H-*benzimidazol-6-yl]oxy]hexansäuremethylester
6-[[1-(4-Methylphenyl)-2-(4-pyridyl)-1*H-*benzimidazol-6-yl]oxy]hexansäuremethylester
6-[[1-(4-Methylphenyl)-2-(2-thienyl)-1*H-*benzimidazol-6-yl]oxy]hexansäuremethylester
6-[[1-(4-Methylphenyl)-2-(3-thienyl)-1*H-*benzimidazol-6-yl]oxy]hexansäuremethylester
6-[[2-(3-Indolyl)-1-(4-methylphenyl)-1*H-*benzimidazol-6-yl]oxy]hexansäuremethylester
6-[[1-(4-Methylphenyl)-2-(2-furyl)-1*H-*benzimidazol-6yl]oxy]hexansäuremethylester
6-[[1-(4-Methylphenyl)-2-(3-furyl)-1*H-*benzimidazol-6-yl]oxy]hexansäuremethylester
6-[[1-(4-Methylphenyl)-2-(5-methyl-2-thienyl)-1*H-*benzimidazol-6-yl]oxy]hexansäuremethylester
6-[[1-(4-Methylphenyl)-2-(3-methyl-2-thienyl)-1*H-*benzimidazol-6-yl]oxy]hexansäuremethylester

12. Eine Verbindung nach einem der Ansprüche 1-11 zur Verwendung im Heilverfahren, in der Prophylaxe oder in der Diagnose.

13. Eine Verbindung nach einem der Ansprüche 1-11 zur Verwendung bei der Behandlung oder Verhütung von Krankheiten, die mit einer Mikroglia-Aktivierung assoziiert sind, ausgewählt aus der Gruppe bestehend aus AIDS-Demenz, Amyotropher Lateralsklerose, Creuzfeld-Jacob-Krankheit, Down-Syndrom, diffuser Lewy-Körperchen-Krankheit, Chorea Huntington, Leukenzephalopathie, multipler Sklerose, neuronaler Dysfunktion und neuronaler Degeneration, Parkinsonscher Krankheit, Pickscher Krankheit, Alzheimerscher Krankheit, Schlaganfall, Temporal-LappenEpilepsie und Tumoren.

14. Verwendung einer Verbindung nach einem der Ansprüche 1-11 zur Herstellung eines Arzneimittels zur Behandlung oder Verhütung von Krankheiten, die mit einer Mikroglia-Aktivierung assoziiert sind, ausgewählt aus der Gruppe bestehend aus AIDS-Demenz, Amyotropher Lateralsklerose, Creuzfeld-Jacob-Krankheit, Down-Syndrom, diffuser Lewy-Körperchen-Krankheit, Chorea Huntington, Leukenzephalopathie, multipler Sklerose, neuronaler Dysfunktion und neuronaler Degeneration, Parkinsonscher Krankheit, Pickscher Krankheit, Alzheimerscher Krankheit, Schlaganfall, Temporal-Lappen-Epilepsie und Tumoren.

15. Pharmazeutisches Mittel **dadurch gekennzeichnet, dass** es eine oder mehrere Verbindungen nach einem der Ansprüche 1-11 und einen oder mehrere Trägerstoffe enthält.

16. Benzimidazol der allgemeinen Formel II 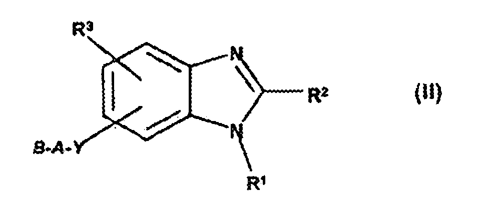 worin
***R***¹ eine mono- oder bicyclische C₀₋₁₂-Arylgruppe oder eine mono- oder bicyclische 5-10 gliedrige Heteroarylgruppe mit 1-4 Heteroatomen ausgewählt aus der Gruppe bestehend aus N, S oder O, wobei die genannte Aryl-oder Heteroarylgruppe mit bis zu drei der folgenden Substituenten unabhängig voneinander substituiert sein kann: F, Cl, Br, I, C(NH)NH₂, C(NH)NHR⁴, C(NH)NR⁴R⁴', C(NR⁴)NH₂, C(NR⁴)NHR^{4'}, C(NR⁴)NR⁴R^{4'}, XOH, XOR⁴, XOCOR⁴, XOCONHR⁴, XOCOOR⁴, XCOR⁴, XC(NOH)R⁴, XC(NOR⁴)R^{4'}, XC(NO(COR⁴))R^{4'}, XCN, XCOOH, XCOOR⁴, XCONH₂, XCONR⁴R^{4'}, XCONHR⁴, XCONHOH, XCONHOR⁴, XCOSR⁴, XSR⁴, XSOR⁴, XSO₂R⁴, SO₂NH₂, SO₂NHR⁴,SO₂NR⁴R^{4'}, NO₂, XNH₂, XNHR⁴, XNR⁴R^{4'}, XNHSO₂R⁴, XN(SO₂R⁴)(SO₂R^{4'}), XNR⁴SO₂R^{4'}, XNHCOR⁴, XNHCOOR⁴,XNHCONHR⁴, Tetrahydro-2,5-dioxopyrrol-1-yl, 2,5- Dihydro-2,5-dioxopyrrol-1-yl, 2,7-Dihydro-2,7-dioxoisoindol-1-yl, R⁴, wobei zwei Substituenten an *R¹*, wenn sie zueinander orthoständig sind, so miteinander verknüpft sein können, dass sie gemeinsam Methandiylbisoxy, Ethan-1,2-diylbisoxy, Propan-1,3-diyl, Butan- 1,4-diyl bilden.
***R²*** eine mono- oder bicyclische C₆₋₁₀-Arylgruppe oder eine mono- oder bicyclische 5-10 gliedrige Heteroarylgruppe mit 1-4 Heteroatomen ausgewählt aus der Gruppe bestehend aus N, S oder O, wobei die genannte Aryl-oder Heteroarylgruppe mit bis zu drei der folgenden Substituenten unabhängig voneinander substituiert sein kann: F, Cl, Br, I, C(NH)NH₂, C(NH)NHF⁴, C(NH)NR⁴R^{4'}, C(NR⁴)NH₂ C(NR⁴)NHR^{4'}, C(NR⁴)NR⁴R^{4'}, XOH, XOR⁴, XOCOR⁴, XOCONHR⁴, XOCOOR⁴, XCOR⁴, XC(NOH)R⁴, XC(NOR⁴)R^{4'}, XC(NO(COR⁴))R^{4'} XCN, XCOOH, XCOOR⁴, XCONH₂, XCONR⁴R^{4'}, XCONHR⁴, XCONHOH, XCONHOR⁴, XCOSR⁴ XSR⁴. XSOR⁴, XSO₂R⁴, SO₂NH₂, SO₂NHR⁴,SO₂NR⁴R⁴', NO₂, XNH₂. XNHR⁴, XNR⁴R^{4'}, XNHSO₂R⁴, XN(SO₂R⁴)(SO₂R^{4'}), XNR⁴SO₂R^{4'}, XNHCOR⁴, XNHCO0R⁴, XNHCONHR⁴, Tetrahydro-2,5-dioxopyrrol-1-yl, 2,5-Dihydro-2,5-dioxopyrrol-1-yl, 2,7-Dihydro-2,7- dioxoisoindol-1-yl, R⁴, wobei zwei Substituenten an ***R***², wenn sie zueinander orthoständig sind, so miteinander verknüpft sein können, dass sie gemeinsam Methandiylbisoxy. Ethan-1,2- diylbisoxy, Propan-1,3-diyl, Butan-1,4-diyl bilden,
***R***³ für ein oder zwei Substituenten steht, die unabhängig voneinander: Wasserstoff, F, Cl, Br, I, XOH, XOR⁴, XOCOR⁴, XOCONHR⁴, XOCOOR⁴, XCOR⁴, XC(NOH)R⁴, XC(NOR⁴)R, XC(NO(COR⁴))R^{4'}, XCN, XCOOH, XCOOR⁴, XCONH₂, XCONHR⁴, XCONR⁴R^{4'}, XCONHOH, XCONHOR⁴, XCOSR⁴, XSR⁴, XSOR⁴, XSO₂R⁴,SO₂NH₂, SO₂NHR⁴, SO₂NR⁴R^{4'}, NO₂, XNH₂, XNHR⁴, XNR⁴R⁴, XNHSO₂R⁴, XNR⁴SO₂R^{4'}, XN(SO₂R⁴)(SO₂R^{4'}), XNHCOR⁴, XNHCOOR⁴, XNHCONHR⁴, Tetrahydro-2,5-dioxopyrrol-yl, 2,5-Dihydro-2,5-dioxopyrrol-1-yl, 2,7-Dihydro-2,7- dioxoisoindol-1-yl, R⁴, wobei zwei Substituenten ***R*³,** wenn sie zueinander orthoständig sind, so miteinander verknüpft sein können, dass sie gemeinsam Methandiylbisoxy, Ethan- 1,2-diylbisoxy, Propan-1,3-diyl, Butan-1,4-diyl bilden,
***R*⁴** und ***R*^{4'}** unabhängig voneinander C₁₋₄ Perfluoralkyl, C₁₋₆ Alkyl, C₂₋₆ Alkenyl, C₂₋₆ Alkinyl, C₃₋₇ Cycloalkyl, (C₁₋₃ Alkyl-C₃₋₇ Cycloalkyl), C₁₋₃ Alkyl-C₆₋₁₀-aryl, C₁₋₃ Alkyl 5-10 gliedriges Heteroaryl mit 1-4 N-, S- oder O-Atomen heteroaryl C₆₋₁₀-Aryl oder 5-10 gliedriges Heteroaryl mit 1-4 N-, S- oder O-Atomen, wobei die C₆₋₁₀-Aryl- und Heteroarylgruppen mit ein oder zwei Substituenten aus der Gruppe bestehend aus F, CI, Br, CH₃, C₂H₅, NO₂, OCH₃, OC₂H₅, CF₃, C₂F₅ substituiert sein können oder auch eine annelierte Methandiylbisoxy, Ethan-1,2-diylbisoxygruppe tragen können, wobei in einem 5-gliedrigen Cycloalkylring ein Ringglied ein N oder ein O sein kann und in einem 6- oder 7-gliedrigen Cycloalkylring ein oder zwei Ringglieder N und/oder O sein können, wobei Ringstickstoffe gegebenenfalls mit C₁₋₃ Alkyl oder C₁₋₃ Alkanoyl substituiert sein können.
***R*⁵** und **R**⁵ unabhängig voneinander Wasserstoff C₁₋₀ Alkyl, C₂₋₀ Alkenyl, C₂₋₆ Alkinyl, wobei ein Kohlenstoffatom gegen O, S, SO, SO₂, NH, N C₁₋₃ Alkyl oder N C₁₋₃ Alkanoyl ausgetauscht sein kann, C₃₋₇ Cycloalkyl-C₀₋₃ Alkyl, wobei in einem 5-gliedrigen Cycloalkylring ein Ringglied ein N oder ein O sein kann und in einem 6- oder 7-gliedrigen Cycloalkylring ein oder zwei Ringglieder N und/oder O sein können, wobei Ringstickstoffe gegebenenfalls mit C₁₋₃ Alkyl oder C₁₋₃ Alkanoyl substituiert sein können, C₆₋₁₀-Aryl oder 5-10 gliedriges Heteroaryl mit 1-4 Heteroatomen aus N, S und O. wobei die genannten Alkyl-, Alkenyl - und Alkinylketten mit einem der zuvor genannten Cycloalkyle, Aryle oder Heteroaryle substituiert sein können, wobei alle zuvor genannten Alkyl- und Cycloalkylreste mit bis zu zwei Substituenten aus CF₃, C₂F₅, OH, O C₁₋₃ Alkyl, NH2, NH C₁₋₃ Alkyl, NH C₁₋₃ Alkanoyl, N (C₁₋₃ Alkyl)₂, N(C₁₋₃ Alkyl)(C₁₋₃ Alkanoyl), COOH, CONH₂, COO C₁₋₃ Alkyl und alle zuvor genannten Aryl- und Heteroarylgruppen mit ein oder zwei Substituenten aus der Gruppe bestehend aus F, Cl, Br, CH₃, C₂H₅, NO₂, OCH₃, OC₂H₅, CF₃, C₂F₅ substituiert sein können oder auch eine annelierte Methandiylbisoxy, Ethan-1,2-diylbisoxygruppe tragen können, oder R⁵ und R⁵ gemeinsam mit dem Stickstoffatom einen 5-7 gliedrigen Heterocyclus bilden, der ein weiteres Sauerstoff-, Stickstoff- oder Schwefelatom enthalten kann und substituiert sein kann mit C₁₋₄-Alkyl, C₁₋₄-Alkoxy-C₀₋₂-alkyl, C₁₋₄-Alkoxy-carbonyl, Aminocarbonyl oder Phenyl, bedeuten.
***A*** C₁₋₁₀ Alkandiyl, C₂₋₁₀ Alkendiyl, C₂₋₁₀ Alkindiyl, (C₀₋₅ Alkandiyl-C₃₋₇ Cycloalkandiyl-C₀₋₅ Alkandiyl), (C₀₋₅ Alkandiylarylen -C₀₋₅ Alkandiyl), (C₀₋₅ Alkandiyl-Heteroarylen-C₀₋₅ Alkandiyl), wobei die Aryl- und Heteroarylgruppen mit ein oder zwei Substituenten aus F, Cl, Br, CH₃, C₂H₅, NO₂, OCH₃, OC₂H₅, CF₃, C₂F₅ substituiert sein können, wobei in einem 5-gliedrigen Cycloalkylring ein Ringglied ein N oder ein O sein kann und in einem 6- oder 7-gliedrigen Cycloalkylring ein oder zwei Ringglieder N und/oder O sein können, wobei Ringstickstoffe gegebenenfalls mit C₁₋₃-Alkyl oder C₁₋₃-Alkanoyl substituiert sein können, wobei in den genannten aliphatischen Ketten ein Kohlenstoffatom oder zwei Kohlenstoffatome, gegen O, NH, NR⁴, NCOR⁴, NSO₂R⁴ ausgetauscht sein können, und wobei Alkyl- oder Cycloalkygruppen mit bis zu zwei Substituenten aus F, OH, OR⁴, OCOR⁴, =O, NH₂, NR⁴R^{4'}, NHCOR⁴, NHCOOR⁴, NHCONHR⁴, NHSO₂R⁴ SH, SR⁴ substituiert sein können,
***B*** Wasserstoff, OH, OCOR⁵, OCONHR⁵, OCOOR⁵, COR⁵, C(NOH)R⁵, C(NOR⁵)R^{5'}, C(NO(COR⁵))R^{5'}, COOH, COOR⁵, CONH₂, CONHNH₂, CONHR⁵, CONR⁵R^{5'}, CONHOH, CONHOR⁵, SO₃H, SO₂NH₂, SO₂NHR⁵, SO₂NR⁵R^{5'}, PO₃H, PO(OH)(OR⁵), PO(OR⁵)(OR^{5'}), PO(OH)(NHR⁵), PO(NHR⁵)(NHR^{5'}), Tetrazolyl, jeweils gebunden an ein Kohlenstoffatom der Gruppe **A,** oder die gesamte Gruppe ***Y-A-B*** N(SO₂R⁴)(SO₂R^{4'}) oder NHSO₂R⁴,
***X*** eine Bindung, CH₂, (CH₂)₂, CH(CH₃), (CH₂)₃, CH(CH₂CH₃), CH(CH₃)CH₂, CH₂CH(CH₃),
***Y*** eine Bindung, O, S, SO, SO₂, NH, NR⁴,NCOR⁴, NSO₂R⁴,
bedeuten,
zur Verwendung bei der Behandlung oder Verhütung von Krankheiten, die mit einer Mikroglia-Aktivierung assoziiert sind, ausgewählt aus der Gruppe bestehend aus AIDS-Demenz, Amyotropher Lateralsklerose, Creuzfeld-Jacob-Krankheit, Down-Syndrom, diffuser Lewy-Körperchen-Krankheit, Chorea Huntington, Leukenzephalopathie, multipler Sklerose, neuronaler Dysfunktion und neuronaler Degeneration, Parkinsonscher Krankheit, Pickscher Krankheit, Alzheimerscher Krankheit, Schlaganfall, Temporal-Lappen-Epilepsie und Tumoren.

17. Verwendung eines Benzimidazols der allgemeinen Formel II 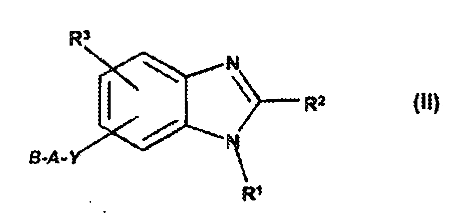 worin
***R*¹** eine mono- oder bicyclische C₆₋₁₂-Arylgruppe oder eine mono- oder bicyclische 5-10 gliedrige Heteroarylgruppe mit 1-4 Heteroatomen ausgewählt aus der Gruppe bestehend aus N, S oder O, wobei die genannte Aryl-oder Heteroarylgruppe mit bis zu drei der folgenden Substituenten unabhängig voneinander substituiert sein kann: F, Cl, Br, I, C(NH)NH₂, C(NH)NHR⁴, C(NH)NR⁴R^{4'}, C(NR⁴)NH₂, C(NR⁴)NHR^{4'}, C(NR⁴)NR⁴R^{4'}, XOH, XOR⁴, XOCOR⁴, XOCONHR⁴, XOCOOR⁴, XCOR⁴, XC(NOH)R⁴, XC(NOR⁴)R^{4'}, XC(NO(COR⁴))R^{4'}, XCN, XCOOH, XCOOR⁴, XCONH₂, XCONR⁴R^{4'}, XCONHR⁴, XCONHOH, XCONHOR⁴, XCOSR⁴, XSR⁴, XSOR⁴,XSO₂R⁴, SO₂NH₂, SO₂NHR⁴,SO₂NR⁴R^{4'}, NO₂,XNH₂, XNHR⁴, XNR⁴R^{4'}, XNHSO₂R⁴, XN(SO₂R⁴)(SO₂R⁴), XNR⁴SO₂R^{4'}, XNHCOR⁴, XNHCOOR⁴, XNHCONHR⁴, Tetrahydro-2,5-dioxopyrrol-1-yl, 2,5- Dihydro-2,5-dioxopyrrol-1-yl, 2,7-Dihydro-2,7-dioxoisoindol-1-yl, R⁴, wobei zwei Substituenten an ***R**¹*, wenn sie zueinander orthoständig sind, so miteinander verknüpft sein können, dass sie gemeinsam Methandiylbisoxy, Ethan-1,2-diylbisoxy, Propan-1,3-diyl, Butan- 1,4-diyl bilden,
***R²*** eine mono- oder bicyclische C₆₋₁₀-Arylgruppe oder eine mono- oder bicyclische 5-10 gliedrige Heteroarylgruppe mit 1-4 Heteroatomen ausgewählt aus der Gruppe bestehend aus N, S oder O, wobei die genannte Aryl-oder Heteroarylgruppe mit bis zu drei der folgenden Substituenten unabhängig voneinander substituiert sein kann: F, Cl, Br, I, C(NH)NH₂, C(NH)NHR⁴, C(NH)NR⁴R^{4'}, C(NR⁴)NH₂, C(NR⁴)NHR^{4'}, C(NR⁴)NR⁴R^{4'}, XOH, XOR⁴, XOCOR⁴, XOCONHR⁴, XOCOOR⁴, XCOR⁴, XC(NOH)R⁴, XC(NOR⁴)R^{4'}, XC(NO(COR⁴))R^{4'}, XCN, XCOOH, XCOOR⁴, XCONH₂, XCONR⁴R^{4'}, XCONHR⁴, XCONHOH, XCONHOR⁴, XCOSR⁴ XSR⁴, XSOR⁴, XSO₂R⁴, SO₂NH₂, SO₂NHR⁴,SO₂NR⁴R^{4'}, NO₂, XNH₂, XNHR⁴, XNR⁴R^{4'}, XNHSO₂R⁴, XN(SO₂R⁴)(SO₂R^{4'}), XNR⁴SO₂R^{4'}, XNHCOR⁴, XNHCOOR⁴, XNHCONHR⁴, Tetrahydro-2,5-dioxopyrrol-1-yl, 2,5-Dihydro-2,5-dioxopyrrol-1-yl, 2,7-Dihydro-2,7- dioxoisoindol-1-yl, R⁴, wobei zwei Substituenten an ***R*²,** wenn sie zueinander orthoständig sind, so miteinander verknüpft sein können, das sie gemeinsam Methandiylbisoxy, Ethan-1,2- diylbisoxy, Propan-1,3-diyl, Butan-1,4-diyl bilden,
***R*³** für ein oder zwei Substituenten steht, die unabhängig voneinander: Wasserstoff, F, Cl, Br, I, XOH, XOR⁴, XOCOR⁴, XCCONHR⁴, XOCOOR⁴, XCOR⁴, XC(NOH)R⁴, XC(NOR⁴)R^{4'}, XC(NO(COR⁴))R^{4'}, XCN, XCOOH, XCOOR⁴, XCONH₂, XCONHR⁴, XCONR⁴R^{4'}, XCONHOH, XCONHOR⁴, XCOSR⁴, XSR⁴, XSOR⁴, XSO₂R⁴,SO₂NH₂, SO₂NHR⁴, SO₂NR⁴R^{4'}, NO₂, XNH₂, XNHR⁴, XNR⁴R^{4'}, XNHSO₂R⁴, XNR⁴SO₂R^{4'}, XN(SO₂R⁴)(SO₂R^{4'}), XNHCOR⁴, XNHCOOR⁴, XNHCONHR⁴, Tetrahydro-2,5-dioxopyrrol-1-yl, 2,5-Dihydro-2,5-dioxopyrrol-1-yl, 2,7-Dihydro-2,7- dioxoisoindol-1-yl, R⁴, wobei zwei Substituenten ***R*³,** wenn sie zueinander orthoständig sind, so miteinander verknüpft sein können, dass sie gemeinsam Methandiylbisoxy, Ethan- 1,2-diylbisoxy, Propan-1,3-diyl, Butan-1,4-diyl bilden,
***R*⁴** und ***R***^{4'} unabhängig voneinander C₁₋₄ Perfluoralkyl, C₁₋₆ Alkyl, C₂₋₈ Alkenyl, C₂₋₈ Alkinyl, C₃₋₇ Cycloalkyl, (C₁₋₃ Alkyl-C₃₋₁ Cycloalkyl), C₁₋₃ Alkyl-C₆₋₁₀-aryl, C₁₋₃ Alkyl 5-10 gliedriges Heteroaryl mit 1-4 N-, S- oder O-Atomen, C₆₋₁₀-Aryl oder 5-10 gliedriges Heteroaryl mit 1-4 N-, S- oder O-Atomen, wobei die C₆₋₁₀-Aryl- und Heteroarylgruppen mit ein oder zwei Substituenten aus der Gruppe bestehend aus F, Cl, Br, CH₃, C₂H₅, NO₂, OCH₃, OC₂H₅, CF₃, C₂F₅ substituiert sein können oder auch eine annelierte Methandiylbisoxy, Ethan-1,2-diylbisaxygruppe tragen können, wobei in einem 5-gliedrigen Cycloalkylring ein Ringglied ein N oder ein O sein kann und in einem 6- oder 7-gliedrigen Cycloalkylring ein oder zwei Ringglieder N und/oder O sein können, wobei Ringstickstoffe gegebenenfalls mit C₁₋₃ Alkyl oder C₁₋₃ Alkanoyl substituiert sein können,
***R*⁵** und **R**^{5'} unabhängig voneinander Wasserstoff C₁₋₆ Alkyl, C₂₋₆ Alkenyl, C₂₋₆ Alkinyl, wobei ein Kohlenstoffatom gegen O, S, SO, SO₂, NH, N C₁₋₃ Alkyl oder N C₁₋₃ Alkanoyl ausgetauscht sein kann, C₃₋₇ Cycloalkyl-C₀₋₃ Alkyl, wobei in einem 5-gliedrigen Cycloalkylring ein Ringglied ein N oder ein O sein kann und in einem 6- oder 7-gliedrigen Cycloalkylring ein oder zwei Ringglieder N und/oder O sein können, wobei Ringstickstoffe gegebenenfalls mit C₁₋₃ Alkyl oder C₁₋₃ Alkanoyl substituiert sein können, C₆₋₁₀-Aryl oder 5-10 gliedriges Heteroaryl mit 1-4 Heteroatomen aus N, S und O, wobei die genannten Alkyl-, Alkenyl- und Alkinylketten mit einem der zuvor genannten Cycloalkyle, Aryle oder Heteroaryle substituiert sein können, wobei alle zuvor genannten Alkyl- und Cycloalkylreste mit bis zu zwei Substituenten aus CF₃, C₂F₅, OH, O C₁₋₃ Alkyl, NH2, NH C₁₋₃ Alkyl, NH C₁₋₃ Alkanoyl, N (C₁₋₃ Alkyl)₂, N(C₁₋₃ Alkyl)(C₁₋₃ Alkanoyl), COOH, CONH₂, COO C₁₋₃ Alkyl und alle zuvor genannten Aryl- und Heteroarylgruppen mit ein oder zwei Substituenten aus der Gruppe bestehend aus F, Cl, Br, CH₃, C₂H₅, NO₂, OCH₃, OC₂H₅, CF₃, C₂F₅ substituiert sein können oder auch eine annelierte Methandiylbisoxy, Ethan-1,2-diylbisoxygruppe tragen können, oder R⁵ und R^{5'} gemeinsam mit dem Stickstoffatom einen 5-7 gliedrigen Heterocyclus bilden, der ein weiteres Sauerstoff-, Stickstoff- oder Schwefelatom enthalten kann und substituiert sein kann mit C₁₋₄-Alkyl, C₁₋₄-Alkoxy-C₀₋₂-alkyl, C₁₋₄-Alkoxy-carbonyl, Aminocarbonyl oder Phenyl, bedeuten.
***A*** C₁₋₁₀ Alkandiyl, C₂₋₁₀ Alkendiyl, C₂₋₁₀ Alkindiyl, (C₀₋₅ Alkandiyl-C₃₋₇ Cycloalkandiyl-C₀₋₅ Alkandiyl), (C₀₋₅ Alkandiylarylen -C₀₋₅ Alkandiyl), (C₀₋₅ Alkandiyl-Heteroarylen-C₀₋₅ Alkandiyl), wobei die Aryl- und Heteroarylgruppen mit ein oder zwei Substituenten aus F, Cl, Br, CH₃, C₂H₅, NO₂, OCH₃, OC₂H₅, CF₃, C₂F₅ substituiert sein können, wobei in einem 5-gliedrigen Cycloalkylring ein Ringglied ein N oder ein O sein kann und in einem 6- oder 7-gliedrigen Cycloalkylring ein oder zwei Ringglieder N und/oder O sein können, wobei Ringstickstoffe gegebenenfalls mit C₁₋₃-Alkyl oder C₁₋₃-Alkanoyl substituiert sein können, wobei in den genannten aliphatischen Ketten ein Kohlenstoffatom oder zwei Kohlenstoffatome, gegen O, NH, NR⁴, NCOR⁴, NSO₂R⁴ ausgetauscht sein können, und wobei Alkyl- oder Cycloalkygruppen mit bis zu zwei Substituenten aus F, OH, OR⁴, OCOR⁴, =O, NH₂, NR⁴R^{4'}, NHCOR⁴, NHCOOR⁴, NHCONHR⁴, NHSO₂R⁴ SH, SR⁴ substituiert sein können,
***B*** Wasserstoff, OH, OCOR⁵, OCONHR⁵, OCOOR⁵, COR⁵, C(NOH)R⁵ ,C(NOR⁵)R^{5'}, C(NO(CC)R⁵))R^{5'}, COOH, COOR⁵, CONH₂, CONHNH₂, CONHR⁵, CONR⁵R^{5'}, CONHOH, CONHOR⁵, SO₃H, SO₂NH₂, SO₂NHR⁵, SO₂NR⁵R^{5'}, PO₃H, PO(OH)(OR⁵), PO(OR⁵)(OR^{5'}), PO(OH)(NHR⁵), PO(NHR⁵)(NHR^{5'}), Tetrazolyl, jeweils gebunden an ein Kohlenstoffatom der Gruppe ***A***, oder die gesamte Gruppe ***Y-A-B*** N(SO²R⁴)(SO₂R^{4'}) oder NHSO₂R⁴,
***X*** eine Bindung, CH₂, (CH₂)₂, CH(CH₃), (CH₂)₃, CH(CH₂CH₃), CH(CH₃)CH₂, CH₂CH(CH₃),
***Y*** eine Bindung, O, S, SO, SO₂, NH, NR⁴,NCOR⁴, NSO₂R⁴,
bedeuten,
zur Herstellung eines Arzneimittels zur Behandlung oder Verhütung von Krankheiten, die mit einer Mikroglia-Aktivierung assoziiert sind, ausgewählt aus der Gruppe bestehend aus AIDS-Demenz, Amyotropher Lateralsklerose, Creuzfeld-Jacob-Krankheit, Down-Syndrom, diffuser Lewy-Körperchen-Krankheit, Chorea Huntington, Leukenzephalopathie, multipler Sklerose, neuronaler Dysfunktion und neuronaler Degeneration, Parkinsonscher Krankheit, Pickscher Krankheit, Alzheimerscher Krankheit, Schlaganfall, Temporal-Lappen-Epilepsie und Tumoren.

18. Verwendung nach Anspruch 16 oder 17, wobei in der allgemeinen Formel II
***R*¹** eine mono- oder bicyclische Arylgruppe oder eine mono- oder bicyclische 5-10 gliedrige Heteroarylgruppe mit 1-2 Heteroatomen ausgewählt aus der Gruppe bestehend aus N, S oder O bedeutet, wobei die genannte Aryl-oder Heteroarylgruppe mit bis zu drei der folgenden Substituenten unabhängig voneinander substituiert sein kann: F, Cl, Br, XOH, XOR⁴, XOCOR⁴, XOCONHR⁴, XOCOOR⁴, XCOR⁴, XCN, XCOOH, XCOOR⁴, XCONH₂, XCONR⁴R⁴', XCONHR⁴, XCONHOH, XCONHOR⁴, XCOSR⁴, XSR⁴, NO₂, XNHR⁴, XNR⁴R⁴, R⁴, wobei zwei Substituenten an ***R¹***, werten sie zueinander orthoständig sind, so miteinander verknüpft sein können, dass sie gemeinsam Methandiylbisoxy, Ethan-1,2-diylbisoxy, Propan- 1,3-diyl, Butan-1,4-diyl bilden.

19. Verwendung nach einem der Ansprüche 16-18, wobei in der allgemeinen Formel II
***R²*** eine mono- oder bicyclische Arylgruppe oder eine mono- oder bicyclische 5-10 gliedrige Heteroarytgruppe mit 1-2 Heteroatomen ausgewählt aus der Gruppe bestehend aus N, S oder O bedeuten, wobei die genannte Aryl-oder Heteroarylgruppe mit bis zu drei der folgenden Substituenten unabhängig voneinander substituiert sein kann: F, Cl, Br, XOH. XOR⁴, XOCOR⁴, XOCONHR⁴, XOCOOR⁴,XCOR⁴, XC(NOH)R⁴, XC(NOR⁴)R⁴, XC(NO)(COR⁴))R⁴, XCN, XCOOH, XCOOR⁴, XCONH₂, XCONR⁴R⁴, XCONHR⁴, XCONHOH, XCONHOR⁴, XCOSR⁴, XSR⁴, XSOR⁴, XSO₂R⁴, SO₂NH₂, SO₂NHR⁴, SO₂NR⁴R⁴, NO₂, XNH₂, XNHR⁴, XNR⁴R⁴, XNHSO₂R⁴, XN(SO₂R⁴)(SO₂R⁴), XNR⁴SO₂R⁴, XNHCOR⁴, XNHCOOR⁴, XNHCONHR⁴, R⁴, wobei zwei Substituenten an ***R²***, wenn sie zueinander orthoständig sind, so miteinander verknüpft sein können, dass sie gemeinsam Methandiylbisoxy, Ethan-1,2-diylbisoxy, Propan- 1,3-diyl, Butan-1,4-diyl bilden.

20. Verwendung nach einem der Ansprüche 16-19, wobei in der allgemeinen Formel II
***R³*** für ein oder zwei Substituenten steht, die unabhängig voneinander. Wasserstoff,F, Cl, Br, XOH, XOR⁴, XOCOR⁴, XOCONHR⁴, XOCOOR⁴, XCOR⁴, XC(NOH)R⁴, XC(NOR⁴)R⁴, XC(NO(COR⁴))R^{4'},XCN, XSR⁴, XSOR⁴, XSO₂R⁴,SO₂NH₂, SO₂NHR⁴, SO₂NR⁴,NO₂, XNH₂, XNHR⁴, XNR⁴R^{4'}, XNHSO₂R⁴, XNR⁴SO₂R^{4'}, XN(SO₂R⁴) (SO₂R^{4'}),XNHCOR⁴, XNHCOOR⁴, XNHCONHR⁴ oder R⁴ bedeuten, wobei zwei Substituenten ***R³***, wenn sie zueinander orthoständig sind, so miteinander verknüpft sein können, dass sie gemeinsam Methandiylbisoxy, Ethan-1,2- diylbisoxy, Propan-1,3-diyl, Butan-1,4-diyl bilden.

21. Verwendung nach einem der Ansprüche 16-20, wobei in der allgemeinen Formel II
***R⁴*** und **R^{4'}** unabhängig voneinander CF₃, C₂F₅, C₁₋₄ Alkyl, C₂₋₄ Alkenyl, C₂₋₄ Alkinyl, C₃₋₆ Cycloalkyl, (C₁₋₂ Alkyl-C₃₋₆ Cycloalkyl), C₁₋₃ Alkylaryl, C₁₋₃ Alkylheteroaryl, monocyclisches Aryl oder 5-6 gliedriges Heteroaryl mit 1-2 N-, S- oder O-Atamen, wobei die Aryl- und Heteroarylgruppen mit ein oder zwei Substituenten aus der Gruppe bestehend aus F, Cl, Br, CH₃, C₂H₅, NO₂, OCH₃, OC₂H₅, CF₃, C₂F₅ substituiert sein können oder auch eine annelierte Methandiylbisoxy- oder Ethan-1,2-diylbisoxygruppe tragen können, und weiterhin in einem 5-gliedrigen Cycloalkylring ein Ringglied ein N oder ein O sein kann und in einem 6-gliedrigen Cycloalkylring ein oder zwei Ringglieder N und/oder O sein können, wobei Ringstickstoffe gegebenenfalls mit C₁₋₃ Alkyl oder C₁₋₃ Alkanoyl substituiert sein können.

22. Verwendung nach einem der Ansprüche 16-21, wobei in der allgemeinen Formel II
***R⁵*** und **R^{5'}** unabhängig voneinander C₁₋₆ Alkyl, wobei ein Kohlenstoffatom gegen O, NH, N C₁₋ ₃ Alkyl, N C₁₋₃ Alkanoyl ausgetauscht sein kann C₃₋₇ Cycloalkyl-C₀₋₃ Alkyl, wobei in einem 5-gliedrigen Cycloalkylring ein Ringglied ein N oder ein O sein kann und in einem 6- oder 7-gliedrigen Cycloalkylring ein oder zwei Ringglieder N und/oder O sein können, wobei Ringstickstoffe gegebenenfalls mit C₁₋₃ Alkyl oder C₁₋₃ Alkanoyl substituiert sein können, wobei der genannte C₁₋₆ Alkylteil mit einem der zuvor genannten Cycloalkyle oder auch einem 5-6 gliedrigen Heteroaromaten mit 1-2 Heteroatomen ausgewählt aus der Gruppe bestehend aus N, S oder O, substituiert sein kann, wobei alle zuvor genannten Alkyl- und Cycloalkylteile mit bis zu zwei Substituenten aus CF₃, OH, O C₁₋₃ Alkyl, und die zuvor genannten Heteroarylgruppen mit ein oder zwei Substituenten aus F, Cl, CF₃, CH₃, C₂H₅, OCH₃, OC₂H₅ substituiert sein können, oder R⁵ und R^{5'} gemeinsam mit dem Stickstoffatom einen 5-7 gliedrigen Heterocyclus bilden, der ein weiteres Sauerstoff-, Stickstoff- oder Schwefelatom enthalten kann und substituiert sein kann mit C₁₋₄-Alkyl, C₁₋₄-Alkoxy-C₀₋₂-alkyl, C₁₋₄-Alkoxy-carbonyl, Aminocarbonyl oder Phenyl, bedeutet.

23. Verwendung nach einem der Ansprüche 16-22, wobei in der allgemeinen Formel II
***A*** C₁₋₁₀ Alkandiyl, C₂₋₁₀ Alkendiyl, C₂₋₁₀ Alkindiyl, (C₀₋₅ Alkandiyl-C₃₋₇ Cycloalkandiyl-C₀₋₅ Alkandiyl) oder (C₀₋₅ Alkandiyl-Heteroarylen-C₀₋₅ Alkandiyl),bedeutet, wobei eine gegebenenfalls vorhandene Heteroarylgruppe mit ein oder zwei Substituenten aus F, Cl, Br, CH₃, C₂H₅, NO₂,OCH₃, OC₂H₅, CF₃, C₂F₅ substituiert sein kann, und weiterhin in einem 5-gliedrigen Cycloalkylring ein Ringglied ein N oder ein O sein kann und in einem 6- oder 7-gliedrigen Cycloalkylring ein oder zwei Ringglieder N und/oder O sein können, wobei Ringstickstoffe gegebenenfalls mit C₁₋₃ Alkyl oder C₁₋₃ Alkanoyl substituiert sein können, wobei in einer aliphatischen Kette ein Kohlenstoffatom oder zwei Kohlenstoffatome, gegen O, NH, N C₁₋₃ Alkyl, N C₁₋₃ Alkanoyl, NSO₂ C₁₋₃ Alkyl ausgetauscht sein können, und wobei Alkyl- oder Cycloalkylteile mit bis zu zwei F Atomen oder einem der Substituenten aus OH, O C₁₋₃ Alkyl, O C₁₋₃ Alkanoyl, =O, NH₂, NH C₁₋₃ Alkyl, N (C₁₋₃ Alkyl)₂, NH C₁₋₃ Alkanoyl, N (C₁₋₃ Alkyl) (C₁₋₃ Alkanoyl), NHCOO C₁₋₃ Alkyl, NHCONH C₁₋₃ Alkyl, NHSO₂ C₁₋₃ Alkyl, SH, S C₁₋₃ Alkyl substituiert sein können.

24. Verwendung nach einem der Ansprüche 16-23, wobei in der allgemeinen Formel II
***B*** Wasserstoff, OH, OCOR⁵, OCONHR⁵, OCOOR⁵ COOH, COOR⁵, CONH₂, CONHR⁵, CONR⁵R^{5'}, CONHOH, CONHOR⁵ oder Tetrazolyl, jeweils gebunden an ein Kohlenstoffatom der Gruppe A bedeutet.

25. Verwendung nach einem der Ansprüche 16-24, wobei in der allgemeinen-Formel II
***X*** eine Bindung oder CH₂ bedeutet.

26. Verwendung nach einem der Ansprüche 16-25, wobei in der allgemeinen Formel **II**
***Y*** eine Bindung, O. S, NH, NR⁴,NCOR⁴ oder NSO₂R⁴ bedeutet.

## Claims

1. Benzimidazoles of the general formula (I) 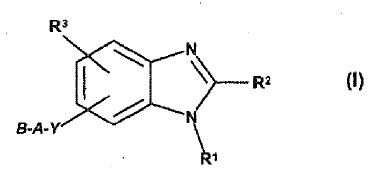 in which
***R¹*** is a mono- or bicyclic C₆₋₁₂-aryl group or a mono- or bicyclic 5-10-membered heteroaryl group with 1-4 heteroatoms selected from the group consisting of N, S or 0, where the specified aryl or heteroaryl group can be substituted with up to three of the following substituents independently of one another: F, Cl, Br, I, C(NH)NH₂, C(NH)NHR⁴, C(NH)NR⁴R^{4'}, C(NR⁴)NH₂, C(NR⁴)NHR^{4'}, C(NR⁴)NR⁴R^{4'}, XOH, XOR⁴, XOCOR⁴, XOCONHR⁴, XOCOOR⁴, XCOR⁴, XC(NOH)R⁴, XC(NOR⁴)R^{4'}, XC(NO(COR⁴))R^{4'}, XCN, XCOOH, XCOOR⁴, XCONH₂, XCONR⁴R^{4'} , XCONHR⁴, XCONHOH, XCONHOR⁴, XCOSR⁴, XSR⁴, XSOR⁴, XSO₂R⁴ SO₂NH₂, SO₂NHR⁴ SO₂NR⁴R^{4'}, NO₂, XNH₂, XNHR⁴, XNR⁴R^{4'}, XNHSO₂R⁴, XN(SO₂R⁴)SO₂R^{4'} , XNR⁴SO₂R^{4'}, XNHCOR⁴, XNHCOOR⁴, XNHCONHR⁴, tetrahydro- 2,5-dioxopyrrol-1-yl, 2,5-dihydro-2,5-dioxopyrrol- 1-yl, 2,7-dihydro-2,7-dioxoisoindol-1-yl, R⁴, where two substituents on ***R¹*,** if they are in the ortho position relative to one another, can be linked with one another such that they together form methanediylbisoxy, ethane-1,2-diylbisoxy, propane-1,3-diyl, butane-1,4-diyl,
***R²*** is a mono- or bicyclic C₆₋₁₀-aryl group or a mono- or bicyclic 5-10-membered heteroaryl group with 1-4 heteroatoms selected from the group consisting of N, S or 0, where the specified aryl or heteroaryl group can be substituted with up to three of the following substituents independently of one another: F, Cl, Br, I, XOH, XOR⁴, XOCOR⁴, XOCONHR⁴, XOCOOR⁴, XCOR⁴, XC(NOH)R⁴, XC(NOR⁴)R^{4'}, XC(NO(COR⁴))R^{4'} XCOOH, XCOOR⁴, XCONH₂, XCONHR⁴, XCONR⁴R^{4'}, XCONHOH, XCONHOR⁴, XCOSR⁴, XSR⁴, XSOR⁴, XSO₂R⁴, SO₂NH₂, SO₂NHR⁴, SO₂NR⁴R^{4'}, NO₂, XNHR⁴, XNR⁴R^{4'}, XNHSO₂R⁴, XN(SO₂R⁴) SO₂R^{4'}, XNR⁴SO₂R^{4'}, tetrahydro-2,5-dioxopyrrol-1-yl, 2,5- dihydro-2,5-dioxopyrrol-1-yl, 2,7-dihydro-2,7- dioxoisoindol-1-yl, R⁴, where two substituents on ***R²*,** if they are in the ortho position relative to one another, can be linked with one another such that they together form methanediylbisoxy, ethane-1,2-diylbisoxy, propane-1,3-diyl, butane-1,4-diyl,
***R³*** is one or two substituents which can be independently of one another: hydrogen, F, Cl, Br, I, XOH, XOR⁴, XOCOR⁴, XOCONHR⁴, XOCOOR⁴, XCOR⁴, XC(NOH)R⁴, XC(NOR⁴)R^{4'}XC(NO(COR⁴))R^{4'}, XCN, XCOOH, XCOOR⁴, XCONH₂, XCONHR⁴, XCONR⁴R^{4'}, XCONHOH, XCONHOR⁴, XCOSR⁴ XSR⁴, XSOR⁴, XSO₂R⁴, SO₂NH₂, SO₂NHR⁴, SO₂NR⁴R^{4'}, NO₂, XNH₂, XNHR⁴, XNR⁴R^{4'}, XNHSO₂R⁴, XNR⁴SO₂R^{4'}, XN(SO₂R⁴)(SO₂R^{4'}), XNHCOR⁴, XNHCOOR⁴, XNHCONHR⁴, tetrahydro-2,5- dioxopyrrol-1-yl, 2,5-dihydro-2,5-dioxopyrrol-1- yl, 2,7-dihydro-2,7-dioxoisoindol-1-yl or R⁴, where two substituents ***R³*,** if they are in the ortho position relative to one another, can be linked with one another such that they together form methanediylbisoxy, ethane-1,2-diylbisoxy, propane- 1,3-diyl, butane-1,4-diyl,
***R⁴*** and ***R^{4'}*,** independently of one another, are C₁₋₄ perfluoroalkyl, C₁₋₆ alkyl, C₂₋₆ alkenyl, C₂₋₆ alkynyl, C₃₋₇ cycloalkyl, (C₁₋₃ alkyl-C₃₋₇ cycloalkyl), C₁₋₃ alkyl-C₆₋₁₀-aryl, C₁₋₃ alkyl-5-10- membered heteroaryl with 1-4 N, S or 0 atoms, C₆₋₁₀- aryl or 5-10-membered heteroaryl with 1-4 N, S or O atoms, where the aryl and heteroaryl groups can be substituted with one or two substituents from the group consisting of F, Cl, Br, CH₃, C₂H₅, NO₂, OCH₃, OC₂H₅, CF₃, C₂F₅ or else can carry a fused methanediylbisoxy, ethane-1,2-diylbisoxy group, and furthermore, in a 5-membered cycloalkyl ring, one ring member can be an N or an 0 and in a 6- or 7-membered cycloalkyl ring, one or two ring members can be N and/or 0, where ring nitrogens can be optionally substituted with C₁₋₃ alkyl or C₁- alkanoyl,
***R⁵*** and ***R^{5'}*,** independently of one another, are C₁₋₆ alkyl, C₂₋₆ alkenyl, C₂₋₆ alkynyl, where one carbon atom can be exchanged for 0, S, SO, SO₂, NH, N C₁₋₃ alkyl or N C₁₋₃ alkanoyl, C₃₋₇ cycloalkyl-C₀₋₃ alkyl, where, in a 5-membered cycloalkyl ring, one ring member can be an N or an O and in a 6- or 7-membered cycloalkyl ring, one or two ring members can be N and/or 0, where ring nitrogens can be optionally substituted with C₁₋₃ alkyl or C₁₋₃ alkanoyl, C₆₋₁₀-aryl or 5-10-membered heteroaryl with 1-4 heteroatoms from N, S and 0, where the specified alkyl, alkenyl and alkynyl chains can be substituted with one of the aforementioned cycloalkyls, aryls or heteroaryls, where all of the aforementioned alkyl and cycloalkyl radicals can be substituted with up to two substituents from CF₃, C₂F₅, OH, 0 C₁₋₃ alkyl, NH₂, NH C₁₋₃ alkyl, NH C₁₋₃ alkanoyl, N (C₁₋₃ alkyl)₂, N(C₁₋₃ alkyl) (C₁₋₃alkanoyl), COOH, CONH₂, COO C₁₋₃ alkyl and all of the aforementioned aryl and heteroaryl groups can be substituted with one or two substituents from the group consisting of F, Cl, Br, CH₃, C₂H₅, NO₂, OCH₃, OC₂H₅, CF₃, C₂F₅ or else can carry a fused methanediylbisoxy, ethane-1,2- diylbisoxy group, or R⁵ and R⁵, together with the nitrogen atom, form a 5-7-membered heterocycle which can contain a further oxygen, nitrogen or sulphur atom and can be substituted with C₁₋₄-alkyl, C₁₋₄-alkoxy-C₀₋₂- alkyl, C₁₋₄-alkoxycarbonyl, aminocarbonyl or phenyl,
***A*** is C₁₋₁₀ alkanediyl, C₂₋₁₀ alkenediyl, C₂₋₁₀ alkynediyl, (C₀₋₅ alkanediyl-C₃₋₇ cycloalkanediyl- C₀₋₅ alkanediyl), where, in a 5-membered cycloalkyl ring, one ring member can be an N or an 0 and in a 6- or 7- membered cycloalkyl ring, one or two ring members can be N and/or 0, where ring nitrogens can be optionally substituted with C₁₋₃ alkyl or C₁₋₃ alkanoyl,
where, in the aforementioned aliphatic chains, one or two carbon atoms can be exchanged for 0, NH, N C₁₋₃ alkyl, N C₁₋₃ alkanoyl and where alkyl or cycloalkyl groups can be substituted with up to two substituents from =O, OH, 0 C₁₋₃ alkyl, NH₂, NH C₁₋₃ alkyl, NH C₁₋₃ alkanoyl, N (C₁₋₃ alkyl)₂, N(C₁₋₃ alkyl)(C₁₋₃ alkanoyl),
***B*** is COOH, COOR⁵, CONH₂, CONHNH₂, CONHR⁵, CONHR⁵R^{5'}, CONHOH, CONHOR⁵, SO₃H, SO₂NH₂, SO₂NHR⁵, S0₂NR⁵R^{5'}, PO₃H, PO(OH)(OR⁵) , PO(OR⁵)(OR^{5'}) , PO(OH)(NHR⁵) , PO(NHR⁵) (NHR^{5'}) , tetrazolyl, in each case bonded to a carbon atom of group ***A*,** or the overall group ***Y-A-B*** is N(SO₂R⁴)(SO₂R^{4'}) or NHSO₂R⁴,
***X*** is a bond, CH₂, (CH₂)₂, CH(CH₃), (CH₂)₃, CH(CH₂CH₃), CH(CH₃) CH₂, CH₂CH(CH₃),
***Y*** is 0, NH, NR⁴, NCOR⁴, NSO₂R⁴,
with the proviso that
A) if Y is NH, NR⁴, NCOR⁴ or NSO₂R⁴,
a) and the substituent ***R²*** contains a nitrogencontaining saturated heterocycle, this heterocycle is not substituted on the imine nitrogen with H, methyl, ethyl, propyl or isopropyl,
or
b) in any present groups XNHR⁴ or XNR ⁴R^{4'} of the substituent ***R²***, R⁴ *and*/*or* R^{4'} are not C₁₋₄-alkyl,
and that
B) ***B*** is not COOH, SO₃H, PO₃H₂ or tetrazolyl, and ***R¹*** and ***R²*,** independently of one another, are not C₅₋₆ heteroaryl or phenyl at the same time if these, independently of one another, are unsubstituted, monosubstituted with C₁₋₆ alkyl, C₁₋₄ perfluoroalkyl, O C₁₋₆ alkyl, O C₁₋₄ perfluoroalkyl, COOH, COO C₁₋₆ alkyl, CO C₁₋₆ alkyl, CONH₂, CONHR⁴, NO₂, NH₂, NHCOR⁴, NHSO₂R⁴ or substituted with one or two halogen atoms from the group F, Cl, Br, I, and where the following compounds are excluded:
methyl [(1,2-diphenyl-1H-benzimidazol-6-yl)oxy]acetate,
methyl 5-[(1,2-diphenyl-1H-benzimidazol-6-yl)oxy]-pentanoate,
ethyl 4-[(1,2-diphenyl-1H-benzimidazol-6-yl)oxy]-butanoate,
methyl 5-[[1-(4-nitrophenyl)-2-phenyl-1H-benzimidazol-6-yl]oxy]pentanoate,
methyl 6-[[1-(4-nitrophenyl)-2-phenyl-1H-benzimidazol-6-yl]oxy]hexanoate,
methyl 5-[[1-(4-aminophenyl)-2-phenyl-1H-benzimidazol-6-yl]oxy]pentanoate,
methyl 5-[[1-[4-[[(4-chlorophenyl)sulphonyl]amino]-phenyl]-2-phenyl-1H-benzimidazol-6-yl]oxy]pentanoate,
methyl 5-[[1-[4-[(acetyl)amino]phenyl]-2-phenyl-1H-benzimidazol-6-yl]oxy]pentanoate,
methyl 5-[[1-(3-nitrophenyl)-2-phenyl-1H-benzimidazol-6-yl]oxy]pentanoate,
methyl 6-[[1-(3-nitrophenyl)-2-phenyl-1H-benzimidazol-6-yl]oxy]hexanoate,
methyl 5-[[1-(3-aminophenyl)-2-phenyl-1H-benzimidazol-6-yl]oxy]pentanoate,
methyl 5-[[1-[3-[[(4-chlorophenyl)sulphonyl]amino]-phenyl]-2-phenyl-1H-benzimidazol-6-yl]oxy]pentanoate,
methyl 5-[[1-[3-[(acetyl)amino]phenyl]-2-phenyl-1H-benzimidazol-6-yl]oxy]pentanoate.

2. Benzimidazoles according to Claim 1, **characterized in that**
***R¹*** is a mono- or bicyclic C₆₋₁₂-aryl group or a mono- or bicyclic 5-10-membered heteroaryl group with 1-2 heteroatoms selected from the group consisting of N, S or 0, where the specified aryl or heteroaryl group can be substituted with up to three of the following substituents independently of one another: F, Cl, Br, XOH, XOR⁴, XOCOR⁴, XOCONHR⁴, XOCOOR⁴, XCOR⁴, XCN, XCOOH, XCOOR⁴, XCONH₂, XCONR⁴R^{4'}, XCONHR⁴, XCONHOH, XCONHOR⁴, XCOSR⁴, XSR⁴, NO₂, XNHR⁴, XNR⁴R^{4'}, R⁴, where two substituents on ***R¹*,** if they are in the ortho position relative to one another, can be linked with one another such that they together form methanediylbisoxy, ethane-1,2-diylbisoxy, propane-1,3-diyl, butane-1,4-diyl.

3. Benzimidazoles according to Claim 1 or 2, **characterized in that**
***R²*** is a mono- or bicyclic C₆₋₁₀-aryl group or a mono- or bicyclic 5-10-membered heteroaryl group with 1-2 heteroatoms selected from the group consisting of N, S or 0, where the specified aryl or heteroaryl group can be substituted with up to three of the following substituents independently of one another: F, Cl, Br, XOH, XOR⁴, XOCOR⁴, XOCONHR⁴, XOCOOR⁴, XCOR⁴, XC(NOH)R⁴, XC(NOR⁴)R⁴ XC(NO(COR⁴))R^{4'}, XCOOH, XCOOR⁴, XCONH₂, XCONHR⁴, XCONR⁴R^{4'}, XCONHOH, XCONHOR⁴, XCOSR⁴, XSR⁴, XSOR⁴, XSO₂R⁴, SO₂NH₂, SO₂NHR⁴ SO₂NR⁴R^{4'}, NO₂, XNHR⁴, XNR⁴R^{4'}, XNHSO₂R⁴, XN(SO₂R⁴) SO₂R^{4'}, XNR⁴SO2R^{4'}, R⁴, where two substituents on ***R²*,** if they are in the ortho position relative to one another, can be linked with one another such that they together form methanediylbisoxy, ethane-1,2-diylbisoxy, propane-1,3-diyl, butane-1,4-diyl.

4. Benzimidazoles according to one of Claims 1-3, **characterized in that**
***R³*** is one or two substituents which can be independently of one another: hydrogen, F, Cl, Br, XOH, XOR⁴, XOCOR⁴, XOCONHR⁴, XOCOOR⁴, XCOR⁴, XC(NOH)R⁴, XC(NOR⁴)R^{4'}, XC(NO(COR⁴))R^{4'}, XCN, XSR⁴, XSOR⁴, XSO₂R⁴, SO₂NH₂, SO₂NHR⁴, SO₂NR⁴R^{4'}, NO₂, XNH₂, XNHR⁴, XNR⁴R^{4'}, XNHSO₂R⁴, XNR⁴SO₂R^{4'}, XN(SO₂R⁴)SO₂R^{4'}, XNHCOR⁴, XNHCOOR⁴, XNHCONHR⁴, or R⁴, where two substituents ***R³*,** if they are in the ortho position relative to one another, can be linked with one another such that they together form methanediylbisoxy, ethane-1,2-diylbisoxy, propane- 1,3-diyl, butane-1,4-diyl.

5. Benzimidazoles according to one of Claims 1-4, **characterized in that**
***R⁴*** and ***R^{4'}***, independently of one another, are CF₃, C₂F₅, C₁₋₄-alkyl, C₂₋₄-alkenyl, C₂₋₄-alkynyl, C₃₋₆- cycloalkyl, (C₁₋₃-alkyl-C₃₋₆-cycloalkyl), phenyl or 5-6-membered heteroaryl with 1-2 N, S or 0 atoms, where the phenyl and heteroaryl groups can be substituted with one or two substituents from the group consisting of F, Cl, Br, CH₃, C₂H₅, OCH₃, OC₂H₅, CF₃, C₂F₅, and furthermore, in a 5-membered cycloalkyl ring, one ring member can be an N or an 0 and in a 6-membered cycloalkyl ring, one or two ring members can be N and/or 0, where ring nitrogens can be optionally substituted with C₁₋₃-alkyl or C₁₋₃-alkanoyl.

6. Benzimidazoles according to one of Claims 1-5, **characterized in that**
***R⁵*** and ***R^{5'}***, independently of one another, are C₁₋₆ alkyl, where one carbon atom can be exchanged for 0, NH, N C₁₋₃ alkyl, N C₁₋₃ alkanoyl, C₃₋₇ cycloalkyl-C₀₋₃ alkyl, where, in a 5-membered cycloalkyl ring, one ring member can be an N or an O and in a 6- or 7-membered cycloalkyl ring, one or two ring members can be N and/or 0, where ring nitrogens can be optionally substituted with C₁₋₃ alkyl or C₁₋₃ alkanoyl, where the specified C₁₋₆ alkyl moiety can be substituted with one of the aforementioned cycloalkyls or else a 5-6-membered heteroaromatic with 1-2 heteroatoms selected from N, S or 0, where all of the aforementioned alkyl and cycloalkyl moieties can be substituted with up to two substituents from CF₃, OH, 0 C₁₋₃ alkyl, and the aforementioned heteroaryl groups can be substituted with one or two substituents from F, Cl, CF₃, CH₃, C₂H₅, OCH₃, OCH₂H₅, or R⁵ and R^{5'}, together with the nitrogen atom, form a 5-7- membered heterocycle which can contain a further oxygen, nitrogen or sulphur atom and can be substituted with C₁₋₄-alkyl, C₁₋₄-alkoxy-C₀₋₂-alkyl, C₁₋₄-alkoxycarbonyl, aminocarbonyl or phenyl.

7. Benzimidazoles according to one of Claims 1-6, **characterized in that**
***A*** is C₁₋₁₀ alkanediyl, C₂₋₁₀ alkenediyl, C₂₋₁₀ alkynediyl, (C₀₋₅ alkanediyl-C₃₋₇ cycloalkanediyl-C₀₋ alkanediyl), where, in a 5-membered cycloalkyl ring, one ring member can be an N or an 0 and in a 6- or 7- membered cycloalkyl ring, one or two ring members can be N and/or 0, where ring nitrogens can be optionally substituted with C₁₋₃ alkyl or C₁₋₃ alkanoyl,
where, in the aforementioned aliphatic chains, one or two carbon atoms can be exchanged for 0, NH, N C₁₋₃ alkyl, N C₁₋₃ alkanoyl.

8. Benzimidazoles according to one of Claims 1-7, **characterized in that**
***B*** is COOH, COOR⁵, CONH₂, CONHR⁵, CONR⁵R^{5'}, CONHOH, CONHOR⁵ or tetrazolyl, in each case bonded to a carbon atom of group ***A.***

9. Benzimidazoles according to one of Claims 1-8, **characterized in that**
***X*** is a bond or methylene.

10. Benzimidazoles according to one of Claims 1-9, **characterized in that**
***Y*** is O.

11. Benzimidazole according to one of Claims 1-10, selected from the group consisting of
isopropyl [(1,2-diphenyl-1H-benzimidazol-6-yl)-oxy]acetate
methyl 3-[(1,2-diphenyl-1H-benzimidazol-6-yl)oxy]-propanoate
methyl 2-[(1,2-diphenyl-1H-benzimidazol-6-yl)oxy]-propanoate
isopropyl 4-[(1,2-diphenyl-1H-benzimidazol-6-yl)-oxy]butanoate
isopropyl 5-[(1,2-diphenyl-1H-benzimidazol-6-yl)-oxy]pentanoate
methyl 6-[(1,2-diphenyl-1H-benzimidazol-6-yl)oxy]-hexanoate
isopropyl 6-[(1,2-diphenyl-1H-benzimidazol-6-yl)-oxy]hexanoate
6-[(1,2-diphenyl-1H-benzimidazol-6-yl)oxy]-hexanamide
N-methoxy-6-[(1,2-diphenyl-1H-benzimidazol-6-yl)oxy]hexanamide
N-(phenylmethoxy)-6-[(1,2-diphenyl-1H-benzimidazol-6-yl)oxy]hexanamide
N-hydroxy-6-[(1,2-diphenyl-1H-benzimidazol-6-yl)oxy]hexanamide
methyl 7-[(1,2-diphenyl-1H-benzimidazol-6-yl)oxy]-heptanoate
isopropyl 6-[[1-(3-nitrophenyl)-2-phenyl-1H-benzimidazol-6-yl]oxy]hexanoate
methyl 6-[[2-phenyl-1-[3-(trifluoromethyl)phenyl]-1H-benzimidazol-6-yl]oxy]hexanoate
isopropyl 6-[[2-phenyl-1-[3-(trifluoromethyl)-phenyl]-1H-benzimidazol-6-yl]oxy]hexanoate
methyl 6-[[1-(3-cyanophenyl)-2-phenyl-1H-benzimidazol-6-yl]oxy]hexanoate
isopropyl 6-[[1-(3-cyanophenyl)-2-phenyl-1H-benzimidazol-6-yl]oxy]hexanoate
6-[[1-(3-cyanophenyl)-2-phenyl-1H-benzimidazol-6-yl]oxy]hexanoic acid
methyl 6-[[1-(4-cyanophenyl)-2-phenyl-1H-benzimidazol-6-yl]oxy]hexanoate
isopropyl 6-[[1-(4-cyanophenyl)-2-phenyl-1H-benzimidazol-6-yl]oxy]hexanoate
methyl 6-[[1-(3-chlorophenyl)-2-phenyl-1H-benzimidazol-6-yl]oxy]hexanoate
isopropyl 6-[[1-(3-chlorophenyl)-2-phenyl-1H-benzimidazol-6-yl]oxy]hexanoate
methyl 6-[[1-(4-chlorophenyl)-2-phenyl-1H-benzimidazol-6-yl]oxy]hexanoate
isopropyl 6-[[1-(4-chlorophenyl)-2-phenyl-1H-benzimidazol-6-yl]oxy]hexanoate
methyl 6-[[1-(3-methylphenyl)-2-phenyl-1H-benzimidazol-6-yl]oxy]hexanoate
isopropyl 6-[[1-(3-methylphenyl)-2-phenyl-1H-benzimidazol-6-yl]oxy]hexanoate
methyl 6-[[1-(4-methylphenyl)-2-phenyl-1H-benzimidazol-6-yl]oxy]hexanoate
isopropyl 6-[[1-(4-methylphenyl)-2-phenyl-1H-benzimidazol-6-yl]oxy]hexanoate
methyl 6-[[1-(3,4-dimethylphenyl)-2-phenyl-1H-benzimidazol-6-yl]oxy]hexanoate
methyl 6-[[1-(3,5-dimethylphenyl)-2-phenyl-1H-benzimidazol-6-yl]oxy]hexanoate
isopropyl 6-[[1-(3,5-dimethylphenyl)-2-phenyl-1H-benzimidazol-6-yl]oxy]hexanoate
methyl 6-[[1-(3-methoxyphenyl)-2-phenyl-1H-benzimidazol-6-yl]oxy]hexanoate
methyl 6-[[1-(4-methoxyphenyl)-2-phenyl-1H-benzimidazol-6-yl]oxy]hexanoate
methyl 6-[[1-(3,4-dimethoxyphenyl)-2-phenyl-1H-benzimidazol-6-yl]oxy]hexanoate
methyl 6-[[1-[3,4-(methylenedioxy)phenyl]-2-phenyl-1H-benzimidazol-6-yl]oxy]hexanoate
6-[[1-[3,4-(methylenedioxy)phenyl]-2-phenyl-1H-benzimidazol-6-yl]oxy]hexanoic acid
methyl 6-[[2-phenyl-1-(3,4,5-trimethoxyphenyl)-1H-benzimidazol-6-yl]oxy]hexanoate
6-[[2-phenyl-1-(3,4,5-trimethoxyphenyl)-1H-benzimidazol-6-yl]oxy]hexanoic acid
isopropyl 6-[[2-phenyl-1-(3,4,5-trimethoxyphenyl)-1H-benzimidazol-6-yl]oxy]hexanoate
methyl 6-[[1-[4-(N,N-dimethylamino)phenyl]-2-phenyl-1H-benzimidazol-6-yl]oxy]hexanoate
6-[[1-[4-(N,N-dimethylamino)phenyl]-2-phenyl-1H-benzimidazol-6-yl]oxy]hexanoic acid
isopropyl 6-[[1-phenyl-2-[3-(trifluoromethyl)-phenyl]-1H-benzimidazol-6-yl]oxy]hexanoate
methyl 6-[[2-(3-chlorophenyl)-1-phenyl-1H-benzimidazol-6-yl]oxy]hexanoate
isopropyl 6-[[2-(3-chlorophenyl)-1-phenyl-1H-benzimidazol-6-yl]oxy]hexanoate
methyl 6-[[2-(4-chlorophenyl)-1-phenyl-1H-benzimidazol-6-yl]oxy]hexanoate
isopropyl 6-[[2-(4-chlorophenyl)-1-phenyl-1H-benzimidazol-6-yl]oxy]hexanoate
methyl 6-[[2-(4-methylphenyl)-1-phenyl-1H-benzimidazol-6-yl]oxy]hexanoate
isopropyl 6-[[2-(4-methylphenyl)-1-phenyl-1H-benzimidazol-6-yl]oxy]hexanoate
methyl 6-[[1-phenyl-2-(4-pyridinyl)-1H-benzimidazol-6-yl]oxy]hexanoate
methyl 6-[(1,2-diphenyl-5-nitro-1H-benzimidazol-6-yl)oxy]hexanoate
isopropyl 6-[(1,2-diphenyl-5-nitro-1H-benzimidazol-6-yl)oxy]hexanoate
isopropyl 6-[[5-[[(4-bromophenyl)sulphonyl]amino]-1,2-diphenyl-1H-benzimidazol-6-yl]oxy]hexanoate
methyl 6-[[5-[[(4-chlorophenyl)sulphonyl]amino]-1,2-diphenyl-1H-benzimidazol-6-yl]oxy]hexanoate
isopropyl 6-[[5-[[(4-chlorophenyl)sulphonyl]-amino]-1,2-diphenyl-1H-benzimidazol-6-yl]oxy]-hexanoate
isopropyl 6-[[1,2-diphenyl-5-[[(3-methylphenyl)-sulphonyl]amino]-1H-benzimidazol-6-yl]oxy]-hexanoate
isopropyl 6-[[1,2-diphenyl-5-[[(4-methylphenyl)-sulphonyl]amino]-1H-benzimidazol-6-yl]oxy]-hexanoate
isopropyl 6-[[1,2-diphenyl-5-[[(4-methoxyphenyl)-sulphonyl]amino]-1H-benzimidazol-6-yl]oxy]-hexanoate
isopropyl 6-[[1,2-diphenyl-5-[[[(4-trifluoromethyl)phenyl]sulphonyl]amino]-1H-benzimidazol-6-yl]oxy]hexanoate
isopropyl 6-[[5-[[[4-(acetylamino)phenyl]-sulphonyl]amino]-1,2-diphenyl-1H-benzimidazol-6-yl]oxy]hexanoate
isopropyl 6-[[5-[[bis(3-chlorophenyl)sulphonyl]-amino]-1,2-diphenyl-1H-benzimidazol-6-yl]oxy]-hexanoate
isopropyl 6-[[1,2-diphenyl-5-[(propylsulphonyl)-amino]-1H-benzimidazol-6-yl]oxy]hexanoate
isopropyl 6-[[5-[(benzylsulphonyl)amino]-1,2-diphenyl-1*H*-benzimidazol-6-yl]oxy]hexanoate
methyl 2-[2-[(1,2-diphenyl-1*H*-benzimidazol-6-yl)-oxy]ethoxy]acetate
methyl 3-[2-[(1,2-diphenyl-1*H*-benzimidazol-6-yl)-oxy]ethoxy]propanoate
ethyl 6-[ [1-(3-nitrophenyl)-2-phenyl-1*H*-benzimidazol-6-yl]oxy]hexanoate
methyl 6-[[4-acetyl-1-(4-methylphenyl)-2-phenyl-1*H*-benzimidazol-6-yl]oxy]hexanoate
methyl 6-[ [1-(4-methylphenyl)-2-phenyl-1*H*-benzimidazol-5-yl]oxy]hexanoate
methyl 6-[[2-phenyl-1-[4-(thiomethyl)phenyl]-1*H-*benzimidazol-5-yl]oxy]hexanoate
methyl 6-[ [2-phenyl-1-[(4-(thiomethyl)phenyl]-1*H-*benzimidazol-6-yl]oxy]hexanoate
methyl 6-[[2-phenyl-1-(3-thienyl)-1*H*-benzimidazol-5-yl]oxy]hexanoate
methyl 6-[[2-phenyl-1-(3-thienyl)-1*H*-benzimidazol-6-yl]oxy]hexanoate
methyl 4-[(1,2-diphenyl-1*H*-benzimidazol-6-yl)oxy]-butanoate
*N-*(phenylmethoxy)-6-[[2-phenyl-1-(3,4,5-trimethoxyphenyl)-1*H*-benzimidazol-6-yl]oxy]hexanamide
*N*,*N-*dimethyl-6-[(1,2-diphenyl-1*H*-benzimidazol-6-yl)oxy]hexanamide
*N-*isopropyl-6-[(1,2-diphenyl-1*H*-benzimidazol-6-yl)oxy]hexanamide
6-[(1,2-diphenyl-1*H*-benzimidazol-6-yl)oxy]-1-pyrrolidin-1-ylhexan-l-one
methyl 5-[[5-[[(4-chlorophenyl)sulphonyl]amino]-1,2-diphenyl-1*H*-benzimidazol-6-yl]oxy]pentanoate
methyl 6-[[5-[[(4-chlorophenyl)sulphonyl]amino]-1-(4-methylphenyl)-2-phenyl-1*H*-benzimidazol-6-yl]oxy]hexanoate
methyl 6-[[5-[[(4-chlorophenyl)sulphonyl]amino]-1-(4-methoxyphenyl)-2-phenyl-1*H*-benzimidazol-6-yl]oxy]hexanoate
methyl 6-[[4-(acetyloxy)-1-(4-methylphenyl)-2-phenyl-1*H*-benzimidazol-6-yl]oxy]hexanoate
methyl 6-[[4-hydroxy-1-(4-methylphenyl)-2-phenyl-1*H*-benzimidazol-6-yl]oxy]hexanoate
6-[[4-hydroxy-1-(4-methylphenyl)-2-phenyl-1*H-*benzimidazol-6-yl]oxy]hexanoic acid
methyl 6-[[7-methyl-1-(4-methylphenyl)-2-phenyl-1*H*-benzimidazol-6-yl]oxy]hexanoate
methyl 6-[[2-phenyl-1-(3-pyridyl)-1*H*-benzimidazol-5-yl]oxy]hexanoate
methyl 6-[[2-phenyl-1-(3-pyridyl)-1*H*-benzimidazol-6-yl]oxy]hexanoate
methyl 6-[[2-phenyl-1-(4-pyridyl)-1*H*-benzimidazol-6-yl]oxy]hexanoate
methyl 6-[[2-(4-fluorophenyl)-1-phenyl-1*H*-benzimidazol-6-yl]oxy]hexanoate
methyl 6-[[2-(4-methoxyphenyl)-1-phenyl-1*H*-benzimidazol-6-yl]oxy]hexanoate
methyl 6-[[2-(4-bromophenyl)-1-phenyl-1*H*-benzimidazol-6-yl]oxy]hexanoate
methyl 6-[[2-[4-(trifluoromethyl)phenyl]-1-phenyl-1*H*-benzimidazol-6-yl]oxy]hexanoate
methyl 6-[[1-phenyl-2-(benzothien-2-yl)-1*H*-benzimidazol-6-yl]oxy]hexanoate
6-[[1-phenyl-2-(benzothien-2-yl)-1*H*-benzimidazol-6-yl]oxy]hexanoic acid
isopropyl 6-[[5-hydroxy-1-(4-methylphenyl)-2-phenyl-1*H*-benzimidazol-6-yl]oxy]hexanoate
6-[[5-hydroxy-1-(4-methylphenyl)-2-phenyl-1*H-*benzimidazol-6-yl]oxy]hexanoic acid
isopropyl 6-[[5-methoxy-1-(4-methylphenyl)-2-phenyl-1*H*-benzimidazol-6-yl]oxy]hexanoate
methyl 6-[[5-hydroxy-1-(4-methylphenyl)-2-phenyl-1*H*-benzimidazol-6-yl]oxy]hexanoate
methyl 6-[[5-methoxy-1-(4-methylphenyl)-2-phenyl-1*H*-benzimidazol-6-yl]oxy]hexanoate
methyl 6-[[5-[[(4-chlorophenyl)sulphonyl]amino]-1-(3,4-dimethylphenyl)-2-phenyl-1*H*-benzimidazol-6-yl]oxy]hexanoate
methyl 6-[[5-[[(4-chlorophenyl)sulphonyl]amino]-2-(4-fluorophenyl)-1-(4-methoxyphenyl)-1*H-*benzimidazol-6-yl]oxy]hexanoate
methyl 6-[[5-[[(4-chlorophenyl)sulphonyl]amino]-1-(4-methoxyphenyl)-2-(4-methoxyphenyl)-1*H-*benzimidazol-6-yl]oxy]hexanoate
methyl 4-[[5-[[(4-chlorophenyl)sulphonyl]amino]-1-(4-methoxyphenyl)-2-phenyl-1*H*-benzimidazol-6-yl]oxy]butanoate
methyl 5-[[5-[[(4-chlorophenyl)sulphonyl]amino]-1-(4-methoxyphenyl)-2-phenyl-1*H*-benzimidazol-6-yl]-oxy]pentanoate
methyl 5-[[5-[[(4-chlorophenyl)sulphonyl]amino]-1, 2-diphenyl-1*H*-benzimidazol-6-yl]oxy]pentanoate
methyl 6-[[5-[[(4-(trifluoromethyl)phenyl)-sulphonyl]amino]-1-(4-methoxyphenyl)-2-phenyl-1*H-*benzimidazol-6-yl]oxy]hexanoate
methyl 6-[[5-[[(4-chlorophenyl)sulphonyl]-methylamino]-1-(4-methoxyphenyl)-2-phenyl-1*H-*benzimidazol-6-yl]oxy]hexanoate
methyl 6-[[1-(indan-5-yl)-2-phenyl-1*H*-benzimidazol-6-yl]oxy]hexanoate
6-[[1-(indan-5-yl)-2-phenyl-1*H*-benzimidazol-6-yl]oxy]hexanoic acid
methyl 6-[[1-(3-fluorophenyl)-2-phenyl-1*H*-benzimidazol-6-yl]oxy]hexanoate
methyl 6-[[2-(4-nitrophenyl)-1-phenyl-1*H*-benzimidazol-6-yl]oxy]hexanoate
methyl 6-[[1-phenyl-2-(3-pyridinyl)-1*H*-benzimidazol-6-yl]oxy]hexanoate
*N-*(cyclopropylmethoxy)-6-[(1,2-diphenyl-1*H-*benzimidazol-6-yl)oxy]hexanamide
*N-*isobutoxy-6-[(1,2-diphenyl-1*H*-benzimidazol-6-yl)oxy]hexanamide
*N-*(cyclopropylmethoxy)-6-[(2-phenyl-1-(3,4,5-trimethoxyphenyl)-1*H*-benzimidazol-6-yl)oxy]hexanamide
*N-*isobutoxy-6-[(2-phenyl-1-(3,4,5-trimethoxyphenyl)-1*H*-benzimidazol-6-yl)oxy]hexanamide *N-*(2-methoxyethyl)-6-[(1,2-diphenyl-1*H-*benzimidazol-6-yl)oxy]hexanamide
*N-*(3-methoxypropyl)-6-[(1,2-diphenyl-1*H-*benzimidazol-6-yl)oxy]hexanamide
*N-*isobutyl-6-[(1,2-diphenyl-1*H*-benzimidazol-6-yl)oxy]hexanamide
6-[(1,2-diphenyl-1*H*-benzimidazol-6-yl)oxy]-1-morpholin-1-ylhexan-l-one
*N*,*N-*di(2-methoxyethyl)-6-[(1,2-diphenyl-1*H-*benzimidazol-6-yl)oxy]hexanamide
*N-*isopentyl-6-[(1,2-diphenyl-1*H*-benzimidazol-6-yl)oxy]hexanamide
*N-*(pyridin-2-yl)-6-[(1,2-diphenyl-1*H*-benzimidazol-6-yl)oxy]hexanamide
*N-*(pyridin-3-yl)-6-[(1,2-diphenyl-1*H*-benzimidazol-6-yl)oxy]hexanamide
*N-*isopropyl-6-[[1-(3,4-dimethylphenyl)-2-phenyl-1*H*-benzimidazol-6-yl]oxy]hexanamide
*N*,*N-*dimethyl-6-[[1-(3,4-dimethylphenyl)-2-phenyl-1*H*-benzimidazol-6-yl]oxy]hexanamide
*N*,*N-*diethyl-6-[[1-(3,4-dimethylphenyl)-2-phenyl-1*H*-benzimidazol-6-yl]oxy]hexanamide
*N-*isobutyl-6-[[1-(3,4-dimethylphenyl)-2-phenyl-1*H-*benzimidazol-6-yl]oxy]hexanamide
*N-*cyclopropyl-6-[[1-(3,4-dimethylphenyl)-2-phenyl-1*H*-benzimidazol-6-yl]oxy]hexanamide
*N-*cyclobutyl-6-[[1-(3,4-dimethylphenyl)-2-phenyl-1*H*-benzimidazol-6-yl]oxy]hexanamide
*N-tert*-butyl-6-[[1-(3,4-dimethylphenyl)-2-phenyl-1*H*-benzimidazol-6-yl]oxy]hexanamide
(*R*)-6-[ [1-(3,4-dimethylphenyl)-2-phenyl-1*H-*benzimidazol-6-yl]oxy]-1-(2-methoxymethyl)-pyrrolidin-1-ylhexan-l-one
*N-*(3-imidazol-1-ylpropyl)-6-[[1-(3,4-dimethylphenyl)-2-phenyl-1*H*-benzimidazol-6-yl]oxy]hexanamide
*N-*(2-pyridin-2-ylethyl)-6-[[1-(3,4-dimethylphenyl)-2-phenyl-1*H*-benzimidazol-6-yl]oxy]hexanamide
*N-*(3-methoxypropyl)-6-[[1-(indan-5-yl)-2-phenyl-1*H*-benzimidazol-6-yl]oxy]heptanamide
methyl 6-[[1-(4-methylphenyl)-2-(3-pyridyl)-1*H-*benzimidazol-6-yl]oxy]hexanoate
methyl 6-[[1-(4-methylphenyl)-2-(4-pyridyl)-1*H-*benzimidazol-6-yl]oxy]hexanoate
methyl 6-[[1-(4-methylphenyl)-2-(2-thienyl)-1*H-*benzimidazol-6-yl]oxy]hexanoate
methyl 6-[[1-(4-methylphenyl)-2-(3-thienyl)-1*H-*benzimidazol-6-yl]oxy]hexanoate
methyl 6-[[2-(3-indolyl)-1-(4-methylphenyl)-1*H-*benzimidazol-6-yl]oxy]hexanoate
methyl 6-[[1-(4-methylphenyl)-2-(2-furyl)-1*H-*benzimidazol-6-yl]oxy]hexanoate
methyl 6-[[1-(4-methylphenyl)-2-(3-furyl)-1*H-*benzimidazol-6-yl]oxy]hexanoate
methyl 6-[[1-(4-methylphenyl)-2-(5-methyl-2-thienyl)-1*H*-benzimidazol-6-yl]oxy]hexanoate
methyl 6-[[1-(4-methylphenyl)-2-(3-methyl-2-thienyl)-1*H*-benzimidazol-6-yl]oxy]hexanoate.

12. Compound according to one of Claims 1-11 for use in healing processes, in prophylaxis or in diagnosis.

13. Compound according to one of Claims 1-11 for use in the treatment or prevention of illnesses associated with a microglia activation, selected from the group consisting of AIDS dementia, amyotrophic lateral sclerosis, Creuzfeld-Jacob disease, Down's syndrome, diffuse Lewy body disease, Huntington's chorea, leukoencephalopathy, multiple sclerosis, neuronal dysfunction and neuronal degeneration, Parkinson's disease, Pick's disease, Alzheimer's disease, stroke, temporal lobe epilepsy and tumours.

14. Use of a compound according to one of Claims 1-11 for producing a drug for the treatment or prevention of illnesses associated with a microglia activation, selected from the group consisting of AIDS dementia, amyotrophic lateral sclerosis, Creutzfeldt-Jacob disease, Down's syndrome, diffuse Lewy body disease, Huntington's chorea, leukoencephalopathy, multiple sclerosis, neuronal dysfunction and neuronal degeneration, Parkinson's disease, Pick's disease, Alzheimer's disease, stroke, temporal lobe epilepsy and tumours.

15. Pharmaceutical composition, **characterized in that** it comprises one or more compounds according to one of Claims 1-11 and one or more excipients.

16. Benzimidazole of the general formula II 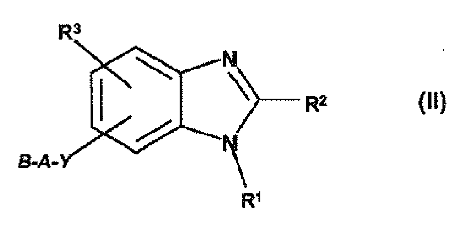 in which
***R¹*** is a mono- or bicyclic C₆₋₁₂-aryl group or a mono- or bicyclic 5-10-membered heteroaryl group with 1-4 heteroatoms selected from the group consisting of N, S or 0, where the specified aryl or heteroaryl group can be substituted with up to three of the following substituents independently of one another: F, Cl, Br, I, C(NH)NH₂, C(NH) NHR⁴, C(NH)NR⁴R^{4'}, C(NR⁴) NH₂, C(NR⁴)NHR^{4'}, C(NR⁴)NR⁴R^{4'}, XOH, XOR⁴, XOCOR⁴, XOCONHR⁴, XOCOOR⁴, XCOR⁴, XC (NOH) R⁴, XC(NOR⁴)R^{4'}, XC(NO(COR⁴))R^{4'} , XCN, XCOOH, XCOOR⁴, XCONH₂, XCONR⁴R^{4'} XCONHR⁴, XCONHOH, XCONHOR⁴, XCOSR⁴, XSR⁴, XSOR⁴, XSO₂R⁴ SO₂NH₂, SO₂NHR⁴, SO₂NR⁴R^{4'}, NO₂, XNH₂, XNHR⁴, XNR⁴R^{4'}, XNHSO₂R⁴, XN(SO₂R⁴) (SO₂R^{4'}),XNR⁴SO₂R^{4'} , XNHCOR⁴, XNHCOOR⁴, XNHCONHR⁴, tetrahydro-2,5-dioxopyrrol-1-yl, 2,5- dihydro-2,5-dioxopyrrol-1-yl, 2,7-dihydro-2,7- dioxoisoindol-1-yl, R⁴, where two substituents on ***R¹,*** if they are in the ortho position relative to one another, can be linked with one another such that they together form methanediylbisoxy, ethane- 1,2-diylbisoxy, propane-1,3-diyl, butane-1,4-diyl,
***R²*** is a mono- or bicyclic C₆₋₁₀-aryl group or a mono- or bicyclic 5-10-membered heteroaryl group with 1-4 heteroatoms selected from the group consisting of N, S or 0, where the specified aryl or heteroaryl group can be substituted with up to three of the following substituents independently of one another: F, Cl, Br, I, C(NH)NH_{2,} C(NH)NHR C(NH)NR⁴R^{4'}, C(NR⁴)NH₂, C(NR⁴)NHR^{4'}, C(NR⁴)NR⁴R^{4'}, XOH, XOR⁴, XOCOR⁴, XOCONHR⁴, XOCOOR⁴, XCOR⁴, XC(NOH)R⁴, XC(NOR⁴)R^{4'}, XC(NO(COR⁴))R^{4'}, XCN, XCOOH, XCOOR⁴, XCONH₂, XCONR⁴R^{4'}, XCONHR⁴, XCONHOH, XCONHOR⁴, XCO_{SR}⁴ XSR⁴, XSOR⁴, XSO₂R⁴, SO₂NH₂, SO₂NHR⁴, SO₂NR⁴R^{4'}, NO₂, XNH₂, XNHR⁴, XNR⁴R^{4'} , XNHSO₂R⁴, XN (SO₂R⁴) (SO₂R^{4'}), XNR⁴SO₂R^{4'}, XNHCOR⁴, XNHCOOR⁴, XNHCONHR⁴, tetrahydro-2,5-dioxopyrrol-1-yl, 2,5-dihydro-2,5- dioxopyrrol-1-yl, 2,7-dihydro-2,7-dioxoisoindol-1- yl, R⁴, where two substituents on ***R²***, if they are in the ortho position relative to one another, can be linked with one another such that they together form methanediylbisoxy, ethane-1,2-diylbisoxy, propane-1,3-diyl, butane-1,4-diyl,
***R³*** is one or two substituents which can be independently of one another:
hydrogen, F, Cl, Br, I, XOH, XOR⁴, XOCOR⁴, XOCONHR⁴, XOCOOR⁴, XCOR⁴, XC(NOH)R⁴, XC(NOR⁴) R^{4'}, XC(NO(COR⁴))R^{4'}, XCN, XCOOH, XCOOR⁴, XCONH₂, XCONHR⁴, XCONR⁴R^{4'}, XCONHOH, XCONHOR⁴, XCOSR⁴, XSR⁴, XSOR⁴, XSO₂R⁴, SO₂NH₂, SO₂NHR⁴, SO₂NR⁴R^{4'}, NO₂, XNH₂, XNHR⁴, XNR⁴R^{4'}, XNHSO₂R⁴, XNR⁴SO_{2R}^{4'}, XN(SO₂R⁴) (SO₂R^{4'}), XNHCOR⁴, XNHCOOR⁴, XNHCONHR⁴, tetrahydro-2,5-dioxopyrrol-1-yl, 2,5-dihydro-2,5- dioxopyrrol-1-yl, 2,7-dihydro-2,7-dioxoisoindol-1-yl, R⁴, where two substituents ***R³***, if they are in the ortho position relative to one another, can be linked with one another such that they together form methanediylbisoxy, ethane-1,2-diylbisoxy, propane-1,3-diyl, butane-1,4-diyl,
***R⁴*** and ***R***^{***4***'}, independently of one another, are C₁₋₄ perfluoroalkyl, C₁₋₆ alkyl, C₂₋₆ alkenyl, C₂-₆ alkynyl, C₃₋₇ cycloalkyl, (C₁₋₃ alkyl-C₃₋₇ cycloalkyl) , C₁₋₃ alkyl-C₆₋₁₀-aryl, C₁₋₃ alkyl-5-10- membered heteroaryl with 1-4 N, S or 0 atoms, C₆₋₁₀- aryl or 5-10-membered heteroaryl with 1-4 N, S or 0 atoms, where the C₆₋₁₀-aryl and heteroaryl groups can be substituted with one or two substituents from the group consisting of F, Cl, Br, CH₃, C₂H₅, NO₂, OCH₃, OC₂H₅, CF₃, C₂F₅ or else can carry a fused methanediylbisoxy, ethane-1,2-diylbisoxy group, where, in a 5-membered cycloalkyl ring, one ring member can be an N or an 0 and in a 6- or 7-membered cycloalkyl ring, one or two ring members can be N and/or O, where ring nitrogens can be optionally substituted with C₁₋₃ alkyl or C₁- alkanoyl,
***R⁵*** and ***R^{5'}***, independently of one another, are hydrogen, C₁₋₆ alkyl, C₂₋₆ alkenyl, C₂₋₆ alkynyl, where one carbon atom can be exchanged for O, S, SO, SO₂, NH, N C₁₋₃ alkyl or N C₁₋₃ alkanoyl, C₃₋₇ cycloalkyl-C₀₋₃ alkyl, where, in a 5-membered cycloalkyl ring one ring, member can be an N or an 0 and in a 6- or 7-membered cycloalkyl ring, one or two ring members can be N and/or O, where ring nitrogens can be optionally substituted with C₁-₃ alkyl or C₁₋₃ alkanoyl, C₆₋₁₀-aryl or 5-10-membered heteroaryl with 1-4 heteroatoms from N, S and O, where the specified alkyl, alkenyl and alkynyl chains can be substituted with one of the aforementioned cycloalkyls, aryls or heteroaryls, where all of the aforementioned alkyl and cycloalkyl radicals can be substituted with up to two substituents from CF₃, C₂F₅, OH, 0 C₁₋₃ alkyl, NH₂, NH C₁₋₃ alkyl, NH C₁₋₃ alkanoyl, N(C₁₋₃ alkyl)₂, N(C₁₋₃ alkyl)(C₁₋₃ alkanoyl), COOH, CONH₂, COO C₁₋₃ alkyl and all of the aforementioned aryl and heteroaryl groups can be substituted with one or two substituents from the group consisting of F, Cl, Br, CH₃, C₂H₅, NO₂, OCH₃, OC₂H₅, CF₃, C₂F₅ or else can carry a fused methanediylbisoxy, ethane-1,2- diylbisoxy group, or R⁵ and R^{5'}, together with the nitrogen atom, form a 5-7-membered heterocycle which can contain a further oxygen, nitrogen or sulphur atom and can be substituted with C₁₋₄- alkyl, C₁₋₄-alkoxy-C₀₋₂-alkyl, C₁₋₄-alkoxycarbonyl, aminocarbonyl or phenyl,
**A** is C₁₋₁₀ alkanediyl, C₂₋₁₀ alkenediyl, C₂₋₁₀ alkynediyl, (C₀₋₅ alkanediyl-C₃₋₇ cycloalkanediyl-C₀- ₅ alkanediyl), (C₀₋₅ alkanediylarylene-C₀₋₅ alkanediyl), (C₀₋₅ alkanediyl-heteroarylene-C₀₋₅ alkanediyl), where the aryl and heteroaryl groups can be substituted with one or two substituents from F, Cl, Br, CH₃, C₂H₅, NO₂, OCH₃, OC₂H₅, CF₃, C₂F₅, where, in a 5-membered cycloalkyl ring, one ring member can be an N or an 0 and in a 6- or 7-membered cycloalkyl ring, one or two ring members can be N and/or O, where ring nitrogens can be optionally substituted with C₁₋₃-alkyl or C₁₋₃-alkanoyl, where, in the specified aliphatic chains, one or two carbon atoms can be exchanged for 0, NH, NR⁴, NCOR⁴, NSO₂R, and where alkyl or cycloalkyl groups can be substituted with up to two substituents from F, OH, OR⁴, OCOR⁴, =O, NH₂, NR⁴R^{4'}, NHCOR⁴, NHCOOR⁴, NHCONHR⁴, NHSO₂R⁴ SH, SR⁴,
***B*** is hydrogen, OH, OCOR⁵, OCONHR⁵, OCOOR⁵, COR⁵, C (NOH) R⁵, C(NOR⁵) R^{5'}, C(NO(COR⁵) ) R^{5'}, COOH, COOR⁵, CONH₂, CONHNH₂, CONHR⁵, CONR⁵R^{5'}, CONHOH, CONHOR⁵, SO₃H, SO₂NH₂, SO₂NHR⁵, SO₂NR⁵R^{5'}, PO₃H, PO(OH)(O⁵), PO(OR⁵) (OR⁵), PO (OH) (NHR⁵), PO (NHR⁵)(NHR^{5'}), tetrazolyl, in each case bonded to a carbon atom of group ***A***, or the overall group ***Y-A-B*** is N (SO₂R⁴) (SO₂R^{4'}) or NHSO₂R⁴,
***X*** is a bond, CH₂, (CH₂)₂, CH(CH₃), (CH₂)₃, CH(CH₂CH₃), CH(CH₃)CH₂, CH₂CH(CH₃),
***Y*** is a bond, 0, S, SO, SO₂, NH, NR⁴, NCOR⁴, NSO₂R⁴, for use in the treatment or prevention of illnesses associated with a microglia activation, selected from the group consisting of AIDS dementia, amyotrophic lateral sclerosis, Creutzfeldt-Jacob disease, Down's syndrome, diffuse Lewy body disease, Huntington's chorea, leukoencephalopathy, multiple sclerosis, neuronal dysfunction and neuronal degeneration, Parkinson's disease, Pick's disease, Alzheimer's disease, stroke, temporal lobe epilepsy and tumours.

17. Use of a benzimidazole of the general formula II 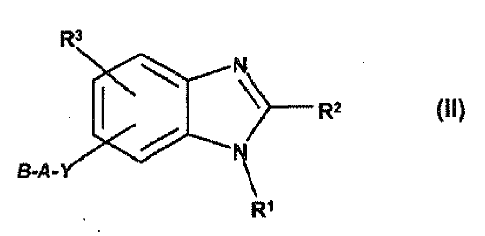 in which
***R¹*** is a mono- or bicyclic C₆₋₁₂-aryl group or a mono- or bicyclic 5-10-membered heteroaryl group with 1-4 heteroatoms selected from the group consisting of N, S or O, where the specified aryl or heteroaryl group can be substituted with up to three of the following substituents independently of one another:
F, Cl, Br, I, C(NH)NH₂, C(NH) NHR⁴ C(NH)NR⁴R^{4'}, C(NR⁴)NH₂, C(NR⁴)NHR^{4'}, C(NR⁴)NR⁴R^{4'}, XOH, XOR⁴, XOCOR⁴, XOCONHR⁴, XOCOOR⁴, XCOR⁴, XC(NOH)R⁴ XC(NOR⁴)R^{4'}, XC(NO(COR⁴))R^{4'}, XCN, XCOOH, XCOOR⁴, XCONH₂, XCONR⁴R^{4'}, XCONHR⁴, XCONHOH, XCONHOR⁴, XCOSR⁴, XSR⁴, XSOR⁴, XSO₂R⁴ SO₂NH₂, SO₂NHR⁴, SO₂NR⁴R^{4'}, NO₂, XNH₂, XNHR⁴, XNR⁴R^{4'}, XNHSO₂R⁴, XN(SO₂R⁴) (SO₂R^{4'}) XNR⁴SO₂R^{4'}, XNHCOR⁴, XNHCOOR⁴, XNHCONHR⁴, tetrahydro-2,5-dioxopyrrol-1-yl, 2,5- dihydro-2,5-dioxopyrrol-1-yl, 2,7-dihydro-2,7- dioxoisoindol-1-yl, R⁴, where two substituents on ***R¹,*** if they are in the ortho position relative to one another, can be linked with one another such that they together form methanediylbisoxy, ethane- 1,2-diylbisoxy, propane-1,3-diyl, butane-1,4-diyl,
***R²*** is a mono- or bicyclic C₆₋₁₀-aryl group or a mono- or bicyclic 5-10-membered heteroaryl group with 1-4 heteroatoms selected from the group consisting of N, S or O, where the specified aryl or heteroaryl group can be substituted with up to three of the following substituents independently of one another: F, Cl, Br, I, C(NH)NH₂, C(NH)NHR⁴, C(NH)NR⁴R^{4'}, C(NR⁴)NH₂, C(NR⁴)NHR^{4'}, C(NR⁴)NR⁴R^{4'}, XOH, XOR⁴, XOCOR⁴, XOCONHR⁴, XOCOOR⁴, XCOR⁴, XC(NOH)R⁴ XC(NOR⁴)R^{4'}, XC(NO(COR⁴))R^{4'}, XCN, XCOOH, XCOOR⁴, XCONH₂, XCONR⁴R^{4'}, XCONHR⁴, XCONHOH, XCONHOR⁴, XCOSR⁴ XSR⁴, XSOR⁴, XSO₂R , SO₂NH₂, SO₂NHR⁴, SO₂NR⁴R^{4'}, NO₂, XNH₂, XNHR⁴, XNR⁴R^{4'} , XNHSO₂R⁴, XN (SO₂R⁴) (SO₂R^{4'}), XNR⁴SO_{2R}^{4'}, XNHCOR⁴, XNHCOOR⁴, XNHCONHR⁴, tetrahydro-2,5-dioxopyrrol-1-yl, 2,5-dihydro-2,5- dioxopyrrol-1-yl, 2,7-dihydro-2,7-dioxoisoindol-1- yl, R⁴, where two substituents on ***R²***, if they are in the ortho position relative to one another, can be linked with one another such that they together form methanediylbisoxy, ethane-1,2-diylbisoxy, propane-1,3-diyl, butane-1,4-diyl,
***R³*** is one or two substituents which can be independently of one another: hydrogen, F, Cl, Br, I, XOH, XOR ⁴ XOCOR⁴, XOCONHR⁴, XOCOOR⁴, XCOR⁴, XC(NOH)R⁴ XC(NOR)⁴R^{4'}, XC(NO(COR⁴))R^{4'}, XCN, XCOOH, XCOOR⁴, XCONH₂, XCONHR⁴, XCONR⁴R^{4'}, XCONHOH, XCONHOR⁴, XCOSR⁴, XSR⁴, XSOR⁴, XSO₂R⁴, SO₂NH₂, SO₂NHR⁴, SO₂NR⁴R^{4'}, NO₂, XNH₂, XNHR⁴, XNR⁴R^{4'}, XNHSO₂R⁴ XNR⁴SO₂R^{4'}, XN(SO₂R⁴) (SO₂R^{4'}), XNHCOR⁴, XNHCOOR⁴, XNHCONHR⁴, tetrahydro-2,5-dioxopyrrol-1-yl, 2,5-dihydro-2,5- dioxopyrrol-1-yl, 2,7-dihydro-2,7-dioxoisoindol-1- yl, R⁴, where two substituents on ***R³***, if they are in the ortho position relative to one another, can be linked with one another such that they together form methanediylbisoxy, ethane-1,2-diylbisoxy, propane-1,3-diyl, butane-1,4-diyl,
***R⁴*** and ***R^{4'},***independently of one another, are C₁₋₄ perfluoroalkyl, C₁₋₆ alkyl, C₂₋₆ alkenyl, C₂₋₆ alkynyl, C₃₋₇ cycloalkyl, (C₁₋₃ alkyl-C₃₋₇ cycloalkyl), C₁₋₃ alkyl-C₆₋₁₀-aryl, C₁₋₃ alkyl 5-10- membered heteroaryl with 1-4 N, S or O atoms, C₆₋₁₀- aryl or 5-10-membered heteroaryl with 1-4 N, S or 0 atoms, where the C₆₋₁₀-aryl and heteroaryl groups can be substituted with one or two substituents from the group consisting of F, Cl, Br, CH₃, C₂H₅, NO₂, OCH₃, OC₂H₅, CF₃, C₂F₅ or else can carry a fused methanediylbisoxy, ethane-1,2-diylbisoxy group, where, in a 5-membered cycloalkyl ring, one ring member can be an N or an O and in a 6- or 7-membered cycloalkyl ring, one or two ring members can be N and/or 0, where ring nitrogens can be optionally substituted with C₁₋₃ alkyl or C₁₋ alkanoyl,
***R⁵*** and ***R⁵,*** independently of one another, are hydrogen, C₁₋₆ alkyl, C₂₋₆ alkenyl, C₂₋₆ alkynyl, where one carbon atom can be exchanged for 0, S, SO, SO₂, NH, N C₁₋₃ alkyl or N C₁₋₃ alkanoyl, C₃₋₇ cycloalkyl-C₀₋₃ alkyl, where, in a 5-membered cycloalkyl ring, one ring member can be an N or an 0 and in a 6- or 7-membered cycloalkyl ring, one or two ring members can be N and/or O, where ring nitrogens can be optionally substituted with C₁₋₃ alkyl or C₁₋₃ alkanoyl, C₆₋₁₀-aryl or 5-10-membered heteroaryl with 1-4 heteroatoms from N, S and 0, where the specified alkyl, alkenyl and alkynyl chains can be substituted with one of the aforementioned cycloalkyls, aryls or heteroaryls, where all of the aforementioned alkyl and cycloalkyl radicals can be substituted with up to two substituents from CF₃, C₂F₅, OH, 0 C₁₋₃ alkyl, NH₂, NH C₁₋₃ alkyl, NH C₁₋₃ alkanoyl, N(C₁₋₃ alkyl)₂, N(C₁₋₃ alkyl)(C₁₋₃ alkanoyl), COOH, CONH₂, COO C₁₋₃ alkyl and all of the aforementioned aryl and heteroaryl groups can be substituted with one or two substituents from the group consisting of F, Cl, Br, CH₃, C₂H₅, NO₂, OCH₃, OC₂H₅, CF₃, C₂F₅ or else can carry a fused methanediylbisoxy, ethane-1,2- diylbisoxy group, or R⁵ and R^{5'}, together with the nitrogen atom, form a 5-7-membered heterocycle which can contain a further oxygen, nitrogen or sulphur atom and can be substituted with C₁₋₄- alkyl, C₁₋₄-alkoxy-C₀₋₂-alkyl, C₁₋₄-alkoxycarbonyl, aminocarbonyl or phenyl,
***A*** is C₁₋₁₀ alkanediyl, C₂₋₁₀ alkenediyl, C₂₋₁₀ alkynediyl, (C₀₋₅ alkanediyl-C₃₋₇ cycloalkanediyl-C₀₋ ₅ alkanediyl), (C₀₋₅ alkanediylarylene-C₀₋₅ alkanediyl), (C₀₋₅ alkanediyl-heteroarylene-C₀₋₅ alkanediyl), where the aryl and heteroaryl groups can be substituted with one or two substituents from F, Cl, Br, CH₃, C₂H₅, NO₂, OCH₃, OC₂H₅, CF₃, C₂F₅, where, in a 5-membered cycloalkyl ring, one ring member can be an N or an 0 and in a 6- or 7-membered cycloalkyl ring, one or two ring members can be N and/or 0, where ring nitrogens can be optionally substituted with C₁₋₃-alkyl or C₁₋₃-alkanoyl, where, in the specified aliphatic chains, one or two carbon atoms can be exchanged for O, NH, NR⁴, NCOR⁴, NSO₂R⁴, and where alkyl or cycloalkyl groups can be substituted with up to two substituents from F, OH, OR⁴, OCOR⁴, -O, NH₂, NR⁴R^{4'}, NHCOR⁴, NHCOOR⁴, NHCONHR⁴, NHSO₂R⁴, SH, SR⁴,
***B*** is hydrogen, OH, OCOR⁵, OCONHR⁵, OCOOR⁵, COR⁵, C(NOH)R⁵ C(NOR⁵) R^{5'}, C(NO(COR⁵)) R^{5'}, COOH, COOR⁵, CONH₂, CONHNH₂, CONHR⁵, CONR⁵R^{5'}, CONHOH, CONHOR⁵, SO₃H, SO₂NH₂, SO₂NHR⁵, SO₂NR⁵R^{5'}, PO₃H, PO (OH) (OR⁵), PO (OR⁵) (OR^{5'}) , PO (OH) (NHR⁵) , PO (NHR⁵) (NHR^{5'}) , tetrazolyl, in each case bonded to a carbon atom of group ***A***, or the overall group ***Y-A-B*** is N(SO₂R⁴) (SO₂R^{4'}) or NHSO₂R⁴
***X*** is a bond, CH₂, (CH₂)₂, CH(CH₃), (CH₂)₃, CH(CH₂CH₃), CH(CH₃)CH₂, CH₂CH(CH₃),
***Y*** is a bond, O, S, SO, SO₂, NH, NR⁴, NCOR⁴, NSO₂R⁴,
for producing a drug for treating or preventing illnesses associated with a microglia activation, selected from the group consisting of AIDS dementia, amyotrophic lateral sclerosis, Creutzfeldt-Jacob disease, Down's syndrome, diffuse Lewy body disease, Huntington's chorea, leukoencephalopathy, multiple sclerosis, neuronal dysfunction and neuronal degeneration, Parkinson's disease, Pick's disease, Alzheimer's disease, stroke, temporal lobe epilepsy and tumours.

18. Use according to Claim 16 or 17, where, in the general formula II,
***R¹*** is a mono- or bicyclic aryl group or a mono- or bicyclic 5-10-membered heteroaryl group with 1-2 heteroatoms selected from the group consisting of N, S or 0, where the specified aryl or heteroaryl group can be substituted with up to three of the following substituents independently of one another: F, Cl, Br, XOH, XOR⁴, XOCOR⁴, XOCONHR⁴, XOCOOR⁴, XCOR⁴, XCN, XCOOH, XCOOR⁴, XCONH₂, XCONR⁴R^{4'}, XCONHR⁴, XCONHOH, XCONHOR⁴, XCOSR⁴, XSR⁴, NO₂, XNHR⁴, XNR⁴R^{4'}, R⁴, where two substituents on ***R¹***, if they are in the ortho position relative to one another, can be linked with one another such that they together form methanediylbisoxy, ethane-1,2-diylbisoxy, propane-1,3-diyl, butane-1,4-diyl.

19. Use according to one of Claims 16-18, where, in the general formula II,
***R²*** is a mono- or bicyclic aryl group or a mono- or bicyclic 5-10-membered heteroaryl group with 1-2 heteroatoms selected from the group consisting of N, S or 0, where the specified aryl or heteroaryl group can be substituted with up to three of the following substituents independently of one another: F, Cl, Br, XOH, XOR⁴, XOCOR⁴, XOCONHR⁴, XOCOOR⁴, XCOR⁴, XC(NOH)R ⁴, XC(NOR⁴)R^{4'}, XC(NO(COR⁴))R^{4'}, XCN, XCOOH, XCOOR⁴, XCONH₂, XCONR⁴R^{4'}, XCONHR⁴, XCONHOH, XCONHOR⁴, XCOSR⁴, XSR⁴, XSOR⁴, XSO₂R⁴, SO₂NH₂, SO₂NHR⁴, SO₂NR⁴R^{4'}, NO₂, XNH₂, XNHR⁴, XNR⁴R^{4'}, XNHSO₂R⁴, XN(SO₂R⁴) (SO₂R^{4'}), XNR⁴SO₂R^{4'}, XNHCOR⁴, XNHCOOR⁴, XNHCONHR⁴, R⁴, where two substituents on ***R²***, if they are in the ortho position relative to one another, can be linked with one another such that they together form methanediylbisoxy, ethane-1,2-diylbisoxy, propane-1,3-diyl, butane-1,4-diyl.

20. Use according to one of Claims 16-19, where, in the general formula II,
***R³*** is one or two substituents which are independently of one another: hydrogen, F, Cl, Br, XOH, XOR⁴, XOCOR⁴, XOCONHR⁴, XOCOOR⁴, XCOR⁴, XC(NOH)R⁴, XC(NOR⁴)R^{4'}, XC(NO(COR⁴) R^{4'}, XCN, XSR⁴, XSOR⁴, XSO₂R⁴, SO₂NH₂, SO₂NHR⁴, SO₂NR⁴R^{4'}, NO₂, XNH₂, XNHR⁴, XNR⁴R^{4'}, XNHSO₂R⁴, XNR⁴SO₂R^{4'}, XN(SO₂R⁴) (SO₂R^{4'}), XNHCOR⁴, XNHCOOR⁴, XNHCONHR⁴ or R⁴, where two substituents ***R³***, if they are in the ortho position relative to one another, can be linked with one another such that they together form methanediylbisoxy, ethane- 1,2-diylbisoxy, propane-1,3-diyl, butane-1,4-diyl.

21. Use according to one of Claims 16-20, where, in the general formula II,
***R⁴*** and ***R^{4'}***, independently of one another, are CF₃, C₂F₅, C₁₋₄ alkyl, C₂₋₄ alkenyl, C₂₋₄ alkynyl, C₃₋₆ cycloalkyl, (C₁₋₃ alkyl-C₃₋₆ cycloalkyl), C₁₋₃ alkylaryl, C₁₋₃ alkylheteroaryl, monocyclic aryl or 5-6-membered heteroaryl with 1-2 N, S or O atoms, where the aryl and heteroaryl groups can be substituted with one or two substituents from the group consisting of F, Cl, Br, CH₃, C₂H₅, NO₂, OCH₃, OC₂H₅, CF₃, C₂F₅, or else can carry a fused methanediylbisoxy or ethane-1,2-diylbisoxy group, and furthermore, in a 5-membered cycloalkyl ring, one ring member can be an N or an O and in a 6-membered cycloalkyl ring, one or two ring members can be N and/or O, where ring nitrogens can be optionally substituted with C₁₋₃ alkyl or C₁₋₃ alkanoyl.

22. Use according to one of Claims 16-21, where, in the general formula II,
***R⁵*** and ***R^{5'}***, independently of one another, are C₁₋₆ alkyl, where one carbon atom can be exchanged for O, NH, N C₁₋₃ alkyl, N C₁₋₃ alkanoyl, C₃₋₇ cycloalkyl-C₀₋₃ alkyl, where, in a 5-membered cycloalkyl ring, one ring member can be an N or an O and in a 6- or 7-membered cycloalkyl ring, one or two ring members can be N and/or O, where ring nitrogens can be optionally substituted with C₁₋₃ alkyl or C₁₋₃ alkanoyl, where the specified C₁₋₆ alkyl moiety can be substituted with one of the aforementioned cycloalkyls or else a 5-6-membered heteroaromatic with 1-2 heteroatoms selected from the group consisting of N, S or O, where all of the aforementioned alkyl and cycloalkyl moieties can be substituted with up to two substituents from CF₃, OH, O C₁₋₃ alkyl, and the aforementioned heteroaryl groups can be substituted with one or two substituents from F, Cl, CF₃, CH₃, C₂H₅, OCH₃, OC₂H₅, or R⁵ and R^{5'}, together with the nitrogen atom, form a 5-7-membered heterocycle which can contain a further oxygen, nitrogen or sulphur atom and can be substituted with C₁₋₄-alkyl, C₁₋₄-alkoxy-C₀₋₂- alkyl, C₁₋₄-alkoxycarbonyl, aminocarbonyl or phenyl.

23. Use according to one of Claims 16-22, where, in the general formula II,
***A*** is C₁₋₁₀ alkanediyl, C₂₋₁₀ alkenediyl, C₂₋₁₀ alkynediyl, (C₀₋₅ alkanediyl-C₃₋₇ cycloalkanediyl- C₀₋₅ alkanediyl), or (C₀₋₅ alkanediyl-heteroarylene- C₀₋₅ alkanediyl), where any present heteroaryl group can be substituted with one or two substituents from F, Cl, Br, CH₃, C₂H₅, NO₂, OCH₃, OC₂H₅, CF₃, C₂F₅, and furthermore, in a 5-membered cycloalkyl ring, one ring member can be an N or an O and in a 6- or 7-membered cycloalkyl ring, one or two ring members can be N and/or O, where ring nitrogens can be optionally substituted with C₁₋₃ alkyl or C₁₋₃ alkanoyl, where, in an aliphatic chain, one or two carbon atoms can be exchanged for O, NH, N C₁₋₃ alkyl, N C₁₋₃ alkanoyl, NSO₂ C₁₋₃ alkyl, and where alkyl or cycloalkyl moieties can be substituted with up to two F atoms or one of the substituents from OH, O C₁₋₃ alkyl, O C₁₋₃ alkanoyl, =0, NH₂, NH C₁₋₃ alkyl, N (C₁₋₃ alkyl)₂, NH C₁₋₃ alkanoyl, N (C₁₋₃ alkyl) (C₁₋₃ alkanoyl) , NHCOO C₁₋₃ alkyl, NHCONH C₁₋₃ alkyl, NHSO₂ C₁₋₃ alkyl, SH, S C₁₋₃ alkyl.

24. Use according to one of Claims 16-23, where, in the general formula II,
***B*** is hydrogen, OH, OCOR⁵, OCONHR⁵, OCOOR⁵, COOH, COOR⁵, CONH₂, CONHR⁵_{,} CONR⁵R^{5'}, CONHOH, CONHOR⁵ or tetrazolyl, in each case bonded to a carbon atom of group ***A.***

25. Use according to one of Claims 16-24, where, in the general formula II,
***X*** is a bond or CH₂.

26. Use according to one of Claims 16-25, where, in the general formula II,
***Y*** is a bond, O, S, NH, NR⁴, NCOR⁴ or NSO₂R⁴.

## Revendications

1. Benzimidazoles de formule générale (I) 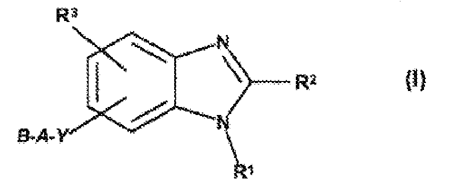 où
***R¹*** signifie un groupe C₆₋₁₂-aryle monocyclique ou bicyclique ou un groupe hétéroaryle de 5-10 chaînons, monocyclique ou bicyclique comprenant 1 à 4 hétéroatomes choisis dans le groupe constitué par N, S ou O, où le groupe aryle ou hétéroaryle mentionné peut être substitué, indépendamment l'un de l'autre, par jusqu'à trois des substituants suivants :
F, Cl, Br, I,
C(NH)NH₂, C(NH)NHR⁴, C(NH)NR⁴R^{4'}, C(NR⁴)NH₂, C(NR⁴)NHR^{4'}, C(NR⁴)NR⁴R^{4'},
XOH, XOR⁴, XOCOR⁴, XOCONHR⁴, XOCOOR⁴,
XCOR⁴, XC(NOH)R⁴, XC(NOR⁴)R^{4'}, XC(NO(COR⁴))R^{4'}
XCN, XCOOH, XCOOR⁴, XCONH₂, XCONR⁴R^{4'}, XCONHR⁴,
XCONHOH, XCONHOR⁴, XCOSR⁴
XSR⁴, XSOR⁴, XSO₂R⁴,
SO₂NH₂, SO₂NHR⁴, SO₂NR⁴R^{4'},
NO₂, XNH₂, XNHR⁴, XNR⁴R^{4'}, XNHSO₂R⁴,
XN(SO₂R⁴)SO₂R^{4'}, XNR⁴SO₂R^{4'}, XNHCOR⁴, XNHCOOR⁴,
XNHCONHR⁴, tétrahydro-2,5-dioxopyrrol-1-yle,
2,5-dihydro-2,5-dioxopyrrol-1-yle, 2,7- dihydro-2,7-dioxoisoindol-1-yle, R⁴,
où deux substituants sur R¹, lorsqu'ils sont en position ortho l'un par rapport à l'autre, peuvent être liés l'un à l'autre de manière telle qu'ils forment ensemble méthanediylbisoxy, éthane-1,2- diylbisoxy, propane-1,3-diyle, butane-1,4-diyle,
***R²*** signifie un groupe C₆₋₁₀-aryle monocyclique ou bicyclique ou un groupe hétéroaryle de 5-10 chaînons, monocyclique ou bicyclique comprenant 1 à 4 hétéroatomes choisis dans le groupe constitué par N, S ou O, où le groupe aryle ou hétéroaryle mentionné peut être substitué, indépendamment l'un de l'autre, par jusqu'à trois des substituants suivants :
F, Cl, Br, I,
XOH, XOR⁴, XOCOR⁴, XOCONHR⁴, XOCOOR⁴,
XCOR⁴, XC(NOH)R⁴, XC(NOR⁴)R^{4'}, XC(NO(COR⁴))R^{4'},
XCOOH, XCOOR⁴, XCONH₂, XCONHR⁴, XCONR⁴R^{4'},
XCONHOH, XCONHOR⁴, XCOSR⁴, XSR⁴, XSOR⁴, XSO₂R⁴,
SO₂NH₂, SO₂NHR⁴, SO₂NR⁴R^{4'},
NO₂, XNHR⁴, XNR⁴R^{4'}, XNHSO₂R⁴, XN(SO₂R⁴)SO₂R^{4'},
XNR⁴SO₂R^{4'}, tétrahydro-2,5-dioxopyrrol-1-yle,
2,5-dihydro-2,5-dioxopyrrol-1-yle, 2,7- dihydro-2,7-dioxoisoindol-1-yle, R⁴,
où deux substituants sur R², lorsqu'ils en position ortho l'un par rapport à l'autre, peuvent être liés l'un à l'autre de manière telle qu'ils forment ensemble méthanediylbisoxy, éthane-1,2-diylbisoxy, propane-1,3-diyle, butane-1,4-diyle,
***R³*** signifie un ou deux substituants, qui peuvent représenter, indépendamment l'un de l'autre :
hydrogène,
F, Cl, Br, I,
XOH, XOR⁴, XOCOR⁴, XOCONHR⁴, XOCOOR⁴,
XCOR⁴, XC(NOH)R⁴, XC(NOR⁴)R^{4'}, XC(NO(COR⁴))R^{4'},
XCN, XCOOH, XCOOR⁴, XCONH₂, XCONHR⁴, XCONR⁴R^{4'},
XCONHOH, XCONHOR⁴, XCOSR⁴, XSR⁴, XSOR⁴, XSO₂R⁴,
SO₂NH₂, SO₂NHR⁴, SO₂NR⁴R^{4'},
NO₂, XNH₂, XNHR⁴, XNR⁴R^{4'},
XNHSO₂R⁴, XNR⁴SO₂R^{4'}, XN(SO₂R⁴)(SO₂R^{4'}),
XNHCOR⁴, XNHCOOR⁴, XNHCONHR⁴, tétrahydro-2,5- dioxopyrrol-1-yle, 2,5-dihydro-2,5- dioxopyrrol-1-yle, 2,7-dihydro-2,7- dioxoisoindol-1-yle, ou R⁴,
où deux substituants R³, lorsqu'ils sont en position ortho l'un par rapport à l'autre, peuvent être liés l'un à l'autre de manière telle qu'ils forment ensemble méthanediylbisoxy, éthane-1,2-diylbisoxy, propane-1,3-diyle, butane-1,4-diyle,
***R⁴*** et ***R^{4'}*** signifient indépendamment l'un de l'autre C₁₋₄-perfluoroalkyle, C₁₋₆-alkyle, C₂₋₆-alcényle, C₂₋₆-alcynyle, C₃₋₇-cycloalkyle, (C₁₋₃-alkyl-C₃₋₇- cycloalkyle), C₁₋₃-alkyl-C₆₋₁₀-aryle, C₁₋₃-alkyl- hétéroaryle de 5-10 chaînons comprenant 1-4 atomes de N, S ou O, C₆₋₁₀-aryle ou hétéroaryle de 5-10 chaînons comprenant 1-4 atomes de N, S ou O, où les groupes aryle et hétéroaryle peuvent être substitués par un ou deux substituants du groupe constitué par F, Cl, Br, CH₃, C₂H₅, NO₂, OCH₃, OC₂H₅, CF₃, C₂F₅ ou peuvent également porter un groupe méthanediylbisoxy, éthane-1,2-diylbisoxy annelé, et en outre, dans un cycle cycloalkyle de 5 chaînons, un chaînon peut être un N ou un O et, dans un cycle cycloalkyle de 6 ou 7 chaînons, un ou deux chaînons peuvent être N et/ou O, où les azotes du cycle peuvent le cas échéant être substitués par C₁₋₃-alkyle ou C₁₋₃-alcanoyle,
***R⁵*** et ***R^{5'}*** signifient indépendamment l'un de l'autre C₁₋₆-alkyle, C₂₋₆-alcényle, C₂₋₆-alcynyle, un atome de carbone pouvant être remplacé par O, S, SO, SO₂, NH, NC₁₋₃-alkyle ou NC₁₋₃-alcanoyle, C₃₋₇-cycloalkyl-C₀₋₃-alkyle, où, dans un cycle cycloalkyle de 5 chaînons, un chaînon peut être un N ou un O et, dans un cycle cycloalkyle de 6 ou 7 chaînons, un ou deux chaînons peuvent être N et/ou O, où les azotes du cycle peuvent le cas échéant être substitués par C₁₋₃-alkyle ou C₁₋₃-alcanoyle C₆₋₁₀-aryle ou hétéroaryle de 5-10 chaînons comprenant 1-4 hétéroatomes parmi N, S et O, où les chaînes alkyle, alcényle et alcynyle mentionnées peuvent être substituées par des cycloalkyles, aryles ou hétéroaryles susmentionnés, où tous les radicaux alkyle et cycloalkyle susmentionnés peuvent être substitués par jusqu'à deux substituants parmi CF₃, C₂F₅, OH, OC₁₋₃-alkyle, NH₂, NHC₁₋₃-alkyle, NHC₁₋₃-alcanoyle, N(C₁₋₃-alkyle)₂, N(C₁₋₃-alkyl)(C₁₋₃-alcanoyle), COOH, CONH₂, COOC₁₋₃- alkyle et tous les groupes aryle et hétéroaryle susmentionnés peuvent être substitués par un ou deux substituants du groupe constitué par F, Cl, Br, CH₃, C₂H₅, NO₂, OCH₃, OC₂H₅, CF₃, C₂F₅ ou peuvent également porter un groupe méthanediylbisoxy, éthane-1,2-diylbisoxy annelé, ou R⁵ et R^{5'} forment, ensemble avec l'atome d'azote, un hétérocycle de 5-7 chaînons, qui peut contenir un autre atome d'oxygène, d'azote ou de soufre et qui peut être substitué par C₁₋₄-alkyle, C₁₋₄-alcoxy-C₀₋₂-alkyle, C₁₋₄-alcoxycarbonyle, aminocarbonyle ou phényle,
***A*** signifie C₁₋₁₀-alcanediyle, C₂₋₁₀-alcènediyle, C₂₋₁₀- alcynediyle, (C₀₋₅-alcanediyl-C₃₋₇-cycloalcanediyl- C₀₋₅-alcanediyle), où, dans un cycle cycloalkyle de 5 chaînons, un chaînon peut être un N ou un O et, dans un cycle cycloalkyle de 6 ou 7 chaînons, un ou deux chaînons peuvent être N et/ou O, où les azotes du cycle peuvent le cas échéant être substitués par C₁₋₃-alkyle ou C₁₋₃-alcanoyle où, dans les chaînes aliphatiques susmentionnées, un atome de carbone ou deux atomes de carbone peuvent être remplacés par O, NH, NC₁₋₃-alkyle, NC₁₋₃-alcanoyle et où les groupes alkyle ou cycloalkyle peuvent être substitués par jusqu'à deux substituants parmi =O, OH, OC₁-₃-alkyle, NH₂, NHC₁₋₃-alkyle, NHC₁₋₃-alcanoyle, N(C₁₋₃-alkyle)₂, N(C₁₋₃-alkyl)(C₁-₃-alcanoyle),
***B*** signifie COOH, COOR⁵, CONH₂, CONHNH₂, CONHR⁵, CONR⁵R^{5'}, CONHOH, CONHOR⁵, SO₃H, SO₂NH₂, SO₂NHR⁵, SO₂NR⁵R^{5'}, PO₃H, PO(OH)(OR⁵), PO(OR⁵)(OR⁵'), PO(OH)(NHR⁵), PO(NHR⁵)(NHR^{5'}), tétrazolyle, à chaque fois lié à un atome de carbone du groupe ***A,***
ou tout le groupe ***Y-A-B*** signifie N(SO₂R⁴)(SO₂R^{4'}) ou NHSO₂R⁴,
***X*** signifie une liaison, CH₂, (CH₂)₂, CH(CH₃), (CH₂)₃, CH(CH₂CH₃), CH(CH₃)CH₂, CH₂CH(CH₃),
***Y*** signifie O, NH, NR⁴, NCOR⁴, NSO₂R⁴,
étant entendu que
A) si Y signifie NH, NR⁴, NCOR⁴ ou NSO₂R⁴,
***a)*** et que le substitué ***R²*** contient un hétérocycle saturé contenant de l'azote, cet hétérocycle n'est pas substitué sur l'azote de fonction imine par H, méthyle, éthyle, propyle ou isopropyle, ou
***b)*** dans les groupes XNHR⁴ ou XNR⁴R^{4'} le cas échéant présents du substituant ***R²***, R⁴ et/ou R^{4'} ne signifient pas C₁₋₄-alkyle,
et que
B) simultanément, ***B*** ne signifie pas COOH, SO₃H, PO₃H₂ ou tétrazolyle et ***R¹*** et ***R²***, indépendamment l'un de l'autre C₅₋₆-hétéroaryle ou phényle, lorsque ceuxci, indépendamment l'un de l'autre, sont non substitués ou monosubstitués par C₁₋₆-alkyle, C₁₋₄-perfluoroalkyle, OC₁₋₆-alkyle, OC₁₋₄-perfluoroalkyle, COOH, COOC₁₋₆-alkyle, COC₁₋₆-alkyle, CONH₂, CONHR⁴, NO₂, NH₂, NHCOR⁴, NHSO₂R⁴ ou substitués par 1 ou 2 atomes d'halogène du groupe F, Cl, Br, I, et
les composés suivants étant exclus :
ester méthylique de l'acide [(1,2-diphényl-1H-benzimidazol-6-yl)oxy]acétique,
ester méthylique de l'acide 5-[(1,2-diphényl-1H-benzimidazol-6-yl)oxy]pentanoïque,
ester éthylique de l'acide 4-[(1,2-diphényl-1H-benzimidazol-6-yl)oxy]butanoïque,
ester méthylique de l'acide 5-[[1-(4-nitrophényl)-2-phényl-1H-benzimidazol-6-yl]oxy]pentanoïque,
ester méthylique de l'acide 6-[[1-(4-nitrophényl)-2-phényl-1H-benzimidazol-6-yl]oxy]hexanoïque,
ester méthylique de l'acide 5-[[1-(4-aminophényl)-2-phényl-1H-benzimidazol-6-yl]oxy]pentanoïque,
ester méthylique de l'acide 5-[[1-[4-[[(4-chlorophényl)sulfonyl]amino]phényl]-2-phényl-1H-benzimidazol-6-yl]oxy]pentanoïque,
ester méthylique de l'acide 5-[[1-[4-[(acétyl)amino]phényl]-2-phényl-1H-benzimidazol-6-yl]oxy]pentanoïque,
ester méthylique de l'acide 5-[[1-(3-nitrophényl)-2-phényl-1H-benzimidazol-6-yl]oxy]pentanoïque,
ester méthylique de l'acide 6-[[1-(3-nitrophényl)-2-phényl-1H-benzimidazol-6-yl]oxy]hexanoïque,
ester méthylique de l'acide 5-[[1-(3-aminophényl)-2-phényl-1H-benzimidazol-6-yl]oxy]pentanoïque,
ester méthylique de l'acide 5-[[1-[3-[[(4-chlorophényl)sulfonyl]amino]phényl]-2-phényl-1H-benzimidazol-6-yl]oxy]pentanoïque,
ester méthylique de l'acide 5-[[1-[3-[(acétyl)amino]phényl]-2-phényl-1H-benzimidazol-6-yl]oxy]pentanoïque.

2. Benzimidazoles selon la revendication 1, **caractérisés en ce que**
***R¹*** signifie un groupe C₆₋₁₂-aryle monocyclique ou bicyclique ou un groupe hétéroaryle de 5-10 chaînons, monocyclique ou bicyclique comprenant 1 à 2 hétéroatomes choisis dans le groupe constitué par N, S ou O, où le groupe aryle ou hétéroaryle mentionné peut être substitué, indépendamment l'un de l'autre, par jusqu'à trois des substituants suivants :
F, Cl, Br,
XOH, XOR⁴, XOCOR⁴, XOCONHR⁴, XOCOOR⁴,
XCOR⁴, XCN, XCOOH, XCOOR⁴, XCONH₂, XCONR⁴R^{4'},
XCONHR⁴, XCONHOH, XCONHOR⁴, XCOSR⁴, XSR⁴, NO₂,
XNHR⁴, XNR⁴R^{4'}, R⁴,
où deux substituants sur ***R¹***, lorsqu'ils sont en position ortho l'un par rapport à l'autre, peuvent être liés l'un à l'autre de manière telle qu'ils forment ensemble méthanediylbisoxy, éthane-1,2- diylbisoxy, propane-1,3-diyle, butane-1,4-diyle.

3. Benzimidazoles selon la revendication 1 ou 2, **caractérisés en ce que**
***R²*** signifie un groupe C₆₋₁₀-aryle monocyclique ou bicyclique ou un groupe hétéroaryle de 5-10 chaînons, monocyclique ou bicyclique comprenant 1 à 2 hétéroatomes choisis dans le groupe constitué par N, S ou O, où le groupe aryle ou hétéroaryle mentionné peut être substitué, indépendamment l'un de l'autre, par jusqu'à trois des substituants suivants :
F, Cl, Br,
XOH, XOR⁴, XOCOR⁴, XOCONHR⁴, XOCOOR⁴,
XCOR⁴, XC(NOH)R⁴, XC(NOR⁴)R^{4'}, XC(NO(COR⁴))R^{4'},
XCOOH, XCOOR⁴, XCONH₂, XCONHR⁴, XCONR⁴R^{4'},
XCONHOH, XCONHOR⁴, XCOSR⁴, XSR⁴, XSOR⁴, XSO₂R⁴,
SO₂NH₂, SO₂NHR⁴, SO₂NR⁴R^{4'},
NO₂, XNHR⁴, XNR⁴R^{4'}, XNHSO₂R⁴, XN(SO₂R⁴)SO₂R^{4'},
XNR⁴SO₂R^{4'}, R⁴,
où deux substituants sur ***R²***, lorsqu'ils sont en position ortho l'un par rapport à l'autre, peuvent être liés l'un à l'autre de manière telle qu'ils forment ensemble méthanediylbisoxy, éthane-1,2- diylbisoxy, propane-1,3-diyle, butane-1,4-diyle.

4. Benzimidazoles selon l'une quelconque des revendications 1-3, **caractérisés en ce que**
***R³*** signifie un ou deux substituants, qui peuvent représenter, indépendamment l'un de l'autre : hydrogène, F, Cl, Br, XOH, XOR⁴, XOCOR⁴, XOCONHR⁴, XOCOOR⁴, XCOR⁴, XC(NOH)R⁴, XC(NOR⁴)R^{4'}, XC(NO(COR⁴))R^{4'}, XCN, XSR⁴, XSOR⁴, XSO₂R⁴, SO₂NH₂, SO₂NHR⁴, SO₂NR⁴R^{4'}, NO₂, XNH₂, XNHR⁴, XNR⁴R^{4'}, XNHSO₂R⁴, XNR⁴SO₂R^{4'}, XN(SO₂R⁴)SO₂R^{4'}, XNHCOR⁴, XNHCOOR⁴, XNHCONHR⁴, ou R⁴, où deux substituants ***R³***, lorsqu'ils sont en position ortho l'un par rapport à l'autre, peuvent être liés l'un à l'autre de manière telle qu'ils forment ensemble méthanediylbisoxy, éthane-1,2-diylbisoxy, propane-1,3-diyle, butane-1,4-diyle.

5. Benzimidazoles selon l'une quelconque des revendications 1-4, **caractérisés en ce que**
***R⁴*** et ***R^{4'}*** signifient, indépendamment l'un de l'autre, CF₃, C₂F₅, C₁₋₄-alkyle, C₂₋₄-alcényle, C₂₋₄-alcynyle, C₃₋₆-cycloalkyle, (C₁₋₃-alkyl-C₃₋₆-cycloalkyle), phényle ou hétéroaryle de 5-6 chaînons comprenant 1-2 atomes de N, S ou O, où les groupes phényle et hétéroaryle peuvent être substitués par un ou deux substituants du groupe constitué par F, Cl, Br, CH₃, C₂H₅, OCH₃, OC₂H₅, CF₃, C₂F₅, et en outre, dans un cycle cycloalkyle de 5 chaînons, un chaînon peut être un N ou un O et, dans un cycle cycloalkyle de 6 chaînons, un ou deux chaînons peuvent être N et/ou O, où les azotes de cycle peuvent le cas échéant être substitués par C₁₋₃-alkyle ou C₁₋₃-alcanoyle.

6. Benzimidazoles selon l'une quelconque des revendications 1-5, **caractérisés en ce que**
***R⁵*** et ***R^{5'}*** signifient indépendamment l'un de l'autre C₁₋₆-alkyle, un atome de carbone pouvant être remplacé par O, NH, NC₁₋₃-alkyle ou NC₁₋₃-alcanoyle, C₃₋₇-cycloalkyl-C₀₋₃-alkyle, où, dans un cycle cycloalkyle de 5 chaînons, un chaînon peut être un N ou un O et, dans un cycle cycloalkyle de 6 ou 7 chaînons, un ou deux chaînons peuvent être N et/ou O, où les azotes du cycle peuvent le cas échéant être substitués par C₁₋₃-alkyle ou C₁₋₃-alcanoyle, où la partie C₁₋₆-alkyle mentionnée peut être substituée par un des cycloalkyles susmentionnés ou également par un hétéroaromatique de 5-6 chaînons comprenant 1-2 hétéroatomes choisis parmi N, S ou O, où toutes les parties alkyle et cycloalkyle susmentionnées peuvent être substituées par jusqu'à deux substituants parmi CF₃, OH, OC₁₋₃- alkyle, et les groupes hétéroaryle susmentionnés peuvent être substitués par un ou deux substituants parmi F, Cl, CF₃, CH₃, C₂H₅, OCH₃, OC₂H₅ ou R⁵ et R^{5'} forment ensemble avec l'atome d'azote un hétérocycle de 5-7 chaînons, qui peut contenir un autre atome d'oxygène, d'azote ou de soufre et qui peut être substitué par C₁₋₄-alkyle, C₁₋₄-alcoxy-C₀₋₂-alkyle, C₁₋₄-alcoxycarbonyle, aminocarbonyle ou phényle.

7. Benzimidazoles selon l'une quelconque des revendications 1-6, **caractérisés en ce que**
***A*** signifie C₁₋₁₀-alcanediyle, C₂₋₁₀-alcènediyle, C₂₋₁₀- alcynediyle, (C₀₋₅-alcanediyl-C₃₋₇-cycloalcanediyl- C₀₋₅-alcanediyle), où, dans un cycle cycloalkyle de 5 chaînons, un chaînon peut être un N ou un O et, dans un cycle cycloalkyle de 6 ou 7 chaînons, un ou deux chaînons peuvent être N et/ou O, où les azotes du cycle peuvent le cas échéant être substitués par C₁₋₃-alkyle ou C₁₋₃-alcanoyle où, dans les chaînes aliphatiques susmentionnées, un atome de carbone ou deux atomes de carbone peuvent être remplacés par O, NH, NC₁₋₃-alkyle, NC₁₋₃-alcanoyle.

8. Benzimidazoles selon l'une quelconque des revendications 1-7, **caractérisés en ce que**
***B*** signifie COOH, COOR , CONH₂, CONHR⁵, CONR⁵R^{5'}, CONHOH, CONHOR⁵ ou tétrazolyle, à chaque fois lié à un atome de carbone du groupe ***A***.

9. Benzimidazoles selon l'une quelconque des revendications 1-8, **caractérisés en ce que**
***X*** signifie une liaison ou méthylène.

10. Benzimidazoles selon l'une quelconque des revendications 1-9, **caractérisés en ce que**
***Y*** signifie O.

11. Benzimidazole selon l'une quelconque des revendications 1-10, choisi dans le groupe constitué par :
ester isopropylique de l'acide [(1,2-diphényl-1H-benzimidazol-6-yl)oxy]acétique
ester méthylique de l'acide 3-[(1,2-diphényl-1H-benzimidazol-6-yl)oxy]propanoïque
ester méthylique de l'acide 2-[(1,2-diphényl-1H-benzimidazol-6-yl)oxy]propanoïque
ester isopropylique de l'acide 4-[(1,2-diphényl-1H-benzimidazol-6-yl)oxy]butanoïque
ester isopropylique de l'acide 5-[(1,2-diphényl-1H-benzimidazol-6-yl)oxy]pentanoïque
ester méthylique de l'acide 6-[(1,2-diphényl-1H-benzimidazol-6-yl)oxy]hexanoïque
ester isopropylique de l'acide 6-[(1,2-diphényl-1H-benzimidazol-6-yl)oxy]hexanoïque
6-[(1,2-diphényl-1H-benzimidazol-6-yl)oxy]hexanamide N-méthoxy-6-[(1,2-diphényl-1H-benzimidazol-6-yl)oxy]hexanamide
N-(phénylméthoxy)-6-[(1,2-diphényl-1H-benzimidazol-6-yl)oxy]hexanamide
N-hydroxy-6-[(1,2-diphényl-1H-benzimidazol-6-yl)oxy]hexanamide
ester méthylique de l'acide 7-[(1,2-diphényl-1H-benzimidazol-6-yl)oxy]heptanoïque
ester isopropylique de l'acide 6-[[1-(3-nitrophényl)-2-phényl-1H-benzimidazol-6-yl]oxy]hexanoïque ester méthylique de l'acide 6-[[2-phényl-1-[3-(trifluorométhyl)phényl]-1H-benzimidazol-6-yl]oxy]hexanoïque
ester isopropylique de l'acide 6-[[2-phényl-1-[3-(trifluorométhyl)phényl]-1H-benzimidazol-6-yl]oxy]hexanoïque
ester méthylique de l'acide 6-[[1-(3-cyanophényl)-2-phényl-1H-benzimidazol-6-yl]oxy]hexanoïque ester isopropylique de l'acide 6-[[1-(3-cyanophényl)-2-phényl-1H-benzimidazol-6-yl]oxy]hexanoïque acide 6-[[1-(3-cyanophényl)-2-phényl-1H-benzimidazol-6-yl]oxy]hexanoïque
ester méthylique de l'acide 6-[[1-(4-cyanophényl)-2-phényl-1H-benzimidazol-6-yl]oxy]hexanoïque ester isopropylique de l'acide 6-[[1-(4-cyanophényl)-2-phényl-1H-benzimidazol-6-yl]oxy]hexanoïque ester méthylique de l'acide 6-[[1-(3-chlorophényl)-2-phényl-1H-benzimidazol-6-yl]oxy]hexanoïque ester isopropylique de l'acide 6-[[1-(3-chlorophényl)-2-phényl-1H-benzimidazol-6-yl]oxy]hexanoïque ester méthylique de l'acide 6-[[1-(4-chlorophényl)-2-phényl-1H-benzimidazol-6-yl]oxy]hexanoïque ester isopropylique de l'acide 6-[[1-(4-chlorophényl)-2-phényl-1H-benzimidazol-6-yl]oxy]]hexanoïque ester méthylique de l'acide 6-[[1-(3-méthylphényl)-2-phényl-1H-benzimidazol-6-yl]oxy]hexanoïque ester isopropylique de l'acide 6-[[1-(3-méthylphényl)-2-phényl-1H-benzimidazol-6-yl]oxy]hexanoïque ester méthylique de l'acide 6-[[1-(4-méthylphényl)-2-phényl-1H-benzimidazol-6-yl]oxy]hexanoïque ester isopropylique de l'acide 6-[[1-(4-méthylphényl)-2-phényl-1H-benzimidazol-6-yl]oxy]hexanoïque ester méthylique de l'acide 6-[[1-(3,4-diméthylphényl)-2-phényl-1H-benzimidazol-6-yl]oxy]hexanoïque ester méthylique de l'acide 6-[[1-(3,5-diméthylphényl)-2-phényl-1H-benzimidazol-6-yl]oxy]hexanoïque ester isopropylique de l'acide 6-[[1-(3,5-diméthylphényl)-2-phényl-1H-benzimidazol-6-yl]oxy]hexanoïque
ester méthylique de l'acide 6-[[1-(3-méthoxyphényl)-2-phényl-1H-benzimidazol-6-yl]oxy]hexanoïque ester méthylique de l'acide 6-[[1-(4-méthoxyphényl)-2-phényl-1H-benzimidazol-6-yl]oxy]hexanoïque ester méthylique de l'acide 6-[[1-(3,4-diméthoxyphényl)-2-phényl-1H-benzimidazol-6-yl]oxy]hexanoïque
ester méthylique de l'acide 6-[[1-[3,4-(méthylènedioxy)phényl]-2-phényl-1H-benzimidazol-6-yl]oxy]hexanoïque
acide 6-[[1-[3,4-(méthylènedioxy)phényl]-2-phényl-1H-benzimidazol-6-yl]oxy]hexanoïque
ester méthylique de l'acide 6-[[2-phényl-1-(3,4,5-triméthoxyphényl)-1H-benzimidazol-6-yl]oxy]hexanoïque acide 6-[[2-phényl-1-(3,4,5-triméthoxyphényl)-1H-benzimidazol-6-yl]oxy]hexanoïque
ester isopropylique de l'acide 6-[[2-phényl-1-(3,4,5-triméthoxyphényl)-1H-benzimidazol-6-yl]oxy]hexanoïque ester méthylique de l'acide 6-[[1-[4-(N,N-diméthylamino)phényl]-2-phényl-1H-benzimidazol-6-yl]oxy]hexanoïque
acide 6-[[1-(4-(N,N-diméthylamino)phényl]-2-phényl-1H-benzimidazol-6-yl]oxy]hexanoïque
ester isopropylique de l'acide 6-[[1-phényl-2-[3-(trifluorométhyl)phényl]-1H-benzimidazol-6-yl]oxy]hexanoïque
ester méthylique de l'acide 6-[[2-(3-chlorophényl)-1-phényl-1H-benzimidazol-6-yl]oxy]hexanoïque ester isopropylique de l'acide 6-[[2-(3-chlorophényl)-1-phényl-1H-benzimidazol-6-yl]oxy]hexanoïque ester méthylique de l'acide 6-[[2-(4-chlorophényl)-1-phényl-1H-benzimidazol-6-yl]oxy]hexanoïque ester isopropylique de l'acide 6-[[2-(4-chlorophényl)-1-phényl-1H-benzimidazol-6-yl]oxy]hexanoïque ester méthylique de l'acide 6-[[2-(4-méthylphényl)-1-phényl-1H-benzimidazol-6-yl]oxy]hexanoïque ester isopropylique de l'acide 6-[[2-(4-méthylphényl)-1-phényl-1H-benzimidazol-6-yl]oxy]hexanoïque ester méthylique de l'acide 6-[[1-phényl-2-(4-pyridinyl)-1H-benzimidazol-6-yl]oxy]hexanoïque ester méthylique de l'acide 6-[(1,2-diphényl-5-nitro-1H-benzimidazol-6-yl)oxy]hexanoïque
ester isopropylique de l'acide 6-[(1,2-diphényl-5-nitro-1H-benzimidazol-6-yl)oxy]hexanoïque ester isopropylique de l'acide 6-[[5-[[(4-bromophényl)sulfonyl]amino]-1,2-diphényl-1H-benzimidazol-6-yl]oxy]hexanoïque
ester méthylique de l'acide 6-[[5-[[(4-chlorophényl)sulfonyl]amino]-1,2-diphényl-1H-benzimidazol-6-yl]oxy]hexanoïque
ester isopropylique de l'acide 6-[[5-[[(4-chlorophényl)sulfonyl]amino]-1,2-diphényl-1H-benzimidazol-6-yl]oxy]hexanoïque
ester isopropylique de l'acide 6-[[1,2-diphényl-5-[[(3-méthylphényl)sulfonyl]amino]-1H-benzimidazol-6-yl]oxy]hexanoïque
ester isopropylique de l'acide 6-[[1,2-diphényl-5-[[(4-méthylphényl)sulfonyl]amino]-1H-benzimidazol-6-yl]oxy]hexanoïque
ester isopropylique de l'acide 6-[[1,2-diphényl-5-[[(4-méthoxyphényl)sulfonyl]amino]-1H-benzimidazol-6-yl]oxy]hexanoïque
ester isopropylique de l'acide 6-[[1,2-diphényl-5-[[[(4-trifluorométhyl)phényl]sulfonyl]amino]-1H-benzimidazol-6-yl]oxy]hexanoïque
ester isopropylique de l'acide 6-[[5-[[[4-(acétylamino)phényl]sulfonyl]amino]-1,2-diphényl-1H-benzimidazol-6-yl]oxy]hexanoïque
ester isopropylique de l'acide 6-[[5-[[bis(3-chlorophényl)sulfonyl]amino]-1,2-diphényl-1H-benzimidazol-6-yl]oxy]hexanoïque
ester isopropylique de l'acide 6-[[1,2-diphényl-5-[(propylsulfonyl)amino]-1H-benzimidazol-6-yl]oxy]hexanoïque
ester isopropylique de l'acide 6-[[5-[(benzylsulfonyl)amino]-1,2-diphényl-1H-benzimidazol-6-yl]oxy]hexanoïque
ester méthylique de l'acide 2-[2-[(1,2-diphényl-1H-benzimidazol-6-yl)oxy]éthoxy]acétique
ester méthylique de l'acide 3-[2-[(1,2-diphényl-1H-benzimidazol-6-yl)oxy]éthoxy]propanoïque ester éthylique de l'acide 6-[[1-(3-nitrophényl)-2-phényl-1H-benzimidazol-6-yl]oxy]hexanoïque ester méthylique de l'acide 6-[[4-acétyl-1-(4-méthylphényl)-2-phényl-1H-benzimidazol-6-yl]oxy]hexanoïque
ester méthylique de l'acide 6-[[1-(4-méthylphényl)-2-phényl-1H-benzimidazol-5-yl]oxy]hexanoïque ester méthylique de l'acide 6-[[2-phényl-1[4-(thiométhyl)phényl]-1H-benzimidazol-5-yl]oxy]hexanoïque ester méthylique de l'acide 6-[[2-phényl-1-[(4-thiométhyl)phényl]-1H-benzimidazol-6-yl]oxy]hexanoïque ester méthylique de l'acide 6-[[2-phényl-1-(3-thiényl)-1H-benzimidazol-5-yl]oxy]hexanoïque
ester méthylique de l'acide 6-[[2-phényl-1-(3-thiényl)-1H-benzimidazol-6-yl]oxy]hexanoïque
ester méthylique de l'acide 4-[(1,2-diphényl-1H-benzimidazol-6-yl)oxy]butanoïque
N-(phénylméthoxy)-6-[[2-phényl-1-(3,4,5-triméthoxyphényl)-1H-benzimidazol-6-yl]oxy]hexanamide N,N-diméthyl-6-[(1,2-diphényl-1H-benzimidazol-6-yl)oxy]hexanamide
N-isopropyl-6-[(1,2-diphényl-1H-benzimidazol-6-yl)oxy]hexanamide
6-[(1,2-diphényl-1H-benzimidazol-6-yl)oxy]-1-pyrrolidin-1-ylhexan-1-one
ester méthylique de l'acide 5-[[5-[[(4-chlorophényl)sulfonyl]amino]-1,2-diphényl-1H-benzimidazol-6-yl]oxy]pentanoïque
ester méthylique de l'acide 6-[[5-[[(4-chlorophényl)sulfonyl]amino]-1-(4-méthylphényl)-2-phényl-1H-benzimidazol-6-yl]oxy]hexanoïque ester méthylique de l'acide 6-[[5-[[(4-chlorophényl)sulfonyl]amino]-1-(4-méthoxyphényl)-2-phényl-1H-benzimidazol-6-yl]oxy]hexanoïque ester méthylique de l'acide 6-[[4-(acétyloxy)-1-(4-méthylphényl)-2-phényl-1H-benzimidazol-6-yl]oxy]hexanoïque
ester méthylique de l'acide 6-[[4-hydroxy-1-(4-méthylphényl)-2-phényl-1H-benzimidazol-6-yl]oxy]hexanoïque
acide 6-[[4-hydroxy-1-(4-méthylphényl)-2-phényl-1H-benzimidazol-6-yl]oxy]hexanoïque
ester méthylique de l'acide 6-[[7-méthyl-1-(4-méthylphényl)-2-phényl-1H-benzimidazol-6-yl]oxy]hexanoïque
ester méthylique de l'acide 6-[[2-phényl-1-(3-pyridyl)-1H-benzimidazol-5-yl]oxy]hexanoïque
ester méthylique de l'acide 6-[[2-phényl-1-(3-pyridyl)-1H-benzimidazol-6-yl]oxy)hexanoïque
ester méthylique de l'acide 6-[[2-phényl-1-(4-pyridyl)-1H-benzimidazol-6-yl]oxy]hexanoïque
ester méthylique de l'acide 6-[[2-(4-fluorophényl)-1-phényl-1H-benzimidazol-6-yl]oxy]hexanoïque ester méthylique de l'acide 6-[[2-(4-méthoxyphényl)-1-phényl-1H-benzimidazol-6-yl]oxy]hexanoïque ester méthylique de l'acide 6-[[2-(4-bromophényl)-1-phényl-1H-benzimidazol-6-yl]oxy]hexanoïque ester méthylique de l'acide 6-[[2-[4-(trifluorométhyl)phényl]-1-phényl-1H-benzimidazol-6-yl]oxy]hexanoïque
ester méthylique de l'acide 6-[[1-phényl-2-(benzothién-2-yl)-1H-benzimidazol-6-yl]oxy]hexanoïque acide 6-[[1-phényl-2-(benzothién-2-yl)-1H-benzimidazol-6-yl]oxy]hexanoïque
ester isopropylique de l'acide 6-[[5-hydroxy-1-(4-méthylphényl)-2-phényl-1H-benzimidazol-6-yl]oxy]hexanoïque
acide 6-[[5-hydroxy-1-(4-méthylphényl)-2-phényl-1H-benzimidazol-6-yl]oxy]hexanoïque
ester isopropylique de l'acide 6-[[5-méthoxy-1-(4-méthylphényl)-2-phényl-1H-benzimidazol-6-yl]oxy]hexanoïque
ester méthylique de l'acide 6-[[5-hydroxy-1-(4-méthylphényl)-2-phényl-1H-benzimidazol-6-yl]oxy]hexanoïque
ester méthylique de l'acide 6-[[5-méthoxy-1-(4-méthylphényl)-2-phényl-1H-benzimidazol-6-yl]oxy]hexanoïque
ester méthylique de l'acide 6-[[5-[[(4-chlorophényl)sulfonyl]amino]-1-(3,4-diméthylphényl)-2-phényl-1H-benzimidazol-6-yl]oxy]-hexanoïque ester méthylique de l'acide 6-[[5-[[(4-chlorophényl)sulfonyl]amino]-2-(4-fluorophényl)-1-(4-méthoxyphényl)-1H-benzimidazol-6-yl]oxy]hexanoïque ester méthylique de l'acide 6-[[5-[[(4-chlorophényl)sulfonyl]amino]-1-(4-méthoxyphényl)-2-(4-méthoxyphényl)-1H-benzimidazol-6-yl]oxy]hexanoïque ester méthylique de l'acide 4-[[5-[[(4-chlorophényl)sulfonyl]amino]-1-(4-méthoxyphényl)-2-phényl-1H-benzimidazol-6-yl]oxy]-butanoïque ester méthylique de l'acide 5-[[5-[[(4-chlorophényl)sulfonyl]amino]-1-(4-méthoxyphényl)-2-phényl-1H-benzimidazol-6-yl]oxy]pentanoïque ester méthylique de l'acide 5-[[5-[[(4-chlorophényl)sulfonyl]amino]-1,2-diphényl-1H-benzimidazol-6-yl]oxy]pentanoïque
ester méthylique de l'acide 6-[[5-[[(4-(trifluorométhyl)phényl)sulfonyl]amino]-1-(4-méthoxyphényl)-2-phényl-1H-benzimidazol-6-yl]oxy]hexanoïque
ester méthylique de l'acide 6-[[5-[[(4-chlorophényl)sulfonyl]méthylamino]-1-(4-méthoxyphényl)-2-phényl-1H-benzimidazol-6-yl]oxy]hexanoïque ester méthylique de l'acide 6-[[1-(indan-5-yl)-2-phényl-1H-benzimidazol-6-yl]oxy]hexanoïque acide 6-[[1-(indan-5-yl)-2-phényl-1H-benzimidazol-6-yl]oxy]hexanoïque
ester méthylique de l'acide 6-[[1-(3-fluorophényl)-2-phényl-1H-benzimidazol-6-yl]oxy]hexanoïque ester méthylique de l'acide 6-[[2-(4-nitrophényl)-1-phényl-1H-benzimidazol-6-yl]oxy]hexanoïque ester méthylique de l'acide 6-[[1-phényl-2-(3-pyridinyl)-1H-benzimidazol-6-yl]oxy]hexanoïque N-(cyclopropylméthoxy)-6-[(1,2-diphényl-1H-benzimidazol-6-yl)oxy]hexanamide
N-isobutoxy-6[(1,2-diphényl-1H-benzimidazol-6-yl)oxy]hexanamide
N-(cyclopropylméthoxy)-6-[2-phényl-1-(3,4,5-triméthoxyphényl)-1H-benzimidazol-6-yl]oxy]hexanamide N-isobutoxy-6-[2-phényl-1-(3,4,5-triméthoxyphényl)-1H-benzimidazol-6-yl]oxy]hexanamide
N-(2-méthoxyéthyl)-6-[(1,2-diphényl-1H-benzimidazol-6-yl)oxy]hexanamide
N-(3-méthoxypropyl)-6-[(1,2-diphényl-1H-benzimidazol-6-yl]oxy]hexanamide
N-isobutyl-6-[(1,2-diphényl-1H-benzimidazol-6-yl)oxy]hexanamide
6-[(1,2-diphényl-1H-benzimidazol-6-yl)oxy]-1-morpholin-1-ylhexan-1-one
N,N-di(-2-méthoxyéthyl)-6-[(1,2-diphényl-1H-benzimidazol-6-yl)oxy]hexanamide
N-isopentyl-6-[(1,2-diphényl-1H-benzimidazol-6-yl)oxy]hexanamide
N-(pyridin-2-yl)-6-[(1,2-diphényl-1H-benzimidazol-6-yl)oxy]hexanamide
N-(pyridin-3-yl)-6-[(1,2-diphényl-1H-benzimidazol-6-yl)oxy]hexanamide
N-isopropyl-6-[[1-(3,4-diméthylphényl)-2-phényl-1H-benzimidazol-6-yl]oxy]hexanamide
N,N-diméthyl-6-[[1-(3,4-diméthylphényl)-2-phényl-1H-benzimidazol-6-yl]oxy]hexanamide
N,N-diéthyl-6-[[1-(3,4-diméthylphényl)-2-phényl-1H-benzimidazol-6-yl]oxy]hexanamide
N-isobutyl-6-[[1-(3,4-diméthylphényl)-2-phényl-1H-benzimidazol-6-yl]oxy]hexanamide
N-cyclopropyl-6-[[1-(3,4-diméthylphényl)-2-phényl-1H-benzimidazol-6-yl]oxy]hexanamide
N-cyclobutyl-6-[[1-(3,4-diméthylphényl)-2-phényl-1H-benzimidazol-6-yl]oxy]hexanamide
N-tert-butyl-6-[[1-(3,4-diméthylphényl)-2-phényl-1H-benzimidazol-6-yl]oxy]hexanamide
(R)-6-[[1-(3,4-diméthylphényl)-2-phényl-1H-benzimidazol-6-yl]oxy]1-(2-méthoxyméthyl)pyrrolidin-1-ylhexan-1-one
N-(3-imidazol-1-ylpropyl)-6-[[1-(3,4-diméthylphényl)-2-phényl-1H-benzimidazol-6-yl]oxy]hexanamide N-(2-pyridin-2-yléthyl)-6-[[1-(3,4-diméthylphényl)-2-phényl-1H-benzimidazol-6-yl]oxy]hexanamide N-(3-méthoxypropyl)-6-[[1-(indan-5-yl)-2-phényl-1H-benzimidazol-6-yl]oxy]heptanamide
ester méthylique de l'acide 6-[[1-(4-méthylphényl)-2-(3-pyridyl)-1H-benzimidazol-6-yl]oxy]hexanoïque ester méthylique de l'acide 6-[[1-(4-méthylphényl)-2-(4-pyridyl)-1H-benzimidazol-6-yl]oxy]hexanoïque ester méthylique de l'acide 6-[[1-(4-méthylphényl)-2-(2-thiényl)-1H-benzimidazol-6-yl]oxy]hexanoïque ester méthylique de l'acide 6-[[1-(4-méthylphényl)-2-(3-thiényl)-1H-benzimidazol-6-yl]oxy]hexanoïque ester méthylique de l'acide 6-[[2-(3-indolyl)-1-(4-méthylphényl)-1H-benzimidazol-6-yl]oxy]hexanoïque ester méthylique de l'acide 6-[[1-(4-méthylphényl)-2-(2-furyl)-1H-benzimidazol-6-yl]oxy]hexanoïque ester méthylique de l'acide 6-[[1-(4-méthylphényl)-2-(3-furyl)-1H-benzimidazol-6-yl]oxy]hexanoïque ester méthylique de l'acide 6-[[1-(4-méthylphényl)-2-(5-méthyl-2-thiényl)-1H-benzimidazol-6-yl]oxy]hexanoïque
ester méthylique de l'acide 6-[[1-(4-méthylphényl)-2-(3-méthyl-2-thiényl)-1H-benzimidazol-6-yl]oxy]hexanoïque.

12. Composé selon l'une quelconque des revendications 1-11 pour une utilisation dans un processus de guérison, dans la prophylaxie ou dans le diagnostic.

13. Composé selon l'une quelconque des revendications 1-11 destiné à une utilisation lors du traitement ou de la prévention de maladies qui sont associées à une activation de la microglie, choisies dans le groupe constitué par la démence associée au SIDA, la sclérose latérale amyotrophique, la maladie de Creutzfeld-Jacob, le syndrome de Down, la démence à corps de Lewy, la chorée de Huntington, la leuco-encéphalopathie, la sclérose en plaques, le dysfonctionnement et la dégénérescence neuronal(e), la maladie de Parkinson, la maladie de Pick, la maladie d'Alzheimer, une attaque, l'épilepsie temporale et les tumeurs.

14. Utilisation d'un composé selon l'une quelconque des revendications 1-11 pour la préparation d'un médicament destiné au traitement ou à la prévention de maladies qui sont associées à une activation de la microglie, choisies dans le groupe constitué par la démence associée au SIDA, la sclérose latérale amyotrophique, la maladie de Creutzfeld-Jacob, le syndrome de Down, la démence à corps de Lewy, la chorée de Huntington, la leuco-encéphalopathie, la sclérose en plaques, le dysfonctionnement et la dégénérescence neuronal(e), la maladie de Parkinson, la maladie de Pick, la maladie d'Alzheimer, une attaque, l'épilepsie temporale et les tumeurs.

15. Agent pharmaceutique, **caractérisé en ce qu'**il contient un ou plusieurs composés selon l'une quelconque des revendications 1-11 et une ou plusieurs substances support.

16. Benzimidazole de formule générale II 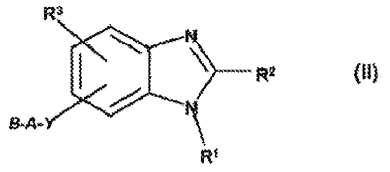 où
***R¹*** signifie un groupe C₆₋₁₂-aryle monocyclique ou bicyclique ou un groupe hétéroaryle de 5-10 chaînons, monocyclique ou bicyclique comprenant 1 à 4 hétéroatomes choisis dans le groupe constitué par N, S ou O, où le groupe aryle ou hétéroaryle mentionné peut être substitué, indépendamment l'un de l'autre, par jusqu'à trois des substituants suivants :
F, Cl, Br, I, C(NH)NH₂, C(NH)NHR⁴, C(NH)NR⁴R^{4'}, C(NR⁴)NH², C(NR⁴)NHR^{4'}, C(NR⁴)NR⁴R^{4'}, XOH, XOR⁴, XOCOR⁴, XOCONHR⁴, XOCOOR⁴, XCOR⁴, XC(NOH)R⁴, XC(NOR⁴)R^{4'}, XC(NO(COR⁴))R^{4'}, XCN, XCOOH, XCOOR⁴, XCONH₂, XCONR⁴R^{4'}, XCONHR⁴, XCONHOH, XCONHOR⁴, XCOSR⁴, XSR⁴, XSOR⁴, XSO₂R⁴, SO₂NH₂, SO₂NHR⁴, SO₂NR⁴R^{4'}, NO₂, XNH₂, XNHR⁴, XNR⁴R^{4'}, XNHSO₂R⁴, XN(SO₂R⁴)(SO₂R^{4'}), XNR⁴SO₂R^{4'}, XNHCOR⁴, XNHCOOR⁴, XNHCONHR⁴, tétrahydro-2,5-dioxopyrrol-1-yle, 2,5- dihydro-2,5-dioxopyrrol-1-yle, 2,7-dihydro-2,7- dioxoisoindol-1-yle, R⁴,
où deux substituants sur ***R¹***, lorsqu'ils sont en position ortho l'un par rapport à l'autre, peuvent être liés l'un à l'autre de manière telle qu'ils forment ensemble méthanediylbisoxy, éthane-1,2- diylbisoxy, propane-1,3-diyle, butane-1,4-diyle,
***R²*** signifie un groupe C₆₋₁₀-aryle monocyclique ou bicyclique ou un groupe hétéroaryle de 5-10 chaînons, monocyclique ou bicyclique comprenant 1 à 4 hétéroatomes choisis dans le groupe constitué par N, S ou O, où le groupe aryle ou hétéroaryle mentionné peut être substitué, indépendamment l'un de l'autre, par jusqu'à trois des substituants suivants :
F, Cl, Br, I, C(NH)NH₂, C(NH)NHR⁴, C(NH)NR⁴R^{4'}, C(NR⁴)NH₂ , C(NR⁴)NHR^{4'}, C(NR⁴)NR⁴R^{4'}, XOH, XOR⁴, XOCOR⁴, XOCONHR⁴, XOCOOR⁴,
XCOR⁴, XC(NOH)R⁴, XC(NOR⁴)R^{4'}, XC(NO(COR⁴))R^{4'}, XCN, XCOOH, XCOOR⁴, XCONH₂, XCONR⁴R^{4'}, XCONHR⁴, XCONHOH, XCONHOR⁴, XCOSR⁴
XSR⁴, XSOR⁴, XSO₂R⁴, SO₂NH₂, SO₂NHR⁴, SO₂NR⁴R^{4'}, NO₂, XNH₂, XNHR⁴, XNR⁴R^{4'}, XNHSO₂R⁴, XN(SO₂R⁴)(SO₂R^{4'}), XNR⁴SO₂R^{4'}, XNHCOR⁴, XNHCOOR⁴, XNHCONHR⁴,
tétrahydro-2,5-dioxopyrrol-1-yle, 2,5- dihydro-2,5-dioxopyrrol-1-yle, 2,7-dihydro- 2,7-dioxoisoindol-1-yle, R⁴,
où deux substituants sur ***R²***, lorsqu'ils sont en position ortho l'un par rapport à l'autre, peuvent être liés l'un à l'autre de manière telle qu'ils forment ensemble méthanediylbisoxy, éthane-1,2-diylbisoxy, propane-1,3-diyle, butane-1,4-diyle,
***R³*** représente un ou deux substituants, qui peuvent représenter, indépendamment l'un de l'autre :
hydrogène, F, Cl, Br, I, XOH, XOR⁴, XOCOR⁴, XOCONHR⁴, XOCOOR⁴,
XCOR⁴, XC(NOH)R⁴, XC(NOR⁴)R^{4'}, XC(NO(COR⁴))R^{4'}, XCN, XCOOH, XCOOR⁴,
XCONH₂, XCONHR⁴, XCONR⁴R^{4'}, XCONHOH, XCONHOR⁴, XCOSR⁴, XSR⁴, XSOR⁴, XSO₂R⁴, SO₂NH₂, SO₂NHR⁴, SO₂NR⁴R^{4'}, NO₂, XNH₂, XNHR⁴, XNR⁴R^{4'},
XNHSO₂R⁴, XNR⁴SO₂R^{4'}, XN(SO₂R⁴)(SO₂R^{4'}), XNHCOR⁴, XNHCOOR⁴, XNHCONHR⁴,
tétrahydro-2,5-dioxopyrrol-1-yle, 2,5- dihydro-2,5-dioxopyrrol-1-yle, 2,7-dihydro- 2,7-dioxoisoindol-1-yle, R⁴,
où deux substituants ***R³***, lorsqu'ils sont en position ortho l'un par rapport à l'autre, peuvent être liés l'un à l'autre de manière telle qu'ils forment ensemble méthanediylbisoxy, éthane-1,2-diylbisoxy, propane-1,3-diyle, butane-1,4-diyle,
***R⁴*** et ***R^{4'}*** signifient indépendamment l'un de l'autre C₁₋₄-perfluoroalkyle, C₁₋₆-alkyle, C₂₋₆-alcényle, C₂₋₆-alcynyle, C₃₋₇-cycloalkyle, (C₁₋₃-alkyl-C₃₋₇- cycloalkyle), C₁₋₃-alkyl-C₆₋₁₀-aryle, C₁₋₃-alkyl- hétéroaryle de 5-10 chaînons comprenant 1-4 atomes de N, S ou 0, C₆₋₁₀-aryle ou hétéroaryle de 5-10 chaînons comprenant 1-4 atomes de N, S ou 0, où les groupes C₆₋₁₀-aryle et hétéroaryle peuvent être substitués par un ou deux substituants du groupe constitué par F, Cl, Br, CH₃, C₂H₅, NO₂, OCH₃, OC₂H₅, CF₃, C₂F₅ ou peuvent également porter un groupe méthanediylbisoxy, éthane-1,2-diylbisoxy annelé, et en outre, dans un cycle cycloalkyle de 5 chaînons, un chaînon peut être un N ou un 0 et, dans un cycle cycloalkyle de 6 ou 7 chaînons, un ou deux chaînons peuvent être N et/ou 0, où les azotes du cycle peuvent le cas échéant être substitués par C₁₋₃-alkyle ou C₁₋₃-alcanoyle,
***R⁵*** et ***R^{5'}*** signifient indépendamment l'un de l'autre hydrogène, C₁₋₆-alkyle, C₂₋₆-alcényle, C₂₋₆-alcynyle, un atome de carbone pouvant être remplacé par 0, S, SO, SO₂, NH, NC₁₋₃-alkyle ou NC₁₋₃-alcanoyle, C₃₋₇-cycloalkyl-C₀₋₃-alkyle, où, dans un cycle cycloalkyle de 5 chaînons, un chaînon peut être un N ou un 0 et, dans un cycle cycloalkyle de 6 ou 7 chaînons, un ou deux chaînons peuvent être N et/ou 0, où les azotes du cycle peuvent le cas échéant être substitués par C₁₋₃-alkyle ou C₁₋₃-alcanoyle C₆₋₁₀-aryle ou hétéroaryle de 5-10 chaînons comprenant 1-4 hétéroatomes parmi N, S et 0, où les chaînes alkyle, alcényle et alcynyle mentionnées peuvent être substituées par des cycloalkyles, aryles ou hétéroaryles susmentionnés, où tous les radicaux alkyle et cycloalkyle susmentionnés peuvent être substitués par jusqu'à deux substituants parmi CF₃, C₂F₅, OH, OC₁₋₃-alkyle, NH₂, NHC₁₋₃-alkyle, NHC₁₋₃-alcanoyle, N(C₁₋₃-alkyle)₂, N(C₁₋₃-alkyl)(C₁₋₃-alcanoyle), COOH, CONH₂, COOC₁₋₃- alkyle et tous les groupes aryle et hétéroaryle susmentionnés peuvent être substitués par un ou deux substituants du groupe constitué par F, Cl, Br, CH₃, C₂H₅, NO₂, OCH₃, OC₂H₅, CF₃, C₂F₅ ou peuvent également porter un groupe méthanediylbisoxy, éthane-1,2-diylbisoxy annelé, ou R⁵ et R^{5'} forment, ensemble avec l'atome d'azote, un hétérocycle de 5-7 chaînons, qui peut contenir un autre atome d'oxygène, d'azote ou de soufre et qui peut être substitué par C₁₋₄-alkyle, C₁₋₄-alcoxy-C₀₋₂-alkyle, C₁₋₄-alcoxycarbonyle, aminocarbonyle ou phényle,
***A*** signifie C₁₋₁₀-alcanediyle, C₂₋₁₀-alcènediyle, C₂₋₁₀- alcynediyle, (C₀₋₅-alcanediyl-C₃₋₇-cycloalcanediyl- C₀₋₅-alcanediyle), (C₀₋₅-alcanediylarylène-C₀₋₅- alcanediyle), (C₀₋₅-alcanediyl-hétéroarylène-C₀₋₅- alcanediyle), où les groupes aryle et hétéroaryle peuvent être substitués par un ou deux substituants parmi F, Cl, Br, CH₃, C₂H₅, NO₂, OCH₃, OC₂H₅, CF₃, C₂F₅, où, dans un cycle cycloalkyle de 5 chaînons, un chaînon peut être un N ou un 0 et, dans un cycle cycloalkyle de 6 ou 7 chaînons, un ou deux chaînons peuvent être N et/ou 0, où les azotes du cycle peuvent le cas échéant être substitués par C₁₋₃-alkyle ou C₁₋₃-alcanoyle où, dans les chaînes aliphatiques susmentionnées, un atome de carbone ou deux atomes de carbone peuvent être remplacés par 0, NH, NR⁴, NCOR⁴, NSO₂R⁴, et où les groupes alkyle ou cycloalkyle peuvent être substitués par jusqu'à deux substituants parmi F, OH, OR⁴, OCOR⁴, =O, NH₂, NR⁴R^{4'}, NHCOR⁴, NHCOOR⁴, NHCONHR⁴, NHSO₂R⁴ SH, SR⁴,
***B*** signifie hydrogène, OH, OCOR⁵, OCONHR⁵, OCOOR⁵, COR⁵, C(NOH)R⁵, C(NOR⁵)R^{5'}, C(NO(COR⁵))R⁵, COOH, COOR₅ CONH₂, CONHNH₂, CONHR⁵, CONR⁵R^{5'}, CONHOH, CONHOR⁵, SO₃H, SO₂NH₂, SO₂NHR⁵, SO₂NR⁵R^{5'}, PO₃H, PO(OH)(OR⁵), PO(OR⁵)(OR^{5'}), PO(OH)(NHR⁵), PO( NHR⁵ )(NHR^{5'}), tétrazolyle, à chaque fois lié à un atome de carbone du groupe ***A***, ou tout le groupe ***Y-A-B*** signifie N(SO2R⁴)(SO₂R^{4'}) ou NHSO₂R⁴,
***X*** signifie une liaison, CH₂, (CH₂)₂, CH(CH₃), (CH₂)₃, CH(CH₂CH₃), CH(CH₃)CH₂, CH₂CH(CH₃),
***Y*** signifie une liaison, 0, S, SO, SO₂, NH, NR⁴, NCOR⁴, NSO₂R⁴, destiné à une utilisation lors du traitement ou de la prévention de maladies qui sont associées à une activation de la microglie, choisies dans le groupe constitué par la démence associée au SIDA, la sclérose latérale amyotrophique, la maladie de Creutzfeld-Jacob, le syndrome de Down, la démence à corps de Lewy, la chorée de Huntington, la leuco-encéphalopathie, la sclérose en plaques, le dysfonctionnement et la dégénérescence neuronal(e), la maladie de Parkinson, la maladie de Pick, la maladie d'Alzheimer, une attaque, l'épilepsie temporale et les tumeurs.

17. Utilisation d'un benzimidazole de formule générale II 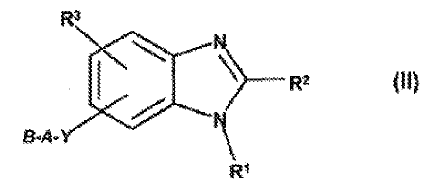 où
***R¹*** signifie un groupe C₆₋₁₂-aryle monocyclique ou bicyclique ou un groupe hétéroaryle de 5-10 chaînons, monocyclique ou bicyclique comprenant 1 à 4 hétéroatomes choisis dans le groupe constitué par N, S ou 0, où le groupe aryle ou hétéroaryle mentionné peut être substitué, indépendamment l'un de l'autre, par jusqu'à trois des substituants suivants :
F, Cl, Br, I, C(NH)NH₂, C(NH)NHR⁴, C(NH)NR⁴R^{4'}, C(NR⁴)NH₂, C(NR⁴)NHR^{4'}, C(NR⁴)NR⁴R^{4'}, XOH, XOR⁴, XOCOR⁴, XOCONHR⁴, XOCOOR⁴, XCOR⁴, XC(NOH)R⁴, XC(NOR⁴)R^{4'}, XC(NO(COR⁴))R^{4'}, XCN, XCOOH, XCOOR⁴, XCONH₂, XCONR⁴R^{4'}, XCONHR⁴, XCONHOH, XCONHOR⁴, XCOSR⁴, XSR⁴, XSOR⁴, XSO₂R⁴, SO₂NH₂, SO₂NHR⁴, SO₂NR⁴R^{4'}, NO₂, XNH₂, XNHR⁴, XNR⁴R^{4'}, XNHSO₂R⁴, XN(SO₂R⁴)(SO₂R^{4'}), XNR⁴SO₂R^{4'}, XNHCOR⁴, XNHCOOR⁴, XNHCONHR⁴, tétrahydro-2,5-dioxopyrrol-1-yle, 2,5- dihydro-2,5-dioxopyrrol-1-yle, 2,7-dihydro-2,7- dioxoisoindol-1-yle, R⁴,
où deux substituants sur ***R¹***, lorsqu'ils sont en position ortho l'un par rapport à l'autre, peuvent être liés l'un à l'autre de manière telle qu'ils forment ensemble méthanediylbisoxy, éthane-1,2- diylbisoxy, propane-1,3-diyle, butane-1,4-diyle,
***R²*** signifie un groupe C₆₋₁₀-aryle monocyclique ou bicyclique ou un groupe hétéroaryle de 5-10 chaînons, monocyclique ou bicyclique comprenant 1 à 4 hétéroatomes choisis dans le groupe constitué par N, S ou 0, où le groupe aryle ou hétéroaryle mentionné peut être substitué, indépendamment l'un de l'autre, par jusqu'à trois des substituants suivants :
F, Cl, Br, I, C (NH) NH₂, C(NH) NHR⁴, C(NH) NR⁴R^{4'}, C(NR⁴)NH₂, C(NR⁴)NHR^{4'}, C(NR⁴)NR⁴R^{4'}, XOH, XOR⁴, XOCOR⁴, XOCONHR⁴, XOCOOR⁴, XCOR⁴, XC (NOH) R⁴, XC(NOR⁴)R^{4'}, XC(NO(COR⁴))R^{4'}, XCN, XCOOH, XCOOR⁴, XCONH₂, XCONR⁴R^{4'}, XCONHR⁴, XCONHOH, XCONHOR⁴, XCOSR⁴, XSR⁴, XSOR⁴, XSO₂R⁴,SO₂NH₂, SO₂NHR⁴, SO₂NR⁴R^{4'}, NO₂, XNH₂, XNHR⁴, XNR⁴R^{4'}, XNHSO₂R⁴, XN(SO²R⁴ )(SO₂R^{4'}), XNR⁴SO₂R^{4'}, XNHCOR⁴, XNHCOOR⁴, XNHCONHR⁴, tétrahydro-2,5-dioxopyrrol-1-yle, 2,5- dihydro-2,5-dioxopyrrol-1-yle, 2,7-dihydro-2,7- dioxoisoindol-1-yle, R⁴,
où deux substituants sur ***R²***, lorsqu'ils sont en position ortho l'un par rapport à l'autre, peuvent être liés l'un à l'autre de manière telle qu'ils forment ensemble méthanediylbisoxy, éthane-1,2- diylbisoxy, propane-1,3-diyle, butane-1,4-diyle,
***R³*** représente un ou deux substituants, qui peuvent représenter, indépendamment l'un de l'autre : hydrogène, F, Cl, Br, I, XOH, XOR⁴, XOCOR⁴, XOCONHR⁴, XOCOOR⁴, XCOR⁴, XC(NOH)R⁴, XC(NOR⁴)R^{4'}, XC(NO(COR⁴))R^{4'}, XCN, XCOOH, XCOOR⁴, XCONH₂, XCONHR⁴, XCONR⁴R^{4'}, XCONHOH, XCONHOR⁴, XCOSR⁴, XSR⁴, XSOR⁴, XSO₂R⁴, SO₂NH₂, SO₂NHR⁴, SO₂NR⁴R^{4'}, NO₂, XNH₂, XNHR⁴, XNR⁴R^{4'}, XNHSO₂R⁴, XNR⁴SO₂R^{4'}, XN(SO₂R⁴)(SO₂R^{4'}), XNHCOR⁴, XNHCOOR⁴, XNHCONHR⁴, tétrahydro-2,5-dioxopyrrol-1-yle, 2,5-dihydro-2,5- dioxopyrrol-1-yle, 2,7-dihydro-2,7-dioxoisoindol- 1-yle, R⁴, où deux substituants ***R³***, lorsqu'ils sont en position ortho l'un par rapport à l'autre, peuvent être liés l'un à l'autre de manière telle qu'ils forment ensemble méthanediylbisoxy, éthane-1,2- diylbisoxy, propane-1,3-diyle, butane-1,4-diyle,
***R⁴*** et ***R^{4'}*** signifient indépendamment l'un de l'autre C₁₋₄-perfluoroalkyle, C₁₋₆-alkyle, C₂₋₆-alcényle, C₂₋₆-alcynyle, C₃₋₇-cycloalkyle, (C₁₋₃-alkyl-C₃₋₇- cycloalkyle), C₁₋₃-alkyl-C₆₋₁₀-aryle, C₁₋₃-alkyl- hétéroaryle de 5-10 chaînons comprenant 1-4 atomes de N, S ou 0, C₆₋₁₀-aryle ou hétéroaryle de 5-10 chaînons comprenant 1-4 atomes de N, S ou 0, où les groupes C₆₋₁₀-aryle et hétéroaryle peuvent être substitués par un ou deux substituants du groupe constitué par F, Cl, Br, CH₃, C₂H₅, NO₂, OCH₃, OC₂H₅, CF₃, C₂F₅ ou peuvent également porter un groupe méthanediylbisoxy, éthane-1,2-diylbisoxy annelé, et en outre, dans un cycle cycloalkyle de 5 chaînons, un chaînon peut être un N ou un 0 et, dans un cycle cycloalkyle de 6 ou 7 chaînons, un ou deux chaînons peuvent être N et/ou 0, où les azotes du cycle peuvent le cas échéant être substitués par C₁₋₃-alkyle ou C₁₋₃-alcanoyle,
***R⁵*** et ***R^{5'}*** signifient indépendamment l'un de l'autre hydrogène, C₁₋₆-alkyle, C₂₋₆-alcényle, C₂₋₆- alcynyle, un atome de carbone pouvant être remplacé par O, S, SO, SO₂, NH, NC₁₋₃-alkyle ou NC₁₋₃-alcanoyle, C₃₋₇-cycloalkyl-C₀₋₃-alkyle, où, dans un cycle cycloalkyle de 5 chaînons, un chaînon peut être un N ou un 0 et, dans un cycle cycloalkyle de 6 ou 7 chaînons, un ou deux chaînons peuvent être N et/ou 0, où les azotes du cycle peuvent le cas échéant être substitués par C₁₋₃-alkyle ou C₁₋₃-alcanoyle C₆₋₁₀-aryle ou hétéroaryle de 5-10 chaînons comprenant 1-4 hétéroatomes parmi N, S et 0, où les chaînes alkyle, alcényle et alcynyle mentionnées peuvent être substituées par des cycloalkyles, aryles ou hétéroaryles susmentionnés, où tous les radicaux alkyle et cycloalkyle susmentionnés peuvent être substitués par jusqu'à deux substituants parmi CF₃, C₂F₅, OH, OC₁₋₃-alkyle, NH₂, NHC₁₋₃-alkyle, NHC₁₋₃-alcanoyle, N(C₁₋₃-alkyle)₂, N(C₁₋₃-alkyl)(C₁₋₃-alcanoyle), COOH, CONH₂, COOC₁₋₃- alkyle et tous les groupes aryle et hétéroaryle susmentionnés peuvent être substitués par un ou deux substituants du groupe constitué par F, Cl, Br, CH₃, C₂H₅, NO₂, OCH₃, OC₂H₅, CF₃, C₂F₅ ou peuvent également porter un groupe méthanediylbisoxy, éthane-1,2-diylbisoxy annelé, ou R⁵ et R^{5'} forment, ensemble avec l'atome d'azote, un hétérocycle de 5-7 chaînons, qui peut contenir un autre atome d'oxygène, d'azote ou de soufre et qui peut être substitué par C₁₋₄-alkyle, C₁₋₄-alcoxy-C₀₋₂-alkyle, C₁₋₄-alcoxycarbonyle, aminocarbonyle ou phényle,
***A*** signifie C₁₋₁₀-alcanediyle, C₂₋₁₀-alcènediyle, C₂₋₁₀- alcynediyle, (C₀₋₅-alcanediyl-C₃₋₇-cycloalcanediyl- C₀₋₅-alcanediyle) , (C₀₋₅-alcanediylarylène-C₀₋₅- alcanediyle), (C₀₋₅-alcanediyl-hétéroarylène-C₀₋₅- alcanediyle), où les groupes aryle et hétéroaryle peuvent être substitués par un ou deux substituants parmi F, Cl, Br, CH₃, C₂H₅, NO₂, OCH₃, OC₂H₅, CF₃, C₂F₅, où, dans un cycle cycloalkyle de 5 chaînons, un chaînon peut être un N ou un 0 et, dans un cycle cycloalkyle de 6 ou 7 chaînons, un ou deux chaînons peuvent être N et/ou 0, où les azotes du cycle peuvent le cas échéant être substitués par C₁₋₃-alkyle ou C₁₋₃-alcanoyle où, dans les chaînes aliphatiques susmentionnées, un atome de carbone ou deux atomes de carbone peuvent être remplacés par 0, NH, NR⁴, NCOR⁴, NSO₂R⁴, et où les groupes alkyle ou cycloalkyle peuvent être substitués par jusqu'à deux substituants parmi F, OH, OR⁴, OCOR⁴, =O, NH₂, NR⁴R^{4'}, NHCOR⁴, NHCOOR⁴, NHCONHR⁴, NHSO₂R⁴, SH, SR⁴,
***B*** signifie hydrogène, OH, OCOR⁵, OCONHR⁵, OCOOR⁵, COR⁵, C(NOH)R⁵, C(NOR⁵)R^{5'}, C(NO(COR⁵))R^{5'}, COOH, COOR⁵, CONH₂, CONHNH₂, CONHR⁵, CONR⁵R^{5'}, CONHOH, CONHOR⁵, SO₃H, SO₂NH₂, SO₂NHR⁵, SO₂NR⁵R^{5'}, PO₃H, PO(OH)(OR⁵), PO(OR⁵)(OR^{5'}), PO(OH)(NHR⁵), PO(NHR⁵)(NHR^{5'}), tétrazolyle, à chaque fois lié à un atome de carbone du groupe ***A***,
ou tout le groupe ***Y-A-B*** signifie N(SO₂R⁴)(SO₂R^{4'}) ou NHSO₂R⁴,
***X*** signifie une liaison, CH₂, (CH₂)₂, CH(CH₃), (CH₂)₃, CH(CH₂CH₃), CH(CH₃)CH₂, CH₂CH(CH₃),
***Y*** signifie une liaison, O, S, SO, SO₂, NH, NR⁴, NCOR⁴, NSO₂R⁴, pour la préparation d'un médicament destiné au traitement ou à la prévention de maladies qui sont associées à une activation de la microglie, choisies dans le groupe constitué par la démence associée au SIDA, la sclérose latérale amyotrophique, la maladie de Creutzfeld-Jacob, le syndrome de Down, la démence à corps de Lewy, la chorée de Huntington, la leuco-encéphalopathie, la sclérose en plaques, le dysfonctionnement et la dégénérescence neuronal(e), la maladie de Parkinson, la maladie de Pick, la maladie d'Alzheimer, une attaque, l'épilepsie temporale et les tumeurs.

18. Utilisation selon la revendication 16 ou 17, où,
dans la formule générale II
***R¹*** signifie un groupe aryle monocyclique ou bicyclique ou un groupe hétéroaryle de 5-10 chaînons, monocyclique ou bicyclique comprenant 1 à 2 hétéroatomes choisis dans le groupe constitué par N, S ou 0, où le groupe aryle ou hétéroaryle mentionné peut être substitué, indépendamment l'un de l'autre, par jusqu'à trois des substituants suivants :
F, Cl, Br,
XOH, XOR⁴, XOCOR⁴, XOCONHR⁴, XOCOOR⁴,
XCOR⁴, XCN, XCOOH, XCOOR⁴, XCONH₂, XCONR⁴R^{4'}, XCONHR⁴, XCONHOH, XCONHOR⁴, XCOSR⁴, XSR⁴, NO₂, XNHR⁴, XNR⁴R^{4'}, R⁴,
où deux substituants sur ***R¹***, lorsqu'ils sont en position ortho l'un par rapport à l'autre, peuvent être liés l'un à l'autre de manière telle qu'ils forment ensemble méthanediylbisoxy, éthane-1,2-diylbisoxy, propane-1,3-diyle, butane-1,4-diyle.

19. Utilisation selon l'une quelconque des revendications 16-18, où, dans la formule générale II
***R²*** signifie un groupe aryle monocyclique ou bicyclique ou un groupe hétéroaryle de 5-10 chaînons, monocyclique ou bicyclique comprenant 1 à 2 hétéroatomes choisis dans le groupe constitué par N, S ou 0, où le groupe aryle ou hétéroaryle mentionné peut être substitué, indépendamment l'un de l'autre, par jusqu'à trois des substituants suivants :
F, Cl, Br, XOH, XOR⁴, XOCOR⁴, XOCONHR⁴, XOCOOR⁴, XCOR⁴, XC(NOH)R⁴, XC(NOR⁴)R^{4'}, XC(NO(COR⁴))R^{4'}, XCN, XCOOH, XCOOR⁴, XCONH₂, XCONR⁴R^{4'}, XCONHR⁴, XCONHOH, XCONHOR⁴, XCOSR⁴, XSR⁴, XSOR⁴, XSO₂R⁴, SO₂NH₂, SO₂NHR⁴, SO₂NR⁴R^{4'}, NO₂, XNH₂, XNHR⁴,XNR⁴R^{4'}, XNHSO₂R⁴, XN(SO₂R⁴)(SO₂R^{4'}), XNR⁴SO₂R^{4'}, XNHCOR⁴, XNHCOOR⁴, XNHCONHR⁴, R⁴,
où deux substituants sur ***R²***, lorsqu'ils sont en position ortho l'un par rapport à l'autre, peuvent être liés l'un à l'autre de manière telle qu'ils forment ensemble méthanediylbisoxy, éthane-1,2- diylbisoxy, propane-1,3-diyle, butane-1,4-diyle.

20. Utilisation selon l'une quelconque des revendications 16-19, où, dans la formule générale II
***R³*** représente un ou deux substituants, qui peuvent représenter, indépendamment l'un de l'autre :
hydrogène, F, Cl, Br, XOH, XOR⁴, XOCOR⁴, XOCONHR⁴, XOCOOR⁴, XCOR⁴, XC(NOH)R⁴, XC(NOR⁴)R^{4'}, XC(NO(COR⁴))R^{4'}, XCN, XSR⁴, XSOR⁴, XSO₂R⁴, SO₂NH₂, SO₂NHR⁴, SO₂NR⁴R^{4'}, NO₂, XNH₂, XNHR⁴, XNR⁴R⁴, XNHSO₂R⁴, XNR⁴SO₂R^{4'}, XN(SO₂R⁴)(SO₂R^{4'}), XNHCOR⁴, XNHCOOR⁴, XNHCONHR⁴ ou R⁴,
où deux substituants ***R³***, lorsqu'ils sont en position ortho l'un par rapport à l'autre, peuvent être liés l'un à l'autre de manière telle qu'ils forment ensemble méthanediylbisoxy, éthane-1,2-diylbisoxy, propane-1,3-diyle, butane-1,4-diyle.

21. Utilisation selon l'une quelconque des revendications 16-20, où, dans la formule générale II
***R⁵*** et ***R^{4'}*** signifient, indépendamment l'un de l'autre CF₃, C₂F₅, C₁₋₄-alkyle, C₂₋₄-alcényle, C₂₋₄-alcynyle, C₃₋₆-cycloalkyle, (C₁₋₃-alkyl-C₃₋₆-cycloalkyle), C₁₋₃- alkylaryle, C₁₋₃-alkylhétéroaryle, aryle monocyclique ou hétéroaryle de 5-6 chaînons, comprenant 1-2 atomes de N, S ou 0, où les groupes aryle et hétéroaryle peuvent être substitués par un ou deux substituants du groupe constitué par F, Cl, Br, CH₃, C₂H₅, NO₂, OCH₃, OC₂H₅, CF₃, C₂F₅ ou peuvent également porter un groupe méthanediylbisoxy ou éthane-1,2-diylbisoxy annelé, et en outre, dans un cycle cycloalkyle de 5 chaînons, un chaînon peut être un N ou un 0 et, dans un cycle cycloalkyle de 6 chaînons, un ou deux chaînons peuvent être N et/ou 0, où les azotes du cycle peuvent le cas échéant être substitués par C₁₋₃-alkyle ou C₁₋₃-alcanoyle.

22. Utilisation selon l'une quelconque des revendications 16-21, où, dans la formule générale II
***R⁵*** et ***R^{5'}*** signifient indépendamment l'un de l'autre C₁₋₆-alkyle, un atome de carbone pouvant être remplacé par 0, NH, NC₁₋₃-alkyle ou NC₁₋₃-alcanoyle, C₃₋₇-cycloalkyl-C₀₋₃-alkyle, où dans un cycle cycloalkyle de 5 chaînons, un chaînon peut être un N ou un 0 et dans un cycle cycloalkyle de 6 ou 7 chaînons, un ou deux chaînons peuvent être N et/ou 0, où les azotes du cycle peuvent le cas échéant être substitués par C₁₋₃-alkyle ou C₁₋₃-alcanoyle où la partie C₁₋₆-alkyle mentionnée peut être substituée par un des cycloalkyles susmentionnés ou également par un hétéroaromatique de 5-6 chaînons comprenant 1-2 hétéroatomes choisis parmi N, S ou 0,
où tous les radicaux alkyle et cycloalkyle susmentionnés peuvent être substitués par jusqu'à deux substituants parmi CF₃, OH, OC₁₋₃-alkyle et les groupes hétéroaryle susmentionnés peuvent être substitués par un ou deux substituants parmi F, Cl, CF₃, CH₃, C₂H₅, OCH₃, OC₂H₅, ou R⁵ et R^{5'} forment, ensemble avec l'atome d'azote, un hétérocycle de 5-7 chaînons, qui peut contenir un autre atome d'oxygène, d'azote ou de soufre et qui peut être substitué par C₁₋₄-alkyle, C₁₋₄-alcoxy-C₀₋₂-alkyle, C₁₋₄-alcoxycarbonyle, aminocarbonyle ou phényle.

23. Utilisation selon l'une quelconque des revendications 16-22, où, dans la formule générale II
***A*** signifie C₁₋₁₀-alcanediyle, C₂₋₁₀-alcènediyle, C₂₋₁₀- alcynediyle, (C₀₋₅-alcanediyl-C₃₋₇-cycloalcanediyl- C₀₋₅-alcanediyle) ou (C₀₋₅-alcanediyl-hétéroarylène- C₀₋₅-alcanediyle), où un groupe hétéroaryle le cas échéant présent peut être substitué par un ou deux substituants parmi F, Cl, Br, CH₃, C₂H₅, NO₂, OCH₃, OC₂H₅, CF₃, C₂F₅,
où en outre, dans un cycle cycloalkyle de 5 chaînons, un chaînon peut être un N ou un 0 et, dans un cycle cycloalkyle de 6 ou 7 chaînons, un ou deux chaînons peuvent être N et/ou 0, où les azotes du cycle peuvent le cas échéant être substitués par C₁₋₃-alkyle ou C₁₋₃-alcanoyle
où, dans une chaîne aliphatique susmentionnée, un atome de carbone ou deux atomes de carbone peuvent être remplacés par 0, NH, NC₁₋₃-alkyle, NC₁₋₃-alcanoyle, NSO₂-C₁₋₃-alkyle,
et où les parties alkyle ou cycloalkyle peuvent être substituées par jusqu'à deux atomes de F ou un des substituants parmi OH, OC₁₋₃-alkyle, OC₁₋₃-alcanoyle, =O, NH₂, NHC₁₋₃-alkyle, N(C₁₋₃-alkyle)₂, NHC₁₋₃-alcanoyle, N(C₁₋₃-alkyl)(C₁₋₃-alcanoyle), NHCOOC₁₋₃-alkyle, NHCONHC₁₋₃-alkyle, NHSO₂C₁₋₃-alkyle, SH, SC₁₋₃-alkyle.

24. Utilisation selon l'une quelconque des revendications 16-23, où, dans la formule générale II
***B*** signifie hydrogène, OH, OCOR⁵, OCONHR⁵, OCOOR⁵, COOH, COOR⁵, CONH₂, CONHR⁵, CONR⁵R^{5'}, CONHOH, CONHOR⁵ ou tétrazolyle, à chaque fois lié à un atome de carbone du groupe ***A***.

25. Utilisation selon l'une quelconque des revendications 16-24, où, dans la formule générale II
***X*** signifie une liaison ou CH₂.

26. Utilisation selon l'une quelconque des revendications 16-25, où, dans la formule générale II
***Y*** signifie une liaison, O, S, NH, NR⁴, NCOR⁴ ou NSO₂R⁴.
